# EUROPEAN PATENT SPECIFICATION

(11) **EP 3 443 149 B1**
(45) Date of publication and mention of the grant of the patent: **14.08.2024**
(21) Application number: 17781661.8
(22) Date of filing: 13.04.2017
(51) Int. Cl.: C12Q 1/6869, C12Q 1/6855, C12Q 1/6881

(54) **HYBRID-CAPTURE SEQUENCING FOR DETERMINING IMMUNE CELL CLONALITY**
HYBRIDERFASSUNGSSEQUENZ ZUR BESTIMMUNG VON IMMUNZELLENKLONALITÄT
SÉQUENÇAGE DE CAPTURE HYBRIDE POUR DÉTERMINER LA CLONALITÉ DE CELLULES IMMUNITAIRES

(30) Priority: 15.04.2016 US 201662322999 P
(43) Date of publication of application: 20.02.2019
(73) Proprietor: University Health Network (UHN), Toronto, Ontario M5G 2C4 (CA)
(72) Inventor: PUGH, Trevor John, Toronto Ontario M4K 1G3 (CA); MULDER, David Thomas, Hamilton Ontario L8P 1Z6 (CA); MAHE, Etienne Raymond G. A., Calgary Alberta T3Z 3C6 (CA)
(74) Representative: Carpmaels & Ransford LLP
(86) International application number: PCT/CA2017/000084
(87) International publication number: WO 2017/177308

(56) References cited:
- WO-A1-2014/008447
- US-A1- 2008 125 333
- US-A1- 2015 218 620
- ELIANA RUGGIERO ET AL: "High-resolution analysis of the human T-cell receptor repertoire", NATURE COMMUNICATIONS, vol. 6, no. 1, 1 September 2015 (2015-09-01), XP055446483, DOI: 10.1038/ncomms9081
- DANIEL S HERMAN ET AL: "Filter-based hybridization capture of subgenomes enables resequencing and copy-number detection", NATURE METHODS, vol. 6, no. 7, 21 June 2009 (2009-06-21), pages 507 - 510, XP055197367, ISSN: 1548-7091, DOI: 10.1038/nmeth.1343
- XIAO LIU ET AL: "Systematic Comparative Evaluation of Methods for Investigating the TCR&bgr; Repertoire", PLOS ONE, vol. 11, no. 3, 28 March 2016 (2016-03-28), pages e0152464, XP055302912, DOI: 10.1371/journal.pone.0152464
- ANNA GAZZOLA ET AL: "The evolution of clonality testing in the diagnosis and monitoring of hematological malignancies", THER ADV HEMATOL, 1 January 2014 (2014-01-01), pages 35 - 47, XP055640777, Retrieved from the Internet <URL:https://www.ncbi.nlm.nih.gov/pmc/articles/PMC3949299/pdf/10.1177_2040620713519729.pdf> [retrieved on 20191108], DOI: 10.1177/2040620713519729Therapeutic
- HE J ET AL.: "IgH Gene Rearrangements as Plasma Biomarkers in Non-Hodgkin's Lymphoma Patients", ONCOTARGET, vol. 2, no. 3, 8 March 2011 (2011-03-08), pages 178 - 185, XP055247543
- AN INTRODUCTION TO NEXT-GENERATION SEQUENCING TECHNOLOGY, 28 February 2012 (2012-02-28), XP055431981, Retrieved from the Internet <URL:https://www.illumina.com/Documents/products/Illumina_Sequencing_Introduction.pdf> [retrieved on 20171008]

## Description

### FIELD OF THE INVENTION

The invention relates to methods of capturing and sequencing immune-associated nucleotide sequences, and more particularly to methods of determining clonality of immune cells.

### BACKGROUND OF THE INVENTION

The maturation of lymphocytes is a fascinating process that is marked not only by immunophenotypic changes, but also by discrete and regulated molecular events ⁽¹⁻³⁾. As T-cells mature, an important part of the associated molecular "maturation" involves the somatic alteration of the germline configuration of the T-cell receptor (TR) genes to a semi-unique configuration in order to permit the development of a clone of T-cells with an extracellular receptor specific to a given antigen ⁽¹⁻³⁾. B-cells undergo a similar maturation process involving different loci that encode the antibody-containing B-cell receptor (BC). These clones, when considered together as a population, produce a repertoire of antigen sensitivity orders of magnitude larger than would be possible by way of inherited immunological diversity alone ⁽³⁾. Indeed, the somatic rearrangement of the TR and BR genes is one of the key ontological events permitting the adaptive immune response ⁽³⁾.

When molecular carcinogenesis occurs in a lymphoid cell lineage, the result is the selective growth and expansion of the tumoural lymphocytes relative to their normal counterparts ⁽²⁾. The so-called precursor (historically termed "lymphoblastic") lesions are believed to reflect molecular carcinogenesis in lymphoid cells at a relatively immature stage of maturation⁽²⁾. In contrast, if molecular carcinogenesis occurs at a point during or after the process of T-cell receptor gene re-arrangement (TRGR), the result is a "mature" (often also termed "peripheral") T-cell lymphoma in which the tumour contains a massively expanded population of malignant T-cells with an immunophenotype reminiscent of mature lymphocytes, most if not all bearing an identical TR gene configuration ⁽⁴⁾. It is this molecular "homogeneity" of the TR configuration within a T-cell neoplasm that defines the concept of clonality in T-cell neoplasia^{(1,2,4)}.

The T-cell receptor is a heteroduplex molecule anchored to the external surface of T lymphocytes ^{(5,21)}; there the TR, in cooperation with numerous additional signalling and structural proteins, functions to recognize an antigen with a high degree of specificity. This specificity, and indeed the vast array of potential antigenic epitopes that may be recognized by the population of T-cells on the whole, is afforded by (1) the number of TR encoding regions of a given T-cell receptor's genes as present in the germline; and (2) the intrinsic capacity of the TR gene loci to undergo somatic re-arrangement ⁽³⁾. There are four TR gene loci, whose protein products combine selectively to form functional TRs: T-cell receptor alpha (TRA) and T-cell receptor beta (TRB) encode the α and β chains, respectively, whose protein products pair to form a functional α/β TR; T-cell receptor gamma (TRG) and T-cell receptor delta (TRD) encode the γ and δ chains, respectively, whose protein products pair to form a functional γ/δ TR. The vast majority (>95%) of circulating T-cells are of the α/β type ^{(21,22)}; for reasons as yet not fully understood, γ/δ T-cells tend to home mainly to epithelial tissues (e.g. skin and mucosae) and appear to have a different function than the more common α/β type T-cells.

The TRA locus is found on the long arm of chromosome 14 in band 14q11.2 and spans a total of 1000 kilobases (kb) ⁽²³⁾; interestingly, sandwiched between the TRA V and J domains, is the TRD locus (14q11.2), itself spanning only 60 kb ⁽²⁴⁾. The TRB locus is found on the long arm of chromosome 7 in band 7q35 and spans a total of 620 kb ⁽²⁵⁾. The TRG locus is found on the short arm of chromosome 7 in region 7p15-p14 and spans 160 kb ⁽²⁶⁾.

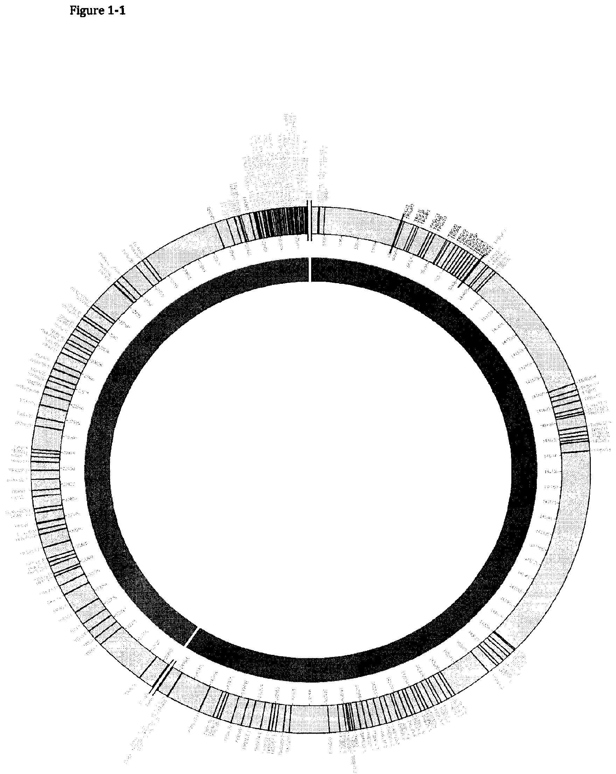

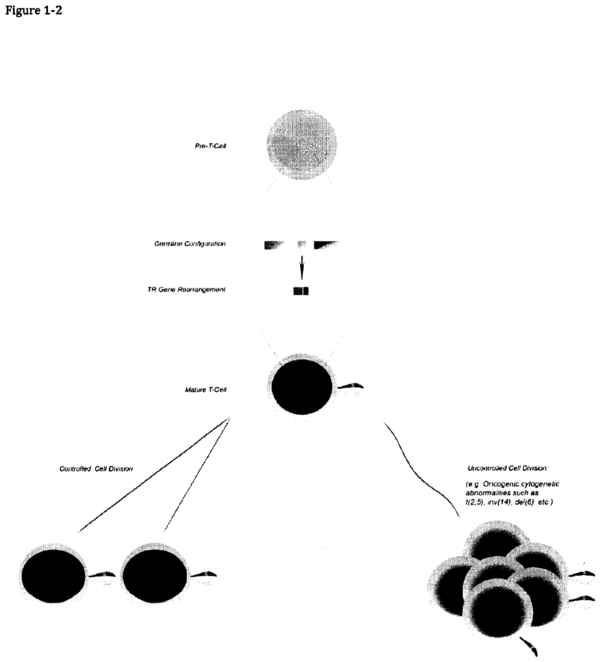

Within each TR gene locus are a variable number of variable (V) and join (J) segments ⁽²³⁻²⁶⁾; additional diversity (D) segments are present within the TRB and TRD loci ^{(24,25)}. These V, D and J segments are grouped into respective V, D and J regions (see Figure 1-1). In the germline configuration, a full complement of V (numbering from 4-6 in TRG to 45-47 in TRA), D (2 in TRB and 3 in TRD) and J (numbering as few as 4 in TRD to as many as 61 in TRA) segments can be detected, varying based on inheritance ⁽²³⁻²⁶⁾. In this configuration, the specificity of any resulting coding sequence would be uniformly based on inherited variation. During maturation, however, somatic mutation (i.e. rearrangement) occurs such that there is semi-random recombination of variable numbers of the V, D and J segments to produce a lineage of cells with a "re-arranged" configuration of TR gene segments. This gene re-arrangement, when later subject to gene transcription and translation, produces a TR unique to the given T-lymphocyte (and its potential daughter cells). This process is represented pictorially in Figure 1-2. Although the specific details of this re-arrangement process are far beyond the scope of this work, the process is at least partly mediated by enzymes of similar function to those used to perform splicing ^{(21,22)}.

BIOMED-2 ⁽²⁹⁾ is a product of several years of collaborative expert study, resulting in a thoroughly studied consensus T-cell clonality assay. The BIOMED-2 assay includes multiplexed primer sets for both Immunoglobulin (IG) and TR clonality assessment and can be implemented with commercially available electrophoresis systems (e.g. Applied Biosystems fluorescence electrophoresis platforms) ⁽²⁹⁾. These commercially available primer sets have the advantage of standardization and ease of implementation. In addition, by virtue of the extensive study performed by the BIOMED consortium, the BIOMED-2 assay has the well-documented advantage of capturing the mono-clonality of the vast majority of control lymphomas bearing productive T-cell receptors (i.e. flow-sorted positive for either α/β or γ/δ T-cell receptors) using the specified TRB and TRG primer sets ⁽²⁹⁾. Of note, having been in use for over a decade, the BIOMED-2 has been globally accepted as the diagnostic assay primer set of choice.

The current approach to TRGR testing is subject to a number of technical and practical caveats that dilute the applicability of TRGR testing to the full breadth of real-world contexts.

Because the PCR-based techniques that are employed in TRGR assays are subject to amplicon size restrictions ^{(21,14)}, the sheer size of the TRA locus prevents a complete assay of the TRA gene in clinical settings. Indeed, although of smaller size, the TRB locus as a whole is also prohibitively large to sequence in its germline configuration. It is therefore of no surprise that much of the published data pertaining to the utility and validity of TRGR assays has stemmed from assays specific to only subparts of TRB as well as TRG, a locus of size much more amenable to a single-assay. In addition, since the TRD locus is often deleted after TR gene rearrangement (since it is contained within the TRA locus and excised whenever the TRA locus is rearranged), assays for TRD have also not been as rigorously studied. For this reason, any BIOMED-2-based T-cell clonality assay aimed at directing immunotherapy, requiring a complete sequence-based understanding of the TR genes involved, would be insufficient.

The BIOMED-2 assay is subject to additional technical challenges. As part of the standard TRGR assay, most laboratories rely on the demonstration of electrophoretic migration patterns for the determination of TR clonality. Interpretation of the assay depends on the demonstration (or lack thereof) of a dominant amplicon of specific (albeit not pre-defined) molecular weight, rather than the normal Gaussian distribution of amplicons of variable size. This approach, as has been described previously ⁽³⁵⁻³⁷⁾, is subject to interpretative error and other technical problems. Also, given the large amounts of DNA required for the multitude of multiplex tubes making up the assay, the overall assay can very quickly deplete DNA supplies, especially when obtained from limited sample sources.

Finally, and arguably of greatest import, is the issue of diagnostic bias used in the study of TRGR assay performance. More precisely, when laboratories seek to validate a TRGR assay, the requirement of "standard" samples will typically require that the laboratory utilize previously established clonal samples or samples previously diagnosed and accepted to represent clonal entities (e.g. previously diagnosed cases of lymphoma); these samples are in turn compared to "normal" controls. In contrast, the demographics of subsequent "real-life" test samples are unlikely to be so decidedly parsed into "normal" and "abnormal" subsets.

Current T-Cell Receptor (TCR) rearrangement profiling assays rely on targeted PCR amplification of rearranged TCR genomic loci. The simplest method for assessing clonality of T-cells involves qualitative assessment through multiplexed amplification of the individual loci using defined primer sets and interpretation of fragment size distributions according to the BIOMED2 protocol ^{A1,2}. Next-generation sequencing can be used as a read-out to provide quantitative assessment of the TCR repertoire including detection of low abundance rearrangements from bulk immune cells, or even pairing of the heterodimeric chain sequences with single cell preparation methods ^{A3,4}. Hybrid-capture based library subsetting is an alternative method to PCR-based amplification that can improve coverage uniformity and library complexity when sample is not limiting and allows for targeted enrichment of genetic loci of interest from individual genes to entire exomes ^{A5}. In hybrid-capture methods, the formation of probe-library fragment DNA duplexes are used to recover regions of interest ^{A6 7,8}.

Similar to T-cells, B-cells involved in adaptive immunity also undergo somatic rearrangement of germline DNA to encode a functional B-cell receptor (BR). Like TRs, these sequences comprise by discrete V, D, J segments that are rearranged and potentially altered during B-cell maturation to encode a diversity of unique immunoglobulin proteins. The clonal diversity of B-cell populations may have clinical utility and, similar to T-cell lymphomas, several cancers are characterized by clonal expansion of specific BR/lg sequences.

He et al. (Oncotarget, 2(3), 2011) discloses a method of capturing and characterising rearranged immunoglobulin (IgH) and B-cell DNA fragments in lymphoma and plasma samples of patients with Non-Hodgkin's Lymphoma. Illumina Inc. (Publication No. 770-2012-008, 2012) discloses Illumina Next-Generation sequencing techniques adapted for short read sequencing. US2015/0218620 discloses methods to capture and/or remove highly abundant RNAs from a heterogeneous RNA sample. US2008/0125333 discloses methods and kits for hybrid capture using a depletion technique to allow isolation of capture-associated nucleotides.

### SUMMARY OF THE INVENTION

There is described herein, the development of a novel NGS-based T-cell clonality assay, incorporating all four TR loci. The assay was both analytically and clinically validated. For the former, a series of idealized specimens was used, with combined PCR/Electrophoresis and Sanger Sequencing to confirm NGS-data. The latter validation compared NGS results to the current gold standard for clinical T-cell clonality testing (i.e. the BIOMED-2 primer PCR method) on an appropriately-sized minimally-biased sample of hematopathology specimens. In the latter dataset also, the patterns of T-cell clonality were also correlated with clinical, pathologic, and outcome data.

In an aspect, there is provided, a method of capturing a population of T-Cell receptor and/or immunoglobulin sequences with variable regions within a patient sample, said method comprising: extracting/preparing DNA fragments from the patient sample; ligating a nucleic acid adapter to the DNA fragments, the nucleic acid adapter suitable for recognition by a pre-selected nucleic acid probe; capturing DNA fragments existing in the patient sample using a collection of nucleic acid hybrid capture probes, wherein each capture probe is designed to hybridize to a known V gene segment and/or a J gene segment within the T cell receptor and/or immunoglobulin genomic loci, wherein the hybrid capture probes are designed based on all unique V genes and J genes and these probe sets specifically target the 3' regions of V gene coding regions and the 5' regions of J gene coding regions.

A method of immunologically classifying a population of T-Cell receptor and/or immunoglobulin sequences can comprise:
(a) identifying all sequences containing a V gene segment from the sequences of the DNA fragments by aligning the sequences of the DNA fragments to a library of known V gene segment sequences;
(b) trimming the identified sequences in (a) to remove any sequences corresponding to V gene segments to produce a collection of V-trimmed nucleotide sequences;
(c) identifying all sequences containing a J gene segment in the population of V-trimmed nucleotide sequences by aligning the V-trimmed nucleotide sequences to a library of known J gene segment sequences;
(d) trimming the V-trimmed nucleotide sequences identified in (c) to remove any sequences corresponding to J gene segments to produce VJ-trimmed nucleotide sequences;
(e) identifying any D gene segment comprised in the VJ-trimmed nucleotide sequences identified in (d) by aligning the VJ-trimmed nucleotide sequences to a library of known D gene segment sequences;
(f) for each VJ-trimmed nucleotides sequence identified in (d), assembling a nucleotide sequence comprising the V gene segment, any D gene segment, and the J gene segment identified in steps (a), (e) and (c) respectively;
(g) selecting from the nucleotide sequence assembled in step (f) a junction nucleotide sequence comprising at least the junction between the V gene segment and the J gene segment, including any D gene segment, the junction nucleotide sequence comprising between 18bp and 140bp, preferably 40-100bp, further preferably about 80bp;
and optionally (h) and (i):
(h) translating each reading frame of the junction nucleotide sequence and its complementary strand to produce 6 translated sequences; and
(i) comparing the 6 translated sequences to a library of known CDR3 regions of T-Cell receptor and/or immunoglobulin sequences to identify the CDR3 region in the DNA fragments.

A method of identifying CDR3 regions in T-Cell receptor and/or immunoglobulin sequences can comprise:
(a) identifying a V gene segment comprised in the immunoglobulin sequence by aligning the immunoglobulin sequence to a library of known V gene segment sequences;
(b) identifying a J gene segment comprised in the immunoglobulin sequence by aligning the immunoglobulin sequence to a library of known J gene segment sequences;
(c) if V and J gene segments are identified, then comparing the immunoglobulin sequence to a library of known CDR3 regions of T-Cell receptor and/or immunoglobulin sequences to identify any CDR3 region in the immunoglobulin sequence.

### BRIEF DESCRIPTION OF FIGURES

These and other features of the methods described herein will become more apparent in the following detailed description in which reference is made to the appended drawings wherein:
**Figure 1-1****:** Genomic distribution of the TRA, TRB, TRD and TRG locus genes. The inner ring highlights the relevant portions of chromosome 7 (blue) and chromosome 14 (red); the relative positions of each of the genes is denoted in the ideogram, indexed by chromosome position (bp x 1000), with the accompanying HUGO accepted gene symbols.
**Figure 1-2****:** TRGR Situated Relative to Controlled & Uncontrolled (Malignant) T-cell Expansion. The path of maturation from Pre T-cell to mature T-cell is outlined, including the TR gene rearrangement; additionally, accumulated mutations might then lead to the uncontrolled cell growth, characteristic of mature T-cell lymphoma.
**Figure 2-1A****:** TRGR Assay Wet-Bench Work-Flow Schematic. 1, DNA isolation; 2, Shearing (~200 bp); 3, Library Production; 4, Hybridization with Biotinylated DNA Probes; 5, Enrichment with Streptavidin-Bound Paramagnetic Beads; 6, PCR; 7, Illumina sequencing.

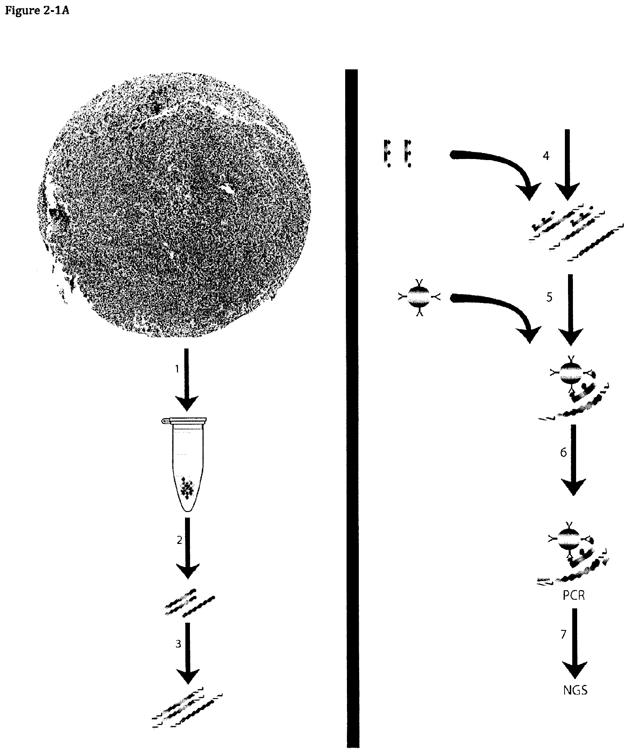

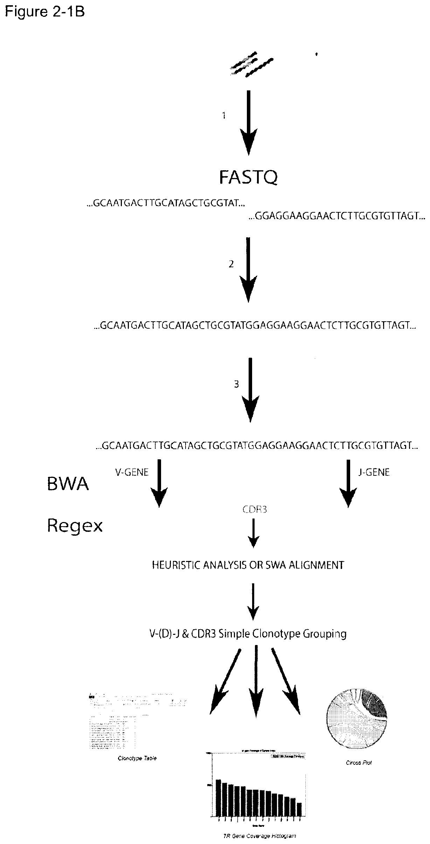

**Figure 2-1B****:** TRGR Assay Informatic Work-Flow Schematic. 1, Paired-end 150 bp DNA sequencing is performed; 2, Merging of paired ends (e.g. PEAR pipeline); 3, TRSeq pipeline (outputs may include Clonotype table, Coverage histograms and Circos plots).

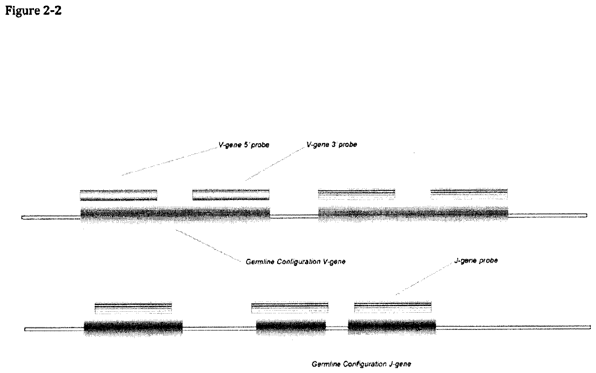

**Figure 2-2****:** Schematic Representation of V and J Gene Probe Placement Relative to the Germline. The germline V-genes are highlighted in solid red, with 100 bp probe placement shown above; probes are oriented inward and abut the 5' & 3' ends of the germline V-gene configuration. The germline J-genes are highlighted in solid blue, with 100 bp probe placement shown above; J-gene probes cover the entire J-gene, and on occasion some flanking extragenic sequence.

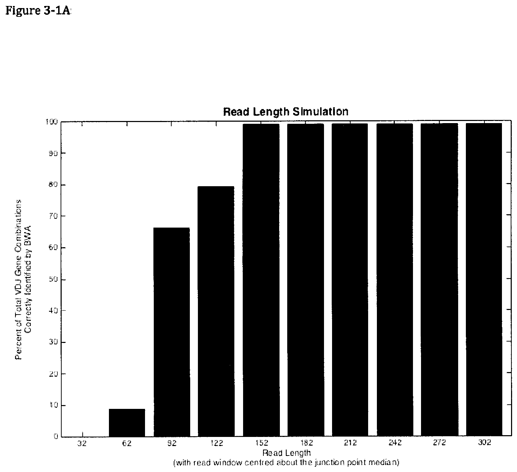

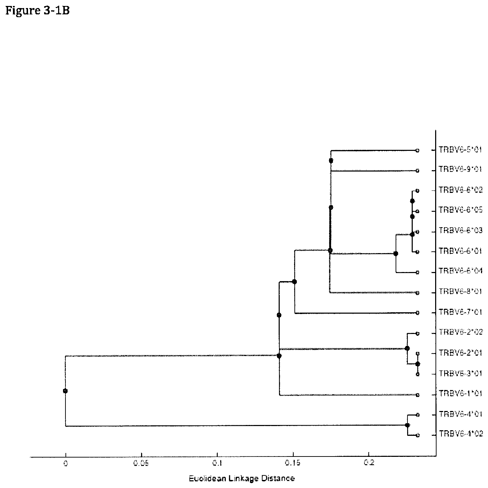

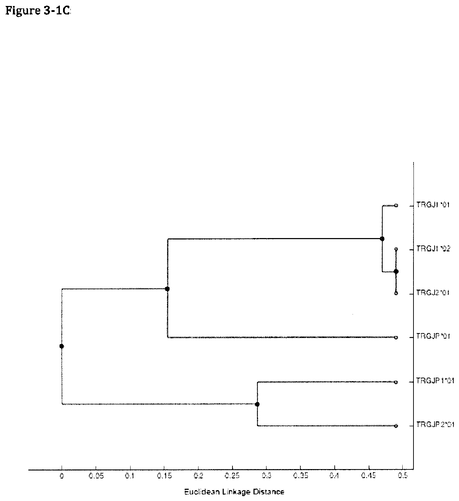

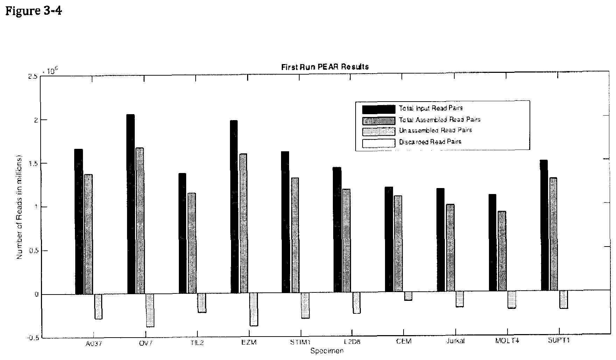

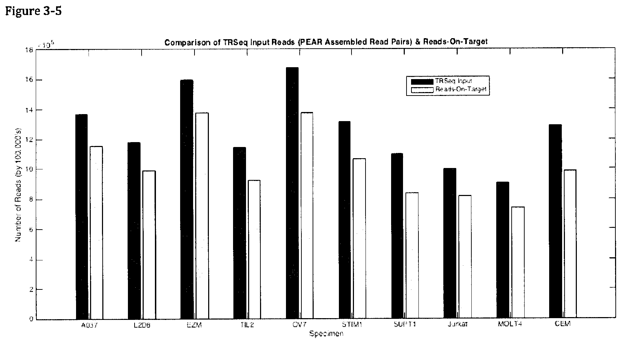

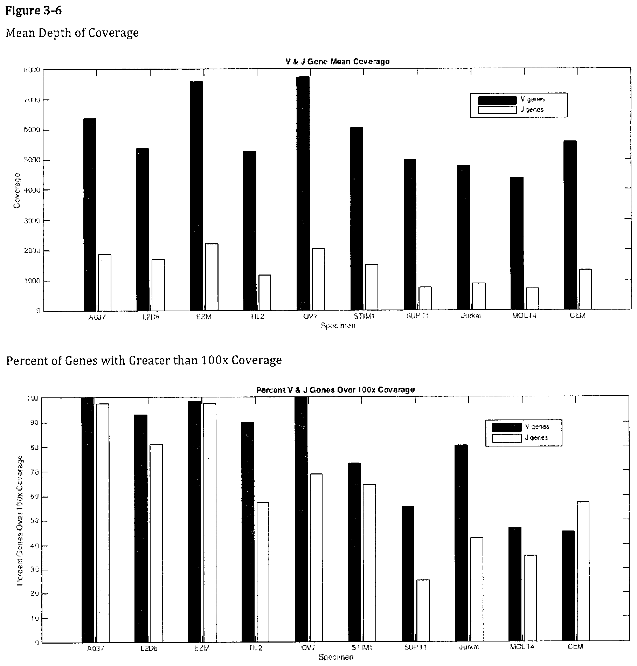

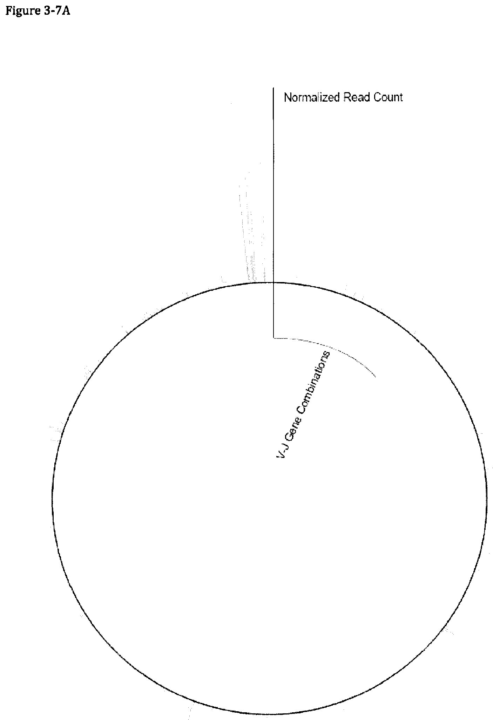

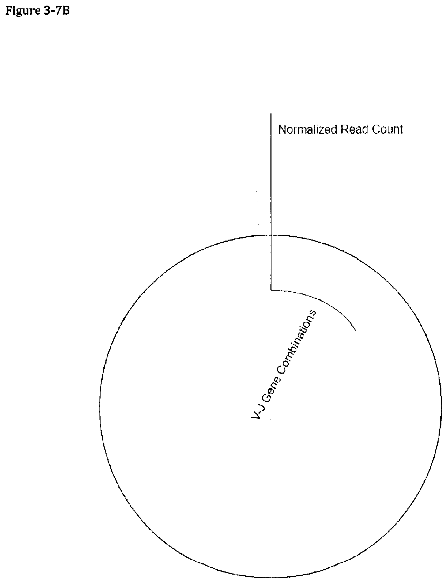

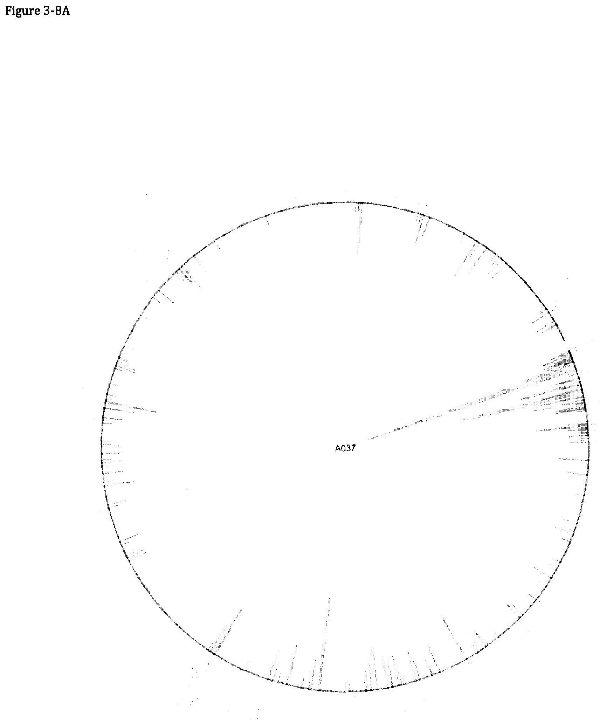

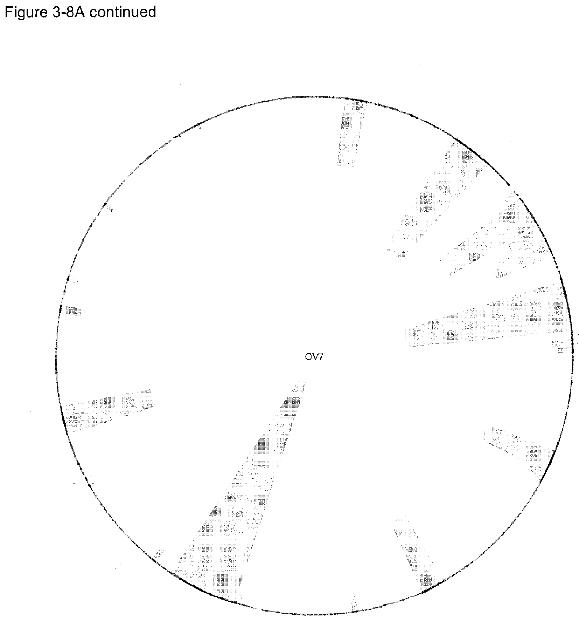

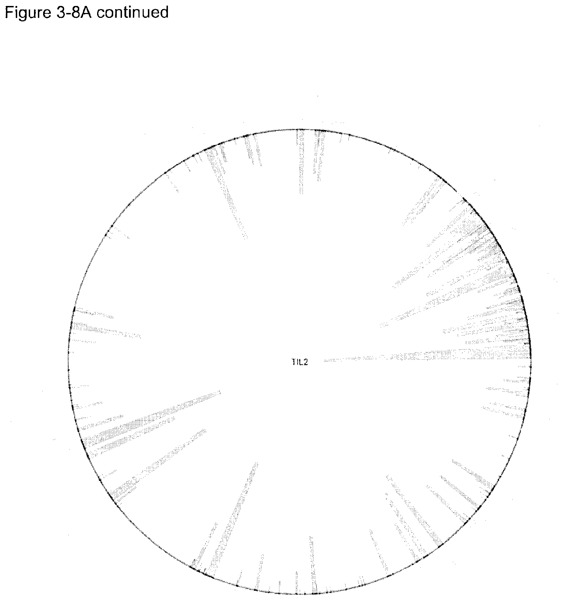

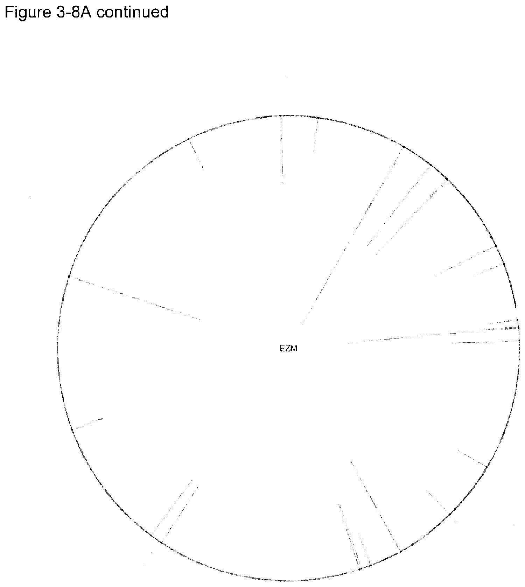

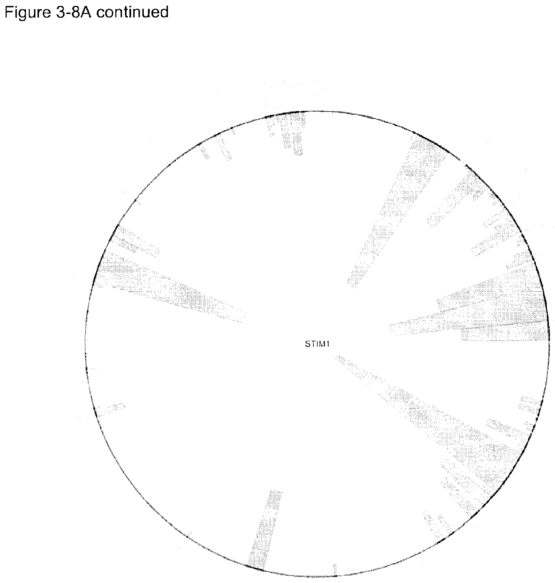

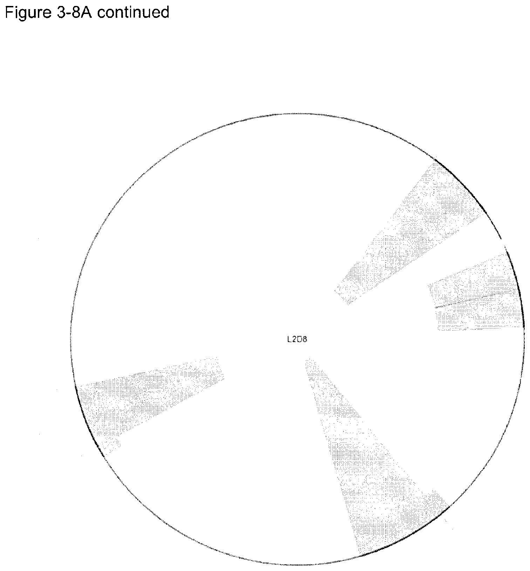

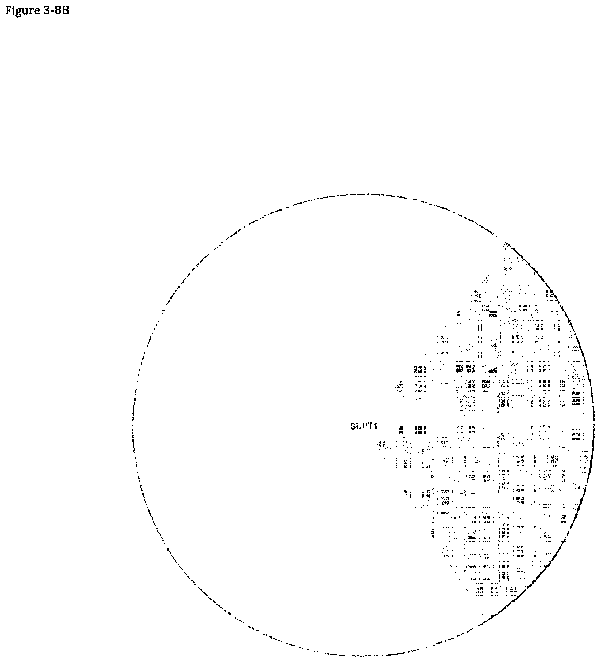

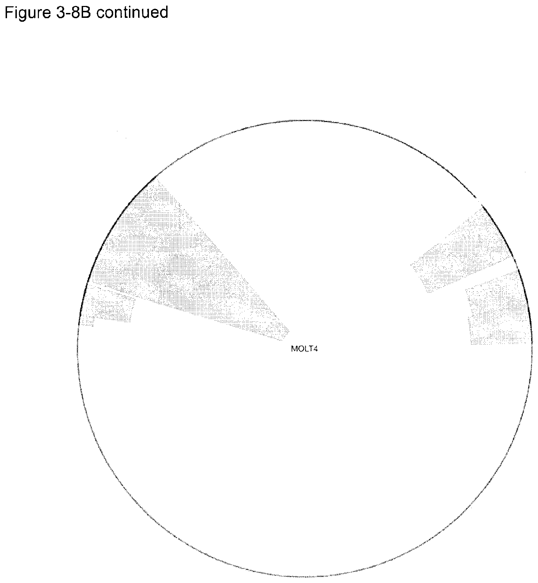

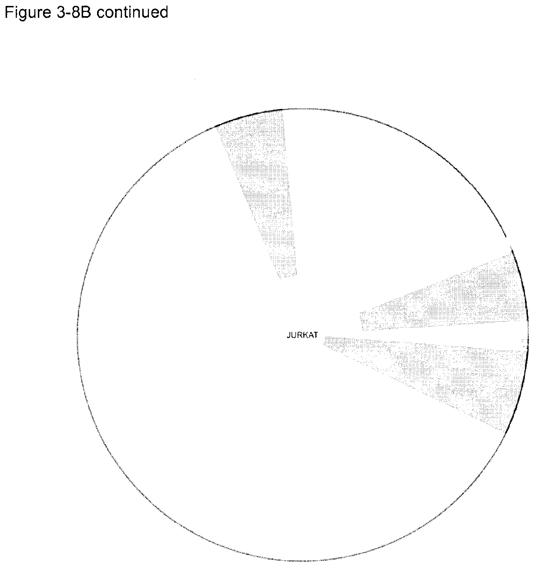

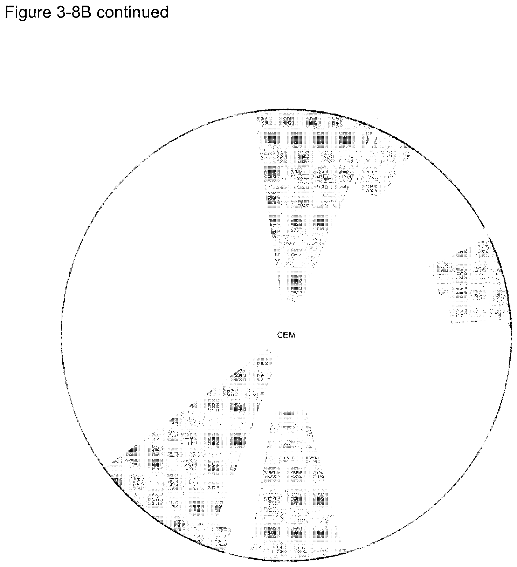

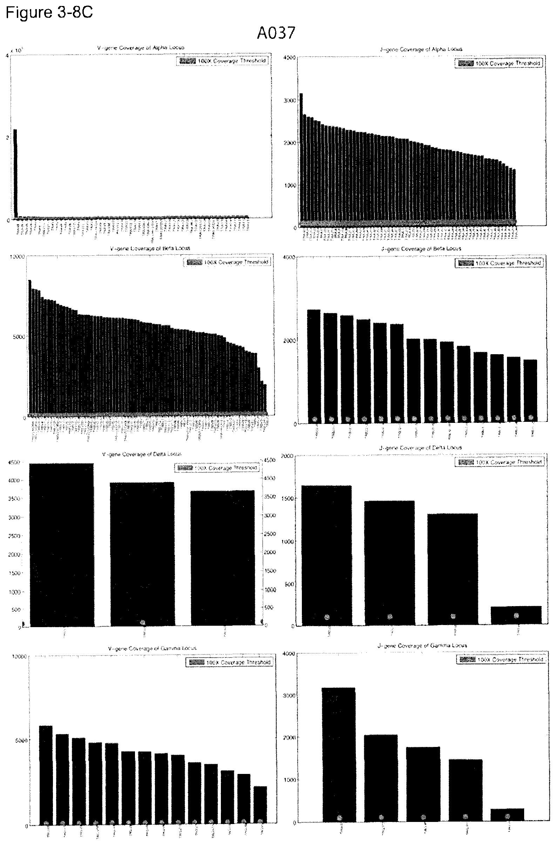

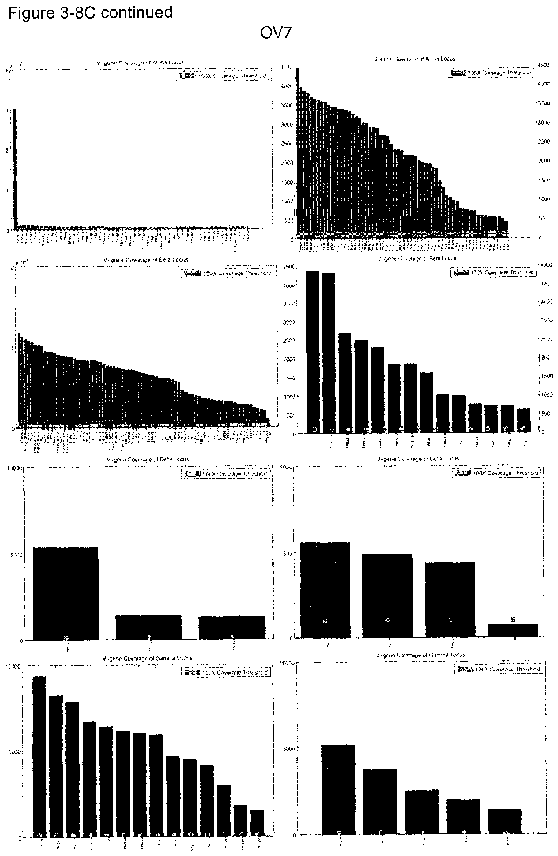

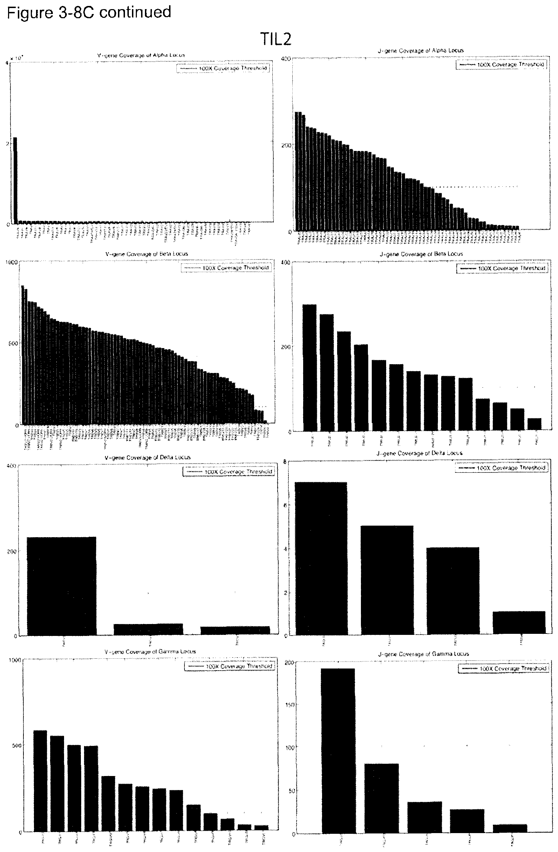

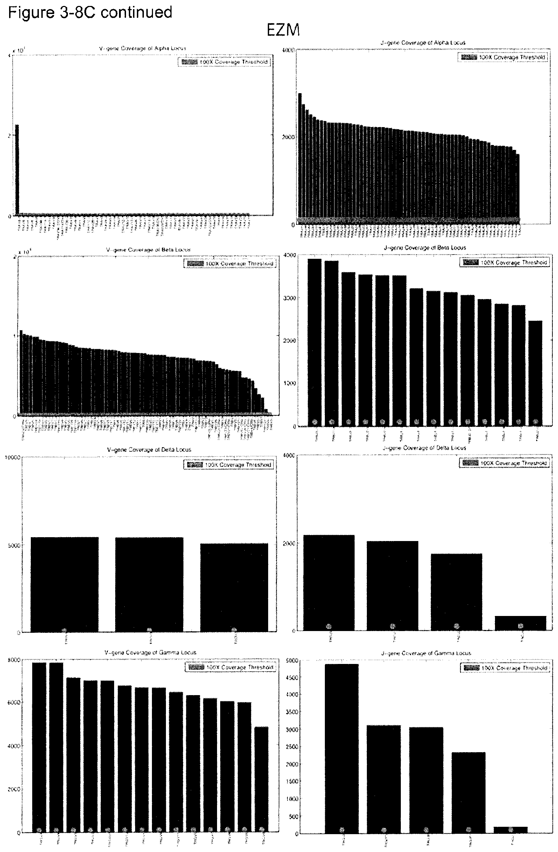

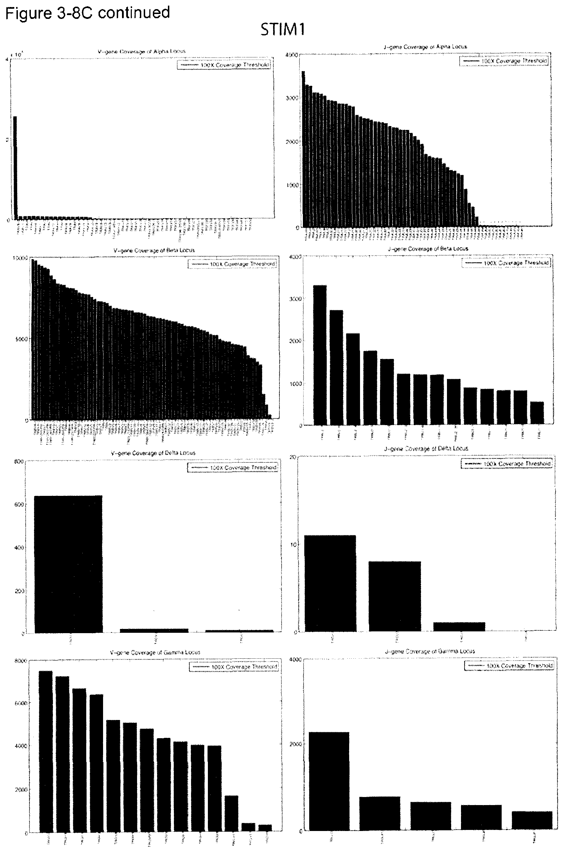

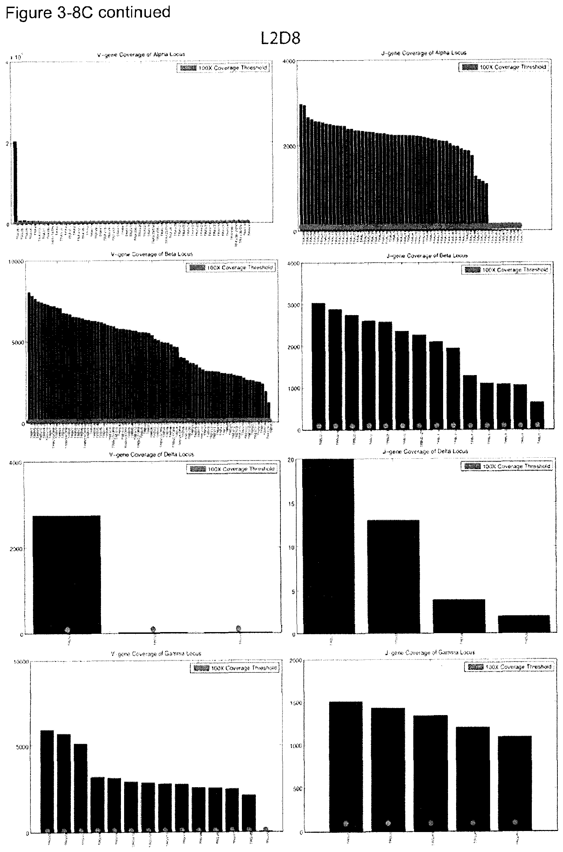

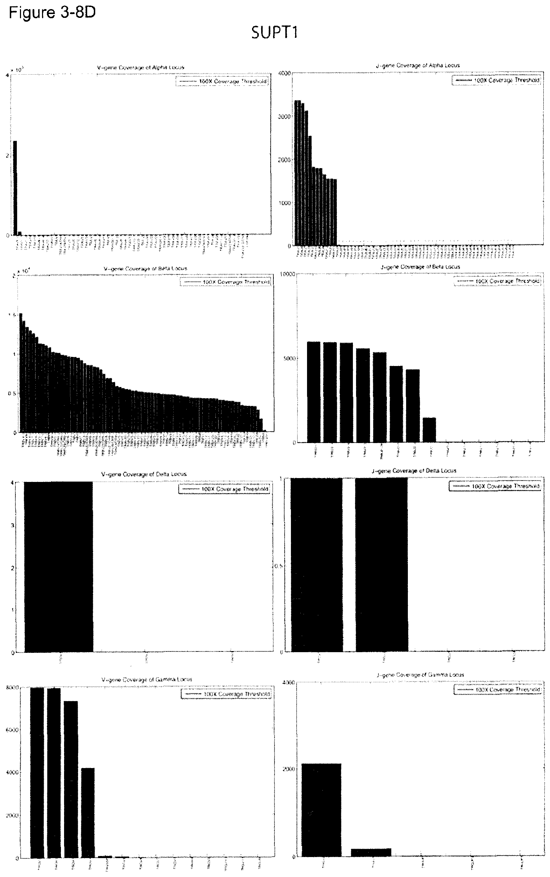

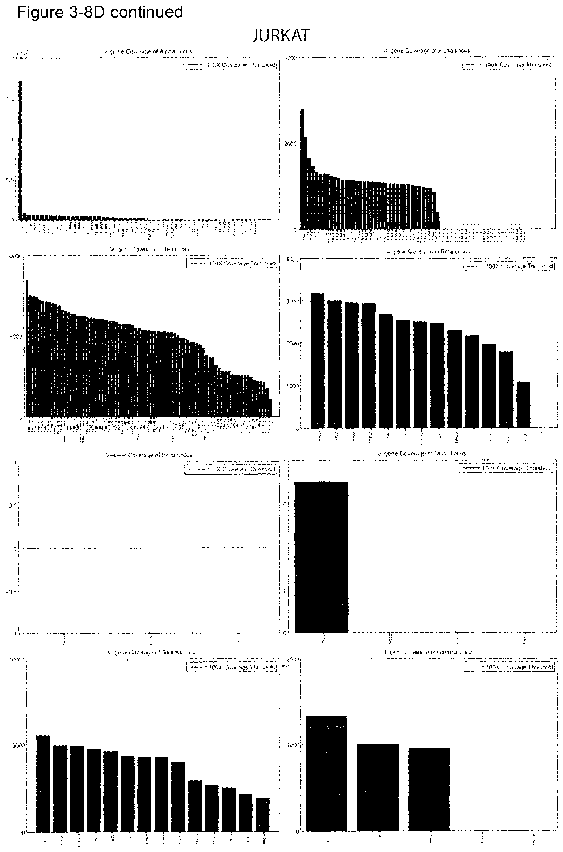

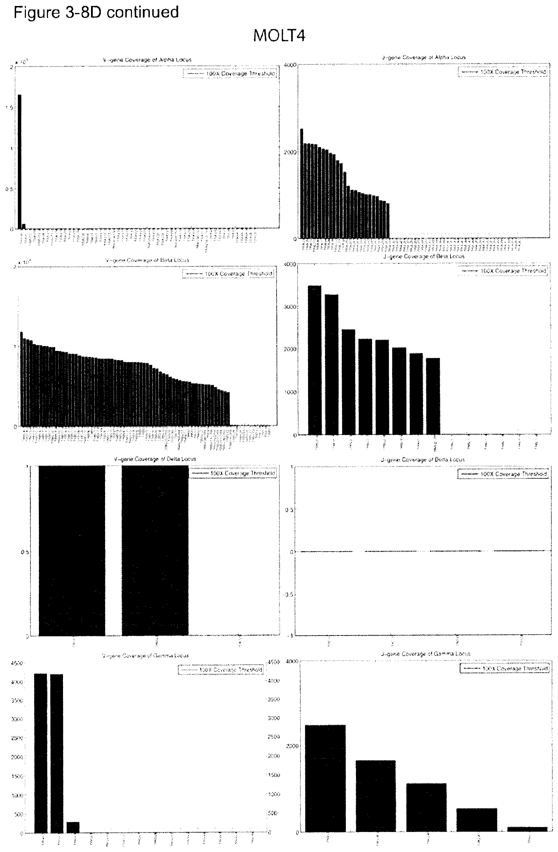

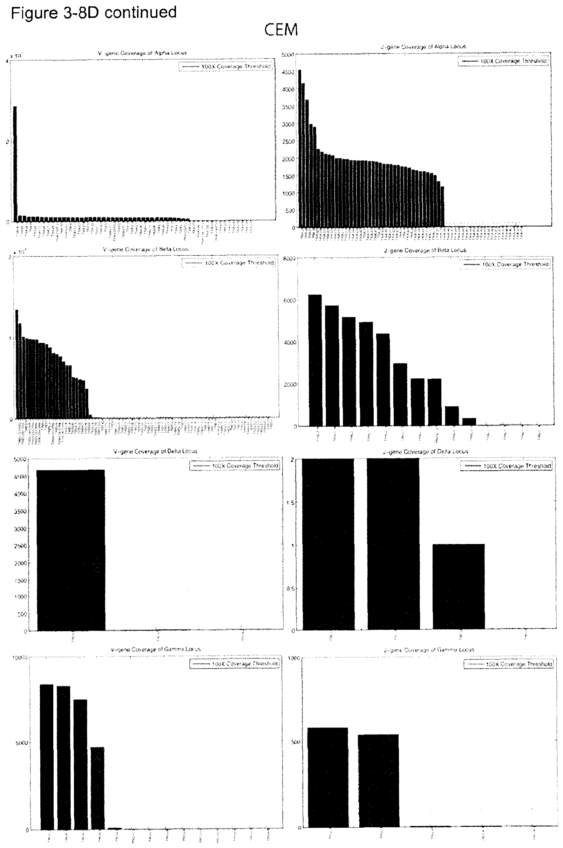

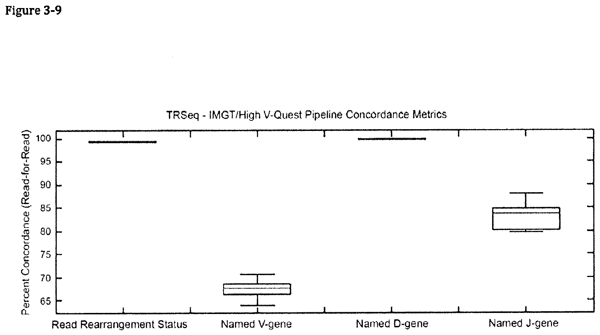

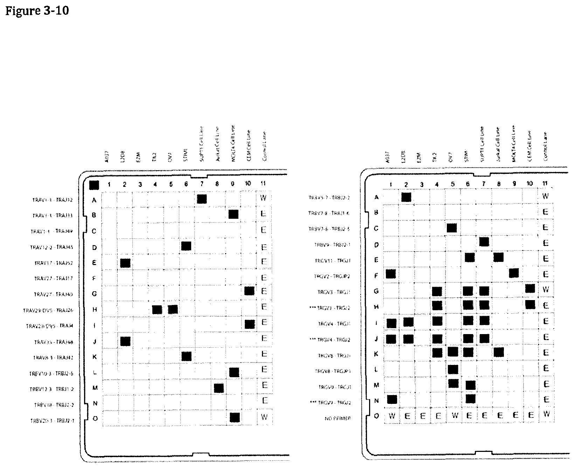

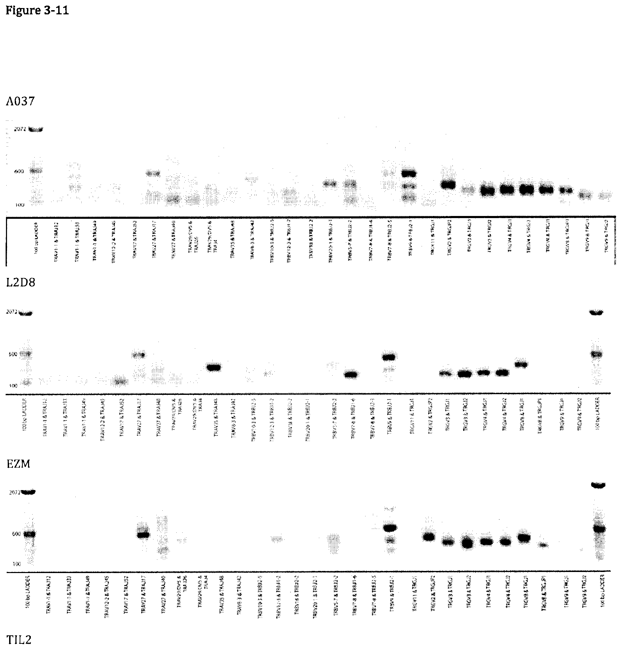

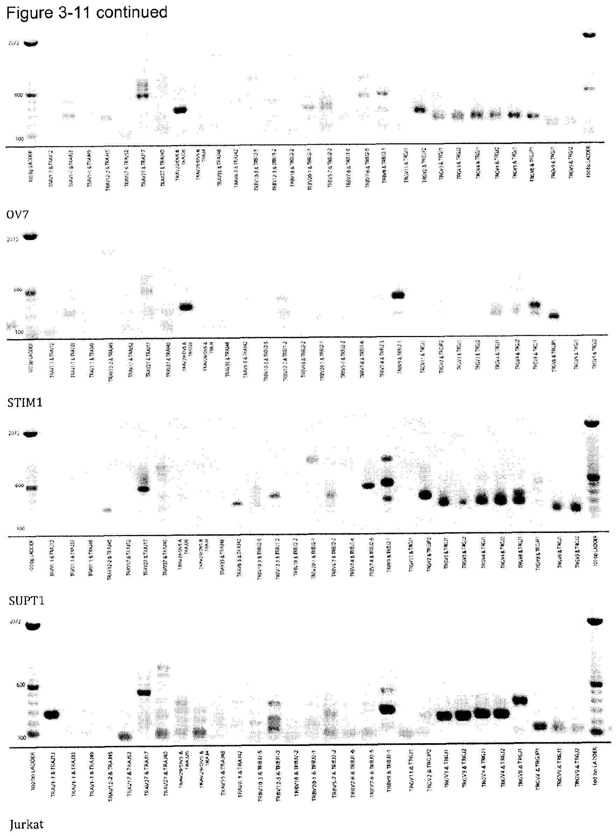

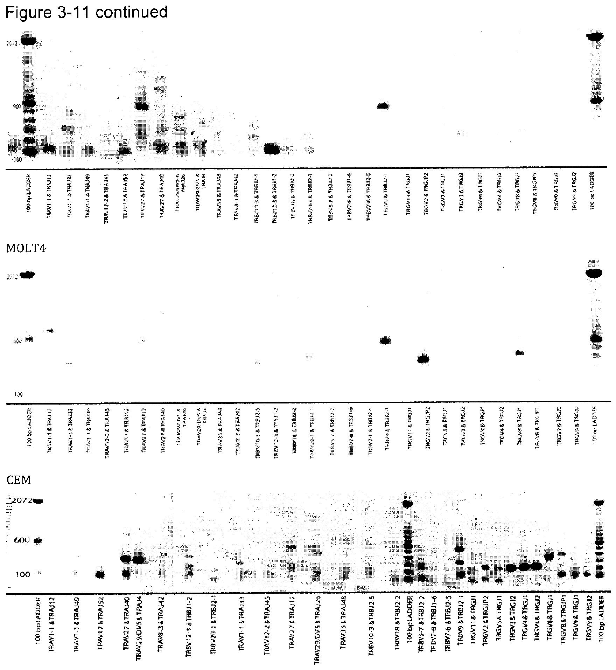

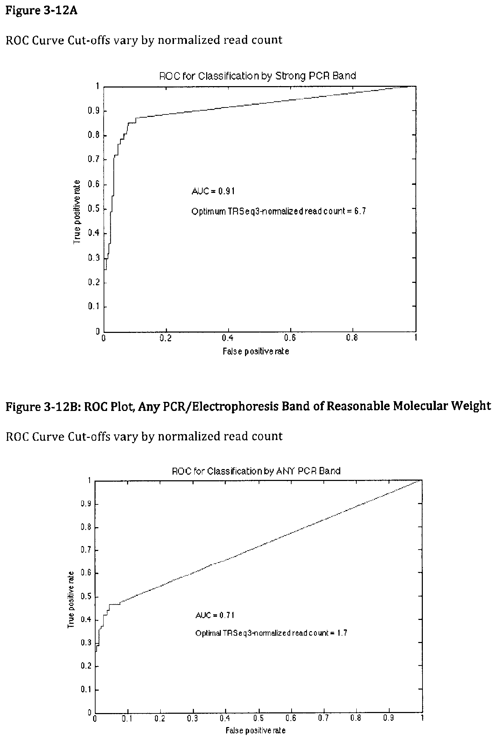

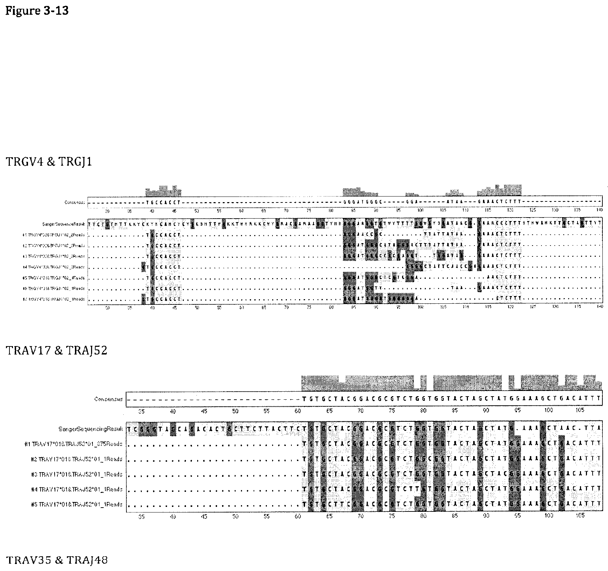

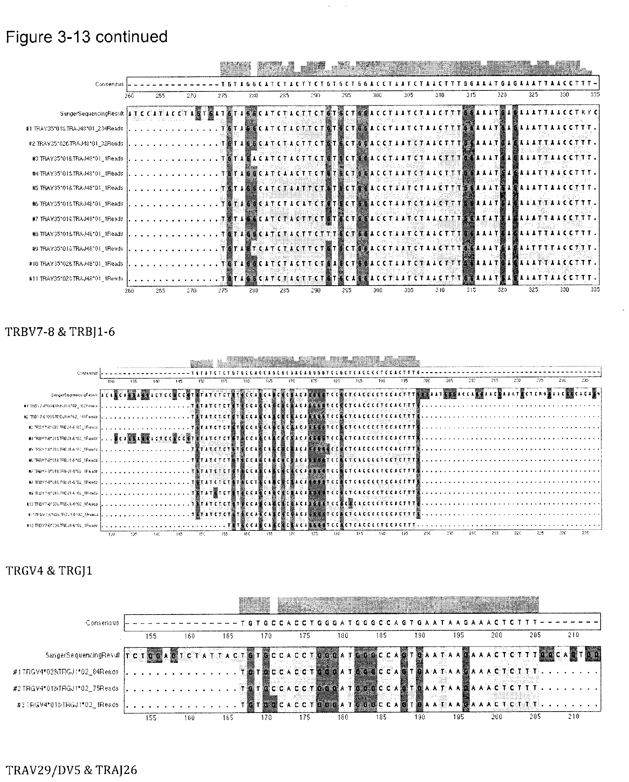

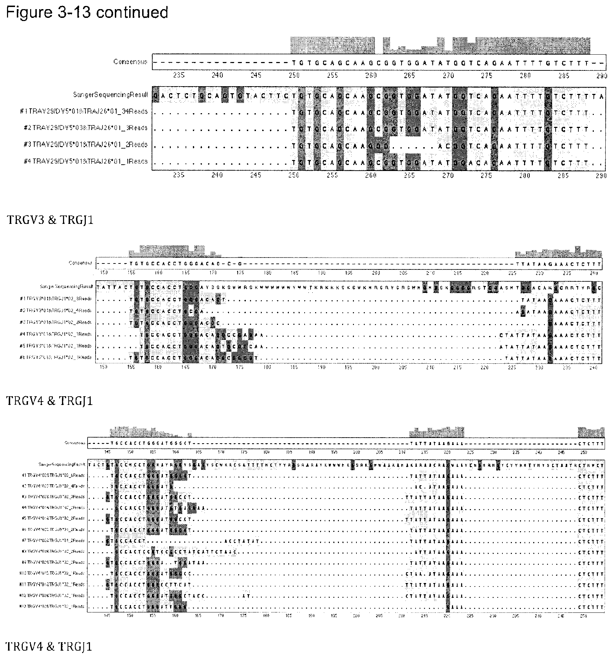

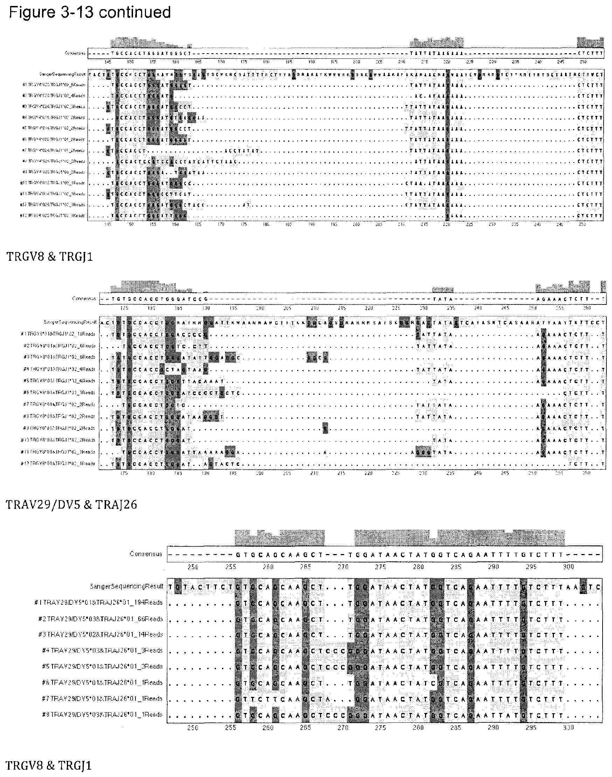

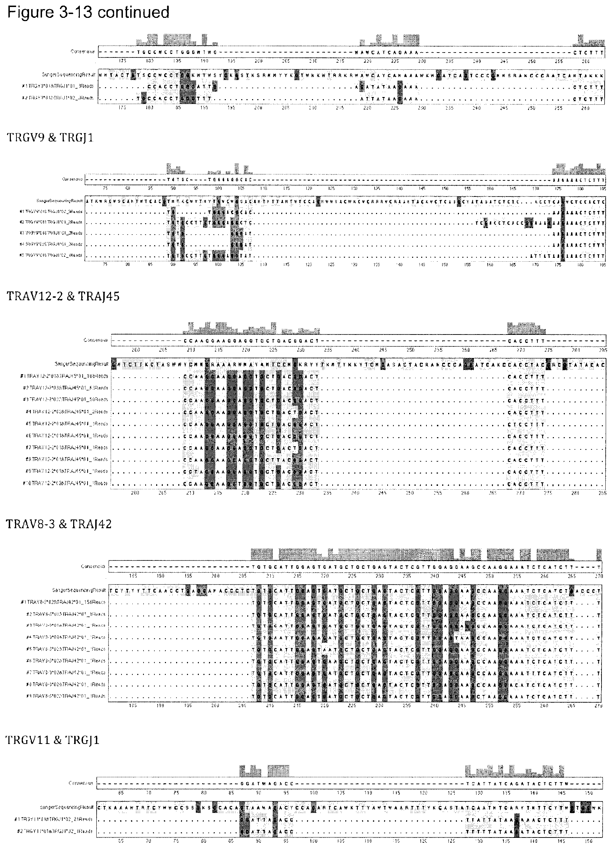

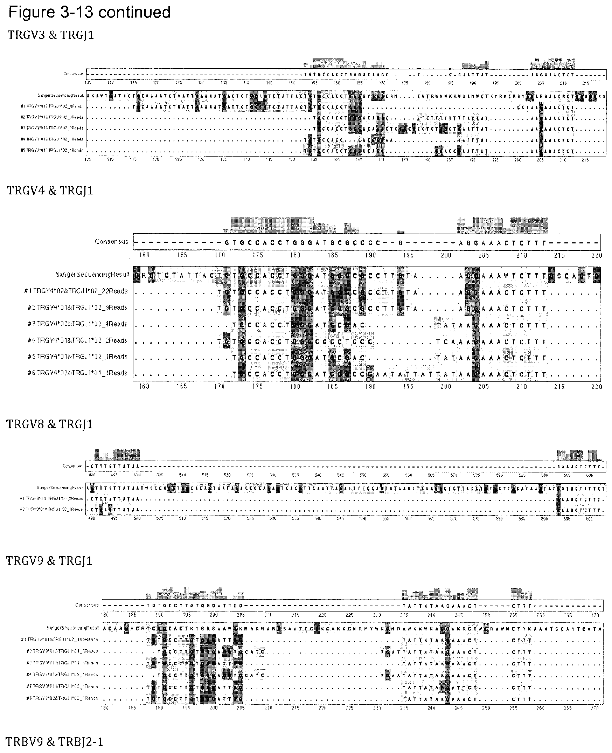

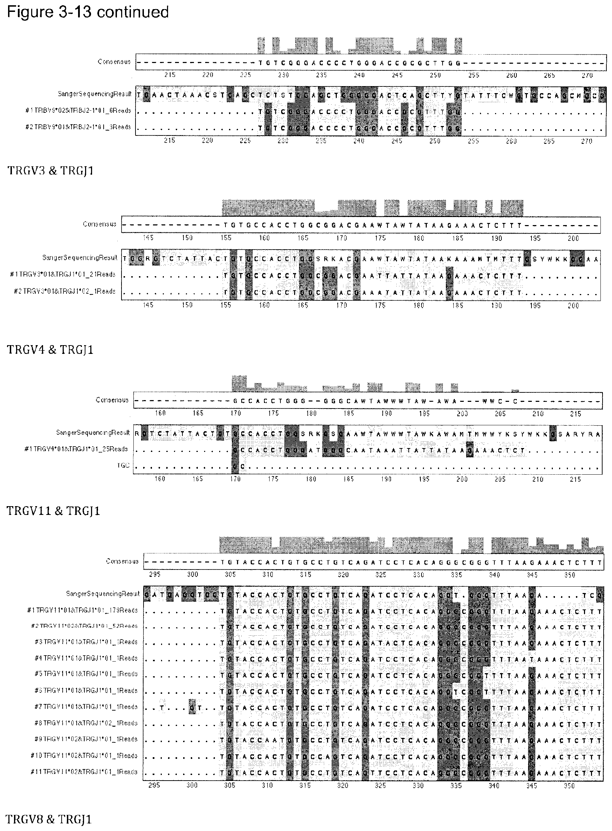

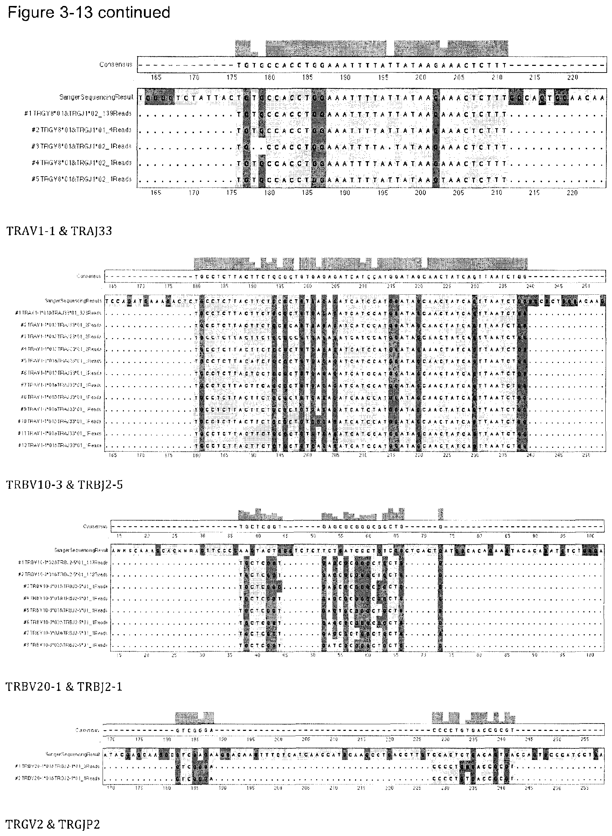

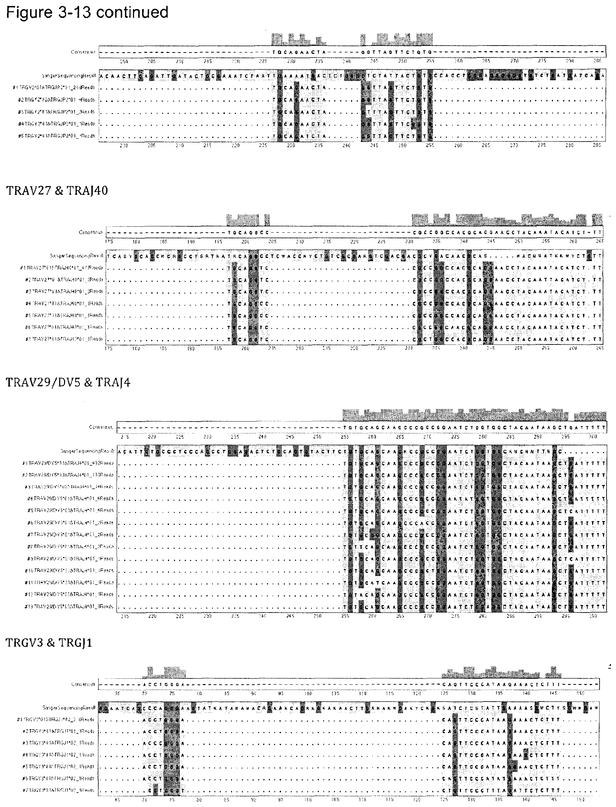

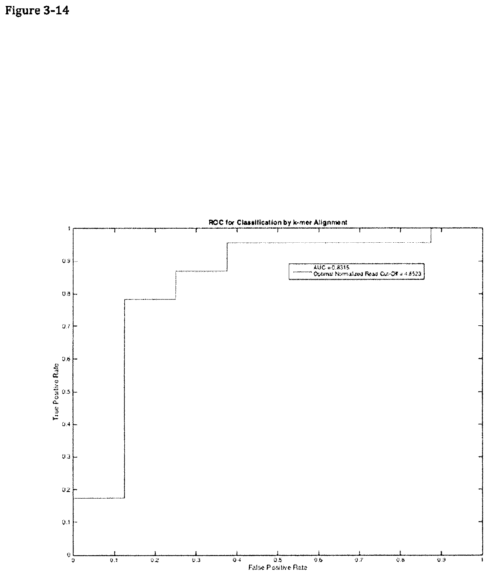

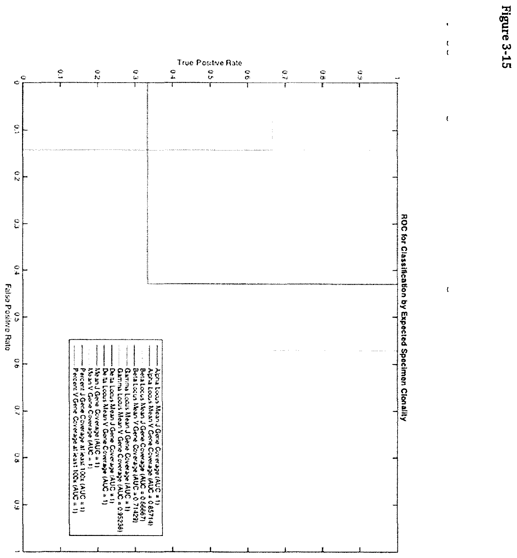

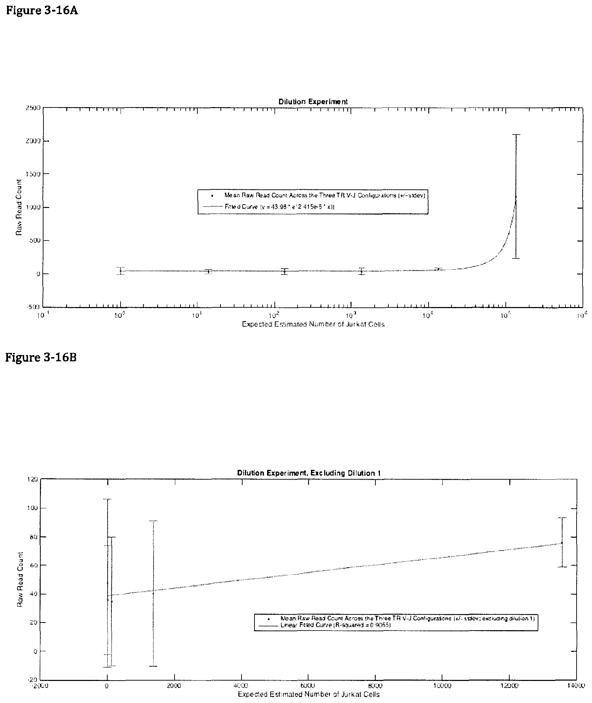

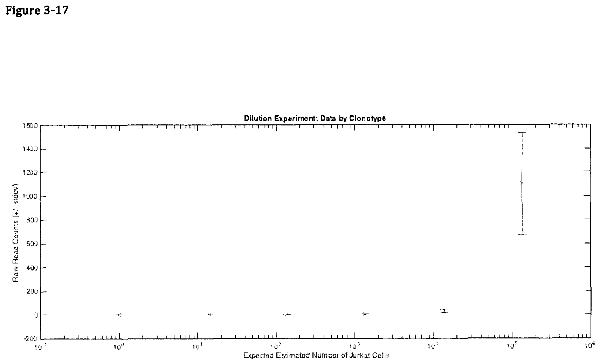

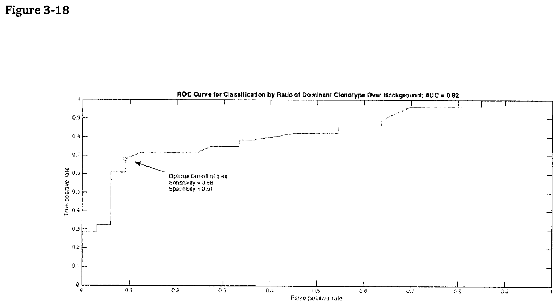

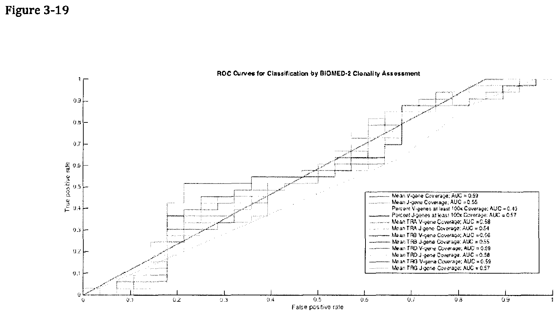

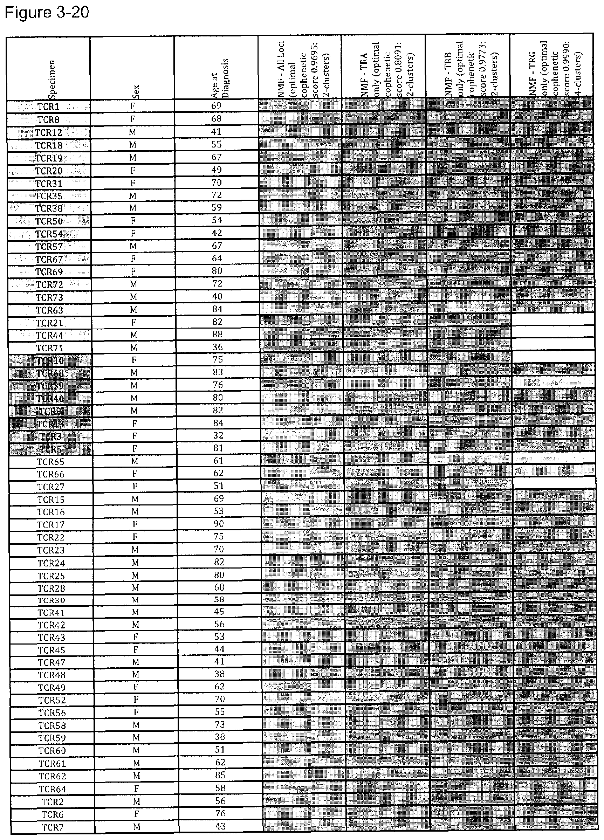

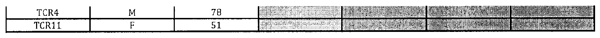

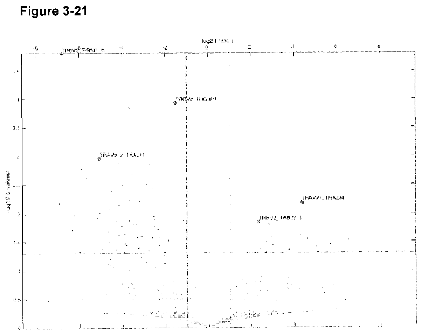

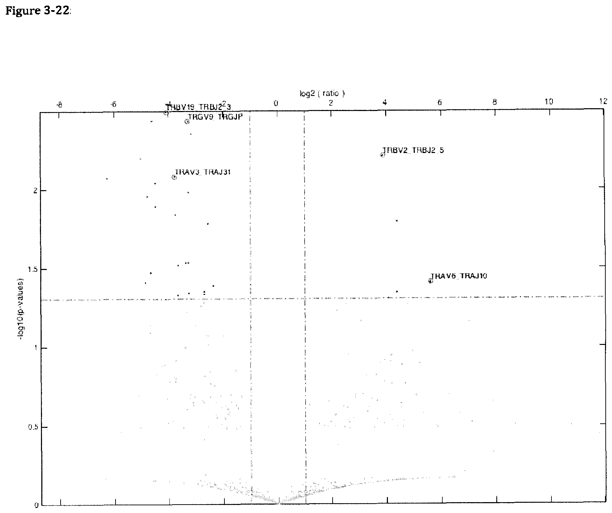

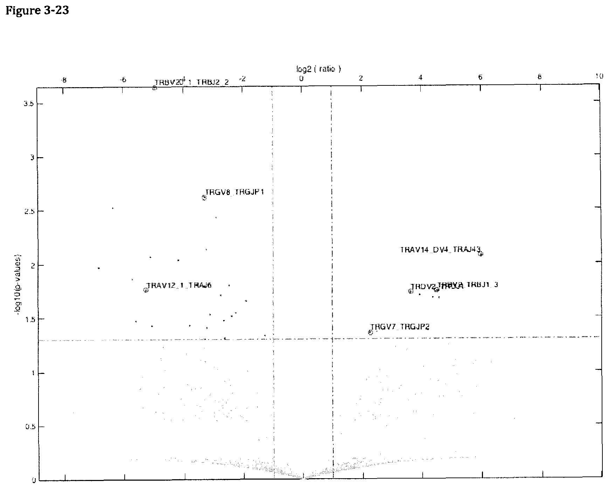

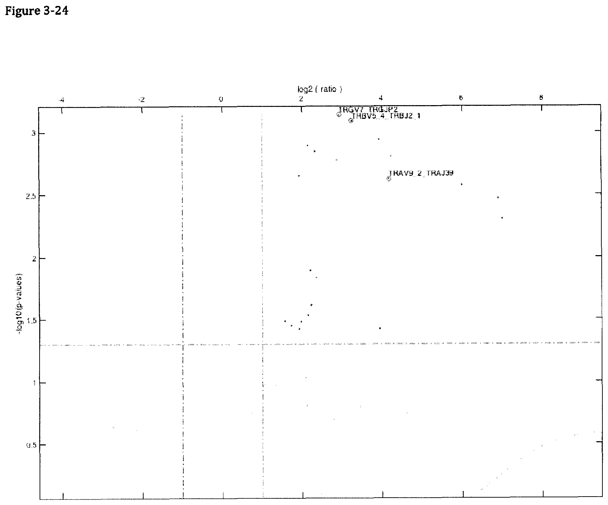

**Figure 3-1A****:** Read Length Simulation Results. In this simulation, the percent of total BWA-detectable VDJ gene combinations obtained by reference sequence concatenation was computed. Note that a plateau of maximal sensitivity could be inferred with a read length of approximately 200 bp or more.
**Figure 3-1B****:** TRBV6 Group Phylogenetic Sequence Alignment. A post-hoc analysis of the TRBV6 group by phylogenetic comparison of reference sequences suggested that the TRBV6-2 01-allele and TRBV6-3 gene are more closely related than the TRBV6-2 01 & 02 alleles, a seeming violation of the IMGT naming/numbering system.
**Figure 3-1C****:** TRGJ Group Phylogenetic Sequence Alignment. A post-hoc analysis of the TRGJ group by phylogenetic comparison of reference sequences suggested that the TRGJ1 02-allele and TRGJ2 gene are more closely related than the TRGJ1 01 & 02-alleles, a seeming violation of the IMGT naming/numbering system.

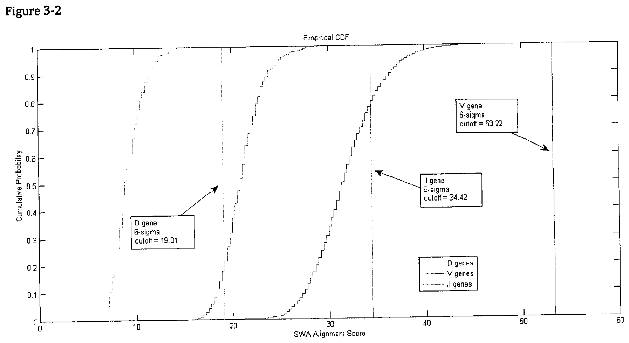

**Figure 3-2****:** Empirical determination of MATLAB alignment score cut-off values.

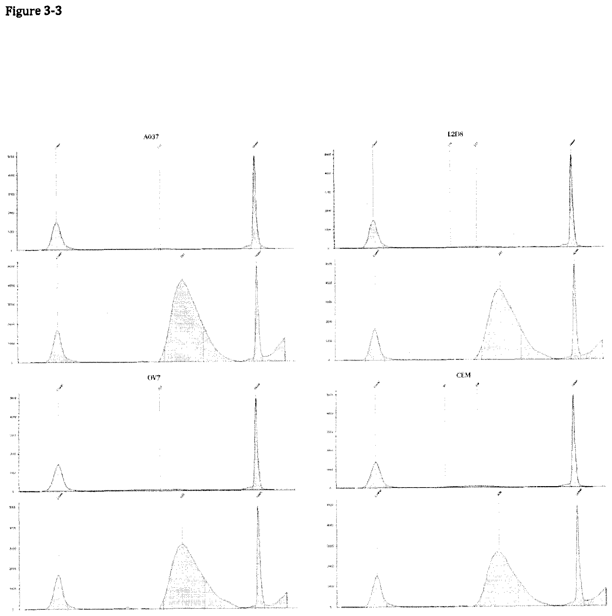

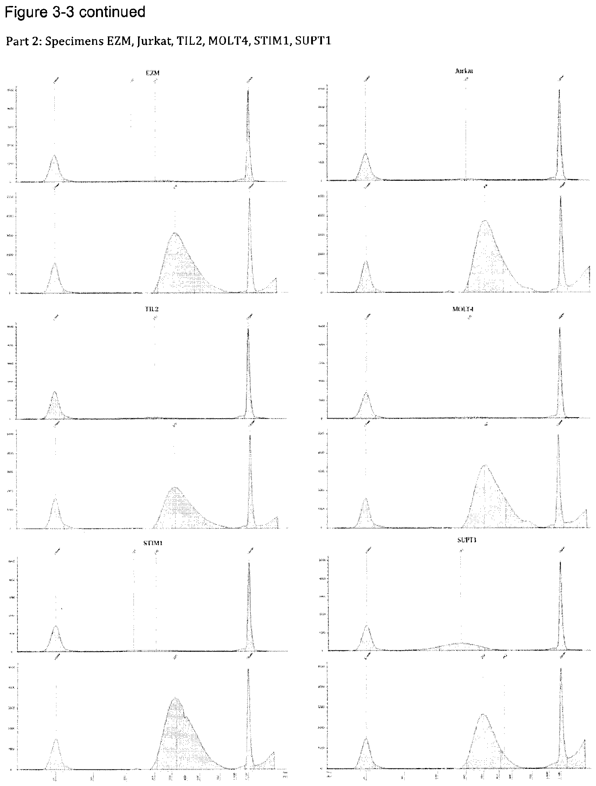

**Figure 3-3****:** First Run TapeStation tracings Pre-Library (post-shearing) vs. Post-Library Preparation. In this tableau, each specimen's electropherogram tracing before & after library preparation is displayed (one above the other) in order to compare the library preparation adapter/barcode ligation success & expected increase in average fragment length of approximately 100 bp. Part 1: Specimens A037, L2D8, OV7 & CEM. Part 2: Specimens EZM, Jurkat, TIL2, MOLT4, STIM1, SUPT1.

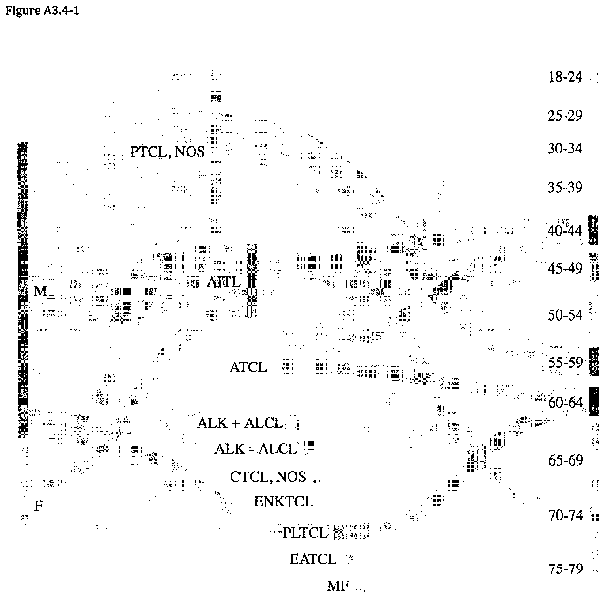

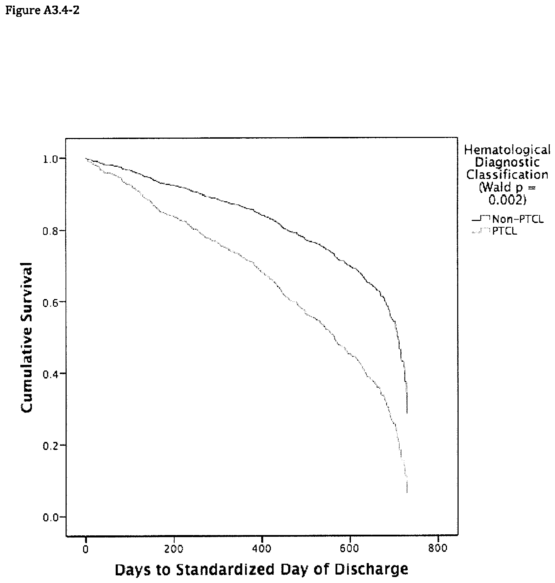

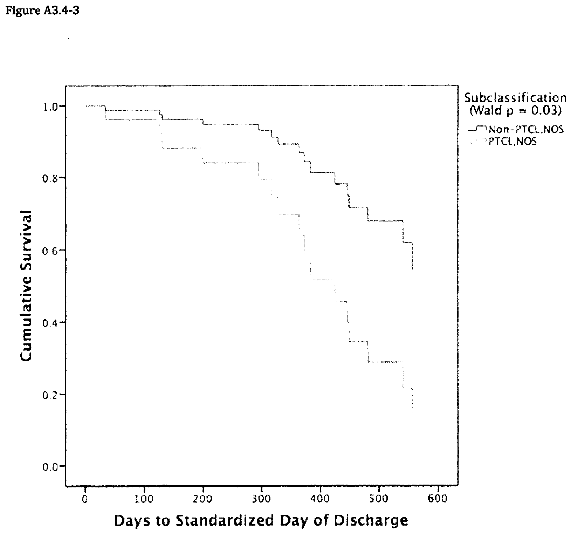

**Figure 3-4****:** PEAR Algorithm Read-Merge & Assembly Results for each first-run specimen.
**Figure 3-5****:** First Run Comparison of PEAR-produced input Reads (blue) vs. Reads-on-Target (yellow).
**Figure 3-6****:** First Run Summary Coverage Statistics. Mean Depth of Coverage and Percent of Genes with Greater than 100x Coverage shown.
**Figure 3-7A****:** Histogram of V-J gene alignment pair counts for sample A037. Healthy patient peripheral blood specimen demonstrating a "polyclonal" process, for comparison with a clonal specimen delineated in Figure 3-7B.
**Figure 3-7B****:** Histogram of V-J gene alignment pair counts for a selected clonal specimen. CEM cell line demonstrating a "clonal" process, for comparison with the polyclonal specimen of Figure 3-7A. **Figure 3-8A****:** First Run Lymphocyte Sample Circos Plots. The ideogram represents all intra-locus V-J combinations (color coded by locus: TRA red; TRB blue; TRD yellow; TRG green); the height and width of the gray bars are determined by read counts of identical V & J gene name and CDR3 sequence triads.
**Figure 3-8B****:** First Run Cell Line Circos Plots. The ideogram represents all intra-locus V-J combinations (color coded by locus: TRA red; TRB blue; TRD yellow; TRG green); the height and width of the gray bars are determined by read counts of identical V & J gene name and CDR3 sequence triads.
**Figure 3-8C****:** Tableaus of coverage histograms for V and J genes across all four TR loci for each of the six lymphocyte samples. Specimens more characteristically "polyclonal" show a uniform coverage across most if not all genes, at greater than 100x; specimens more seemingly "clonal" tend to show at least a subset of genes at coverage less than 100x.
**Figure 3-8D****:** Tableau of coverage histograms for V and J genes across all four TR loci for each of the four cell line samples. These clonal specimens uniformly show at least a subset of genes at coverage less than 100x.
**Figure 3-9****:** First Run TRSeq algorithm performance metrics relative to the IMGT/High V-Quest Pipeline. This boxplot highlights the percent concordance of calls made by the TRSeq pipeline across all four loci and over all 10 specimens for each of overall read rearrangement status, and named V, D, and J-gene concordance relative to the calls made by the IMGT/High V-Quest system.
**Figure 3-10****:** Analytical Validation PCR/Electrophoresis Design. The experiment was performed in a 384-well plate with samples listed by column and primer combinations (V-gene forward & J-gene reverse complement) listed by row; W = water; E = empty; ■ = reaction selected for PCR purification & Sanger Sequencing; *** = excluded from subsequent analyses due to primer sequence redundancy (see methods/results).
**Figure 3-11****:** Analytical Validation Electrophoresis Composite Gel Photographs. Gels are listed by Specimen Name. Primer Combinations (V-gene forward & J-gene reverse complement) are listed along the x-axes; 100 bp ladders are shown along the y-axes. Interpretation of the banding patterns, by expected amplicon size and by intensity, is outlined in Table 3.1A.
**Figure 3-12A****:** ROC Plot by Strong PCR/Electrophoresis Band. ROC Curve Cut-offs vary by normalized read count shown.
**Figure 3-12B****:** ROC Plot, Any PCR/Electrophoresis Band of Reasonable Molecular Weight. ROC Curve Cut-offs vary by normalized read count shown.
**Figure 3-13****:** Analytical Validation Sanger Sequencing Results. In this analysis, the CDR3 sequence from each TRGR configuration is aligned to the corrected Sanger Sequence (with the number of reads of each configuration also tallied); the diagrams below delineate this alignment process for each of the PCR reactions submitted for Sanger Sequencing, as highlighted in Figure 3-10, excluding those cases rejected due to false-positive amplification using the TRGJ2 primer *and* cases not containing TRSeq-identifiable CDR3 sequences (for a total of 32 of 47 reactions).
**Figure 3-14****:** Sanger Sequencing Receiver-Operating Characteristic Curve. Using a k-mer-based analysis, the TRSeq-generated CDR3 sequences were compared to the Sanger Sequence results. For each applicable primer configuration, the corresponding TRSeq-generated CDR3 sequence was aligned using PHRED-based quality-score adjustment as a k-mer across the length of the Sanger ("reference") Sequence. If the optimal alignment from this process was present within the sequence window in which a CDR3 was predicted to exist, the CDR3 read configuration was classified as "compatible." This "compatibility" scoring system was then compared to the read counts of the appertaining TRSeq configuration to generate a ROC curve.
**Figure 3-15****:** Coverage ROC Curve. Classification by expected specimen clonality, with curves for each coverage metric included, defined by varying gene coverage counts, as indicated in the legend.
**Figure 3-16A****:** Dilution Experiment Curve by V-J Configurations. In this experiment, mean raw read counts (+/- standard deviation) of the various Jurkat-specific V-J combinations are tallied for each of the dilutions.
**Figure 3-16B****:** Dilution Experiment Curve by V-J Configurations, Excluding Dilution 1. In this plot, the data from Figure 3-16A are re-analyzed after excluding dilution 1, in order to highlight an apparently linear correlation between raw read counts and expected number of Jurkat cells at the lower end of the dilution series.
**Figure 3-17****:** Dilution Experiment Curve by Clonotype. In this experiment, mean raw read counts (+/- standard deviation) of the various Jurkat-specific clonotypes (i.e. V & J-gene & specific CDR3 sequence), allowing for acceptable CDR3 sequence error per the methods of Bolotin, et al. (27), are tallied for each of the dilutions.
**Figure 3-18****:** NTRA - BIOMED-2 Comparison. ROC analysis for classification by maximum TRB and TRG dominant clonotype read count-to-background ratio relative to overall BIOMED-2 results (taken as positive or negative for a clonal population).
**Figure 3-19****:** Coverage ROC Curve: Classification by BIOMED-2 Clonality Assessment. Cut-offs vary by coverage, as set-out in the legend.
**Figure 3-20****:** Unsupervised NMF clustering of V-J gene combinations. Red highlighted samples represent malignant entities, whereas green highlighted samples represent clonal but non-malignant entities. Colors are arbitrarily assigned to the four cluster designations.
**Figure 3-21****:** Volcano Plot: V-J gene combination usage differences between those cases classified as "clonal" and "polyclonal" by the BIOMED-2 assay. (the top enriched (right) and depleted (left) V-J combinations from each applicable locus are highlighted).
**Figure 3-22****:** Volcano Plot: V-J gene combination usage differences between malignant and non-malignant BIOMED-2-clonal cases, (the top enriched (right) and depleted (left) V-J combinations from each applicable locus are highlighted).
**Figure 3-23****:** Volcano Plot: V-J gene combination usage differences between LGL and non-LGL T-LPDs. (the top enriched (right) and depleted (left) V-J combinations from each applicable locus are highlighted).
**Figure 3-24****:** Volcano Plot: V-J gene combination usage differences between malignant LGL and non-malignant LGLs. (the top depleted V-J combinations from each applicable locus are highlighted).
**Figure A3.4-1:** Sankey plot of relevant CIHI DAD TLPD epidemiology. The TLPD cases are segregated by sex, diagnostic category, as well as age category in relative proportions.
**Figure A3.4-2:** Cox Proportional Hazards Model Survival Curve: TLPD "survival" vs. "survival" of other hematolymphoid entities. Based on anonymized CIHI "survival" estimated by the difference in the de-identified DAD day of disposition from the reference day for all "new" diagnoses.
**Figure A3.4-3:** Cox Proportional Hazards Model Survival Curve: PTCL, NOS "survival" vs. "survival" of other TLPDs.

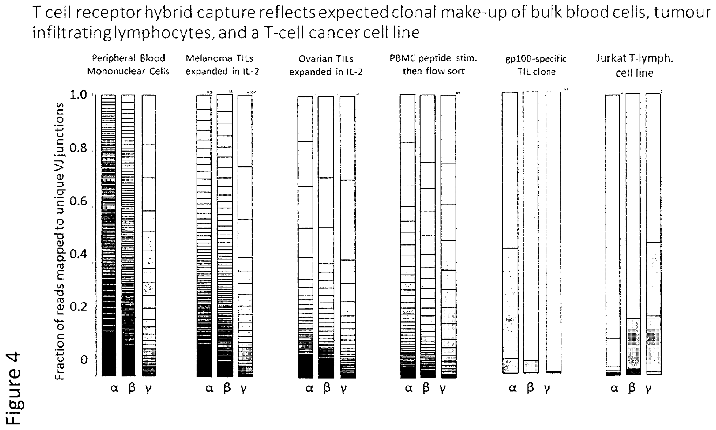

**Figure 4****:** T cell receptor hybrid capture reflects expected clonal make-up of bulk blood cells, tumour infiltrating lymphocytes, T-cell cancer cell line.

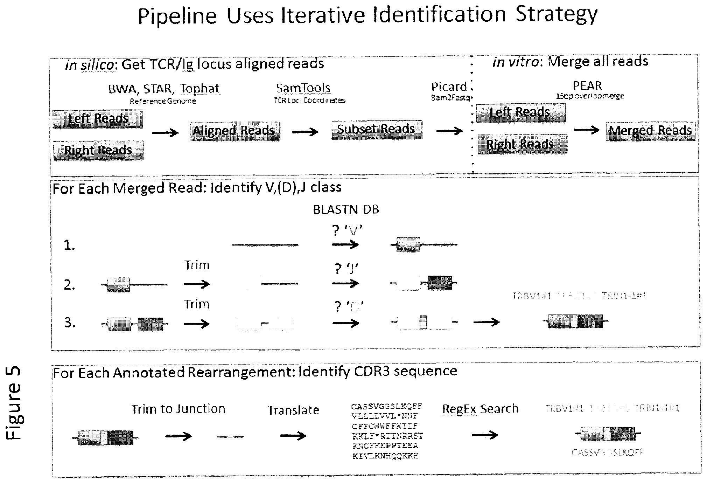

**Figure 5****:** A custom Bash/Python/R pipeline is employed for analysis of paired read sequencing data generated by Illumina DNA sequencing instruments from the hybrid-capture products. This pipeline consists of four major steps: (1) Merging of the paired reads; (2) Identification of specific V, J, and D genes within the fragment sequence; (3) identification of the V/J junction position as well as the antigen specificity determining Complementarity Determining Region 3 (CDR3) sequence at this site; (4) Calculation and visualization of capture efficiency and clone frequency within and across individual samples.

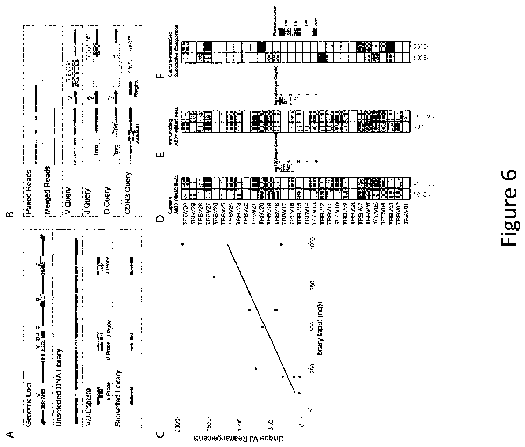

**Figure 6****:** An overview of the CapTCR-Seq hybrid-capture method. (A) Hybrid-capture method experimental flow diagram. Fragments are colored based on whether they contain V-region targets (blue), J-region targets (red), D-regions (green), constant regions (yellow) or non-TCR coding regions (black). (B) V(D)J rearrangement and CDR3 sequence detection algorithm flow diagram. (C) Number of unique VJ pairs recovered relative to library DNA input amount for one-step V capture of A037 PBMC derived libraries. (D) A037 polyclonal human beta locus VJ rearrangements determined by CapTCR-seq. (E) A037 polyclonal human beta locus VJ rearrangements determined by a PCR-based profiling service. (F) Subtractive comparison between CapTCR-seq and PCR-based profiling service. Red indicates relative enrichment of indicated pair by CapTCR-seq while blue indicates relative enrichment of indicated pair by PCR-based profiling.

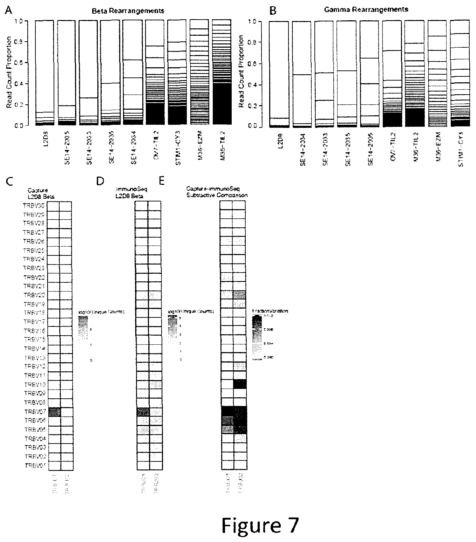

**Figure 7****:** Cell line and tumor isolate T-cell clonality. Boxes represent individual unique VJ pairs and box size reflects abundance in sample. Samples ordered by decreasing clonality. (A) Beta chain VJ rearrangements. (B) Gamma chain VJ rearrangements. (C) L2D8 Gp100 antigen specific beta locus VJ rearrangements determined by CapTCR-seq. (D) L2D8 Gp100 antigen specific beta locus VJ rearrangements determined by a PCR-based profiling service. (E) Subtractive comparison between CapTCR-seq and PCR-based profiling service. Red indicates relative enrichment of indicated pair by CapTCR-seq while blue indicates relative enrichment of indicated pair by PCR-based profiling.

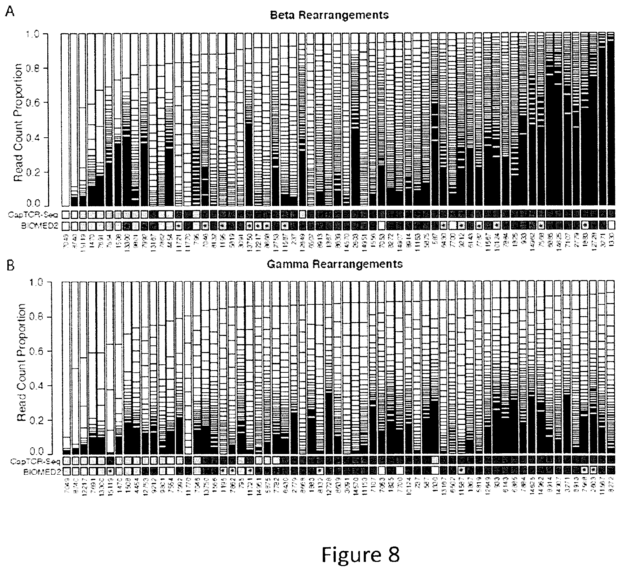

**Figure 8****:** Clinical sample T-cell clonality. Boxes represent individual clones with unique VJ rearrangements and box size reflects abundance in sample. Clonality assessments are indicated as either green (clonal), red (polyclonal), or yellow (not performed). Samples are ordered left to right in terms of increasing CapTCR-Seq clonality with an asterisk indicating disagreement between CapTCR-Seq and BIOMED2 assessments. (A) Beta chain VJ rearrangements. (B) Gamma chain VJ rearrangements.

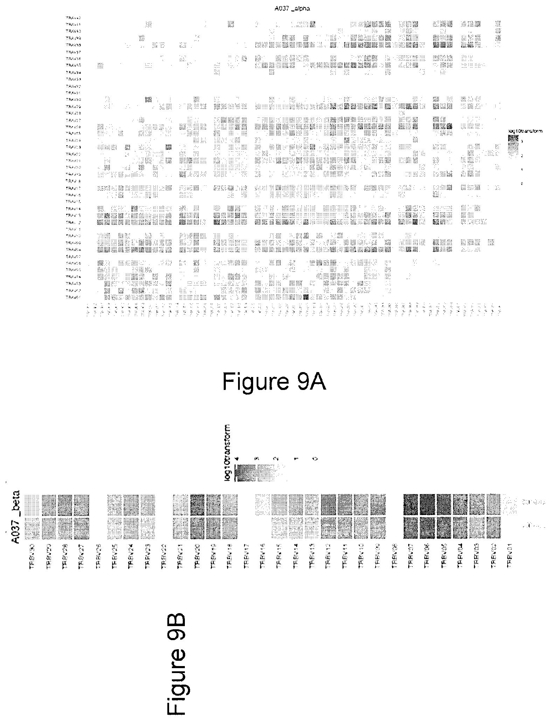

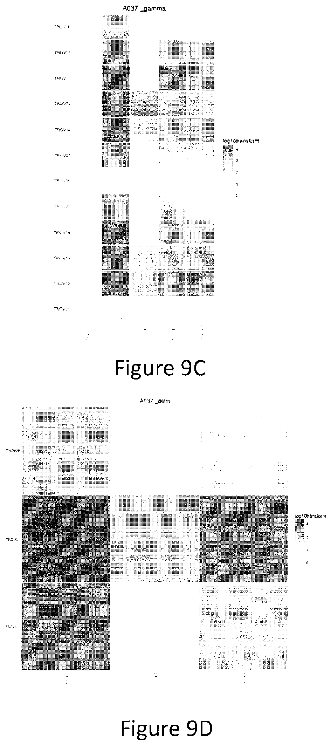

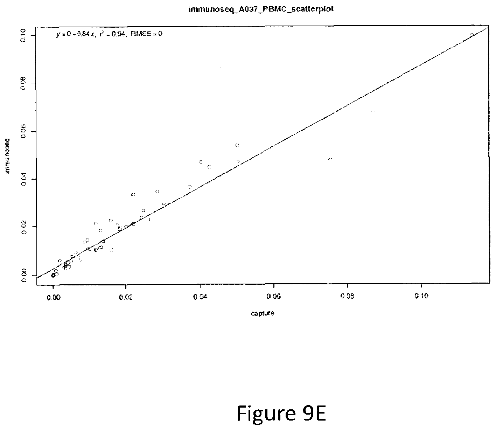

**Figure 9****:** (A) A037 healthy reference sample: Unique alpha chain VJ combinatorial counts. (B) A037 healthy reference sample: Unique beta chain VJ combinatorial counts. (C) A037 healthy reference sample: Unique gamma chain VJ combinatorial counts. (D) A037 healthy reference sample: Unique delta chain VJ combinatorial counts. (E) Comparison of unique VJ fraction prevalence between A037 samples assessed by ImmunoSEQ and CapTCR-seq. Each point represents fraction of total observed rearrangements for each V or J allele.

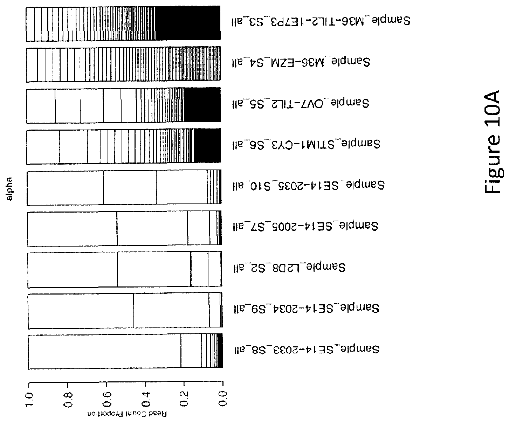

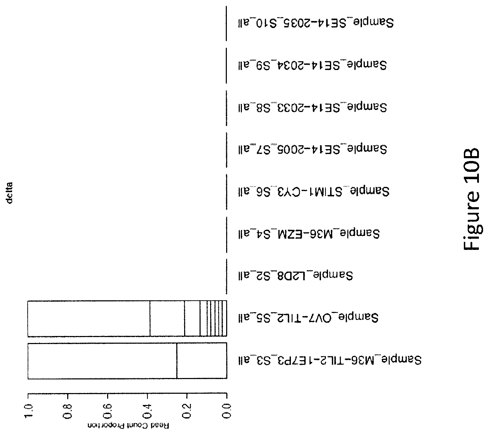

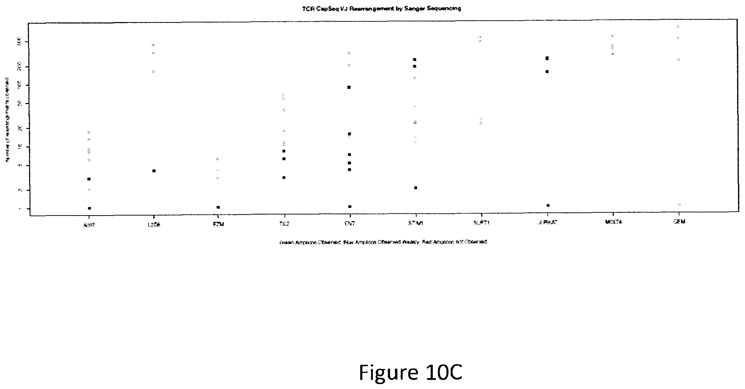

**Figure 10****:** (A) Alpha chain VJ rearrangements. Boxes represent individual unique VJ pairs and box size reflects abundance in sample. Samples are ordered left to right in terms of decreasing clonality based on prevalence of top clone. (B) Delta chain VJ rearrangements. Boxes represent individual unique VJ pairs and box size reflects abundance in sample. Delta rearrangements were not observed for all samples. Samples are ordered left to right in terms of decreasing clonality based on prevalence of top clone. (C) Sanger sequencing validation of individual VJ rearrangements from hybrid-capture sample data with the number of times the given VJ rearrangement was observed plotted on the y-axis. VJ rearrangements that failed to generate a dominant band upon PCR amplification tended to be those with low observation counts. Green: Amplicon observed; Blue: Amplicon observed weakly; Red: Amplicon not observed.

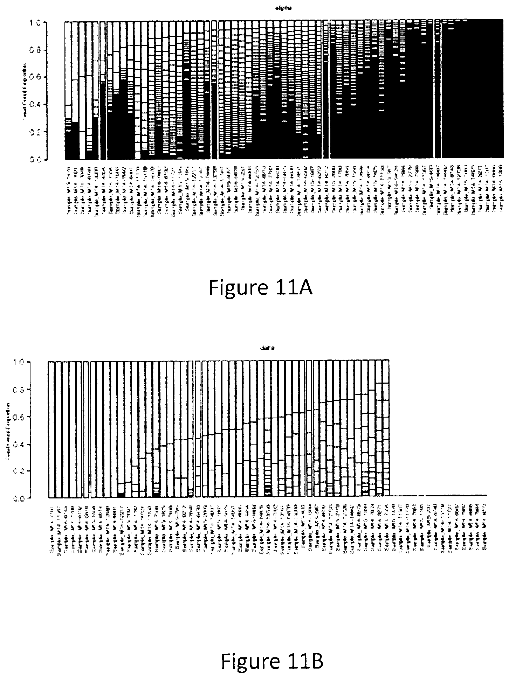

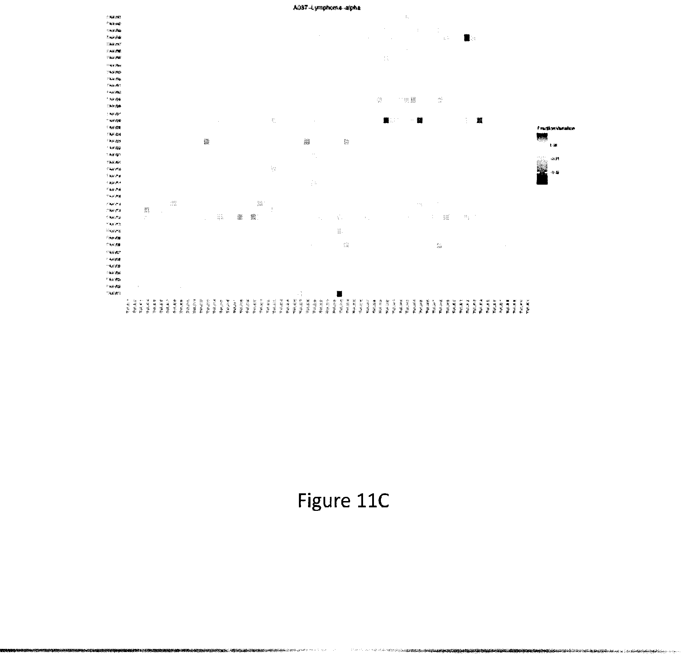

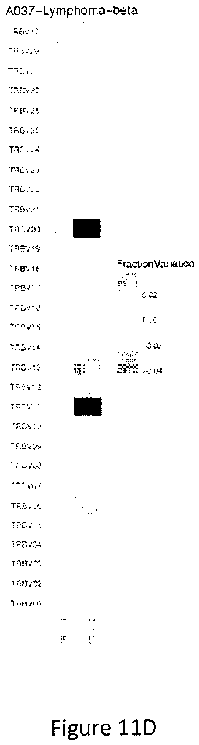

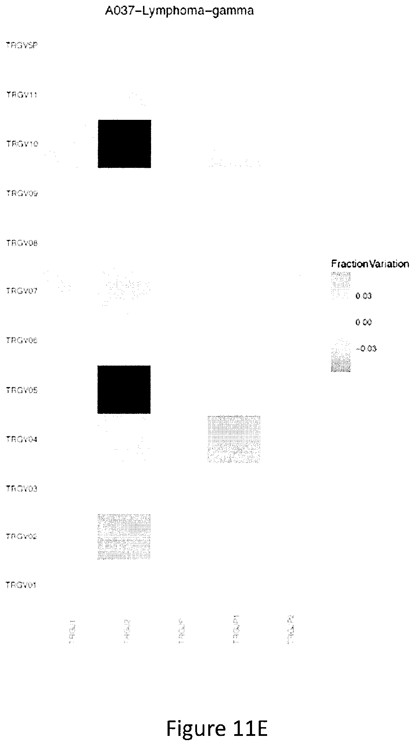

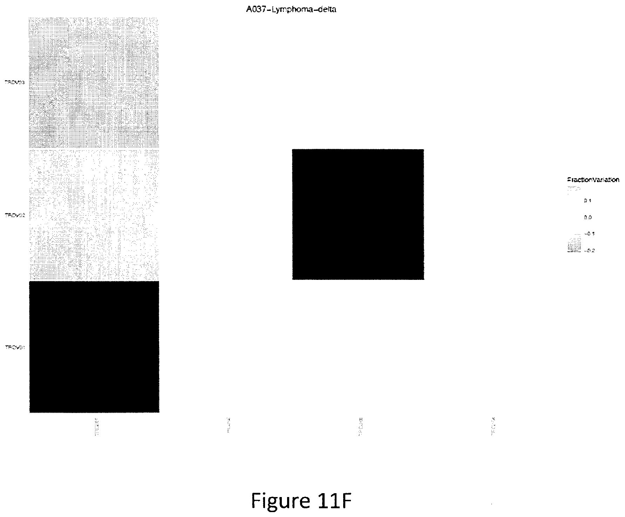

**Figure 11****:** (A) Alpha chain. Boxes represent individual VJ rearrangements and box size reflects abundance in sample. Samples are ordered left to right in terms of decreasing clonality based on prevalence of top clone. (B) Delta chain. Boxes represent individual VJ rearrangements and box size reflects abundance in sample. Samples are ordered left to right in terms of decreasing clonality based on prevalence of top clone. Delta rearrangements were not observed for all samples. (C) Subtractive comparison between polyclonal A037 and collective lymphoma data set alpha VJ rearrangements. Red indicates relative enrichment in capture data while blue indicates relative enrichment in lymphoma data. (D) Subtractive comparison between polyclonal A037 and collective lymphoma data set beta VJ rearrangements. Red indicates relative enrichment in capture data while blue indicates relative enrichment in lymphoma data. (E) Subtractive comparison between polyclonal A037 and collective lymphoma data set gamma VJ rearrangements. Red indicates relative enrichment in capture data while blue indicates relative enrichment in lymphoma data. (F) Subtractive comparison between polyclonal A037 and collective lymphoma data set delta VJ rearrangements. Red indicates relative enrichment in capture data while blue indicates relative enrichment in lymphoma data.

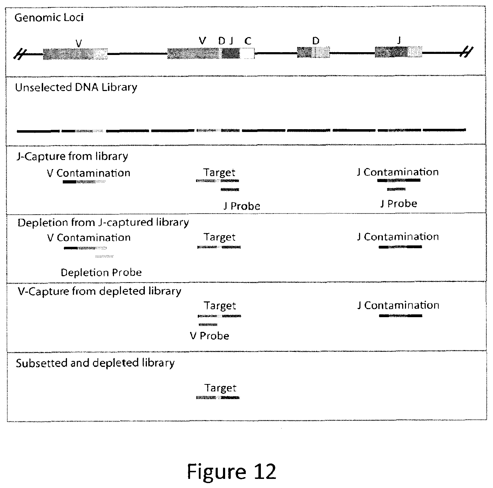

**Figure 12****:** Overview of the capture method. Panel 1: A representative TCR locus with unrearranged and rearranged V, D, J, C gene segments. Panel 2: The TCR locus when sheared and represented in a sequencing library. Panel 3: Subsetting of J-containing regions with the J-probe library. Panel 4: Removal (depletion) of non-rearranged V-containing regions from the library with the depletion-probe library. Panel 5: Subsetting of V-containing regions with the V-probe library. Panel 6: Final subsetted library.

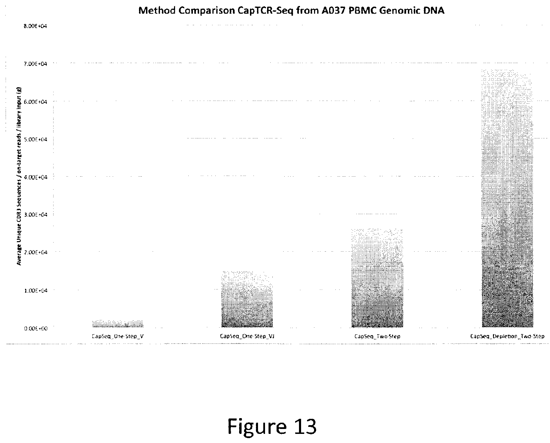

**Figure 13****:** Comparison of different method variants in terms of yielded average unique CDR3 sequences (normalized to reads and library input).

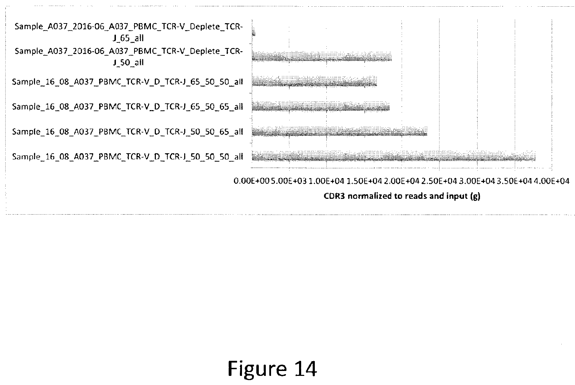

**Figure 14****:** Comparison of different hybridization and capture temperatures in terms of yielded average unique CDR3 sequences (normalized to reads and library input).

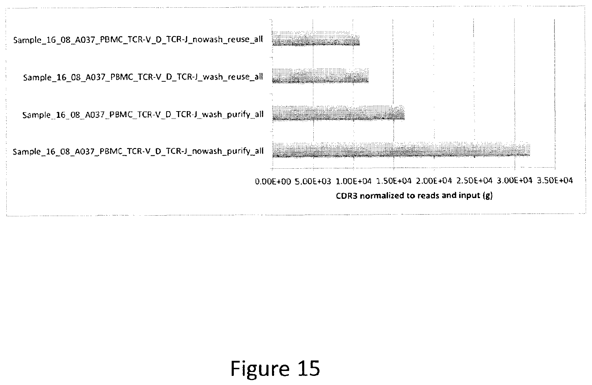

**Figure 15****:** Comparison of different depletion clean-up steps in terms of yielded average unique CDR3 sequences (normalized to reads and library input).

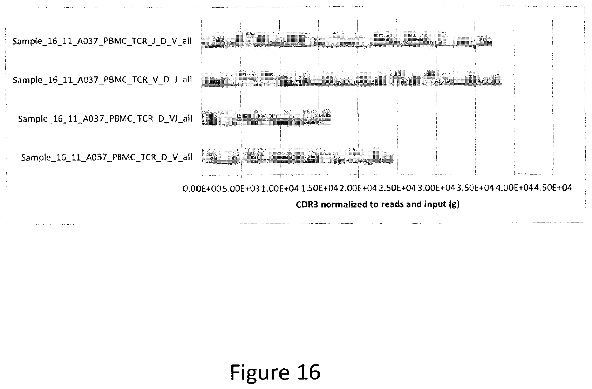

**Figure 16****:** Comparison of different permutations of iterative captures in terms of yielded average unique CDR3 sequences (normalized to reads and library input).

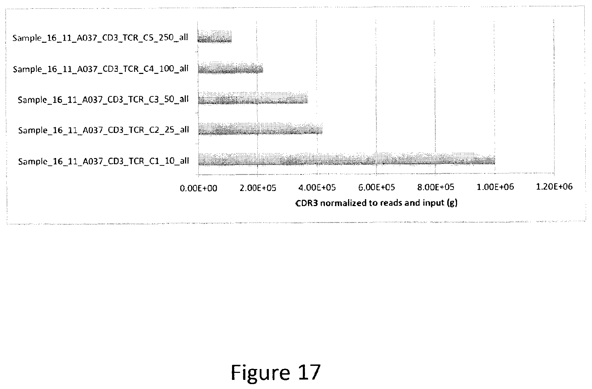

**Figure 17****:** CD3+ T cell fraction dilution curve. Comparison of average unique CDR3 sequences (normalized to reads and library input) for samples with varying amounts of source material added to generate the library (10ng-250ng).

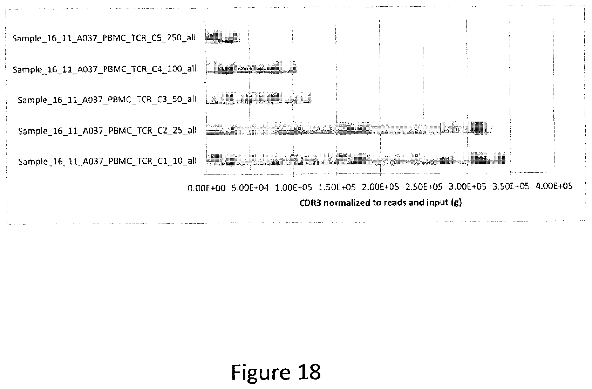

**Figure 18****:** PBMC fraction dilution curve. Comparison of average unique CDR3 sequences (normalized to reads and library input) for samples with varying amounts of source material added to generate the library (10ng-250ng).

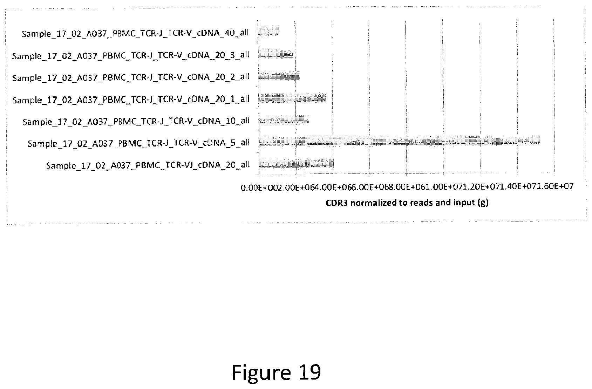

**Figure 19****:** PBMC fraction cDNA dilution curve. Comparison of average unique CDR3 sequences (normalized to reads and library input) for samples with varying amounts of source material added to generate the library (5ng-40ng).

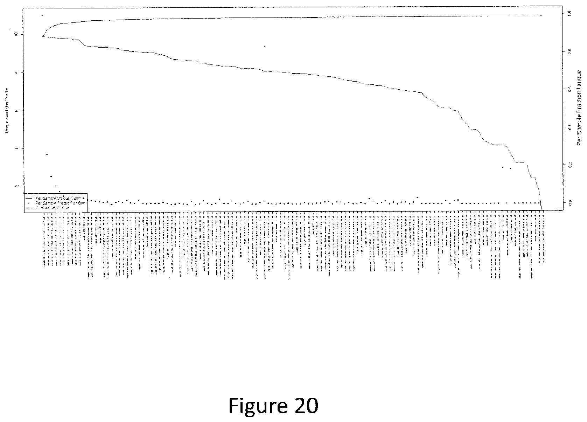

**Figure 20****:** A037 VJ repertoire saturation curve. All samples derived from a single patient blood draw. Samples are drawn on the X-axis and black dots represents the fraction of new VJ combinations not seen before in previous samples from left to right and graphed on the right axis.
Blue curve represents total combined number of unique VJ combinations across all samples from left to right and graphed on the left axis (log). Red curve represents per sample number of unique VJ combinations graphed on the left axis (log).

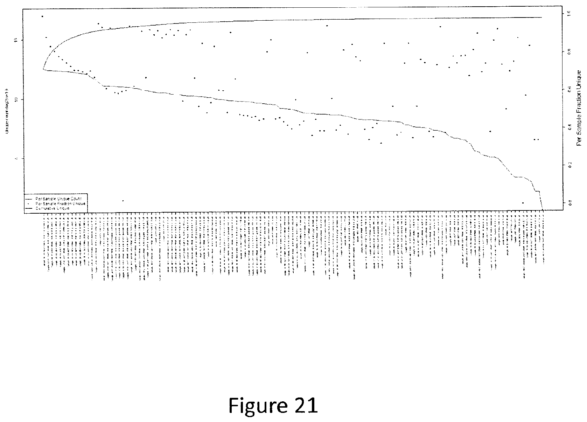

**Figure 21****:** A037 CDR3 repertoire saturation curve. All samples derived from a single patient blood draw. Samples are drawn on the X-axis and black dots represents the fraction of new CDR3 combinations not seen before in previous samples from left to right and graphed on the right axis. Blue curve represents total combined number of unique CDR3 combinations across all samples from left to right and graphed on the left axis (log). Red curve represents per sample number of unique CDR3 combinations graphed on the left axis (log).

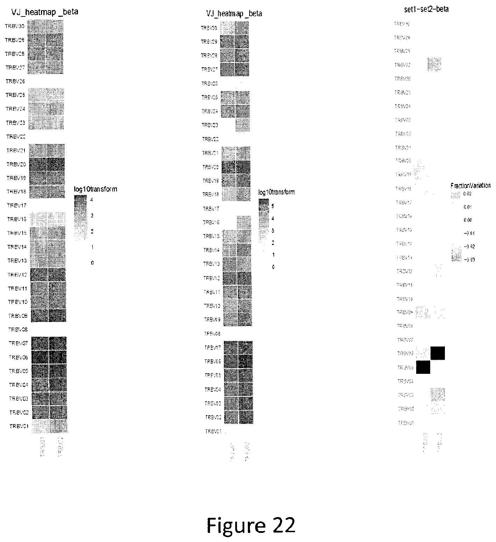

**Figure 22****:** Comparison of VJ beta locus repertoire for A037 sample derived from genomic DNA (panel 1) and from cDNA (panel 2). A subtractive heatmap is shown in panel 3 that shows differences in overall repertoire between the two samples. Red indicates deviation for genomic, while blue indicates deviation for cDNA.

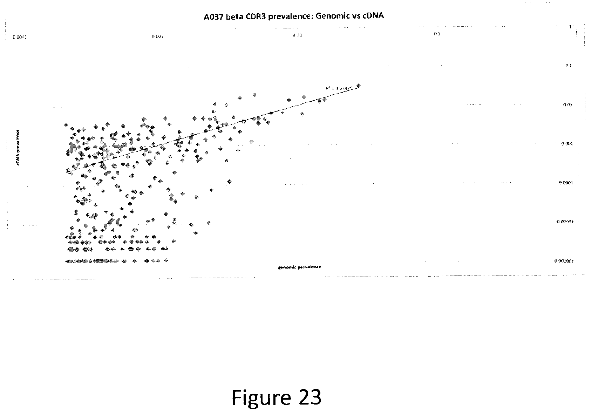

**Figure 23****:** Prevalence comparison of the top 1000 beta locus CDR3 in the genomic DNA set compared with their prevalences in the cDNA set.

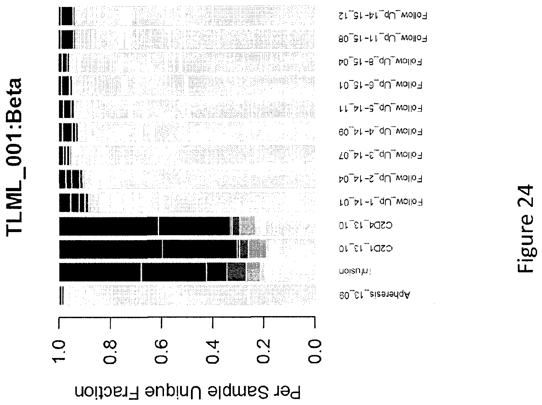

**Figure 24****:** Beta locus VJ repertoire of an adoptive cell transfer immunotherapy patient over time.
Samples are indicated on the X axis ordered by date of sample. VJ clones are ordered in all samples according to prevalence in the TIL infusion product and the top nine most prevalent TIL infusion clones are colored.

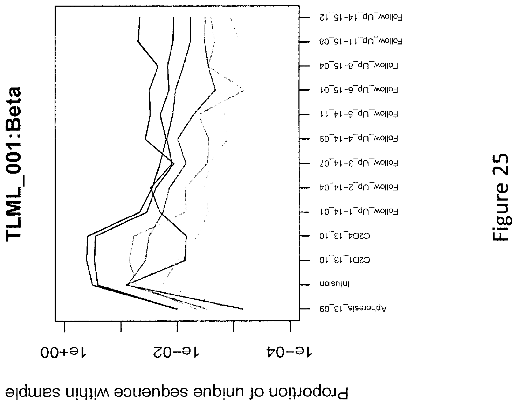

**Figure 25****:** Nine most prevalent TIL infusion clones at the Beta locus of an adoptive cell transfer immunotherapy patient over time. Samples are indicated on the X axis ordered by date of sample.

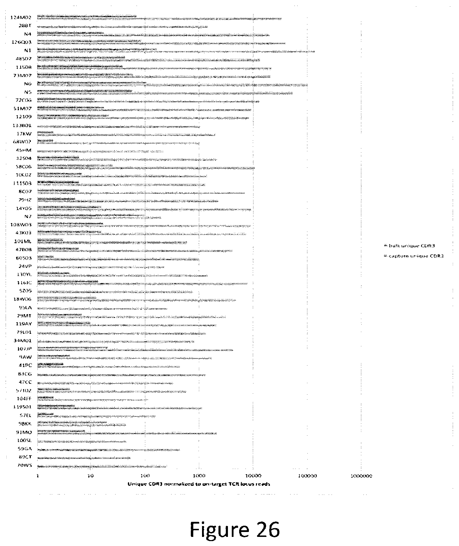

**Figure 26****:** TCR signal from an unselected cDNA library (red) and the same library following capture CapTCR-Seq (blue). Samples are indicated on the Y axis, while unique CDR3 counts is graphed on the X axis (log).

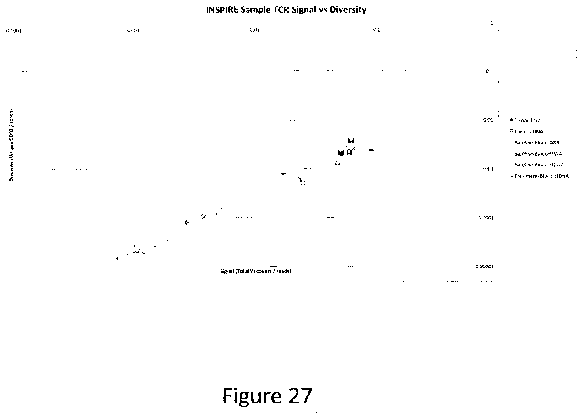

**Figure 27****:** TCR total signal (VJ counts) and repertoire diversity (unique CDR3 counts) for all samples from five patients.

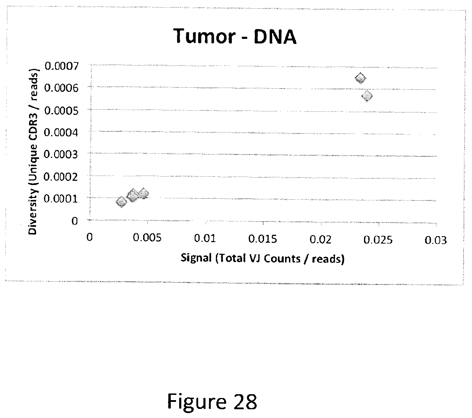

**Figure 28****:** TCR total signal (VJ counts) and repertoire diversity (unique CDR3 counts) for all tumor samples from five patients.

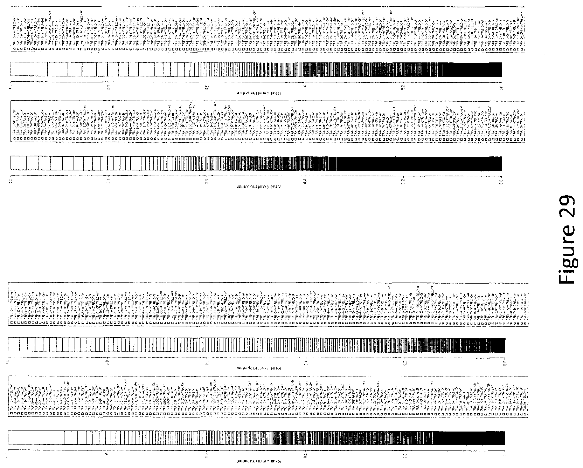

**Figure 29****:** Patient A: Stacked barplots of unique VJ rearrangements for alpha locus tumor (panel 1), beta locus tumor (panel 2), alpha locus baseline blood (panel 3), and beta locus baseline blood (panel 4). Each box represents a VJ rearrangement and box size corresponds to prevalence within sample (Y axis).

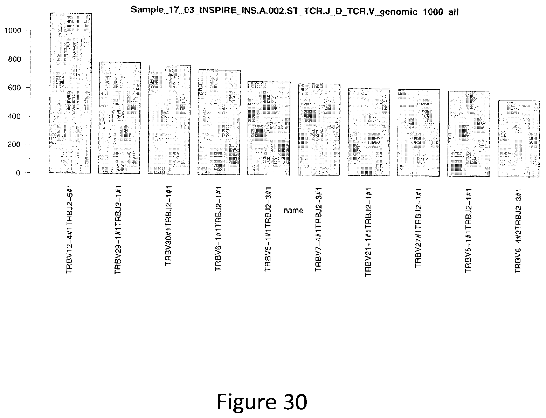

**Figure 30****:** Top ten most prevalent beta locus rearrangements from patient A tumor.

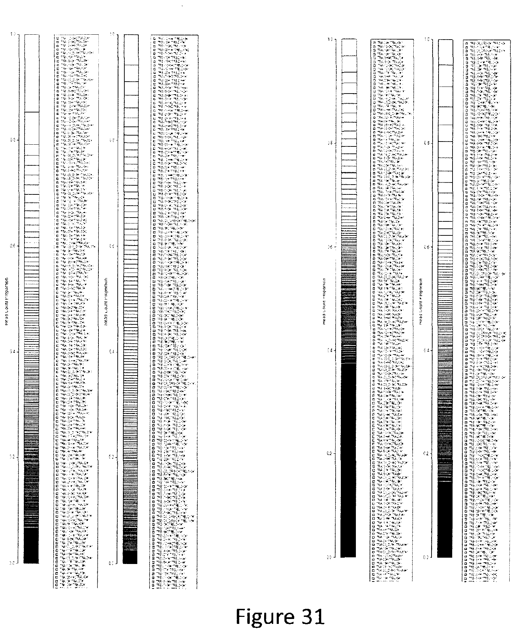

**Figure 31****:** Patient B: Stacked barplots of unique VJ rearrangements for alpha locus tumor (panel 1), beta locus tumor (panel 2), alpha locus baseline blood (panel 3), and beta locus baseline blood (panel 4). Each box represents a VJ rearrangement and box size corresponds to prevalence within sample (Y axis).

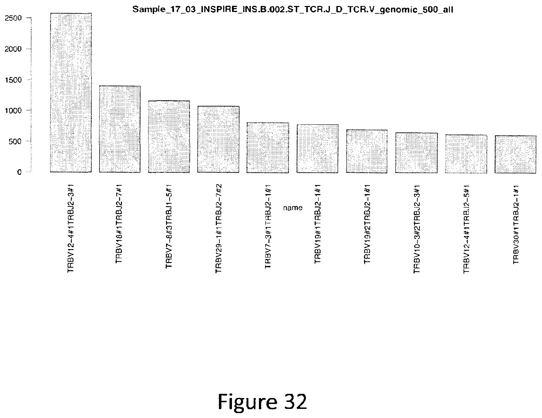

**Figure 32****:** Top ten most prevalent beta locus rearrangements from patient B tumor.

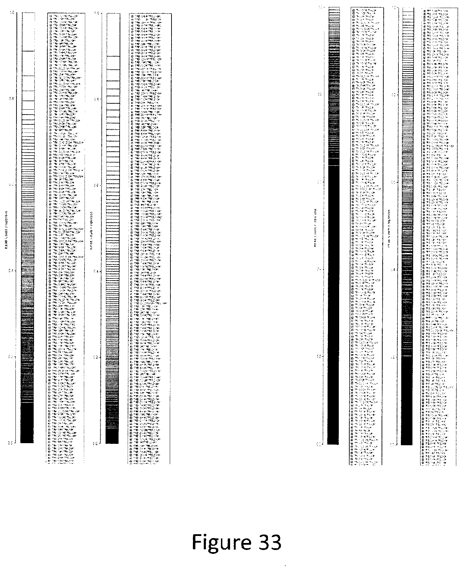

**Figure 33****:** Patient C: Stacked barplots of unique VJ rearrangements for alpha locus tumor (panel 1), beta locus tumor (panel 2), alpha locus baseline blood (panel 3), and beta locus baseline blood (panel 4). Each box represents a VJ rearrangement and box size corresponds to prevalence within sample (Y axis).

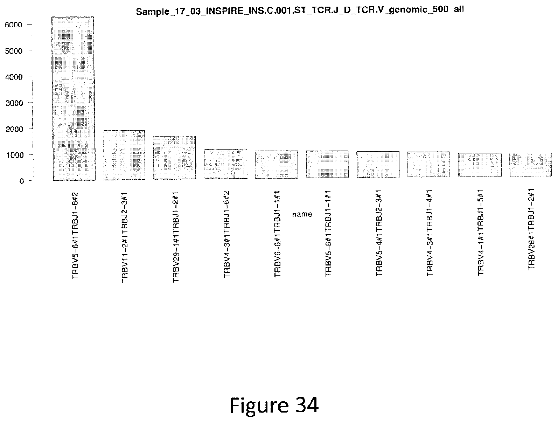

**Figure 34****:** Top ten most prevalent beta locus rearrangements from patient C tumor.

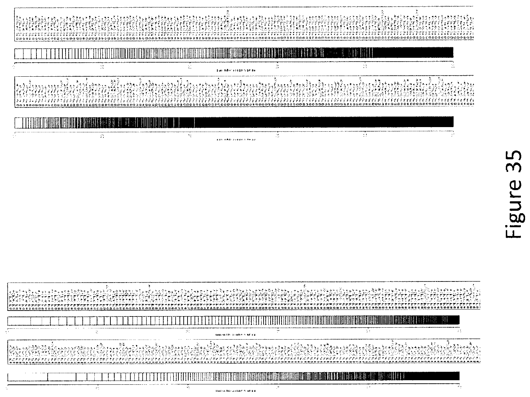

**Figure 35****:** Patient D: Stacked barplots of unique VJ rearrangements for alpha locus tumor (panel 1), beta locus tumor (panel 2), alpha locus baseline blood (panel 3), and beta locus baseline blood (panel 4). Each box represents a VJ rearrangement and box size corresponds to prevalence within sample (Y axis).

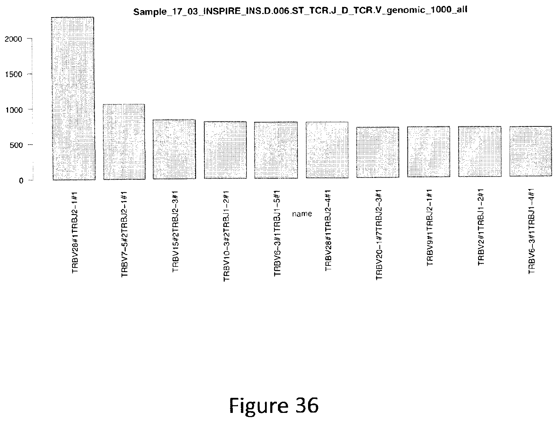

**Figure 36****:** Top ten most prevalent beta locus rearrangements from patient D tumor.

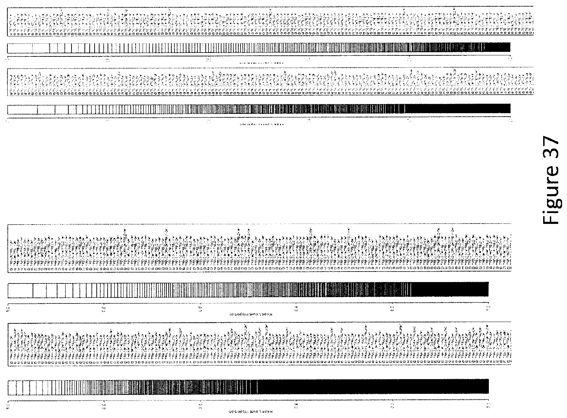

**Figure 37****:** Patient E: Stacked barplots of unique VJ rearrangements for alpha locus tumor (panel 1), beta locus tumor (panel 2), alpha locus baseline blood (panel 3), and beta locus baseline blood (panel 4). Each box represents a VJ rearrangement and box size corresponds to prevalence within sample (Y axis).

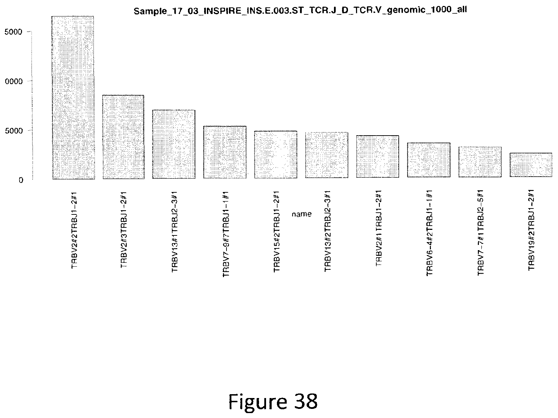

**Figure 38****:** Top ten most prevalent beta locus rearrangements from patient E tumor.

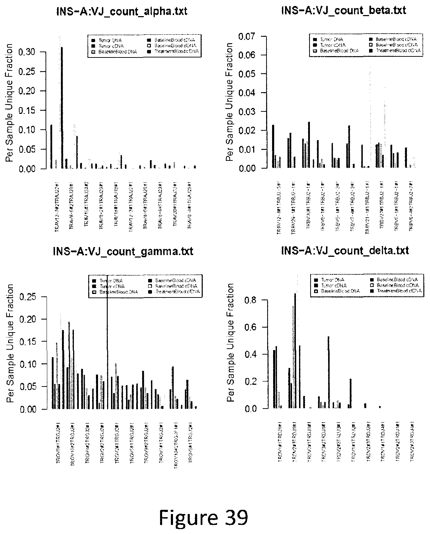

**Figure 39****:** Sample fractions within all patient A samples for top ten most prevalent VJ rearrangements in tumor. Alpha locus (panel 1), beta locus (panel 2), gamma locus (panel 3), delta locus (panel 4).

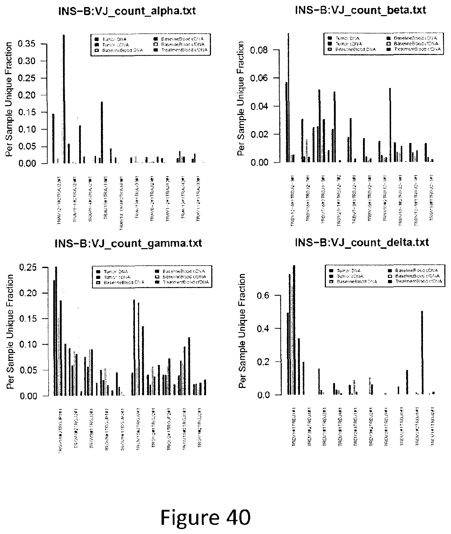

**Figure 40****:** Sample fractions within all patient B samples for top ten most prevalent VJ rearrangements in tumor. Alpha locus (panel 1), beta locus (panel 2), gamma locus (panel 3), delta locus (panel 4).

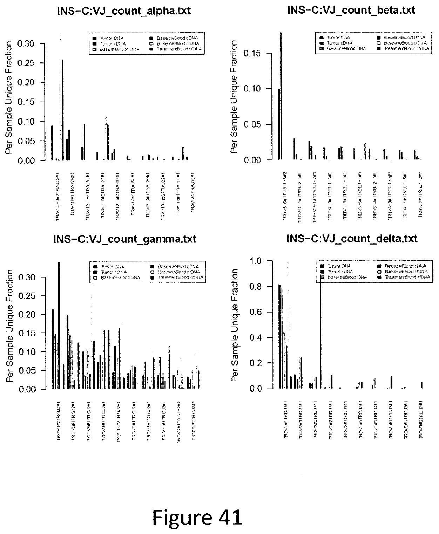

**Figure 41****:** Sample fractions within all patient C samples for top ten most prevalent VJ rearrangements in tumor. Alpha locus (panel 1), beta locus (panel 2), gamma locus (panel 3), delta locus (panel 4).

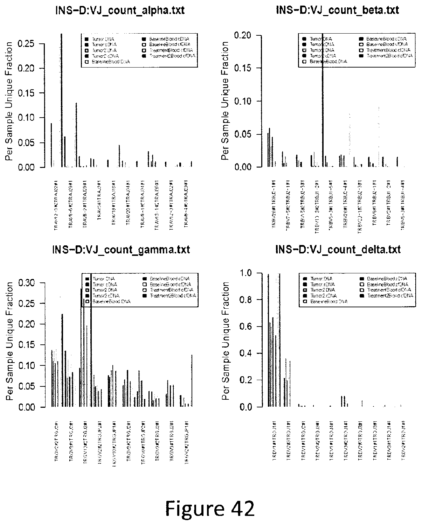

**Figure 42****:** Sample fractions within all patient D samples for top ten most prevalent VJ rearrangements in tumor. Alpha locus (panel 1), beta locus (panel 2), gamma locus (panel 3), delta locus (panel 4).

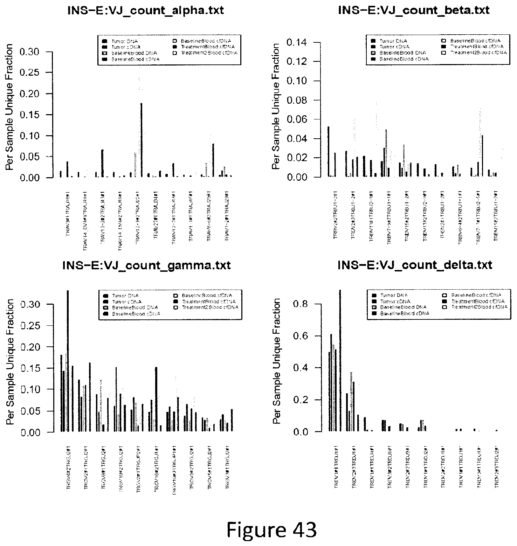

**Figure 43****:** Sample fractions within all patient E samples for top ten most prevalent VJ rearrangements in tumor. Alpha locus (panel 1), beta locus (panel 2), gamma locus (panel 3), delta locus (panel 4).

### DETAILED DESCRIPTION

In the following description, numerous specific details are set forth to provide a thorough understanding of the invention. However, it is understood that the invention may be practiced without these specific details.

The advantages of high-throughput DNA sequencing technologies could potentially be applied to T-cell clonality testing. The nature of T-cell gene diversity, requiring the consideration of potential variability arising from four distinct gene loci, makes obvious the benefit of multiplexing; what has traditionally required multiple separate tests could be combined in a single reaction. The capacity of modern DNA sequencing technologies to query longer contiguous segments of DNA in greater quantities relative to traditional techniques also provides an opportunity to explore the potential meaning of TRA and TRB sequence rearrangements. Sequence-level data might afford a greater ease of assay result interpretation. Indeed, the generation of sequence-level data in a TRGR assay would likely be much more informative than gross estimates of DNA electrophoretic migration patterns when disease trends are being studied; the high-level analysis of such data might help the identification of heretofore hidden patterns of TR rearrangement in specific T-cell lymphoma subtypes. The issue of replicate numbers for establishing test sensitivity/specificity can be easily overcome by exploiting the high-throughput capacity of modern DNA sequencing platforms; for a comparable investment of time (and possibly cost), sequencing-based approach to TRGR could perform a greater number of individual tests, thereby potentially allowing a more statistically robust estimate of test performance.

Traditional sequencing uses PCR-based techniques to markedly amplify input template DNA, thus improving the sensitivity of detection during the sequencing step. Indeed, many sequencing-based technologies still perform directed library preparation using PCR-based techniques to isolate and sequence regions of interest ⁽³⁸⁾. By this approach, one might employ specific primer sets to enrich for regions of interest in the library preparation step. In the context of TRGR, however, a primer-based approach to library preparation would be challenging: in order to provide the sufficient breath of coverage required to interrogate the status of the vast number of TR genes (especially in the TRA locus), a massive array of primers would be required. Although it is theoretically possible to prime multiple regions in tandem, previous data suggest that such an approach might open the door to the possibility of technical error (for a more thorough review of the details of these errors and the studies that have supported this evidence, see ⁽³⁸⁾). In the context of TRGR, furthermore, a primer-based approach to library preparation introduces the possibility of allele dropout when the assay attempts to prime a rearranged gene based on the known germline configuration (an easily digestible review to this effect may be found here ⁽³⁹⁾).

A paradigm shift away from PCR primer-directed amplification of genomic areas of interest was required for sequencing experiments aimed at large numbers of genes. Indeed most sequencing-based technologies rather employ the upfront production of vast libraries of template oligonucleotides followed by a series of template enrichment steps ⁽³⁸⁾. These latter steps may simply involve the extraction of DNA of specific lengths or quality, or rather the focus may be to enrich DNA containing specific *sequences* of interest. In the latter scenario, when specific sequence motifs are enriched for during library preparation, the resulting sequencing data will be enriched for the sequences of interest. Additionally, using the above stepwise approach, library preparation may be generalized to permit the enrichment of specific sequences out of a mix of "all" sequences produced from the primary non-specific amplification step; it is easy to see how this approach may be used to permit multiple separate assays using different enrichment approaches applied to a single input library ⁽⁴⁰⁾.

Hybrid capture is a form of library enrichment in which a library is probed for known sequences of interest using tagged nucleic acid probes followed by a subsequent "pull-down" of the tagged hybrids ⁽³⁸⁾; for example, DNA probes tagged with biotin can be efficiently enriched when hybridization is followed by a streptavidin enrichment step ^{(38,40-43)}. The biotin/streptavidin enrichment procedure is schematized in Figure 2-1A. In reference to the assessment of TRGR, this approach has the advantage of enriching TR genes based on the available well-defined germline TR gene sequences, which can be performed in a massively parallel fashion using several hundred probes. Notably, this approach also allows for enrichment of rearranged sequences as the hybrid-capture probes can also hybridize to (and therefore enrich for) subsequences of the rearrangement product. This latter "pull-down" of rearranged TR genes would be difficult using a primer-only approach to library preparation.

Rather than restricting the assessment of test performance of the above DNA sequencing approaches to a pre-set (and potentially biased) sample of "malignant" and "benign" T-cell lymphoproliferative disorders, a more prudent sampling rubric might use a "real-world" series of consecutive samples taken from a population as similar to the "test population" as possible. In the context of TRGR validation, such a sample might consist of a series of consecutive tissue samples from patients being worked-up by a hematologist and submitted for molecular (i.e. T-cell clonality) assessment. The overall sample size could be established based on an estimate of the historical incidence of T-cell lymphomas in such a population, such that the total size of the sample is adequately large to include a sufficient "expected" number of clonal T-cell lymphoproliferative disorders.

In many validation studies, the final pathology diagnosis is used as the gold standard against which the novel test is measured ⁽⁴⁴⁾. While not unreasonable, there are arguments against employing such an approach. Of foremost concern is the potential for diagnostic or interpretative error, by which "true positivity" of disease could be misappropriated ⁽⁴⁴⁾. In the realm of T-cell lymphomas, given at least partly due to their rarity, the frequent lack of pathologist experience might make this problem more likely. Furthermore, evidence indicates that even when diagnoses are based on consensus or panel based interpretation, the possibility of diagnostic bias by dominant opinion should be considered ⁽⁴⁵⁾.

When a single clearly-defined outcome measure does not exist (or is limited by bias), a composite gold-standard might be more appropriate ⁽⁴⁶⁾. Composite gold-standards might include a number of individual test results or clinical observations logically combined to produce "positive" or "negative" composites ⁽⁴⁶⁾; of key import is that (1) well-defined rules of composition be set out a *priori* and (2) the number of samples or subjects with each of the composite test results should be well-described ⁽⁴⁶⁾. Ideally, all samples or subjects should be evaluated using each of the composite tests ⁽⁴⁶⁾.

In order to best study a novel test of TLPDs, rather than limiting the reference test to the gold-standard BIOMED-2 T-cell clonality assay or to pathology diagnoses, a series of both individual and composite references might be considered. From the perspective of analytical validity, one might consider validating an sequencing-based TRGR assay using standard PCR techniques followed by Sanger sequence verification. Since the sequences of each of the TR V and J genes are known, forward and reverse primer sets for each V and J genes, respectively, identified by the capture and sequencing assay could be used to verify that the detected result is valid; this could be followed by Sanger sequencing to validate the result of the DNA sequencing result (with deference specifically to the CDR3 variability-defining region).

In another experiment, one might consider comparing a sequencing-based TRGR result to the BIOMED-2 result (with each test applied to all specimens under study). The primary limitation of this approach would be that the BIOMED-2 assay, as explained above, does not test for any TRA rearrangements; thus this comparison alone would be insufficient. Additional comparisons might involve assessment of the sensitivity and specificity of each of the BIOMED-2 and sequencing-based TRGR assays at identifying benign or malignant TLPDs. For this, a composite gold-standard including histologic features (i.e. pathology diagnosis), immunophenotypic features, additional molecular features (as available, e.g. cytogenetic changes), clinical observations (e.g. presence or absence of features of malignancy), and outcome results (e.g. significant deviation in individual patient survival from the median) might be considered. The clinical validity of the sequencing results could thus be assessed against the current diagnostic standard by means of a much more thorough evaluation.

T-cell lymphomas are cancers of immune cell development that result in clonal expansion of malignant clones that dominate the T-cell repertoire of affected patients. Therefore, clonality assessment of these cell populations is essential for the identification and monitoring of T-cell lymphomas. We have developed a hybrid-capture method that recovers rearranged sequences of T-cell receptor (TCR) chains from all four classes (alpha, beta, gamma, and delta loci) in a single reaction from an Illumina sequencing library. We use this method to describe the TCR V(D)J repertoire of monoclonal cancer cell lines, tumor-derived lymphocyte cultures, and peripheral blood mononuclear cells from a healthy donor, as well as a set of 63 clinical isolates sent for clinical clonality testing for suspected T-cell lymphoma. PCR amplification and Sanger sequencing confirmed cell line and tumor predominant rearrangements, individual beta locus V and J allele prevalence was well correlated with results from a commercial PCR-based DNA sequencing assay with an r² value of 0.94, and BIOMED2 PCR fragment size beta and gamma locus clonotyping of clinical isolates showed 73% and 77% agreement respectively. Our method allows for rapid, high-throughput and low cost characterization of TCR repertoires that will enhance sensitivity of tumor surveillance as well as facilitate serial analysis of patient samples with a quantitative read-out during clinical immunotherapy interventions.

In an aspect, there is provided, a method of capturing a population of T-Cell receptor and/or immunoglobulin sequences with variable regions within a patient sample, said method comprising: extracting/preparing DNA fragments from the patient sample; ligating a nucleic acid adapter to the DNA fragments, the nucleic acid adapter suitable for recognition by a pre-selected nucleic acid probe; capturing DNA fragments existing in the patient sample using a collection of nucleic acid hybrid capture probes, wherein each capture probe is designed to hybridize to a known V gene segment and/or a J gene segment within the T cell receptor and/or immunoglobulin genomic loci, wherein the hybrid capture probes are designed based on all unique V genes and J genes and these probe sets specifically target the 3' regions of V gene coding regions and the 5' regions of J gene coding regions.

As used herein, "T-Cell Receptor" or "TCR" means a molecule found on the surface of T lymphocytes (or T cells), preferably human, that is responsible for recognizing fragments of antigen as peptides bound to major histocompatibility complex (MHC) molecules. The TCR is a disulfide-linked membrane-anchored heterodimeric protein normally consisting of the highly variable alpha (α) and beta (β) chains expressed as part of a complex with the invariant CD3 chain molecules. T cells expressing this receptor are referred to as α:β (or αβ) T cells, though a minority of T cells express an alternate receptor, formed by variable gamma (γ) and delta (δ) chains, referred as γδ T cells. Each chain is composed of two extracellular domains: Variable (V) region and a Constant (C) region. The variable domain of both the TCR α-chain and β-chain each have three hypervariable or complementarity determining regions (CDRs). CDR3 is the main CDR responsible for recognizing processed antigen.

The terms "antibody" and "immunoglobulin", as used herein, refer broadly to any immunological binding agent or molecule that comprises a human antigen binding domain, including polyclonal and monoclonal antibodies. Depending on the type of constant domain in the heavy chains, whole antibodies are assigned to one of five major classes: IgA, IgD, IgE, IgG, and IgM. Several of these are further divided into subclasses or isotypes, such as IgG1, IgG2, IgG3, IgG4, and the like. The heavy-chain constant domains that correspond to the difference classes of immunoglobulins are termed α, δ, ε, γ and µ, respectively. The subunit structures and three-dimensional configurations of different classes of immunoglobulins are well known. The "light chains" of mammalian antibodies are assigned to one of two clearly distinct types: kappa (κ) and lambda (λ), based on the amino acid sequences of their constant domains and some amino acids in the framework regions of their variable domains. The variable domains comprise the complementarity determining regions (CDRs). The methods described herein may be applied to immunoglobulin sequences, including B-cell immunoglobulin sequences.

"V gene segments", "J gene segments" and "D gene segments" as used herein, refer to the variable (V), joining (J), and diversity (D) gene segments involved in V(D)J recombination, less commonly known as somatic recombination. V(D)J recombination is the mechanism of genetic recombination that occurs in developing lymphocytes during the early stages of T and B cell maturation. The process results in the highly diverse immune repertoire of antibodies/immunoglobulins (Igs) and T cell receptors (TCRs) found on B cells and T cells, respectively.

The term "nucleic acid" includes DNA and RNA and can be either double stranded or single stranded.

The term "probe" as used herein refers to a nucleic acid sequence that will hybridize to a nucleic acid target sequence. In one example, the probe hybridizes to the RNA biomarker or a nucleic acid sequence complementary thereof. The length of probe depends on the hybridization conditions and the sequences of the probe and nucleic acid target sequence. In one embodiment, the probe is at least 8, 10, 15, 20, 25, 50, 75, 100, 150, 200, 250, 400, 500 or more nucleotides in length.

The term "adapter" as used herein refers a moiety capable of conjugation to a nucleic acid sequence for a particular purpose. For example, the adapter may be used to identify or barcode the nucleic acid. Alternatively, the adapter may be a primer which can be used to amplify the nucleic acid sequence.

The term "hybridize" or "hybridizable" refers to the sequence specific non-covalent binding interaction with a complementary nucleic acid. In a preferred embodiment, the hybridization is under stringent conditions. Appropriate stringency conditions which promote hybridization are known to those skilled in the art, or can be found in Current Protocols in Molecular Biology, John Wiley & Sons, N.Y. (1989), 6.3.1 6.3.6. For example, 6.0 x sodium chloride/sodium citrate (SSC) at about 45°C, followed by a wash of 2.0 x SSC at 50°C may be employed.

In some embodiments, the method further comprises sequencing the captured DNA fragments, wherein the sequencing can be used to determine clonotypes within the patient sample. Various sequencing techniques are known to the person skilled in the art, such as polymerase chain reaction (PCR) followed by Sanger sequencing. Also available are next-generation sequencing (NGS) techniques, also known as high-throughput sequencing, which includes various sequencing technologies including: Illumina (Solexa) sequencing, Roche 454 sequencing, Ion torrent: Proton / PGM sequencing, SOLiD sequencing. NGS allow for the sequencing of DNA and RNA much more quickly and cheaply than the previously used Sanger sequencing. In some embodiments, said sequencing is optimized for short read sequencing.

In some embodiments, the method further comprises amplifying the population of sequences using nucleic acid amplification probes/oligonucleotides that recognize the adapter prior to said sequencing.

In some embodiments, the method further comprises fragmenting DNA extracted from the patient sample to generate the DNA fragments.

In some embodiments, the ligating step is performed before the capturing step.

In some embodiments, the capturing step is performed before the ligating step.

The term "patient" as used herein refers to any member of the animal kingdom, preferably a human being and most preferably a human being that has AML or that is suspected of having AML.

The term "sample" as used herein refers to any fluid, cell or tissue sample from a subject which can be assayed for nucleic acid sequences. In some embodiments, the patient sample comprises tissue, urine, cerebral spinal fluid, saliva, feces, ascities, pleural effusion, blood or blood plasma.

In some embodiments, the patient sample comprises cell-free nucleic acids in blood plasma.

In some embodiments, the clonality analyses described herein may be use to track clonality across samples types.

In some embodiments, the hybrid capture probes are at least 30bp in length. In a further embodiment, the hybrid capture probes are between 60bp and 150bp in length. In a further embodiment, the hybrid capture probes are between 80bp and 120bp in length. In a further embodiment, the hybrid capture probes are about 100bp in length.

In some embodiments, the hybrid capture probes hybridize to at least 30bp, preferably 50bp, more preferably 100bp of the V gene segment and/or J gene segment.

In some embodiments, the screening probes hybridize to at least a portion of the V gene segment.

In some embodiments, the screening probes hybridize to at least a portion of the V gene segment at the 3' end.

In some embodiments, hybridizing comprises hybridizing under stringent conditions, preferably very stringent conditions.

In some embodiments, the collection of nucleic acid hybrid capture probes comprise at least 2, 5, 10, 20, 30, 80, 100, 300, 400, 500, 600, 700, 800 or 900 unique hybrid capture probes.

In some embodiments, the collection of nucleic acid hybrid capture probes is sufficient to capture at least 50%, 60%, 70%, 80%, 90% or 99% of known T-Cell receptor and/or immunoglobulin loci clonotypes.

In some embodiments, the hybrid capture probes are immobilized on an array.

In some embodiments, the hybrid capture probes comprise a label. In a further embodiment, the label is used to distinguish between sequences bound to the screening probes and unbound double stranded fragments, and preferably the capture is performed in solution.

In some embodiments, preparing the DNA fragments comprises extracting RNA from the patient sample and preparing corresponding cDNA.

In some embodiments, the method further comprises a depletion step, comprising depleting the DNA fragments of non-rearranged sequences using probes that recognize nucleic acid sequences adjacent to V and/or J gene segments in the genome. In some embodiments, the capturing of DNA fragments using V gene segment and J gene segment hybrid capture probes is performed in separate steps, and in any order with the depletion step, preferably in the following order: J gene capture , depletion , then V gene capture.

A method of immunologically classifying a population of T-Cell receptor and/or immunoglobulin sequences may comprise:
(a) identifying all sequences containing a V gene segment from the sequences of the DNA fragments by aligning the sequences of the DNA fragments to a library of known V gene segment sequences;
(b) trimming the identified sequences in (a) to remove any sequences corresponding to V gene segments to produce a collection of V-trimmed nucleotide sequences;
(c) identifying all sequences containing a J gene segment in the population of V-trimmed nucleotide sequences by aligning the V-trimmed nucleotide sequences to a library of known J gene segment sequences;
(d) trimming the V-trimmed nucleotide sequences identified in (c) to remove any sequences corresponding to J gene segments to produce VJ-trimmed nucleotide sequences;
(e) identifying any D gene segment comprised in the VJ-trimmed nucleotide sequences identified in (d) by aligning the VJ-trimmed nucleotide sequences to a library of known D gene segment sequences;
(f) for each VJ-trimmed nucleotides sequence identified in (d), assembling a nucleotide sequence comprising the V gene segment, any D gene segment, and the J gene segment identified in steps (a), (e) and (c) respectively ;
(g) selecting from the nucleotide sequence assembled in step (f) a junction nucleotide sequence comprising at least the junction between the V gene segment and the J gene segment, including any D gene segment, the junction nucleotide sequence comprising between 18bp and 140bp, preferably 40-100bp, further preferably about 80bp;
and optionally (h) and (i):
(h) translating each reading frame of the junction nucleotide sequence and its complementary strand to produce 6 translated sequences; and
(i) comparing the 6 translated sequences to a library of known CDR3 regions of T-Cell receptor and/or immunoglobulin sequences to identify the CDR3 region in the DNA fragments.

Alternatively, step (h) may be searching the 6 translated sequences for flanking invariable anchor sequences to define the intervening T-Cell receptor and/or B-cell receptor CDR3 sequences encoded by the DNA fragments.

This method may further comprise, prior to step (a), aligning left and right reads of overlapping initial DNA fragments to produce the DNA fragments on which step (a) is performed.

Steps (a), (c), (e) may be performed with BLASTn and step (i) may be performed using expression pattern matching to known sequences and IMGT annotated data.

A method of identifying CDR3 regions in T-Cell receptor and/or immunoglobulin sequences may comprise:
(a) identifying a V gene segment comprised in the immunoglobulin sequence by aligning the immunoglobulin sequence to a library of known V gene segment sequences;
(b) identifying a J gene segment comprised in the immunoglobulin sequence by aligning the immunoglobulin sequence to a library of known J gene segment sequences;
(c) if V and J gene segments are identified, then comparing the immunoglobulin sequence to a library of known CDR3 regions of T-Cell receptor and/or immunoglobulin sequences to identify any CDR3 region in the immunoglobulin sequence.

Alternatively, step (c) may be if V and J gene segments are identified, then searching the immunoglobulin sequence for flanking invariable anchor sequences to define the intervening T-Cell receptor and/or immunoglobulin CDR3 sequences.

Steps (a) and (b) may be performed using the Burrows-Wheeler Alignment or other sequence alignment algorithm.

If a CDR3 region is identified in step (c), then the method may further comprise determining whether the identified V and J gene segments could be rearranged in the same locus using a heuristic approach.

If a CDR3 region is not identified in step (c), then the method may further comprise determining if a combination of V(D)J gene segments is present based on Smith Waterman Alignment scores.

In an aspect, there is provided, a method for characterizing the immune repertoire of a subject, the immune repertoire comprising the subject's T-Cell population, the method comprising any of the hybrid capture methods described herein, followed by a method comprising:
(j) identifying all sequences containing a V gene segment from the sequences of the DNA fragments by aligning the sequences of the DNA fragments to a library of known V gene segment sequences;
(k) trimming the identified sequences in (j) to remove any sequences corresponding to V gene segments to produce a collection of V-trimmed 5 nucleotide sequences;
(I) identifying all sequences containing a J gene segment in the population of V-trimmed nucleotide sequences by aligning the V-trimmed nucleotide sequences to a library of known J gene segment sequences;
(m) trimming the V-trimmed nucleotide sequences identified in (I) to remove any 10 sequences corresponding to J gene segments to produce VJ-trimmed nucleotide sequences;
(n) identifying any D gene segment comprised in the VJ-trimmed nucleotide sequences identified in (m) by aligning the VJ-trimmed nucleotide sequences to a library of known D gene segment sequences; 15
(o) for each VJ-trimmed nucleotides sequence identified in (m), assembling a nucleotide sequence comprising the V gene segment, any D gene segment, and the J gene segment identified in steps (j), (n) and (I) respectively ;
(p) selecting from the nucleotide sequence assembled in step (o) a junction nucleotide sequence comprising at least the junction between the V gene 20 segment and the J gene segment, including any D gene segment, the junction nucleotide sequence comprising between 18bp and 140bp;
and optionally (q) and (r):
(q) translating each reading frame of the junction nucleotide sequence and its complementary strand to produce 6 translated sequences; and 25
(r) comparing the 6 translated sequences to a library of known CDR3 regions of T-Cell receptor and/or immunoglobulin sequences to identify the CDR3 region in the DNA fragments.

Any of the methods described herein may be used to capture a population of T-Cell receptor sequences, for immunologically classifying a population of T-Cell receptor sequences or for identifying CDR3 regions in T-Cell receptor.

In an aspect, the methods described herein are for characterizing T-cell clonality for a disease in the subject.

In some embodiments, the T-Cell receptor sequences are from tumour infiltrating lymphocytes.

In an aspect, the methods described herein are for identifying therapeutic tumour infiltrating lymphocytes for the purposes of expansion and reinfusion into a patient and/or adoptive cell transfer immunotherapy.

In an aspect, the methods described herein are for monitoring T-cell populations/turnover in a subject, preferably a subject with cancer during cancer therapy, preferably immunotherapy.

In an aspect, the methods described herein are for characterizing the immune repertoire of a subject, the immune repertoire comprising the subject's B-Cell population.

In an aspect, the methods described herein are for capturing a population of B-Cell receptor sequences with variable regions within a patient sample. The methods described herein may also be used for immunologically classifying a population of B-Cell receptor sequences, or for identifying CDR3 regions in B-Cell receptor sequences.

In an aspect, the methods described herein are for characterizing B-cell clonality as a feature of a disease in the subject.

The present methods may be used in subjects who have cancer. Cancers include adrenal cancer, anal cancer, bile duct cancer, bladder cancer, bone cancer, brain/cns tumors, breast cancer, castleman disease, cervical cancer, colon/rectum cancer, endometrial cancer, esophagus cancer, ewing family of tumors, eye cancer, gallbladder cancer, gastrointestinal carcinoid tumors, gastrointestinal stromal tumor (gist), gestational trophoblastic disease, hodgkin disease, kaposi sarcoma, kidney cancer, laryngeal and hypopharyngeal cancer, leukemia (acute lymphocytic, acute myeloid, chronic lymphocytic, chronic myeloid, chronic myelomonocytic), liver cancer, lung cancer (non-small cell, small cell, lung carcinoid tumor), lymphoma, lymphoma of the skin, malignant mesothelioma, multiple myeloma, myelodysplastic syndrome, nasal cavity and paranasal sinus cancer, nasopharyngeal cancer, neuroblastoma, non-hodgkin lymphoma, oral cavity and oropharyngeal cancer, osteosarcoma, ovarian cancer, pancreatic cancer, penile cancer, pituitary tumors, prostate cancer, retinoblastoma, rhabdomyosarcoma, salivary gland cancer, sarcoma - adult soft tissue cancer, skin cancer (basal and squamous cell, melanoma, merkel cell), small intestine cancer, stomach cancer, testicular cancer, thymus cancer, thyroid cancer, uterine sarcoma, vaginal cancer, vulvar cancer, waldenstrom macroglobulinemia, and wilms tumor.

In embodiments relating to T-cells, the subject may have a T-cell related disease, such as a T-cell lymphoma.

T-cell lymphomas are types of lymphoma affecting T cells, and can include peripheral T-cell lymphoma not otherwise specified, extranodal T cell lymphoma, cutaneous T cell lymphoma, including Sézary syndrome and Mycosis fungoides, anaplastic large cell lymphoma, angioimmunoblastic T cell lymphoma, adult T-cell Leukemia/Lymphoma (ATLL), blastic NK-cell Lymphoma, enteropathy-type T-cell lymphoma, hematosplenic gamma-delta T-cell Lymphoma, lymphoblastic Lymphoma, nasal NK/T-cell Lymphomas, treatment-related T-cell lymphomas.

In other embodiments relating to B-cells, the subject may have a B-cell related disease, plasma cell disorder, preferably a B-cell lymphoma.

B-cell are types of lymphoma affecting B cells and can include, diffuse large B-cell lymphoma (DLBCL), follicular lymphoma, marginal zone B-cell lymphoma (MZL) or mucosa-associated lymphatic tissue lymphoma (MALT), small lymphocytic lymphoma (also known as chronic lymphocytic leukemia, CLL), mantle cell lymphoma (MCL), DLBCL variants or sub-types of primary mediastinal (thymic) large B cell lymphoma, T cell/histiocyte-rich large B-cell lymphoma, primary cutaneous diffuse large B-cell lymphoma, leg type (Primary cutaneous DLBCL, leg type), EBV positive diffuse large B-cell lymphoma of the elderly, diffuse large B-cell lymphoma associated with inflammation, Burkitt's lymphoma, lymphoplasmacytic lymphoma, which may manifest as Waldenström's macroglobulinemia, nodal marginal zone B cell lymphoma (NMZL), splenic marginal zone lymphoma (SMZL), intravascular large B-cell lymphoma, primary effusion lymphoma, lymphomatoid granulomatosis, primary central nervous system lymphoma, ALK-positive large B-cell lymphoma, plasmablastic lymphoma, large B-cell lymphoma arising in HHV8-associated multicentric Castleman's disease, B-cell lymphoma, unclassifiable with features intermediate between diffuse large B-cell lymphoma and Burkitt lymphoma, B-cell lymphoma, unclassifiable with features intermediate between diffuse large B-cell lymphoma and classical Hodgkin lymphoma, AIDS-related lymphoma, classic Hodgkin's lymphoma and nodular lymphocyte predominant Hodgkin's lymphoma.

In an aspect, the methods described herein are for identifying therapeutic B-cells for the purposes of expansion and reinfusion into a patient.

In an aspect, the methods described herein are for monitoring B-cell populations/turnover in a subject, preferably a subject with cancer during cancer therapy, preferably immunotherapy.

In an aspect, the methods described herein are for detecting minimal residual disease, whereby TCR or immunoglobulin rearrangements may be used as a marker of disease.

In an aspect, there is provided a library of probes comprising the depletion probes in Table D or at least one of the V-gene and J-gene probes set forth in any of Tables 2.1, 4, B1, or B2.

In some embodiments, the clonality analyses described herein may be performed serially.

In some embodiments, the clonality analyses described herein may be used to distinguish between samples.

The advantages of the present invention and the additional methods described herein are further illustrated by the following examples. The examples and their particular details set forth herein are presented for illustration only and should not be construed as a limitation on the claims of the present invention.

### EXAMPLE 1

### Methods and Materials

### Assay development

Several important theoretical considerations were entertained during the design phase of our novel sequecing-based TRGR assay (heretofore referred to as the NTRA).

Unlike the current BIOMED approach, we wished to avoid a gene-specific primer-based approach to signal amplification. To accomplish this, we chose a "hybrid capture" target enrichment approach by which input genomic DNA containing the TR genes might be enriched (or "captured") relative to other segments of the genome. Several methodological approaches to target enrichment already exist, with multiple commercially available and rigorously optimized kits capable of enriching nearly any well-defined gene target(s) ^{(47,48)}.

The NTRA needed to be robust enough to accommodate sample types of variable DNA quality; this requirement reflects the clinical need to apply TRGR assays to a wide variety of specimens in a wide variety of contexts. Knowing that Formalin-fixed paraffin-embedded (FFPE) specimens typically contain degraded and often poor quality DNA (as such representing the "lowest common denominator" of specimen quality) ⁽⁴⁹⁾, it was deemed necessary to specifically evaluate NTRA performance on FFPE specimens. Furthermore, the use of hybrid capture is also amenable to highly fragmented DNA specimens such as those from circulating cell-free DNA.

Likewise, the most useful NTRA should allow users to both accurately assess the "clonality" of an input sample (as can be done using BIOMED-2 based assays) but also fully characterize the clonotypes of constituent TRGR configurations. Thus it was essential that the NTRA not simply produce a binary "clonal" vs. "polyclonal" result but also provide a much more robust and quantitative data output, including the genes and CDR3 regions present within identified TRGR configurations.

We recognized that much of the utility of the NTRA would depend on the design of a robust bioinformatic analysis pipeline. Of note, at the time at which this project was undertaken, only a single widely-used pipeline existed (the International standard source for ImMunoGeneTics sequences & metadata (IMGT) V-QUEST system), mainly designed around 5'RACE PCR followed by Roche 454 sequencing ⁽⁵¹⁾. As outlined below, several methodological and logistic motivations demanded a novel pipeline of our own design.

Current sequencing-based applications generally require that resultant sequence data (i.e. reads) be mapped to a reference (typically the genome of the organism of interest) using some form of alignment algorithm. Once this alignment is complete, secondary and tertiary tools are used to search for and catalogue sequence deviation from the reference. For our purposes, however, using the entire human genome as a reference map would be unnecessarily cumbersome, especially since the presence of closely juxtaposed V(D)J sequence within a single short (i.e. <500 basepairs (bp)) fragment of DNA is tantamount to evidence of TRGR. Furthermore, aligning to a single reference genome raises the informatics challenge of detecting gene rearrangements from a single alignment step. As such, a strategy of mapping sequence reads to *only* the reference genes in a parallel fashion (i.e. one mapping procedure to the V genes, and one separate mapping procedure to the J genes) was selected, along with an integrated TRGR detection algorithm

This strategy required the theoretical consideration that short sequence read input might result in excessive false negatives (i.e. artificially low TRGR detection rates). This problem might be mitigated, in theory at least, by ensuring that input DNA fragment lengths (and the resulting sequencing read lengths) are carefully set to within a reasonable range of sensitivity for the detection of TRGR in a given sequence. Since all possible TRGRs are combinatorially vast, this process could only be simulated using, for our purposes, an artificial test set of simply-concatenated sequences of all catalogued V, D, and J genes (a test set numbering 197400). By evaluating k-mer subsequences over a range of lengths (k), centred (without loss of generality) about the median of each artificial junction, an estimate of the sensitivity of TRGR detection for variable sequencing windows can be produced. This sequencing window can then be used as an "evidence-based" DNA insert length.

### Insert Length Simulation

Appendix 2.0 outlines a MATLAB script designed to estimate the optimal DNA insert length (a value also generalizable to optimal shearing length and minimal Paired-end rEAd mergeR (PEAR)-assembled sequencing length) for the purposes of the NTRA. This optimum is subject to an important restriction: for our purposes, using the Illumina NextSeq platform, read lengths are limited to paired-ended reads of 150 bp each-this translates to <300 bp read lengths when paired-ends are joined by overlapping sequence (using, in our case, the PEAR algorithm ⁽⁵²⁾). Briefly, the code produces a simulation read set of all possible combinations of V-D-J sequences by way of simple concatenation (with the caveat that a much larger diversity of sequence is found in nature stemming from alterations of junctional sequence by way of splicing inconsistencies); next, the algorithm selects a k-mer (of length from k = 32 to 302, in intervals of 30 bp) from within each simulation sequence; the resulting k-mer (centred, without loss of generality, at the junction median) is then subject to Burrows-Wheeler Alignment algorithm (BWA) alignment against the known reference V and J genes (as in the TRSeq pipeline) to evaluate how well the k-mers of each of the artificial reads can be mapped to *both* V and J genes (representing bioinformatic identification of TRGR within the sequence in question). A histogram of percent detection vs. read length was then produced; analysis of those artificial V-D-J read combinations that could be reliably detected was also performed.

### DNA probe design

We began by reviewing the sequence and metadata of all reference TR genes obtained by way of a (FASTA-formatted) data download from the IMGT database. All sequences were subjected to a series of Clustal W ⁽⁵³⁾ alignment analyses to verify that sequence alignment was limited to known reference motifs (i.e. the J-gene F/W-G-X-G motif and V-gene conserved Cysteine ⁽⁵⁴⁾) and to allele-to-allele overlap.

DNA probe design was then performed using the IMGT reference sequences (including all annotated V and J gene functional, pseudogene and open reading frame sequences) using the xGen Lockdown probe technology. Briefly, this technology is a hybrid-capture-based technology by which biotin-tagged DNA probes (complementary to known sequences/genomic regions set at a 1x depth of coverage) are allowed to hybridize with sample DNA, followed by a streptavidin elution procedure performed to enrich the target sequences ⁽⁴⁰⁻⁴³⁾.

In line with previous studies employing xGen Lockdown probes ⁽⁴⁰⁻⁴³⁾, each DNA probe was designed to a length as close to 100 bp as possible. Using the IMGT database, germline-configuration sequences were extracted for all alleles of all J-genes, with additional leading and trailing IMGT nucleotides added (as necessary) to obtain 100 bp probe lengths; for those instances in which the IMGT data was insufficient to prepare 100 bp probes, additional random nucleotides were added to the leading and trailing ends of the available sequences. Again using the IMGT database, germline-configuration sequences were extracted for all alleles of all V-genes, with additional leading and trailing IMGT nucleotides added to ensure that the 5' and 3' ends of the germline-configuration genes were covered by a given probe (this design, it was theorized, would be able to account for gene re-arrangement at either end of a V-gene, regardless of strandedness, while still covering the vast majority of the sequence of each gene/allele). With careful placement of the probes as outlined above, we hoped that this design would also limit any specific stoichiometric bias among the V-genes represented in the target pool.

Table 2.1 outlines the complete list of xGen Lockdown probe design sequences (with relevant associated metadata).

### NTRA work-flow

The NTRA work-flow is summarized in Figures 2-1A & 2-1B. Briefly, the process begins with DNA isolation, performed for the purposes of this study according to the protocol of Appendix 2.1. isolated DNA was retrieved from frozen archives and quantified using the Qubit assay, per Appendix 2.2. input DNA was shorn using a Covaris sonicator (Appendix 2.3) set to a desired mean DNA length of 200 base pairs; adequate shearing was confirmed using TapeStation assessment. Sequence libraries for each specimen were prepared using the protocol outlined in Appendix 2.4; multiplexing was accommodated using either TruSeq or NEXTflex-96 indices (the latter employed in the final validation run to permit large-scale multiplexing). Library preparation results were validated relative to input short DNA using TapeStation assessment. Subsequently, hybrid-capture with the above described xGen Lockdown probes was performed; captures were performed in pools of 9-13 input libraries, based on a pre-calculated balance of input DNA. The captured library fragments were then repeat-amplified, followed by final Qubit and TapeStation QC-steps. Finally, paired-end 150-bp sequencing was performed on the Illumina NextSeq platform using either a mid- or high-output kit (depending on sample throughput), according to the manufacturer's instructions (Appendix 2.5). The resulting read-pair zipped FASTQ-formatted data files were de-compressed and merged using the publically available PEAR alignment algorithm using a minimum of 25 bp overlap; this allowed the 150-bp sequencing maximum to be expanded to at least 200 bp, as suggest by the results of Section 2.1.2. Non-paired results were also tallied as a means of quality assurance. Subsequent analyses were performed using the custom-designed TRSeq analysis pipeline, as described below.

### NTRA data analysis: the TRSeq pipeline

The NTRA TRSeq pipeline was designed around three main algorithmic steps. The first performs local alignment indexed to the TR V and J genes implemented using the Burrows-Wheeler-Alignment (BWA) algorithm ⁽⁵⁵⁾. From this algorithm, two important results are obtained: the first is a "reads-on-target" estimate (since the genes enriched for (i.e. the TR V and J genes) are those genes used as the index reference gene set); second, by way of the resulting Sequence Alignment Map (SAM) file output, the original input reads are filtered to exclude those unlikely to contain any of the TR V or J genes. This latter step reduces the informatic burden of input to the (relatively computationally slow) second algorithm step (using either heuristics or the Smith-Waterman Alignment (SWA)). Of note, the BWA algorithm could be implemented on a UNIX-based platform only ⁽⁵⁵⁾.

The second algorithm step is designed to extract CDR3 sequences wherever present. This algorithm was implemented in MATLAB, guided by previous publications ⁽⁵⁶⁾, and using a regular-expression (regexp) based search algorithm.

The third step combined the above alignment and CDR3 data (where present), to decide whether a given read contains a TRGR. To do this, one of two decision approaches is used: if a CDR3 is identified in a read, a heuristic approach is employed to decide if the BWA-alignment reference genes could be rearranged within the same locus; the second, in the event that a CDR3 is not detected, relies on the SWA-determined alignment scores to determine if a given combination of V(D)J genes is present.

### Bioinformatic Target Enrichment (Burrows-Wheeler-Alignment Algorithm)

Much like the technical aspects of the NTRA function to enrich TR genes at the DNA level, so too can an informatics target-enrichment approach be employed. Using the BWA algorithm ⁽⁵⁵⁾, a series of FASTQ-formatted reads are first mapped relative to a reference index of IMGT TR V and J genes. Any reads containing sequence mapping to any of the reference genes are flagged as such in the SAM-formatted output file as mapped, whereas those not containing any TR V or J gene mapped sequence are assigned the SAM Flag 4. In this context, unmapped reads are unlikely to contain any detectable TR V(D)J gene rearrangements; this predicate is logical inasmuch as sufficient residual germline sequence of a TR V and/or J gene are required in a read to permit TRGR detection.

Reads-on-target and gene-coverage estimates are also derived using the BWA algorithm, since NTRA input probes consist only of TR V and J genes; this measure is calculated as a percentage of the number of unique reads mapped to the IMGT reference TR V and J gene indices relative to the total number of reads in the input FASTQ-formatted file.

### CDR3 sequence extraction and SWA alignment

This part of the TRSeq algorithm was implemented in MATLAB using strategies similar to those employed by the IMGT ⁽⁵⁶⁻⁵⁸⁾. The IMGT/V-QUEST system utilizes a CDR3 sequence extraction algorithm ^{(57,59)} and an SWA ⁽⁶⁰⁾ algorithm performed against the IMGT reference sequences; the IMGT algorithms are all implemented in JAVA and processing is performed on IMGT servers.

As highlighted previously, we were unable to rely solely on the IMGT system for informatics results for several reasons: (1) the export of patient sequence data to an external non-secured network can be risky if insufficiently censored identifying metadata are also included; (2) the IMGT/High V-Quest system has a 500,000 sequence input limit (which may be substantially less than the number of sequence reads that need to be analyzed in the run of even a single high-throughput sequencing run); and (3) the queueing used by the IMGT can be lengthy, requiring a wait of possibly several days for sequence interpretation to begin.

A MATLAB implementation was chosen for convenience, programming familiarity, and because of easy vectorization, parallel computation and object-oriented programming capabilities. In addition, the MATLAB programming and command-line environments are able to easily incorporate UNIX and PERL-based scripts, including the BWA ^{(Li, 2009)} and CIRCOS software ⁽⁶¹⁾ suites, respectively.

The full coding of the analysis algorithm is presented in Appendix 2.6.2. The MATLAB code was written to accommodate FASTQ-formatted data, align each read using BWA to the reference TR V and J gene germline sequences, index the resultant data, test each indexed read for (and extract if present) a CDR3 sequence (using the uniformly present C-X(5...21)-F/W-G-X-G amino acid motif, per the IMGT canonical sequence motif ^{(62,63)}), and perform either an heuristic or SWA alignment-based validation of the reads mapped by BWA as evidence of a rearrangement within the read in question.

The SWA algorithm produces an optimal local alignment ^{(60,64)} of two co-input sequences (in this case, a query sequence relative to an IMGT reference sequence), and provides an alignment score (a unit-less measure of the degree to which the alignment perfectly matches an input sequence to its co-input sequence). For the purpose of this instance of the algorithm, for any case in which multiple possible alignments were produced, the alphabetical highest-scoring alignment was selected as the "correct" alignment, provided that this score was at least greater than the minimum cut-off score.

The minimum SWA alignment cut-off score was empirically determined for each of the three V, D, and J-gene gene groups using a large set of confirmed-negative sequences evaluated using the IMGT/HighV-QUEST system ^{(56,57)}. The MATLAB code required for implementation of this algorithm is outlined in Appendix 2.6.1. A "practice" set obtained from the IMGT database ^{(65,66)} was also employed to test the pipeline, consisting of IMGT PCR-confirmed TRGR sequences with known V-D-J combinations and CDR3 sequences (see Section 3.1.3 for results of this practice set analysis).

### Analytical Validation

A selection of 10 "First-Run" samples formed the basis of the analytical validation. These samples included 6 de-identified actual patient samples, obtained from flow-sorted peripheral blood specimens, tumour-infiltrating lymphocyte populations or in vitro cultures of lymphocytes. These samples were each subjected to flow-cytometric evaluation and cell-counting for basic immunophenotyping and cell-input consistency. In addition, four cell lines with known and well-described TR gene rearrangements (based on references cited by the IMGT database ⁽⁶⁷⁾) were also included (i.e. Jurkat (Deutsche Sammlung von Mikroorganismen und Zellkulturen (DSMZ) ACC-282), SUPT1 (American Type Culture Collection (ATCC) CRL-1942), CEM (ATCC CCL-119) and MOLT4 (ATCC CRL-1582)).

A three-part analytical validation approach was employed. First, the results obtainable by analysis of the sequencing data using the IMGT/High V-Quest pipeline were directly compared with the results of the TRSeq pipeline. Next, a PCR & Gel Electrophoresis experiment was designed to confirm the presence of the upper 90^{th} centile of rearrangement configurations. Finally, the predominant rearrangements with accompanying TRSeq-identified CDR3 sequences were further Sanger-sequenced to validate this latter component of the NTRA analysis.

### Comparison with IMGT Results

Given the limited input size capacity of the IMGT/High V-Quest system, a read-by-read comparison of a 10% random subset of the NTRA sequencing data was performed. From the IMGT analysis, a read was assumed to contain evidence of a rearrangement when the IMGT pipeline Junction analysis yielded an in-frame result. In addition, a read-by-read comparison of the alignment results (by gene name, for all V, D and J genes) was also performed.

### PCR & Gel Electrophoresis Validation

A PCR-based experiment was deemed a reasonable orthogonal validation approach, given the gold standard BIOMED-2 assay methodology. Knowing that the number of possible rearrangements detected by the NTRA might be substantially large, the PCR validation was arbitrarily limited to those TRSeq-detected rearrangements in the upper 90^{th} centile (i.e. percent rearrangement of greater than 10% of total rearrangements). Given this restriction, however, to ensure an adequate denominator of reactions for comparative purposes, all PCR validation experiments were uniformly performed across all 10 first-run samples.

PCR validation primer sets were constructed modeling the standard V-D-J orientation of rearranged TR genes; specifically, the PCR forward primer was set in the V gene and the reverse primer set in the anti-sense strand of the J gene. For each TRSeq-identified rearrangement above 10% of total rearrangements, the V and J genes were identified and the IMGT primer set database searched for gene (*not* allele) specific primers. While the IMGT primer database did contain a number of suggested primers, many of the TR genes did not have an available appertaining primer. As a result, where necessary, the anticipated rearrangement sequence (containing the V gene sequence artificially positioned before the J gene) was used to derive custom primers using the NCBI Primer-Blast tool ⁽⁶⁸⁾. Careful attention was paid to ensure that each resulting theoretical PCR product length was at least 100 bp (the lower limit of fragment size reliably detectable by standard gel electrophoresis) and that a sufficient amount of the anticipated CDR3 region sequence would be preserved in the PCR product. In addition, the theoretical product length was recorded as an approximate size reference for analysis of the resulting electrophoresis migration patterns.

All putative primer pairs were then re-submitted to Primer-Blast ⁽⁶⁸⁾ to assess for the possibility of non-specific products; the final set of putative primers pairs was also evaluated using the UCSC *in silica* PCR algorithm ⁽⁶⁹⁾ to confirm that no germline configuration products of less than 4 kb might be produced. Primer set physicochemical characteristics were evaluated using the IDT OligoAnalyzer Tool (v 3.1); Clustal W ⁽⁵³⁾ alignments were used to identify significant primer sequence overlaps (Clustal W alignments note significant overlap of the TRGJ1 and TRGJ2 primers. This overlap was considered acceptable in order to define which of the TRGJ1 and TRGJ2 genes were present (given the presence of 5' end non-homology). Since the PCR/electrophoresis results suggested the presence of both TRGJ1 and TRGJ2 positive products, the dominant TRGJ1 primer was selected for subsequent analyses and the TRGJ2 results excluded). The final primer-set sequences are listed in Table 2.2.

Custom primer set production was performed commercially by IDT and the forward and reverse primers were then mixed according to the design outlined in Appendix 2.7.2. PCR was performed in a 384-well plate on an Applied Biosystems Veriti thermal cycler using the Thermo Scientific 2X ReddyMix PCR Master Mix kit according to the manufacturer's instructions; several control reactions were included, as highlighted in Appendix 2.7.2. Gel electrophoresis was performed in a 96-well Bio-Rad Sub-Cell Agarose Gel Electrophoresis System (necessitating 4 separate runs); electrophoretic migration was referenced against an Invitrogen Tracklt 1 kb DNA ladder and visualized using ethidium bromide fluorescence, photographed in a Alphalmager Gel Imaging System. Electropherograms were digitally rendered, adjusted and composited using Adobe Photoshop CC 2014. The resulting electrophoretic results were used in Receiver-Operating Characteristic (ROC) curve analyses relative to the corresponding TRSeq normalized read counts.

### Sanger Sequencing Validation

Based on the results of the above PCR & Gel Electrophoresis experiment, rearrangement-positive PCR products were purified using a QIAquick Spin PCR purification kit (100 bp to 1 kb range) according to the manufacturer's instructions (Appendix 2.7.3). Purified PCR products were then quantified by Qubit and 20 ng equivalent aliquots were taken (with an additional volume reduction step using a SpeedVac, as required, for large volumes). The corresponding primer of the original primer pair with the lowest melting point was then selected for the purposes of single-direction Sanger Sequencing (performed at the TCGA Sick Kids Hospital Sequencing Facility).

The resulting sequencing results were analyzed using the FinchTV v 1.4 software suite, with corrections to sequencing error and reverse-complement sequence corrections performed manually as required. The originating TRSeq CDR3 sequences were then compared to the "reference" Sanger Sequence result. This comparison was performed in two ways: first, a basic multi-alignment comparison was performed (using the *multialign* algorithm of the MATLAB Bioinformatics Toolbox); second, a k-mer based PHRED-quality adjusted comparison was performed.

For the k-mer based approach, for a given V and J gene configuration , the most frequently detected TRSeq CDR3 sequences were aligned to the corresponding Sanger Sequencing result. In this context the Sanger Sequencing results were taken to represent a "consensus" of sequence data produced over all possible V and J configuration CDR3 sequences for that V-J gene configuration (reflecting the possibility of variable TRGR subclones). As such, in order to adjust the Sanger sequencing results to account for the potential alignment of a non-dominant subclone, a quality-based alignment algorithm was employed, based on the methods of ⁽⁷⁰⁾. Each input TRSeq CDR3 sequence was aligned along a progressive series of k-mers of the Sanger sequence using a custom quality-based alignment algorithm (code outlined in Appendix 2.8). For each alignment result, if the optimal alignment score occurred within the expected sequencing region (thereby representing an optimal alignment within a region of Sanger sequence expected to contain the actual CDR3 based on flanking primer sets), as outlined in Table 3.1A, the CDR3 sequence was classified as correct (and vice-versa). This classification was then used to perform ROC analysis to determine what number of TRSeq CDR3 sequence read counts might be considered a validated cut-off.

### Coverage Analysis

In addition to the above validation results, more detailed assessment of NTRA technical performance was also performed. Specifically, given that the NTRA relies on target enrichment, an assessment of the gene coverage of the NTRA was required. In addition, given that much of the utility of the NTRA might relate to identifying clonal cell populations, it was necessary to assess the dynamic sensitivity of the NTRA to decreasing numbers of cells bearing specific TR gene rearrangement configurations and, conversely, assess how standardized read counts might correlate with approximate input cell numbers.

### Coverage Dynamics by Specimen Clonality

Given the nature of TRGR, by which genomic components are excised upon rearrangement, we evaluated the coverage dynamics across the first-run specimens. This analysis served not only as a mean of qualitatively comparing how V and J gene coverage might be expected to vary in specific types of specimens, but also to evaluate which coverage metrics might be most predictive of specimen type (i.e. clonal vs not) and what specific cut-off criteria might be used to this effect. To do this, ROC-based analyses of mean overall and locus-specific coverage data for V and J genes was performed, as well as percent genes at least 100x for each of V and J gene types.

### Negative Control Coverage Assessment

For the purposes of this project, a fully germline TR gene configuration was approximated using a cell lines of embryonic origin and a cell line that has been fully sequenced without any known/reported TR gene derangements. The former scenario was approximated using the HEK293 cell line (an embryonic kidney cell line; ATCC CRL-1573) and the latter using a Coriell cell line (whose genome has been well-characterized and is not known to contain TR rearrangements). Use of the latter cell line was incorporated given that, in our hands, this cell line had been previously and purposefully degraded by FFPE treatment, representing a scenario of TR gene coverage assessment in the context of degraded DNA.

Total genomic DNA was extracted from previously cultured HEK293 cells and FFPE treated Coriell cell cultures and subsequently subjected to the NTRA, as outlined in Appendices 2.1 to 2.5. Standard TRSeq analyses were performed for each sample, with special deference paid to the coverage results.

### Dilution Series

A rigorous dilution series experiment, in the context of this project, might involve a flow-sort spike of cells with a known TR gene configuration into a population previously determined to be "polyclonal"; this might be approximated, for example, using a well-characterized cell line spiked into a population of lymphocytes obtained from normal blood. Rather than undertaking this more complex and expensive approach, an approximation of this dilution experiment was undertaken with DNA obtained from the Jurkat cell line spiked into a known-polyclonal lymphocyte population DNA isolate (the A037 sample; see Results section 3.2). Specifically, Jurkat DNA was spiked in at log-decrements (as outlined in Table 2.3) based on a lymphocyte total DNA complement assumed to be 0.7 pg, given the results of previous publications ⁽⁷¹⁻⁷³⁾. The total DNA of each sample in the dilution series was verified (and compared to expected values) using a Qubit assay; the samples were then subjected to the NTRA, as outlined in Appendices 2.1 to 2.5. Standard TRSeq analyses were performed, with special deference to changes in the raw read counts of Jurkat-specific TRGR configurations across the dilution series.

### Alternative Method and Algorithm

### Hybrid-Capture Protocol

For T cell receptor (TCR) diversity and clonality analyses we investigated genomic DNA isolated from flow sorted T cells isolated by affinity magnetic bead isolation, peripheral blood mononuclear cells (PBMC) isolated from blood by density gradient separation, cell-free plasma DNA extracted from blood, or scraped and pelleted immortalized cell lines.

Isolated DNA is sheared to ~275bp fragments by sonication in 130uL volumes (Covaris). DNA libraries are generated for illumina platform sequencing from 100-1000ng of sheared DNA by ligation of sequencing library adaptors (NextFlex) using the KAPA library preparation kit with standard conditions. Libraries are visually assessed (Agilent TapeStation) and quantified (Qubit) for quality.

Hybridization with probes specifically targeting the V and J genes is performed under standard SeqCap (Roche) conditions with xGen blocking oligos (IDT) and human cot-1 blocking DNA (Invitrogen). Hybridization is performed either at 65C overnight. The target capture panel consists of 598 probes (IDT) targeting the 3' and 5' 100bp of all TCR V gene regions, and 95 probes targeting the 5' 100bp of all TCR J gene regions as annotated by IMGT (four loci, 1.8Mb, total targeted 36kb). Hybridization and capture can be performed as a single step with a combined V/J panel, as a single step with only the V panel, or as a three step process when non-rearranged fragment depletion is desired consisting of a V capture, then depletion, then J capture.

For depletion of non-rearranged fragments 500ng-1000ng of library is depleted by hybridization with a panel of 137 probes (IDT) targeting the 5' 120bp of selected TCR V gene region 3' untranslated regions as annotated by IMGT (four loci, 1.8Mb, total targeted 16.5kb) and 131 probes (IDT) targeting the 5' 120bp of selected Ig V gene region 3' untranslated regions as annotated by IMGT (three loci, 3.1Mb, total targeted 15.7kb). A modified and truncated SeqCap protocol is employed wherein following incubation with M-270 streptavidin linked magnetic beads (Invitrogen), the hybridization reaction is diluted with wash buffer I, beads are discarded and the supernatant is cleaned up by standard Agencourt AMPure XP SPRI bead purification (Beckman).

### Algorithm

A custom Bash/Python/R pipeline is employed for analysis of paired read sequencing data generated by Illumina NextSeq 2500 instrument from the hybrid-capture products. Referring to Figure 5, this pipeline consists of four major steps: (1) Merging of the paired reads; (2) identification of specific V, J, and D genes within the fragment sequence; (3) identification of the V/J junction position as well as the antigen specificity determining Complementarity Determining Region 3 (CDR3) sequence at this site; (4) Calculation and visualization of capture efficiency and clone frequency within and across individual samples.
(1) 150bp paired-end reads are merged using PEAR 0.9.6 with a 25bp overlap parameter. This results in an approximate 275bp sequence for each fragment and enhances the sensitivity of V,J,D gene detection using the subsequent search strategies.
(2) Individual BLAST databases are created using all annotated V, D, J gene segments from IMGT. These full-length gene sequences are the targets of the hybrid-capture probe panel. Individual merged reads are iteratively aligned using BLASTn with an e value cut-off of 1 to the V database, J database then D database with word size of 5 for D segment queries. Trimming of identified V or J segments in the query sequence is performed prior to subsequent alignment to reduce false positives and increase specificity, particularly for the D gene query.
(3) In order to identify CDR3 sequences, the V/J junction position is extracted from the previous search data for those fragments containing both a V and J search result. 80bp of DNA sequence flanking this junction is translated to amino acid sequence in all six open reading frames and sequences lacking stop codons are searched for invariable anchor residues using regular expressions specific for each TCR class as determined by sequence alignments of polyclonal hybrid-captured data from a healthy patient as well as TCR polypeptides annotated by IMGT.
(4) Calculation of capture efficiency (on-target/off-target capture ratio) is performed by aligning all recovered, merged reads to the human genome (BWA) and dividing the number of reads aligning to the TCR loci by the total number of reads. The total number of unique TCR clones is determined by finding the unique minimum set of V/J combinations and the number of occurrences of each is tabulated. This data is visualized using R as stacked bar charts to generate figures that can be quickly visually assessed on a sample-by-sample basis for monoclonal or polyclonal signatures or clinically relevant enrichment of particular clones.

### Application of the algorithm to existing sequencing data

The custom pipeline is not dependent on our hybrid-capture protocol and can be performed on non-target captured whole genome or RNA-seq data. In this situation, an *in silico* capture is performed by extracting reads aligning to the four TCR loci (7:38250000-38450000, 7:141950000-142550000, 14:22000000-23100000) or Ig loci (chr2:89,100,000-90,350,000, chr14:106,400,000-107,300,000, chr22:22,350,000-23,300,000) from DNA (BWA) or RNA (STAR) sequence data (SamTools), followed by paired-end nucleotide sequencing data extraction (PicardTools). These reads are then inserted in to the previously described computational pipeline.

### Results and Discussion

### Informatics

### Insert Length Simulation

Figure 3-1A details the DNA Insert Length Simulation results. The analysis suggested a plateau of sensitivity of greater than 99,1% reached after 182 bp. For convenience, an adequately "evidence-based" insert length and informatics read length goal of 200 bp was chosen for the NTRA.

After further analysis excluded extra-locus V-D-J gene combinations (i.e. combinations not likely to result from rearrangements within the same TR locus), the number of missed combinations was reduced from 1752 to 80.

From among the above 80 intra-locus combinations, missed rearrangements originated only from among the TRB and TRG loci, with particular enrichment of TRBV6-2*01 and TRBV6-3*01 within the former (65 of 80) and enrichment of the TRGJ1*02 within the latter (15 of 80).

Analysis by phylogenetic sequence alignment (using the SWA alignment algorithm) within the TRBV6 group showed significant cophenetic linkage between the TRBV6-2*01 and TRBV6-3*01 genes (see Figure 3-1B). Similarly, analysis by phylogenetic sequence alignment within the TRGJ gene group suggested significant cophenetic linkage between TRGJ1*02 and TRGJ2*01 (see Figure 3-1C). These results suggest that combinations within the artificial read set involving either of these TRBV genes were likely misaligned to another TRBV gene (likely the next closest cophenetic "cousin," TRBV6-2*02) and that the TRGJ1*02 gene was likely misaligned to the TRGJ1*01 gene. Of note, the observation of closer cophenetic linkage between TRBV6-2*01 and TRBV6-3*01 rather than between TRBV6-2*01 and TRBV6-2*02 (as would be expected for two alleles of the same TR gene) and of closer cophenetic linkage between TRGJ1*02 and TRGJ2*01 rather than between TRGJ1*01 and TRGJ1*02, suggests error on the part of the IMGT classification.

### MATLAB SWA score cut-off determination

The results of the empirical V, D and J-gene MATLAB alignment score cut-off score experiment are presented in Figure 3-2. This experiment employed the code presented in Appendix 2.6.1 run on a test set of 91375 Illumina sequencing reads obtained from anonymized myeloid leukemia samples enriched for sequences outside of the IG/TR loci. These sequences were "confirmed" negative for V, D, and J gene sequences using the IMGT/High V-QUEST system (Brochet et al., 2008; Giudicelli et al., 2011). Given an experimental number of sequencing reads of at least 1 million, a 6-sigma cut-off score for MATLAB TRSeq analysis suggests 53.23 for the V genes; 19.02 for the D genes; and 34.43 for the J genes. It is easily observed that the cut-off values increase respectively from D, to J, to V genes; this observation parallels the mean length of the reference sequences from D to J to V genes.

### TRSeq Analysis of IMGT-produced TRGR Sample Sequence Reads

A sample of 268 short read sequences was downloaded from the IMGT website. These sequences consist of a variety of previously characterized TR and IG gene rearrangements available for download in FASTA format. After re-formatting into FASTQ format (using arbitrary quality scores), the dataset was analyzed using the TRSeq pipeline. Of the 268 short read sequences, 55 were identified by the IMGT as containing TR genes (either V or J genes); to these reads, there was perfect (100%) TRSeq alignment concordance, both in relation to gene name and allele. The TRSeq algorithm identified 50 of the 55 reads as containing evidence of TRGR; the 5 remaining reads were identified by the IMGT as containing rearrangements within the TRD locus, each with a TRSeq CDR3 region correctly identified. These results suggest that the 5 TRSeq "false-negatives" were informatically rejected by the TRSeq algorithm based on insufficient TRD D-gene SWA alignment score values; this form of error is not alarming given the more stringent means by which the TRSeq SWA alignment score cut-off values were determined relative to the IMGT/High V-QUEST pipeline ^{(56,58)}.

### First-run Results Summary

Table 2.5 outlines the flow-cytometric features of the 6 patient lymphocyte samples. These immunophenotypic features were in keeping with the lymphocyte sample sources of origin (also documented in Table 2.5), varying from normal patient peripheral blood mononuclear cells to highly immuno-sensitized lymphocyte cultures from tumour infiltrating lymphocyte specimens. Notably, the A037 sample served as a model of a "polyclonal" lymphocyte population whereas, for the purposes of qualitative assessment at least, the L2D8 sample could be immunophenotypically interpreted as highly "clonal" in nature.

In addition, model "clonal" samples were included, consisting of the Jurkat, CEM, SUPT1 and MOLT4 cell lines. Table 2.6 lists the previously documented rearrangements, as cited in the IMGT database ⁽⁶⁷⁾.

Prior to target enrichment and sequencing, adequate quality control was assured, as documented by pre and post-library preparation TapeStation tracings (see Figure 3-3). Post-target enrichment quality control was assured in the same manner.

Illumina NextSeq sequencing was then performed on Tapestation-normalized pooled input target-enriched DNA. The appertaining read-pair FASTQ-formatted zipped files were decompressed and the PEAR paired-end merging algorithm was run with a minimum strand sequence overlap of 25 bp. A breakdown of the PEAR results is shown in Figure 3-4. The resulting PEAR-merged FASTQ-formatted read files were input to the TRSeq pipeline.

Figures 3-5, 3-6, and 3-7A & 3-7B summarize the TRSeq metadata for the first-run sample series, including input reads, reads-on-target, summary coverage statistics, and a histogram of read counts for the proportion of each locus contributing to identified TRGR's, respectively.

One important highlight is the variation in coverage seen across the 10 specimens relating to the D locus. As described in the introduction, since the D locus genes are sandwiched within the larger A locus, the D locus genes are often deleted upon A locus rearrangement. The coverage profiles of the D locus therefore paralleled this phenomenon with lower D locus coverage identified in the clearly clonal or oligoclonal samples relative to the polyclonal samples (e.g. L2D8 and cell line samples vs. A037 peripheral blood sample).

Figures 3-8A and 3-8B display composites of the circos plots obtained from the 10 first-run samples. Much as the coverage profiles differed across the samples (as seen in Figures 3-8C & 3-8D), the resulting circos plots demonstrated a clear aesthetic difference from polyclonal to clonal/oligoclonal samples, with emphasis on the number and relative width of the composite circos links (i.e. fewer and broader in width in the more clonal cases and vice versa). Also of note, the color distributions were distinctly different with the more polyclonal cases, containing a larger number of smaller-quantity "subclones" involving a more disparate number of TR genes.

### Analytical Validation

### IMGT/High V-Quest Comparison

The boxplots of Figure 3-9 summarize the comparison of the IMGT/High V-Quest pipeline analysis to the TRSeq results. The degree of concordance of read-to-read interpretation with respect to identifiable rearrangements (as present or not identified) is excellent (99%), as is the degree of concordance of named D genes (99%). A lower degree of concordance is noted for named V and J genes (68% and 84%, respectively). These results may relate to different initial alignment algorithms employed, as well as different gene-identity cut-off values employed in the SWA algorithms of the IMGT/High V-Quest and TRSeq pipelines. In light of the results seen in Section 3.1.1, the possibility of V and J gene phylogenetic sequence misclassification in the publically-available IMGT sequence databases should also be considered as a possible contributing factor.

The high D-gene concordance relative to the V and J-gene values may relate to both the shorter reference sequences of the D-genes relative to the V and J genes, as well as the lower number of reference D-genes available for rearrangement. It is important to point out the possibility of a theoretical bias against D-gene identification in input reads, given that TRGR reads containing D-genes require 3 rather than 2 composite genes, which could be more difficult to detect in the context of restricted average read lengths. This consideration was brought to bear during the NTRA assay design phase (as described in Section 3.1.1), with the conclusion that adequate flanking 5' and 3' sequence would be available on average in the scenario of read input length of 200 bp or more to reliably identify reads containing V-D-J rearrangements.

### PCR & Gel Electrophoresis

PCR primers were mixed according to the design of Figure 3-10 and the results by Agarose gel electrophoresis are shown in Figure 3-11. Note that results obtained from PCR reactions using the TRGJ2 reverse primer are excluded, as noted in Section 2.2.2. Two classification approaches may then be entertained, one based on dark-staining PCR bands only, and the other based on any staining (assuming bands to be of appropriate molecular weights, as set out in Table 3.1A). When these classifiers are compared with the read-count-normalized results of the TRSeq algorithm (as set out in Table 3.1A), the ROC curves of Figures 3-12A & 3-12B are obtained, respectively. In the former scenario, the ROC Area-Under-the-Curve (AUC) = 0.91 and p-value <0.001, with a TRSeq normalized read count of 6.7 or more. Based on the results of Figure 3-12B, a less stringent classification results in a reduced AUC = 0.71 and p-value <0.001, with a TRSeq normalized read count of 1.7 or more.

### Sanger Sequencing Results

Figure 3-10 details those PCR reactions that were post-PCR purified and submitted for Sanger Sequencing. Figure 3-13 denotes the alignment of each corresponding TRSeq CDR3 sequence (and associated raw read count) in relation to the manually-verified/corrected Sanger Sequencing Result; only those Sanger Sequencing specimens containing TRSeq-identified CDR3 regions, those of sufficient quality for interpretation, and those not rejected based on use of the TRGJ2 reverse primer were further considered.

As may be seen in Figure 3-13, there appears to be a trend for each distinct primer configuration inasmuch as TRSeq-identified CDR3 sequence configurations having sufficient associated read counts, as suggested from Section 3.3.2, show the best contiguous alignments to the corresponding "reference" Sanger Sequences.

To better quantify this relationship, we utilized a k-mer based quality-score adjusted alignment analysis. For each relevant primer configuration, the corresponding CDR3 was aligned using PHRED-based quality-score adjustment across the length of the Sanger "reference" sequence. If the optimal alignment from this process was present within the sequence window in which a CDR3 was theoretically predicted to exist, the CDR3 read configuration was classified as "compatible." The resulting classification analysis is represented by the ROC curve of Figure 3-14 (AUC = 0.832, p-value = 0.006). Based on this analysis, the optimal TRSeq normalized read count cut-off is 4.9.

### Coverage Analysis

### Coverage Dynamics by Specimen Clonality

Using the qualitative data of Table 2.5, specimens were classified as either "clonal" or "polyclonal." The resulting ROC curves for the various coverage metrics are shown in Figure 3-15. Of note, a mean V-gene coverage assessment of the gamma locus appeared to suggest the highest non-unity AUC. Further, the ROC analysis suggested that a mean V-gene coverage of greater than/equal to 4366.4 showed optimal sensitivity and specificity (86% and 67%, respectively) for predicting whether a specimen was unlikely to be clonal. Care should be taken not to use these cut-off points without additional validation, however, given the low number of data points constituting the analysis. Rather, these data stand to suggest a need for further evaluation of the potential predictability of "clonal" status derived from coverage analysis within the gamma locus.

### Negative Control Coverage Assessment

The NTRA was tested on samples of previously cultured HEK293 and Coriell cell lines; these analyses aimed mainly at estimating coverage ceilings for the NTRA, but also served as added negative control specimens (i.e. specimens known or expected not to contain any TRGRs).

Applying the PEAR algorithm ⁽⁵²⁾ (with a minimum 25 bp forward-reverse read overlap) resulted in pairing of 83% of input reads in the HEK293 sample and 90% of input reads in the Coriell sample.

In both instances, the number of subsequently identified TRGR configurations did not meet the TRSeq cut-off criteria (TRGRs were identified in 0 of 5,729,205 total input reads in the HEK293 cell line and only 7 of 2,761,466 total input reads in the Coriell cell line). This was in keeping with the anticipated fully-germline configuration of each of these non-lymphoid origin cell types.

For the HEK293 cell line, the percent V and J genes at or above 100x coverage was 100%; the overall TR V gene coverage averaged 29960x; and the overall TR J gene coverage averaged 8789x.

For the Coriell cell line, the percent V and J genes at or above 100x coverage was 100%; the overall TR V gene coverage averaged 13379x; and the overall TR J gene coverage averaged 3925x.

### Dilution Series

A dilution experiment was performed at log-reduction intervals, set up according to the design of Table 2.3, and adjusted according to Table 3.2 to account for Jurkat DNA concentration discrepancies. Three Jurkat cell line unique TRGR configurations were selected for inter-dilution comparison, namely the TRAV8-4 - TRAJ3, TRGV11 - TRGJ1 and TRGV8 - TRGJ2 rearrangements identified & confirmed in Section 3.3. The above configurations were confirmed absent in the polyclonal (A037) sample. In addition, each of these configurations showed a specific dominant CDR3 sequence.

Figure 3-16A details the mean of the raw read-counts (i.e. not normalized) across the three tracked V-J configurations (with error bars for standard deviation) vs. expected approximate Jurkat cell numbers (with adjustments for significant digits) from Table 3.2. An exponential trend line could be applied, with R-squared = 0.9996.

Of note, when the extremum of the first dilution is excluded, the dilution curve is remarkably linear (as seen in Figure 3-16B), but with a positive slope. This suggests a linear direct correspondence between read count and number of cells bearing a given V-J configuration at low levels.

In contrast to the reliable low-level detection by way of V-J configuration, detection narrowed to *absolute* clonotype (by including the CDR3 sequence) was limited to only the first three dilution specimens (i.e. sensitivity down to an approximated 1 in 125 cells; see Figure 3-17).

This limited sensitivity speaks to the sensitivity of the TRSeq junction finder to sequencing error. Indeed, if even a single base is changed relative to the canonical regular expression required for detection of a CDR3 sequence, the junction finder will not identify the sequence correctly; likewise, any non-triplicate base insertion will not be detected as an in-frame CDR3 sequence. In contrast, since the TRSeq V and J gene enumeration scheme uses alignment-based algorithms, the TRSeq results relating to V and J gene enumeration are much more forgiving of higher the higher likelihood of sequencing error in clonotypes with low read counts, thus substantially improving the assay sensitivity for characteristically unique V-J gene configurations.

Support for these suppositions is echoed in part by previous work pertaining to core clonotype analyses ⁽²⁷⁾. Indeed, when the proposed criteria of Bolotin, et. al. ⁽²⁷⁾ for gathering low-level reads of similar but error-prone sequence into common core clonotypes are applied to the dilution experiment (implemented in Appendix 3), it is possible to identify reads comparable to the clonotypes described above in even the most dilute samples.

For example, running the code of Appendix 3 with the input core clonotype of the TRGV8 - TRGJ2 configuration, and allowing for a maximum of 3 sequence mismatches, 3 or more reads of satisfactory clonotype can be identified in dilutions 2-5. If the number of sequence mismatches is increased to 4, reads of satisfactory clonotype can be identified in all dilutions (i.e. down to an estimated sensitivity of 1 in 185646 cells).

The importance of these results stems from the applicability of this form of core clonotype analysis to a more accurate identification of minimal-residual disease, for example, at very low levels with remarkable sensitivity, even in the absence of traditional primer-directed sequence enrichment ⁽⁷⁷⁾.

### NTRA - BIOMED-2 Comparison

In keeping with the general approach used to assess BIOMED-2 results, the NTRA TRB and TRG clonotype tables were analyzed to compare the ratio of the dominant clonotype read count relative to the "background" read count. The largest read count not satisfying the normalized TRSeq read count according to the results of Section 3.3 was taken as the background read count value; alternatively, in the case where the dominant clonotype did not satisfy the normalized TRSeq read count cut-off of Section 3.3, the next largest clonotype read count was taken as "background". From among each of the TRB and TRG loci, the largest dominant clonotype-to-background ratios were compared to the overall BIOMED-2 results using a ROC analysis.

See Figure 3-18; the ROC analysis result could be classified as "good" ⁽⁷⁸⁾ with AUC = 0.82, p-value < 0.001. Of note, this AUC value appears comparable to those observed in Section 3.3. Of even more impressive note is that the ROC-suggested dominant clonotype-to-background cut-off value was also comparable to that outlined in the current BIOMED-2 TRGR assay interpretation guidelines ⁽⁷⁹⁾; indeed, the ROC analysis-suggested value of 3.4, which is effectively the median value of the "indeterminate" range of dominant peak-to-background ratios recommended for BIOMED-2 result interpretation ⁽⁷⁹⁾.

Interestingly, when the above process was broken down into two separate comparisons of the TRB and TRG loci, the TRG locus was found to be the significant driver: the TRG locus comparison alone yielded a ROC AUC = 0.81 (p-value < 0.001) whereas the TRB locus comparison alone yielded a ROC AUC = 0.60 (p-value = 0.17).

### NTRA Coverage Metrics - BIOMED-2 Comparison

As in Section 3.4, an analysis of coverage variation in relating to clonal status was undertaken (see also Figure 3-19). In contrast to the results of Section 3.4, a far less significant series of areas-under-the-curve were observed from this analysis. The greatest AUC was noted by analysis of mean V-gene coverage (i.e. mean V-gene coverage over all four loci) with AUC = 0.59, p-value = 0.213.

Furthermore, the data from Section 3.4 suggested that analysis of coverage from the Gamma locus might be predictive of clonal status. Unfortunately, these hypotheses were not substantiated by way of the clinical validation set, from which the AUC for the TRG locus V-gene analysis and TRG locus J-gene analysis were 0.59 and 0.57, respectively.

The clear discordance between these results and those of Section 3.4 likely relates to several factors. First, the sample size in Section 3.4 is one-sixth that of the clinical validation set, making the results of Section 3.4 much more vulnerable to the effects of outliers. Second, the overall coverage in the analytical validation set was lower, owing to base-output restrictions using the mid-output NextSeq kit; as such, coverage correlations made in Section 3.4 might not necessarily be applicable to experiments performed using the high-output NextSeq kit. Thirdly, the clinical validation experiment was not subject to bias of assumption as to the clonality of each input specimen; rather clonality was specifically assayed using an orthogonal method.

### Summary

Described above is the first hybrid-capture-based T-cell clonality assay designed to assess clonality and provide clonotype data over all four T-cell gene loci. For this purpose, a custom MATLAB-based analysis pipeline was implemented using optimized object-oriented programming integrating the ultra-fast BWA alignment system and the aesthetically-pleasing circos-based genomic data visualization suite. The latter visualization was designed with current methods in mind, in which electropherographic plots serve as the primary means by which clonotypes are visualized.

### Advantages of NTRA over traditional T-cell clonality testing assays

Not only can the NTRA identify clonotypes from all four loci, the use of hybrid capture makes the process platform-agnostic. The laboratory work-flow can be integrated into any standard library preparation work-flow with the addition of a single hybridization step, capable of enriching for sequences containing T-cell genes of a several specimens at a time. In addition, as part of laboratory work-flows already using a hybrid-capture approach for other purposes, the probes used as part of the NTRA are amenable to "spike-in" combined hybridization reactions, provided that there is no significant probe-set sequence overlap or complementarity.

In comparison to the current BIOMED-2 based clonality assays, the NTRA adds a dearth of extra data, especially as pertaining to clonotype data from the gene-rich alpha-locus. This locus has traditionally been too diffusely distributed within the genome to be amenable to primer-based amplification, a challenge easily overcome using a hybrid-capture approach. Akin to the requirements of the IMGT, the NTRA outputs a clonotype table containing data specific to the best aligned allele. In contrast, however, visualized data is restricted to gene-level only, thereby providing a means of visualization comparable to electropherographic output. In addition, included with the latter, is the in-frame CDR3 sequence (where detected), data currently not available using either standard PCR-based techniques or the mainstream sequencing-based solutions (e.g. Invivoscribe).

In addition to validating the wet-bench and informatics using a number of orthogonal approaches, the NTRA was also shown to be theoretically sensitive to low-level clonotypes. This latter observation is an important boon to the hybrid-capture approach, suggesting that carefully performed hybrid-capture methods can provide signal amplification comparable to flow-cytometric ⁽⁸¹⁾ and molecular approaches ⁽³²⁾⁽⁸²⁾⁽⁸³⁾.

### Assay Cost & Efficiency Considerations

As highlighted in Section 3.8, the assay may be considered cost effective, depending on the specific scenario of interest. In addition, the use of a hybrid-capture approach allows for spike-ins of additional probes for other genomic regions of interest. This allows the possibility of running multiple assays from a single library preparation step, requiring only bioinformatic separation of the resulting enriched sequences.

### Applications

Assessment of lymphocyte clonality is integral to the diagnosis of diseases and cancer affecting the immune system. In addition, sequencing of the T-cell repertoire of a patient has gained clinical value with the recent understanding of T-cell mediated recognition and destruction of neoplasms. Further, the development of adoptive cell therapy and recombinatorial engineering of T-cell receptors requires high-throughput molecular characterization of *in vitro* T-cell populations before transplant. PCR-based methods such as BIOMED-2 and Immunoseq are currently in use for TCR characterization however their costs and complexity remain barriers for clinical deployment requiring high-throughput multi-patient, multi-sample work-flows at low cost. We have therefore developed a hybrid-capture-based method that recovers rearranged TCR sequences of heavy and light TCR chains from all four classes in one tube per sample at low cost. TCR clonality and CDR3 prevalence can be rapidly assessed in a three-day turn-around time with an automated pipeline generating summary figures that can be rapidly assessed by clinicians.

Adaptive T-cell immunotherapy has become a field of great interest in the treatment of multiple solid-tumor cancer types. Non-childhood cancers, particularly those linked to chronic exposure of known carcinogens, are driven by the accumulation of mutations. Some of these mutations drive pro-tumorigenic changes, while others result in non-tumorigenic changes to proteins expressed by the carrier cell. During normal protein turnover these modified proteins are broken down in to short polypeptides and make their way to the surface of the cell in association with molecular surveillance molecules (MHC I). In this context these modified polypeptides are recognized as foreign neo-antigens by the host immune system, and in the context of other signals, lead to the activation of T-cells that direct the destruction of cells expressing these modified proteins.

It is now understood that many solid-tumours exist in a state where their presence recruits neo-antigen specific T-cell lymphocytes to the margins however further advance and effective destruction of the tumor is prevented by expression of checkpoint inhibition molecules on the tumor cell surfaces. Therefore immunotherapy has become a major area of advance in cancer therapy wherein such checkpoint inhibition molecules are masked through transfusion of antibodies. This allows recognition of tumor and its destruction by neo-antigen specific T cells. In order to further enhance such anti-tumor activity, tumor infiltrating lymphocytes (TIL) can be isolated from tumor biopsies and expanded *in vitro,* followed by subsequent transfusion in great numbers back in to the patient following immunodepletion to enhance transplant colonization thereby driving a durable antitumor response.

T-cell lymphocytes are fundamental to this process, however due to their exquisite specificity, only neo-antigen specific T-cells are capable of driving anti-tumor activity. As a result there is a need for molecular characterization of circulating T-cells in the patient before and after treatment, infiltrating T-cells in the tumor before and after treatment, and screening of expanded populations *in vitro* for safety and efficacy. Our method provides a high-throughput, low cost and rapid turn-around method for T-cell receptor characterization in order to facilitate clinical deployment and uptake of adoptive cell transfer immunotherapy.

This method is not only of use in immunotherapy applications, as any disease involving expansion of T-cell clones would benefit from its use. The symptoms of autoimmune diseases are driven largely by T-cell mediated cytotoxicity of "self" tissue and therefore the identification and expansion of specific T-cell clones can be monitored using this method. This method would also be useful to follow immune challenges such as infection or immunization in the development of anti-infectives or vaccines.

### Example 2

There is also described herein a laboratory and bioinformatic workflow for targeted hybrid-capture enrichment of T-cell receptor loci followed by Illumina sequencing to assess the clonality of a range of specimens with variable T-cell clonal complexity as well as a set of 63 T-cell isolates referred for clinical testing at our institution.

### Methods and Materials

***Probe design*** - All annotated V, D, J gene segments were retrieved from the IMGT / LIGM-DB website (www.imgt.org ⁹). The 100bp of annotated 3' V gene coding regions and up to 100bp, when available, of annotated 5' J gene coding regions were selected as baits. Probes with duplicate sequences were not included.

***DNA isolation*** - CD3+ T cells were isolated by flow assisted cell sorting of PBMC populations separated from whole blood. Peripheral blood mononuclear cells (PBMC) were isolated from whole blood by centrifugation followed by DNA isolation with a Gentra Puregene kit (Qiagen) according to manufacturer protocol. In the case of fresh/frozen tissues, a Qiagen Allprep (Qiagen) kit was employed, according to the manufacturer's instructions. In contrast, for FFPE samples a previously optimized in-house approach was used. First, sample FFPE tissue blocks were cored with a sterilized Tissue-Tek Quick-Ray punch (Sakura) in a pre-selected area of representative tissue; alternatively, under sterile conditions, 10 x 10 µm DNA curls/unstained slides were obtained for each submitted block of FFPE tissue. In a fumehood, 400-1000 µL xylene was aliquot into each tube (volume increased for larger FFPE fragments), followed by vigorous vortexing for 10 sec, incubation in a 65°C water bath for 5 min, and centrifugation at 13200 rpm for 2 min. The supernatant was then discarded and step an additional xylene treatment step was performed. Subsequently, addition of 400-1000 µL ethanol (volume adjusted for larger input tissue volumes) was performed, followed by vigorous vortexing for 10 sec, and centrifugation at 13200 rpm for 2 min. The supernatant was then discarded and the ethanol treatment step repeated. The resulting pellet was then dried using a SpeedVac (Thermo Scientific) for 5 min, after which 150 µL of QIAamp buffer ATL (Qiagen) was added, followed by 48-hour incubation at 65°C with 50-150 µL of proteinase K (volume increased for higher input volumes). A final ethanol clean-up step was performed, as above, to produce a purified DNA product. Resuspension in TE buffer (Qiagen) was then performed.

***Hybrid capture*** - isolated genomic DNA was diluted in TE buffer to 130uL volumes. Shearing to ~275bp was then performed on either a Covaris M220 Focused-ultrasonicator or E220 Focused-ultrasonicator, depending on sample throughput, with the following settings: for a sample volume of 130 µL and desired peak length of 200 bp, Peak Incident Power was set to 175 W; duty factor was set to 10%; cycles per burst was set to 200; treatment time was set to 180 s. in addition, temperature and water levels were carefully held to manufacturer's recommendations given the instrument in use.

Illumina DNA libraries were generated from 100 - 1000 ng of fragmented DNA using the KAPA HyperPrep Kit (Sigma) library preparation kit following manufacturer's protocol version 5.16 employing NEXTFlex sequencing library adapters (BIOO Scientific). Library fragment size distribution was determined using the Agilent TapeStation D1000 kit and quantified by fluorometry using the Invitrogen Qubit.

Hybridization with probes specifically targeting V and J loci (Supplemental Table 3) was performed under standard SeqCap (Roche) conditions with xGen blocking oligos (IDT) and human Cot-1 blocking DNA (Invitrogen). Hybridization is performed either at 65C overnight. The target capture panel consists of 598 probes (IDT) targeting the 3' and 5' 100bp of all TR V gene regions, and 95 probes targeting the 5' 100bp of all TR J gene regions as annotated by IMGT (four loci, 1.8Mb, total targeted 36kb).

***Capture Analysis*** - A custom Bash/Python/R pipeline was employed for analysis of paired read sequencing data generated by Illumina NextSeq 2500 instrument from the hybrid-capture products. First, 150 bp paired reads were merged using PEAR 0.9.6 with a 25bp overlap parameter ^{A18}. This results in a single 275 bp sequence for each sequenced fragment. Next, specific V, J, and D genes within the fragment sequence were identified by aligning regions against a reference sequence database. Specifically, individual BLAST databases were created using all annotated V, D, J gene segments retrieved from the IMGT / LIGM-DB website (www.imgt.org ^{A9}), as these full-length gene sequences were the source of probes used to design the hybrid-capture probe panel. Individual merged reads are iteratively aligned using BLASTn with an e value cut-off of 1 to the V database, J database then D database with word size of 5 for D segment queries ^{A19}. Trimming of identified V or J segments in the query sequence is performed prior to subsequent alignment. From reads containing V and J sequences, we identified V/J junction position and the antigen specificity determining Complementarity Determining Region 3 (CDR3) sequences. In order to identify CDR3 sequences, the V/J junction position is extracted from the previous search data for those fragments containing both a V and J search result. 80bp of DNA sequence flanking this junction is translated to amino acid sequence in all six open reading frames and sequences lacking stop codons are searched for invariable anchor residues using regular expressions specific for each TR class as determined by sequence alignments of polyclonal hybrid-captured data from rearranged TR polypeptides annotated by IMGT⁹

### Results and Discussion

The CapTCR-seq method employs hybrid capture biotinylated probe sets designed based on all unique Variable (V) gene and Joining (J) gene annotations retrieved from the IMGT database version 1.1, LIGMDB_V12 ⁹. These probe sets specifically target the 3' regions of V gene coding regions and the 5' regions of J gene coding regions that together flank the short Diversity (D) gene fragment in heavy chain encoding loci and which together form the antigen specificity conferring CDR3 (Figure 6A). D regions (absent in alpha and gamma rearrangements) were not probed due to their short lengths, high potential junctional diversity introduced by the recombination process, and to permit a single universal probe set for both light and heavy chain loci. These biotinylated probes are hybridized with a fragmented DNA sequencing library, and probe-target hybrid duplexes are subsequently recovered by way of streptavidin-linked magnetic beads. The subsetted library is PCR amplified from the bead-purified hybrid-duplex population using a single set of adapter-specific amplification primers and the resulting library is subjected to paired read 150bp sequencing on an Illumina NextSeq 500 instrument. A 250bp fragment size was selected as mid-range between the maximum length of a merged fragment from 150bp paired-end read sequencing (275bp) and a lower limit of 182bp based on alignments of simulated reads centered at the VJ junction with variable insert sizes that had successful V and J alignment sensitivity of > 99%.

To identify V(D)J rearrangements from the pool of captured V and J sequences, we used a computational method that performed: (1) Read merging to collapse paired reads in to a single long-read sequence to enhance V(D)J and CDR3 identification, (2) progressive BLASTn-based V, J and D detection utilizing iterative end trimming and (3) CDR3 scoring using regular expression pattern matching (Figure 6B). This BLAST-based sequence alignment approach was employed due to its tolerance for nucleotide mismatches that could arise from junctional diversity or the presence of allelic variants not present in the reference database. We acknowledge that numerous alternative V(D)J and CDR3 calling algorithms are available ^{A10-16} and these may be used in addition or in lieu of our pipeline to analyze V(D)J fragments captured by our laboratory approach. A head-to-head comparison of these methods is beyond the scope of this proof-of-principle report.

We employed this method to identify V(D)J rearrangements and CDR3 sequences in PBMCs isolated from a healthy human. With a single step hybridization and capture reaction employing the probe panel targeting TCR V genes, the number of detected unique VJ rearrangements increased with increasing amount of sample genomic DNA used to generate the initial library, with 52 times more rearrangements detected with an input of 1,000ng compared with 100ng (1925 vs 37) (Figure 6C). The number of unique VJ rearrangements is dependent on the number of T cells in the original sample with an approximate fourfold increase for CD3+ sorted cells over PBMCs (2475 vs 759) (Supplemental Table 1). Addition of the J probe panel to form a single-step capture using a pooled V and J panel improved recovery of unique CDR3 sequences per 1ng of library input by 5 fold (single-step V capture mean: 1.7, single-step VJ capture mean: 8.56) (Supplemental Table 1). This modification also increased the ratio of on-target reads, effectively decreasing the amount of sequencing needed to obtain the same number of rearranged fragments (single-step V capture mean: 14.4%, single-step VJ capture mean: 42.9%). Overall, we saw a diverse representation of alleles for all four classes with 2895 alpha, 1100 beta, 59 gamma, 9 delta unique VJ rearrangements observed from 16 independent captures of independent libraries (Figure 9A-D). This corresponded to 6257 alpha, 4950 beta, 1802 gamma, 109 delta unique CDR3 sequences. We also submitted a portion of these samples for parallel characterization by a commercial PCR-based TCR profiling service and found similar V/J gene usage and representation with no more than 2% variation (Figure 6D-F) and correlation with an r² value of 0.94 (Figure 9E)

To test the ability of CapTCR-seq to assess TCR clonality of samples with a range of clonal signatures, we analyzed libraries derived from CD3+ flow-sorted Tumor Infiltrating Lymphocytes (TIL) expanded cultures (oligoclonal) and lymphoblast cell lines (clonal) (Figure 7A-B; Figure 10A-B). As expected, the cell-lines and antigen-specific cell-sorted samples were more clonal (12-22 unique VJ rearrangements) than the TIL cultures (123-446 unique VJ rearrangements). The predominant alpha rearrangement represented 40-80% of the recovered reads in clonal samples compared to 2.5-17.5% for the latter TIL cultures. Specifically, we detected 12 unique VJ rearrangements in L2D8, a GP100 antigen-specific tumor-infiltrating lymphocyte clone. In OV7, a mixed ovarian tumor-infiltrating lymphocyte population expanded with IL-2 treatment, we found 311 unique VJ rearrangements. We profiled two populations isolated from the same tumor: M36_EZM, a cell suspension of melanoma tumor with brisk CD3 infiltration harbored 123 unique VJ rearrangements, while M36_TIL2, tumor-infiltrating lymphocytes from this tumor expanded in IL-2 harbored 446 unique VJ rearrangements, reflecting a likely expansion of low prevalence T cells. STIM1 is MART1-specific cell line made from peptide stimulation of healthy donor PBMCs, FACS sorting and expansion of tetramer+ cells from which we found 195 unique VJ rearrangements. The cell lines were found to encode previously reported gene rearrangements at the TCR beta and gamma loci, and additional rearrangements not previously reported (Supplemental Table 2) ^{A17}. Targeted PCR amplification of V/J rearrangement pairs, including the most frequently observed for each sample, was performed on these samples. We observed expected product for all prevalent rearrangements with some amplification failures for low prevalence rearrangements (Sample: Observed bands / expected bands; A037: 9/11; L2D8: 4/5; EZM: 3/4; TIL2: 8/9; OV7: 5/9; STIM1: 7/9; SE14 2005: 4/4; SE14 2033: 3/4; SE14 2034: 4/4; SE14 2035: 4/4) (Figure 10C). We also submitted the GP100 antigen specific L2D8 sample for beta locus profiling by a PCR-based commercial service and found VJ repertoire usage to be highly congruent (Figure 7C-E), however the commercial service identified extensive low level VJ gene usage not present in the capture data (Figure 7D). This signal may represent low-level alternative VJ pair antigen specific clones, or sample contamination with non-antigen specific clones.

To demonstrate the potential clinical utility of our approach, we generated DNA sequencing libraries from an unselected cohort of 63 samples submitted for clinical T-cell receptor rearrangement testing and subjected these to capture, sequencing and analysis (Supplemental Table 1). Samples were found to have varying degrees of clonality, with the predominant CDR3 sequence representing up to 40% of the most clonal sample (average 12.2%; median 6.3%%, range 0.8-100%, Figure 8A-B; Figure 11A-B). When a clonal population was defined as having the most abundant to third most abundant rearrangements observed at two or more times the level of the next most abundant rearrangement, we observed three groups of samples: 11 with clonal enrichment of *both* beta and gamma rearrangements, 12 with clonal enrichment of beta *or* gamma rearrangements, and 41 that were polyclonal for both beta and gamma. When 61 of these samples were assessed by BIOMED2 assay we observed 73% agreement for beta (44/60) and 77% for gamma (46/60), 60% of samples were in agreement for both beta and gamma clonality measures (36/60). For the beta locus, 13 samples that were scored as clonal by BIOMED2 were scored as polyclonal based on relative prevalence when assessed by hybrid capture profiling. Six had low top clone prevalence (predominant rearrangement relative proportion of 1.3%, 1.8%, 2.6%, 3.1%, 3.4%, 3.8%) with a median unique VJ rearrangement count of 185. Seven had higher top clone prevalence (predominant rearrangement relative proportion of 7.6%, 8.4%, 8.5%, 8.8%, 11.9%, 12.1%, 16.9%) with a considerably lower median unique VJ rearrangement count of 44. These 13 samples had variable diversity but no predominant rearrangement was more than twofold enriched relative to the next most common rearrangement. Conversely, three samples that were scored as polyclonal by BIOMED2 at the beta locus were scored as clonal based on relative prevalence (predominant rearrangement relative proportion of 25.9%, 18.6%, 6.5%) with a median unique VJ rearrangement count of 191. These discrepancies could be resolved with deeper sequencing of these libraries to determine whether insufficient depth was distorting the interpretation or whether these represent incorrect interpretations by the BIOMED2 protocol. Improvements in the BIOMED2 primer sets have led to reduced false positives compared to previous generations, and can be further diminished through the use of higher resolution gel separation and additional analyses ^{A2}, however if available, sequencing-based methods provide a more quantitative assessment and relative comparison between all rearrangements. To determine whether there was unexpected enrichment in the A037 or lymphoma data sets we compared their gene usages (Figure 11C-F). A037 and the lymphoma collection had similar VJ usage profiles with few individual unique VJ rearrangement proportion enriched in A037 of up to 1% and more enrichments amongst the lymphoma set of up to 3% as expected given the clonal enrichment of select rearrangements in T-cell lymphomas.

In summary, CapTR-Seq allows for rapid, inexpensive and high-throughput profiling of all four loci from multiple samples of diverse types from a given DNA sequencing library with fragment size of 250bp and sequencing length of 150bp. This method will permit intensive monitoring of TR repertoires of patients with T-cell malignancies as well as monitoring of tumor-infiltrating lymphocytes in tumors from patients undergoing immune checkpoint blockade, adoptive cell transfer and other immunotherapies.

### EXAMPLE 3

Adoptive Cell Transfer (ACT) of *in-vitro* expanded Tumour-Infiltrating Lymphocytes (TIL) has emerged as an effective treatment for numerous types of solid tumours, often resulting in a durable response and in some cases a complete remission by the patient^{B1}. This intervention effectively replaces nearly the entire heterogenous T-cell repertoire of the patient with tumour antigen and patient-specific effector T cells. Effector T-cells are integral for the adaptive immune response due to their roles in cellular cytotoxicity and cytokine production, with specificity conferred by the TCR-MHC interaction^{B2}. The CD8+ effector T-cell repertoire consists of alpha/beta and gamma/delta subtypes, both polyclonal and skewing in the incidence of an antigen-specific response or malignancy^{B3}. In high mutation load neoplasms, the MHC molecule often presents tumour-associated neo-antigens generated as a result of mutation that lead to clonal expansion and infiltration of tumour-infiltrating lymphocytes (TILs)^{B4}. These TILs are largely clonal and distinct from the circulating repertoire in multiple types of neoplasia^{B5}. While these TILs are capable of driving an effective anti-tumour response *in vitro,* they are often exhausted within the tumour microenvironment as a result of expression of immunosuppressive cell-surface proteins by the tumour but their activities can be restored with immune checkpoint blockade therapy^{B6}. The combined effect of immunotherapy intervention: immunodepletion, TIL ACT and checkpoint blockade together present an effective treatment for many patients but have a disruptive effect on the endogenous immune repertoire and therefore proper patient care would benefit from longitudinal monitoring of the T-cell repertoire during the course of disease and treatment.

During ACT immunotherapy, both the requisite immunodepletion and T-cell transfer radically disrupt the abundance and diversity of the endogenous T-cell population and therefore molecular profiling methods are required for monitoring of the patient during the course of immunotherapy^{B7}. The TCR repertoire consists of cell-specific heterodimeric receptors uniquely rearranged and expressed from either the alpha/beta or gamma/delta genomic loci^{B8}. The TCR has unique specificity for an antigen presented in the context of the an MHC molecule as defined by the combined interactions of the amino acid residues encoded at the V-(D)-J junction known as the complementarity determining region 3 (CDR3), and by the CDR1 and CDR2 regions in the upstream V gene fragment.

### Methods and Materials

***Probe design*** - All annotated V (V-panel), D, J (J panel) gene segments and V 3'-UTR (depletion panel) sequences were retrieved from the IMGT / LIGM-DB website (www.imgt.org). The 100bp of annotated 3' V gene coding regions, up to 100bp, when available, of annotated 5' J gene coding regions, and 120bp of V 3'-UTR sequences were selected as baits. Probes with duplicate sequences were not included. The V-panel consists of 299 probes (IDT) targeting the 3' and 5' 100bp of all TR V gene regions, and the J-panel consists of 95 probes targeting the 5' 100bp of all TR J gene regions as annotated by IMGT (four loci, 1.8Mb, total targeted 36kb). The depletion-panel consists of 131 probes targeting the 5' 120bp of 3'-UTR Immunoglobulin V regions, and 107 probes tareting the 5' 120bp of 3'-UTR TCR V regions.

***DNA isolation*** - CD3+ T cells were isolated by flow assisted cell sorting of PBMC populations separated from whole blood. Peripheral blood mononuclear cells (PBMC) were isolated from whole blood by centrifugation followed by DNA isolation with a Gentra Puregene kit (Qiagen) according to manufacturer protocol. In the case of fresh/frozen tissues, a Qiagen Allprep kit (Qiagen) was employed to extract DNA and RNA, according to the manufacturer's instructions. The whole blood plasma fraction was then treated with red blood cell lysis buffer and circulating DNA (cfDNA) was extracted using the Qiagen Nucleic Acid kit (Qiagen) according to manufacturer protocol.

***cDNA synthesis*** - mRNA was separated from isolated total RNA using the NEBNext Poly(A) mRNA Magnetic isolation Module (NEB) according to manufacturer's instructions. To generate cDNA, first NEBNext RNA First Strand Synthesis Module (NEB) was used followed by NEBNext RNA Second Strand Synthesis Module (NEB) according to manufacturer's instructions.

***Library preparation*** - Isolated genomic DNA or synthesized cDNA was diluted in TE buffer to 130uL volumes. Shearing to ~275bp was then performed on either a Covaris M220 Focused-ultrasonicator or E220 Focused-ultrasonicator, depending on sample throughput, with the following settings: for a sample volume of 130 µL and desired peak length of 200 bp, Peak Incident Power was set to 175 W; duty factor was set to 10%; cycles per burst was set to 200; treatment time was set to 180 s. in addition, temperature and water levels were carefully held to manufacturer's recommendations given the instrument in use.

Illumina DNA libraries were generated from 100 - 1000 ng of fragmented DNA using the KAPA HyperPrep Kit (Sigma) library preparation kit following manufacturer's protocol version 5.16 employing NEXTFlex sequencing library adapters (BIOO Scientific). Library fragment size distribution was determined using the Agilent TapeStation D1000 kit and quantified by fluorometry using the Invitrogen Qubit.

***Hybrid capture*** - For cDNA derived libraries, hybridization was performed with a pooled panel of probes targeting V and J loci in equimolar concentrations. For genomic DNA derived libraries, hybridization and capture was performed iteratively with probes specifically targeting V loci, 3'-UTR sequences, or J loci under standard SeqCap (Roche) conditions with xGen blocking oligos (IDT) and human Cot-1 blocking DNA (Invitrogen). Hybridization is performed at 50C overnight. The Capture process consisting of bead incubations and washes are performed at 50C.

For the iterative hybridization and capture process, the first J hybridization and capture is performed in completion with terminal PCR amplification with 4 steps. Following clean-up by Agencourt AMPure XP SPRI bead purification (Beckman) this product is used as input for a subsequent depletion step. For depletion, a modified and truncated SeqCap protocol is employed wherein following incubation of the hybridization mixture with M-270 streptavidin linked magnetic beads (Invitrogen), the 15uL hybridization reaction is separated on a magnetic rack, the supernatant is recovered and diluted to 100uL with TE buffer, followed by clean up by standard Agencourt AMPure XP SPRI bead purification (Beckman). The depletion-probe-target-beads are discarded. The purified supernatant is then used as input for a subsequent V-panel capture and hybridization as described above, but with terminal PCR amplification with 16 or amplifications steps to achieve sufficient library for sequencing.

***Capture Analysis*** - A custom Bash/Python/R pipeline was employed for analysis of paired read sequencing data generated by Illumina NextSeq 2500 instrument from the hybrid-capture products. First, 150 bp paired reads were merged using PEAR 0.9.6 with a 25bp overlap parameter. This results in a single 275 bp sequence for each sequenced fragment. Next, specific V, J, and D genes within the fragment sequence were identified by aligning regions against a reference sequence database. Specifically, individual BLAST databases were created using all annotated V, D, J gene segments retrieved from the IMGT / LIGM-DB website (www.imgt.org), as these full-length gene sequences were the source of probes used to design the hybrid-capture probe panel. Individual merged reads are iteratively aligned using BLASTn with an e value cut-off of 1 to the V database, J database then D database with word size of 5 for D segment queries. Trimming of identified V or J segments in the query sequence is performed prior to subsequent alignment. From reads containing V and J sequences, we identified V/J junction position and the antigen specificity determining Complementarity Determining Region 3 (CDR3) sequences. In order to identify CDR3 sequences, the V/J junction position is extracted from the previous search data for those fragments containing both a V and J search result. 80bp of DNA sequence flanking this junction is translated to amino acid sequence in all six open reading frames and sequences lacking stop codons are searched for invariable anchor residues using regular expressions specific for each TR class as determined by sequence alignments of polyclonal hybrid-captured data from rearranged TR polypeptides annotated by IMGT.

### Results and Discussion

### Methods improvement

We experimented with alternate capture methods, using an iterative three-step hybridization and capture, first with a J panel then molecular depletion of unrearranged V-gene sequences, then subsequently with a V panel (Figure 12). The depletion probes (V-gene and J-gene) are shown in Table D. These altered protocols improved recovery of unique CDR3 sequences when normalized to reads. When compared to a one-step V-panel capture, the one-step combined VJ-panel capture increased signal by 6.84x, the two-step J and V iterative capture increased signal by 12x (no significant difference was observed for J-V or V-J iterative order), and the three-step J-depletion-V iterative capture increased signal by 31.2x (Figure 13).

We experimented with reducing hybridization and wash temperatures to improve recovery (Figure 14). When 50C to 65C in 5C increments were tested at each step of the hybridization and capture, 50C yielded the highest signal and diversity.

We determined the best method for depletion (Figure 15). We found that direct reuse of the hybridization mixture following bead-probe-target separation yielded reduced signal than setting up a new reaction following Agencourt XP bead purification of the supernatant. We also found that direct separation rather than separation of the hybridization following addition of wash buffer yielded increased signal.

We tested whether depletion should be preceded by a V or J capture (Figure 16). We found that direct depletion of the library, followed by V or J capture yielded reduced signal compared to either V-Depletion-J or J-Depletion-V, both of which had increased, yet similar yields.

### Input source material comparisons

To determine whether we could characterize the TCR repertoire from both low and high signal samples, we performed a series of dilution curves for CD3+ genomic DNA (Figure 17), PBMC genomic DNA (Figure 18), and PBMC derived cDNA (Figure 19). Less input actually yielded a higher amount of diversity when normalized for input and reads suggesting that high input libraries are being undersequenced or that probes are being saturated and leaving behind less preferable, but still on-target, targets. Additionally, we observed yields for the cDNA samples to be ~100x that of genomic DNA reflecting enrichment of the TCR signal as a consequence of the high level of transcript expression of the rearranged TCR gene relative to other genes. In contrast, signal from genomic DNA is a related to the fraction of the complete genome of the target sequence and capture efficiency.

Since each sequenced sample represents only a snapshot of the TCR repertoire with the extent dependent on the amount of input material and the complexity of the source repertoire, we were interested in whether the method could assay complete VJ or CDR3 saturation of a patient. We looked at unique VJ pair recovery across multiple samples derived from a single patient blood draw (Figure 20). Beta locus VJ saturation was achieved with fewer than ten runs. With sufficient input and sequencing depth, VJ saturation could be achieved in a single run. We also looked at CDR3 saturation across these same samples and were able to achieve approximately 50% beta locus saturation (Figure 21). This level could be achieved with fewer samples by using cDNA libraries as input with deeper sequencing.

We looked at whether the genomic DNA and cDNA samples were recapitulating the same VJ combinations at the beta locus (Figure 22). This was largely the case with only two discordant VJ pairs showing greater (<3% overall) change.

We looked at whether the genomic DNA and cDNA samples were recapitulating the same CDR3 sequences (Figure 23). For the most prevalent 1000 CDR3 sequences detected from genomic DNA, their correlation with cDNA prevalences had an r squared value of 0.67. Many had similar prevalences however a large number had very low or zero prevalence values in cDNA. This is likely explained by the second group consisting of non-productive rearrangements that are encoded on the alternate chromosome and which are not expressed.

### Investigation of samples from adoptive cell transfer immunotherapy

We next applied the CapTCR-Seq methodology to samples derived from expanded Tumor Infiltrating Lymphocyte (TIL) infusion populations and PBMCs from serial blood draws from patients undergoing adoptive cell transfer immunotherapy. We wanted to track clones from the TIL culture over time to determine whether they successfully colonized the patient and the extent of their population over time (Figure 24). Repertoire profiling reveals a polyclonal and diverse baseline repertoire before treatment, a less complex oligoclonal TIL derived culture, less complex oligoclonal repertoires following chemodepletion and transfusion of the TIL infusion, and finally restoration of a more complex polyclonal repertoire over time. When compared to the baseline, highly prevalent clones in the TIL infusion product persist over time albeit in decreasing amounts. The dominant rearrangements decrease in prevalence over time as the native repertoire is reestablished however the TIL product rearrangements persist. We can observe this persistence by graphing the individual profiles for these top nine rearrangements over time (Figure 25). We can see that while they decrease over time, they remain higher than what was found in the apheresis sample after two years.

### Comparison between uncaptured and captured tumor samples

We wished to demonstrate the value of this method for interrogating existing cDNA RNA-Seq libraries (Figure 26). To do this, Illumina cDNA sequencing libraries were generated from FFPE-derived total RNA and subjected to sequencing followed by analysis using the TCR annotation pipeline to identify unique TCR CDR3 sequences (bulk unique CDR3). Residual library then underwent CapTCR-Seq to identify unique TCR CDR3 sequences (capture unique CDR3). The CapTCR-Seq method yielded a greatly increased number of unique CDR3 sequences (mean: 466 fold, median: 353 fold). When normalized to number of total reads sequenced, we observed a 15fold increase in signal per read sequenced (mean:15.2, median:14.5, n=41).

### Investigation of tumor repertoires from different cancer types

We next wanted to characterize tumor repertoires and investigate highly prevalent TIL clones in the blood repertoire before and during anti-PDL1 immunotherapy treatment. We selected five patients, each with a different tumor type: Patient A: Head and neck; Patient B: Breast; Patient C: Ovarian; Patient D: Melanoma; Patient E: Cervical. Each patient had three sample types: Tumor tissue (extracted DNA and RNA), pre-treatment blood (extracted PBMC DNA, PBMC RNA, and plasma cfDNA), on-treatment blood (extracted plasma cfDNA).

We first queried the extent of the TCR signal in the tumor samples in terms of infiltration and clonality. TCR signal is defined as the total number of counts of fragments containing both a V and J gene region (non-unique, reads normalized) while diversity is defined as the total number of unique CDR3 sequences detected (unique, reads normalized). Overall, diversity increased with signal (Figure 27). cfDNA samples had the lowest signal, genomic DNA samples had intermediate signal, while cDNA samples had the highest signal. Blood sample signal and diversity is similar for all five patients, however tumor signal and diversity varied. Two patients had ten-fold higher TCR signal and diversity in their tumors likely reflecting increased infiltration of immune cells (Figure 28).

Next we assessed the clonality of the tumor sample TIL repertoire. Tumors with clonal infiltration have a larger than expected population of one or more VJ rearrangements, the population of which are significantly greater than the next most prevalent clone. Patient A appears to have a large alpha rearrangement population in its tumor compared to baseline blood, while the most prevalent beta rearrangement is only slightly enriched (Figure 29-30). The tumor sample for patient B showed both greatly enriched top alpha and beta VJ rearrangements compared to baseline blood (Figure 31-32). The tumor sample for patient C showed both greatly enriched top alpha and beta VJ rearrangements compared to baseline blood (Figure 33-34). The tumor sample for patient D showed both greatly enriched top alpha (2) and beta VJ (1) rearrangements compared to baseline blood (Figure 35-36). The tumor sample for patient E showed only a slightly enriched top beta VJ rearrangement compared to baseline blood (Figure 37-38).

Next we assessed how the most prevalent tumor VJ rearrangements differed in terms of prevalence across the other patient samples (Figures 39-43). In general, prevalent TIL clones were not prevalent in the blood repertoire demonstrating clonal expansion within the tumor or selective infiltration. However, for a number of the most prevalent TIL clones, we saw very high levels within the plasma samples suggesting that while these clones are actively undergoing cell death. In combination with their high tumor infiltration, this suggests that these are anti-tumor T-cells undergoing active expansion, anti-tumor cytotoxicity and turnover.

### EXAMPLE 4

We performed similar experiments relating to B-cells. Our design targets more than 500 V-regions and 50 J-regions within the IGH, IGK and IGL loci annotated in the IMmunoGeneTics database. This accounts for all known Ig alleles while maximizing depth of coverage in selected regions. A blast-based informatics pipeline calls V(D)J recombinations and an algorithm combining information from large-insert and soft-clipped reads are used to predict candidate rearrangements which are manually verified in Integrated Genome Viewer.

Candidate V(D)J rearrangements and translocations detected through this approach have been validated in three well-characterized cell-lines with publically available whole genome data; an additional 67 MM cell lines have been annotated for V(D)J rearrangements and translocations into IGH, IGL and IGK genes. The limit of detection was established with a cell-line dilution series. We were also able to translate these techniques to cell-free DNA. These methods are applicable to the detection of MRD in mature B-cell malignancies and immunoglobulin repertoire profiling in a many clinical scenarios including cellular immunotherapy and therapeutics with immunomodulatory effects. V(D)J and complex rearrangement annotations in 70 MM cell-lines are highly relevant in further in-vitro studies.

The B-cell V-gene and J-gene capture probes used are shown in Tables B1 and B2 respectively.

### Reference List

### References

1. Bertness V, Kirsch I, Hollis G, Johnson B, Bunn PA Jr. T-cell receptor gene rearrangements as clinical markers of human T-cell lymphomas. N Engl J Med. 1985 Aug 29;313(9):534-8.
2. Swerdlow SH, Cancer IA for R on, Organization WH. WHO classification of tumours of haematopoietic and lymphoid tissues [Internet]. International Agency for Research on Cancer; 2008. Available from: http://books.google.ca/books?id=WqsTAQAAMAAJ
3. van Dongen JJ, Wolvers-Tettero IL. Analysis of immunoglobulin and T cell receptor genes. Part I: Basic and technical aspects. Clin Chim Acta. 1991 Apr;198(1-2):1-91.
4. Aisenberg AC. Utility of gene rearrangements in lymphoid malignancies. Annu Rev Med. 1993;44:75-84.
5. Rezuke WN, Abernathy EC, Tsongalis GJ. Molecular diagnosis of B- and T-cell lymphomas: fundamental principles and clinical applications. Clin Chem. 1997 Oct;43(10):1814-23.
6. Armitage JO. The aggressive peripheral T-cell lymphomas: 2012 update on diagnosis, risk stratification, and management. Am J Hematol. 2012 May;87(5):511-9.
7. Abouyabis AN, Shenoy PJ, Lechowicz MJ, Flowers CR. Incidence and outcomes of the peripheral T-cell lymphoma subtypes in the United States. Leuk Lymphoma. 2008 Nov;49(11):2099-107.
8. Criscione VD, Weinstock MA. Incidence of cutaneous T-cell lymphoma in the United States, 1973-2002. Arch Dermatol. 2007 Jul;143(7):854-9.
9. Ko OB, Lee DH, Kim SW, Lee JS, Kim S, Huh J, et al. Clinicopathologic characteristics of T-cell non-Hodgkin's lymphoma: a single institution experience. Korean J Intern Med. 2009 Jun;24(2):128-34.
10. Luminari S, Cesaretti M, Rashid I, Mammi C, Montanini A, Barbolini E, et al. Incidence, clinical characteristics and survival of malignant lymphomas: a population-based study from a cancer registry in northern Italy. Hematol Oncol. 2007 Dec;25(4):189-97.
11. Vazquez A, Khan MN, Blake DM, Sanghvi S, Baredes S, Eloy JA. Extranodal natural killer/T-Cell lymphoma: A population-based comparison of sinonasal and extranasal disease. Laryngoscope. 2014 Apr;124(4):888-95.
12. Liao JB, Chuang SS, Chen HC, Tseng HH, Wang JS, Hsieh PP. Clinicopathologic analysis of cutaneous lymphoma in taiwan: a high frequency of extranodal natural killer/t-cell lymphoma, nasal type, with an extremely poor prognosis. Arch Pathol Lab Med. 2010 Jul;134(7):996-1002.
13. Mitarnun W, Suwiwat S, Pradutkanchana J. Epstein-Barr virus-associated extranodal non-Hodgkin's lymphoma of the sinonasal tract and nasopharynx in Thailand. Asian Pac J Cancer Prev Apjcp. 2006 Jan;7(1):91-4.
14. Shih LY, Liang DC. Non-Hodgkin's lymphomas in Asia. Hematol - Oncol Clin N Am. 1991 Oct;5(5):983-1001.
15. Ai WZ, Chang ET, Fish K, Fu K, Weisenburger DD, Keegan TH. Racial patterns of extranodal natural killer/T-cell lymphoma, nasal type, in California: a population-based study. Br J Haematol. 2012 Mar;156(5):626-32.
16. Korgavkar K, Xiong M, Weinstock M. Changing incidence trends of cutaneous T-cell lymphoma. JAMA Dermatol. 2013 Nov;149(11):1295-9.
17. Weinstock MA. Epidemiology of mycosis fungoides. Semin Dermatol. 1994 Sep;13(3):154-9.
18. Weiss LM, Arber DA, Strickler JG. Nasal T-cell lymphoma. Ann Oncol. 1994;5 Suppl 1:39-42.
19. Zackheim HS, Vonderheid EC, Ramsay DL, LeBoit PE, Rothfleisch J, Kashani-Sabet M. Relative frequency of various forms of primary cutaneous lymphomas. J Am Acad Dermatol. 2000 Nov;43(5 Pt 1):793-6.
20. United Nations D of E and SA Population Division. International Migration Report 2009: A Global Assessment. United Nations, New York; 2011.
21. Cossman J, Uppenkamp M, Andrade R, Medeiros LJ. T-cell receptor gene rearrangements and the diagnosis of human T-cell neoplasms. Crit Rev Oncol-Hematol. 1990;10(3):267-81.
22. Vantourout P, Hayday A. Six-of-the-best: unique contributions of gammadelta T cells to immunology. Nat Rev Immunol. 2013 Feb;13(2):88-100.
23. Lefranc MP. TRA (T cell receptor alpha). Atlas Genet Cytogenet Oncol Haematol. 2003;7(4):245-8.
24. Lefranc MP. TRD (T cell receptor delta). Atlas Genet Cytogenet Oncol Haematol. 2003;7(4):252-4.
25. Lefranc MP. TRB (T cell receptor beta). Atlas Genet Cytogenet Oncol Haematol. 2003;7(4):249-51.
26. Lefranc MP. TRG (T cell receptor gamma). Atlas Genet Cytogenet Oncol Haematol. 2003;7(4):255-6.
27. Bolotin DA, Mamedov IZ, Britanova OV, Zvyagin IV, Shagin D, Ustyugova SV, et al. Next generation sequencing for TCR repertoire profiling: platform-specific features and correction algorithms. Eur J Immunol. 2012 Nov;42(11):3073-83.
28. Linnemann C, Heemskerk B, Kvistborg P, Kluin RJ, Bolotin DA, Chen X, et al. High-throughput identification of antigen-specific TCRs by TCR gene capture. Nat Med. 2013 Nov; 19(11): 1534-41.
29. van Dongen JJ, Langerak AW, Bruggemann M, Evans PA, Hummel M, Lavender FL, et al. Design and standardization of PCR primers and protocols for detection of clonal immunoglobulin and T-cell receptor gene recombinations in suspect lymphoproliferations: report of the BIOMED-2 Concerted Action BMH4-CT98-3936. Leukemia. 2003 Dec;17(12):2257-317.
30. Amagai M, Hayakawa K, Amagai N, Kobayashi K, Onodera Y, Shimizu N, et al. T cell receptor gene rearrangement analysis in mycosis fungoides and disseminated lymphocytoma cutis. Dermatologica. 1990;181(3):193-6.
31. Dosaka N, Tanaka T, Fujita M, Miyachi Y, Horio T, Imamura S. Southern blot analysis of clonal rearrangements of T-cell receptor gene in plaque lesion of mycosis fungoides. J Invest Dermatol. 1989 Nov;93(5):626-9.
32. Chan DW, Liang R, Chan V, Kwong YL, Chan TK. Detection of T-cell receptor delta gene rearrangement by clonal specific polymerase chain reaction. Leukemia. 1997 Apr;11 Suppl 3:281-4.
33. Lynch JW Jr, Linoilla I, Sausville EA, Steinberg SM, Ghosh BC, Nguyen DT, et al. Prognostic implications of evaluation for lymph node involvement by T-cell antigen receptor gene rearrangement in mycosis fungoides. Blood. 1992 Jun 15;79(12):3293-9.
34. McClure RF, Kaur P, Pagel E, Ouillette PD, Holtegaard CE, Treptow CL, et al. Validation of immunoglobulin gene rearrangement detection by PCR using commercially available BIOMED-2 primers. Leukemia. 2006 Jan;20(1):176-9.
35. Bagg A, Braziel RM, Arber DA, Bijwaard KE, Chu AY. Immunoglobulin heavy chain gene analysis in lymphomas: a multi-center study demonstrating the heterogeneity of performance of polymerase chain reaction assays. J Mol Diagn. 2002 May;4(2):81-9.
36. Cushman-Vokoun AM, Connealy S, Greiner TC. Assay design affects the interpretation of T-cell receptor gamma gene rearrangements: comparison of the performance of a one-tube assay with the BIOMED-2-based TCRG gene clonality assay. J Mol Diagn. 2010 Nov;12(6):787-96.
37. Groenen PJ, Langerak AW, van Dongen JJ, van Krieken JH. Pitfalls in TCR gene clonality testing: teaching cases. J Hematop. 2008 Sep;1(2):97-109.
38. Mamanova L, Coffey AJ, Scott CE, Kozarewa I, Turner EH, Kumar A, et al. Target-enrichment strategies for next-generation sequencing. Nat Methods. 2010 Feb;7(2):111-8.
39. Bossler AVDV. Chapter 4: Conventional and Real-Time Polymerase Chain Reaction. In: Tubbs RR. S M, editor. Cell and Tissue Based Molecular Pathology. Churchill Livingstone Elsevier; 2009. p. 33-49.
40. Rhodenizer D daSilva C; Skinner N; Hegde, M. One library, many tests: The evolution of Next Generation Sequencing panel testing. In 2014.
41. Bowen DC M; Kautzer, C; Landers, T; Mehta, G; Olivares. Improved Performance of Solution-based Target Enrichment with Spike-in of Individually Synthesized Capture DNA Probes. In 2012.
42. Jarosz MZ Z; Lipson D; Frampton, G; Yalensky, R; Parker A; Cronin, M. High Performance Solution-Based Target Selection Using Individually Synthesized Oligonucleotide Capture Probes. In 2011.
43. Shi WC C; Tang, T; Hipolito, L; Srinivasan, P; Chiang, D; Pend, D; Di Tomaso, E; Tangri, S; Lameh, J; Pollner, R. Development of a Clinical Targeted Next-Generation Sequencing (NGS) Test for Formalin-Fixed Paraffin-Embedded (FFPE) Cancer Samples. In 2014.
44. Schmidt RL, Factor RE. Understanding sources of bias in diagnostic accuracy studies. Arch Pathol Lab Med. 2013 Apr;137(4):558-65.
45. Tomaszewski JE, Bear HD, Connally JA, Epstein JI, Feldman M, Foucar K, et al. Consensus conference on second opinions in diagnostic anatomic pathology. Who, What, and When. Am J Clin Pathol. 2000 Sep;114(3):329-35.
46. Naaktgeboren CA, Bertens LC, van Smeden M, de Groot JA, Moons KG, Reitsma JB. Value of composite reference standards in diagnostic research. BMJ. 2013;347:f5605.
47. Duncavage EJ, Magrini V, Becker N, Armstrong JR, Demeter RT, Wylie T, et al. Hybrid capture and next-generation sequencing identify viral integration sites from formalin-fixed, paraffin-embedded tissue. J Mol Diagn. 2011 May;13(3):325-33.
48. Gnirke A, Melnikov A, Maguire J, Rogov P, LeProust EM, Brockman W, et al. Solution hybrid selection with ultra-long oligonucleotides for massively parallel targeted sequencing. Nat Biotechnol. 2009 Feb;27(2):182-9.
49. Gilbert MT, Haselkorn T, Bunce M, Sanchez JJ, Lucas SB, Jewell LD, et al. The isolation of nucleic acids from fixed, paraffin-embedded tissues-which methods are useful when? PLoS One. 2007;2(6):e537.
50. Bolotin DA, Poslavsky S, Mitrophanov I, Shugay M, Mamedov IZ, Putintseva EV, et al. MiXCR: software for comprehensive adaptive immunity profiling. Nat Methods. 2015 Apr 29; 12(5):380-1.
51. Li S, Lefranc M-P, Miles JJ, Alamyar E, Giudicelli V, Duroux P, et al. IMGT/HighV QUEST paradigm for T cell receptor IMGT clonotype diversity and next generation repertoire immunoprofiling. Nat Commun [Internet]. 2013 Sep 2 [cited 2016 Jan 30];4. Available from: http://www.nature.com/doifinder/10.1038/ncomms3333
52. Zhang J, Kobert K, Flouri T, Stamatakis A. PEAR: a fast and accurate Illumina Paired-End read mergeR. Bioinforma Oxf Engl. 2014 Mar 1;30(5):614-20.
53. Larkin MA, Blackshields G, Brown NP, Chenna R, McGettigan PA, McWilliam. H, et al. Clustal W and Clustal X version 2.0. Bioinformatics. 2007 Nov 1;23(21):2947-8.
54. Giudicelli V, Chaume D, Lefranc MP. IMGT/GENE-DB: a comprehensive database for human and mouse immunoglobulin and T cell receptor genes. Nucleic Acids Res. 2005 Jan 1;33(Database issue):D256-61.
55. Li HD R. Fast and accurate short read alignment with Burrows-Wheeler Transform. Bioinformatics. 2009;25:1754-60.
56. Brochet X, Lefranc MP, Giudicelli V. IMGT/V-QUEST: the highly customized and integrated system for IG and TR standardized V-J and V-D-J sequence analysis. Nucleic Acids Res. 2008 Jul 1;36(Web Server issue):W503-8.
57. Giudicelli V, Lefranc MP. IMGT/junctionanalysis: IMGT standardized analysis of the V-J and V-D-J junctions of the rearranged immunoglobulins (IG) and T cell receptors (TR). Cold Spring Harb Protoc. 2011 Jun;2011(6):716-25.
58. Giudicelli V, Brochet X, Lefranc MP. IMGT/V-QUEST: IMGT standardized analysis of the immunoglobulin (IG) and T cell receptor (TR) nucleotide sequences. Cold Spring Harb Protoc. 2011 Jun;2011(6):695-715.
59. Yousfi Monod M, Giudicelli V, Chaume D, Lefranc MP. IMGT/JunctionAnalysis: the first tool for the analysis of the immunoglobulin and T cell receptor complex V-J and V-D-J JUNCTIONs. Bioinformatics. 2004 Aug 4;20 Suppl 1:i379-85.
60. Smith TF, Waterman MS. identification of common molecular subsequences. J Mol Biol. 1981 Mar 25;147(1):195-7.
61. Krzywinski M, Schein J, Birol I, Connors J, Gascoyne R, Horsman D, et al. Circos: an information aesthetic for comparative genomics. Genome Res. 2009 Sep;19(9):1639-45.
62. Lefranc MP. Unique database numbering system for immunogenetic analysis. Immunol Today. 1997 Nov;18(11):509.
63. Lefranc MP, Pommie C, Ruiz M, Giudicelli V, Foulquier E, Truong L, et al. IMGT unique numbering for immunoglobulin and T cell receptor variable domains and Ig superfamily V-like domains. Dev Comp Immunol. 2003 Jan;27(1):55-77.
64. Altschul S, Erickson B. Optimal sequence alignment using affine gap costs. Bull Math Biol. 1986 Sep 1;48(5-6):603-16.
65. Lefranc MP. IMGT-ONTOLOGY and IMGT databases, tools and Web resources for immunogenetics and immunoinformatics. Mol Immunol. 2004 Jan;40(10):647-60.
66. Lefranc MP. IMGT databases, web resources and tools for immunoglobulin and T cell receptor sequence analysis, http://imgt.cines.fr. Leukemia. 2003 Jan;17(1):260-6.
67. Sandberg Y, Verhaaf B, van Gastel-Mol EJ, Wolvers-Tettero IL, de Vos J, Macleod RA, et al. Human T-cell lines with well-defined T-cell receptor gene rearrangements as controls for the BIOMED-2 multiplex polymerase chain reaction tubes. Leukemia. 2007 Feb;21(2):230-7.
68. Ye J, Coulouris G, Zaretskaya I, Cutcutache I, Rozen S, Madden TL. Primer-BLAST: a tool to design target-specific primers for polymerase chain reaction. BMC Bioinformatics. 2012;13:134.
69. Kent WJ. S C.W.; Furey, T.S.; Roskin, K.M.; Pringle, T.H.; Zahler, A.M.; Haussler, D. The human genome browser at UCSC. Genome Res. 2002 Jun;12(6):996-1006.
70. Malde K. The effect of sequence quality on sequence alignment. Bioinformatics. 2008 Apr 1;24(7):897-900.
71. Davidson JN, Leslie I, White JC. Quantitative studies on the content of nucleic acids in normal and leukaemic cells, from blood and bone marrow. J Pathol Bacteriol. 1951 Jul;63(3):471-83.
72. Glen AC. Measurement of DNA and RNA in human peripheral blood lymphocytes. Clin Chem. 1967 Apr;13(4):299-313.
73. Metais P, Mandel P. [Percentage of desoxypentosenucleic acid in leucocytes in normal and pathological conditions]. C R Seances Soc Biol Fil. 1950 Feb;144(3-4):277-9.
74. Jones SR, Carley S, Harrison M. An introduction to power and sample size estimation. Emerg Med J. 2003 Sep;20(5):453-8.
75. Network NCC. NCCN Clinical Practice Guidelines in Oncology. National Comprehensive Cancer Network, Inc.; 2014.
76. Jaffe ES, Organization WH. Pathology and Genetics of Tumours of Haematopoietic and Lymphoid Tissues [Internet]. IARC Press; 2001. Available from: http://books.google.ca/books?id=XSKqcy7TUZUC
77. Gazzola A, Mannu C, Rossi M, Laginestra MA, Sapienza MR, Fuligni F, et al. The evolution of clonality testing in the diagnosis and monitoring of hematological malignancies. Ther Adv Hematol. 2014 Apr 1;5(2):35-47.
78. Tape T. Interpreting Diagnostic Tests [Internet]. University of Nebraska Medical Center; [cited 2015 Nov 8]. Available from: http://gim.unmc.edu/dxtests/Default.htm
79. Hu PC, Hegde MR, Lennon PA, editors. Modern clinical molecular techniques. New York: Springer; 2012. 436 p.
80. Brunet J-P, Tamayo P, Golub TR, Mesirov JP. Metagenes and molecular pattern discovery using matrix factorization. Proc Natl Acad Sci USA. 2004 Mar 23;101(12):4164-9.
81. Tembhare P, Yuan CM, Xi L, Morris JC, Liewehr D, Venzon D, et al. Flow cytometric immunophenotypic assessment of T-cell clonality by Vβ repertoire analysis: detection of T-cell clonality at diagnosis and monitoring of minimal residual disease following therapy. Am J Clin Pathol. 2011 Jun;135(6):890-900.
82. Sufficool KE, Lockwood CM, Abel HJ, Hagemann IS, Schumacher JA, Kelley TW, et al. T-cell clonality assessment by next-generation sequencing improves detection sensitivity in mycosis fungoides. J Am Acad Dermatol. 2015 Aug;73(2):228-36.e2.
83. Cazzaniga G, Biondi A. Molecular monitoring of childhood acute lymphoblastic leukemia using antigen receptor gene rearrangements and quantitative polymerase chain reaction technology. Haematologica. 2005 Mar;90(3):382-90.
84. Lima M, Almeida J, Santos AH, dos Anjos Teixeira M, Alguero MC, Queirós ML, et al. Immunophenotypic analysis of the TCR-Vbeta repertoire in 98 persistent expansions of CD3(+)/TCR-alphabeta(+) large granular lymphocytes: utility in assessing clonality and insights into the pathogenesis of the disease. Am J Pathol. 2001 Nov;159(5):1861-8.
85. Miles JJ, Douek DC, Price DA. Bias in the αβ T-cell repertoire: implications for disease pathogenesis and vaccination. Immunol Cell Biol. 2011 Mar;89(3):375-87.
86. Society CC. Non-Hodgkin Lymphoma Statistics [Internet]. Cancer Information. 2014. Available from: http://www.cancer.ca/en/cancer-information/cancer-type/non-hodgkin-lymphoma/statistics/?region=on
87. Canada S. Population by year, by province and territory [Internet]. 2014 Sep. Available from: www.statcan.gc.ca/tables-tableaux/sum-som/l01/cst01/demo02a-end.htm
88. Information Cl for H. DAD Abstracting Manual, 2012-2013 Edition [Internet]. 2012 Apr. Available from: http://sda.chass.utoronto.ca.myaccess.library.utoronto.ca/sdaweb/cihi/2011to2013/clin/more_doc/ DAD Abstracting_Manual 2012-2013_E.pdf
89. Information Cl for H. CIHI Specifications Form for Research Analytical Files [Internet]. 2014 Feb. Available from: http://sda.chass.utoronto.ca.myaccess.library.utoronto.ca/sdaweb/cihi/2011to2013/clin/more_doc/ Specifications-DAD-RAF-EN.pdf
   A1. van Dongen, J. J. M. et al. Design and standardization of PCR primers and protocols for detection of clonal immunoglobulin and T-cell receptor gene recombinations in suspect lymphoproliferations: Report of the BIOMED-2 Concerted Action BMH4-CT98-3936. Leukemia 17, 2257-2317 (2003).
   A2. Langerak, A. W. et al. EuroClonality/BIOMED-2 guidelines for interpretation and reporting of Ig/TCR clonality testing in suspected lymphoproliferations. Leukemia 26, 2159-2171 (2012).
   A3. Han, A., Glanville, J., Hansmann, L. & Davis, M. M. Linking T-cell receptor sequence to functional phenotype at the single-cell level. Nat Biotech 32, 684-692 (2014).
   A4. Stubbington, M. J. T. et al. T cell fate and clonality inference from single-cell transcriptomes. Nat Meth 13, 329-332 (2016).
   A5. Samorodnitsky, E. et al. Evaluation of Hybridization Capture Versus Amplicon-Based Methods for Whole-Exome Sequencing. Human Mutation 36, 903-914 (2015).
   A6. Mamanova, L. et al. Target-enrichment strategies for next-generation sequencing. Nat. Methods 7, 111-118 (2010).
   A7. Bodi, K. et al. Comparison of Commercially Available Target Enrichment Methods for Next-Generation Sequencing. J Biomol Tech 24, 73-86 (2013).
   A8. Mertes, F. et al. Targeted enrichment of genomic DNA regions for next-generation sequencing. Briefings in Functional Genomics 10, 374-386 (2011).
   A9. Giudicelli, V. et al. IMGT/LIGM-DB, the IMGT comprehensive database of immunoglobulin and T cell receptor nucleotide sequences. Nucleic Acids Res. 34, D781-784 (2006).
   A10. Bolotin, D. A. et al. MiTCR: software for T-cell receptor sequencing data analysis. Nat Meth 10, 813-814 (2013).
   A11. Bolotin, D. A. et al. MiXCR: software for comprehensive adaptive immunity profiling. Nat Meth 12, 380-381 (2015).
   A12. Brochet, X., Lefranc, M.-P. & Giudicelli, V. IMGT/V-QUEST: the highly customized and integrated system for IG and TR standardized V-J and V-D-J sequence analysis. Nucleic Acids Res. 36, W503-508 (2008).
   A13. Thomas, N., Heather, J., Ndifon, W., Shawe-Taylor, J. & Chain, B. Decombinator: a tool for fast, efficient gene assignment in T-cell receptor sequences using a finite state machine. Bioinformatics 29, 542-550 (2013).
   A14. Yu, Y., Ceredig, R. & Seoighe, C. LymAnalyzer: a tool for comprehensive analysis of next generation sequencing data of T cell receptors and immunoglobulins. Nucl. Acids Res. gkv1016 (2015). doi:10.1093/nar/gkv1016
   A15. Zhang, W. et al. IMonitor: A Robust Pipeline for TCR and BCR Repertoire Analysis. Genetics 201, 459-472 (2015).
   A16. Calis, J. J. A. & Rosenberg, B. R. Characterizing immune repertoires by high throughput sequencing: strategies and applications. Trends Immunol 35, 581-590 (2014).
   A17. Sandberg, Y. et al. Human T-cell lines with well-defined T-cell receptor gene rearrangements as controls for the BIOMED-2 multiplex polymerase chain reaction tubes. Leukemia 21, 230-237 (2007).
   A18. Zhang, J., Kobert, K., Flouri, T. & Stamatakis, A. PEAR: a fast and accurate Illumina Paired-End reAd mergeR. Bioinformatics 30, 614-620 (2014).
   A19. Camacho, C. et al. BLAST+: architecture and applications. BMC Bioinformatics 10, 421 (2009).
   B1. Rosenberg, S.A., and Restifo, N.P. (2015). Adoptive cell transfer as personalized immunotherapy for human cancer. Science 348, 62-68.
   B2. Hadrup, S., Donia, M., and thor Straten, P. (2013). Effector CD4 and CD8 T Cells and Their Role in the Tumor Microenvironment. Cancer Microenvironment 6, 123-133.
   B3. Attaf, M., Huseby, E., and Sewell, A.K. (2015). αβ T cell receptors as predictors of health and disease. Cell. Mol. Immunol. 12, 391-399.
   B4. Gubin, M.M., Artyomov, M.N., Mardis, E.R., and Schreiber, R.D. (2015). Tumor neoantigens: building a framework for personalized cancer immunotherapy. Journal of Clinical Investigation 125, 3413-3421.
   B5. Clemente, M.J., Przychodzen, B., Jerez, A., Dienes, B.E., Afable, M.G., Husseinzadeh, H., Rajala, H.L.M., Wlodarski, M.W., Mustjoki, S., and Maciejewski, J.P. (2013). Deep sequencing of the T-cell receptor repertoire in CD8+ T-large granular lymphocyte leukemia identifies signature landscapes. Blood 122, 4077-4085.
   B6. Topalian, S.L., Drake, C.G., and Pardoll, D.M. (2015). Immune checkpoint blockade: a common denominator approach to cancer therapy. Cancer Cell 27, 450-461.
   B7. Novosiadly, R., and Kalos, M. (2016). High-content molecular profiling of T-cell therapy in oncology. Molecular Therapy - Oncolytics 3, 16009.
   B8. Abbey, J.L., and O'Neill, H.C. (2007). Expression of T-cell receptor genes during early T-cell development. Immunol Cell Biol 86, 166-174.
   B9. Emerson, R.O., Sherwood, A.M., Rieder, M.J., Guenthoer, J., Williamson, D.W., Carlson, C.S., Drescher, C.W., Tewari, M., Bielas, J.H., and Robins, H.S. (2013). High-throughput sequencing of T-cell receptors reveals a homogeneous repertoire of tumour-infiltrating lymphocytes in ovarian cancer. J. Pathol. 231, 433-440.
   B10. Gerlinger, M., Quezada, S.A., Peggs, K.S., Furness, A.J.S., Fisher, R., Marafioti, T., Shende, V.H., McGranahan, N., Rowan, A.J., Hazell, S., et al. (2013). Ultra-deep T cell receptor sequencing reveals the complexity and intratumour heterogeneity of T cell clones in renal cell carcinomas. J. Pathol. 231, 424-432.
   B11. Restifo, N.P., Dudley, M.E., and Rosenberg, S.A. (2012). Adoptive immunotherapy for cancer: harnessing the T cell response. Nat. Rev. Immunol. 12, 269-281.
   B12. Silva-Santos, B., Serre, K., and Norell, H. (2015). γδ T cells in cancer. Nat Rev Immunol 15, 683-691.
   B13. Tscharke, D.C., Croft, N.P., Doherty, P.C., and La Gruta, N.L. (2015). Sizing up the key determinants of the CD8(+) T cell response. Nat. Rev. Immunol. 15, 705-716.
   B14. Wherry, E.J., and Kurachi, M. (2015). Molecular and cellular insights into T cell exhaustion. Nat Rev Immunol 15, 486-499.

### List of Abbreviations

| | |
|---|---|
| ATCC | American Type Culture Collection (Biorepository) |
| AUC | Area-under-the-curve |
| bp | basepair |
| BWA | Burrows-Wheeler Alignment algorithm |
| CIHI | Canadian Institutes for Health Information |
| D | T-cell receptor "diversity" type gene |
| DAD | Discharge Abstracts Database (CIHI database) |
| DSMZ | Deutsche Sammlung von Mikroorganismen und Zellkulturen GmbH (German Collection of Microorganisms and Cell Cultures) |
| FDR | False-Discovery Rare |
| FFPE | Formalin-fixed paraffin-embedded |
| ICD-10 | International classification of disease, version 10 |
| IG | Immunoglobulin |
| IMGT | The International standard source for ImMunoGeneTics sequences & metadata |
| J | T-cell receptor gene "join" type gene |
| kb | kilobase |
| LGL | Large-Granular-Lymphocyte (Leukemia/Lymphoma) |
| NGS | Next-generation sequencing (technology) |
| NMF | Non-negative Matrix Factorization |
| NTRA | Novel NGS-based T-cell receptor gene re-arrangement assay |
| PEAR | Paired-end rEAd mergeR |
| PTCL | Peripheral T-cell lymphoma |
| ROC | Receiver-Operating Characteristic (Curve) |
| SAM | Sequence Alignment Map |
| SEER | Surveillance, epidemiology, and end results program (the primary US source of Cancer Statistics) |
| SWA | Smith-Waterman Alignment (algorithm) |
| TGH | Toronto General Hospital |
| TLPD | T-cell lymphoproliferative disorder |
| TR | T-cell receptor |
| TRA | T-cell receptor alpha gene |
| TRB | T-cell receptor beta gene |
| TRD | T-cell receptor delta gene |
| TRG | T-cell receptor gamma gene |
| TRGR | T-cell receptor gene re-arrangement |
| V | T-cell receptor "variable" type gene |
| WHO | World Health Organization |

**Table D**

| **SEQ ID NO** | **Name** | **Sequence** |
|---|---|---|
| SEQ ID NO:1 | IGKV1/OR2-3*01 | cccagtgcgacaagtcataacatcaaccgctaggatagcagatgagtgaggccgggttgccctagatgctcctcctggtgcctcaatctgctgagttgttttccagatgcag ccaagttt |
| SEQ ID NO:2 | IGKV1-22*01 | cctagagtgttacaggtcataaaataaacccccagggaagcagaagtatgactcatggctgccccaggtgcttccactggtgcctccatctgctgagagtgtttctcaggtg cagccaag |
| SEQ ID NO:3 | IGKV1-27*01 | cactgtgatacaagcccgaacataaaccatggagggaagtagatgtgtgaggctgggctgccccagctgctcctcctggtgccgccctctgctgacagcagttctcagatg cagccaagg |
| SEQ ID NO:4 | IGKV1D-37*01 | cacagtgttacaaggcataacataaacccccaaggaagcagatgtatggggctggcctgccccagatactcctcctactgcctccagctgctcagagcgtttctcatattc cagtcaag |
| SEQ ID NO:5 | IGKV1-39*01 | cacagtgttacaagtcataacataaacctccaaggaagcagatgtgtgaggacgagccaccccagatgctcctcctggtgcctccatctgctgagagcatttctcaaactc agtcaggtt |
| SEQ ID NO:6 | IGKV1-35*01 | cacagtgttacaaaccataacaaaccccccccaggaaagcagacatgtgacgctgggctgccccacctgctcttctttgtgcagccatctggtgacaacacttctcagactc agcctgag |
| SEQ ID NO:7 | IGKV1-32*01 | cacagtgttacaaacccaataagctccccaaggaagcagatatgtgagggtgggctgccccagctgcttctcctgtttcctccatctgctgagagtgtttctcagactcagcc acactct |
| SEQ ID NO:8 | IGHV7-81*01 | caccatgtggaaacccacatcctgagagtgtcagaaatcctgatgtgggaggcagctgtgctgagctgaggcagtgatgcagcagtttccttaacttccatcttatctcattt tgcatcg |
| SEQ ID NO:9 | IGHV1-14*01 | cacagtgtgaaaacccacatcctgagagagtcagaaatcctgagggaggtggcagcagtgctaggcttgagagatgacagggattttatttgctttaaaggctttttttaga aagcgagg |
| SEQ ID NO:10 | IGHV1-69*01 | gacacagtgtgaaaacccacatcctgagagtgtcagaaaccctgagggagaaggcagctgtgccgggctgaggagatgacagggtttattaggtttaaggctgtttacaa aatgggttat |
| SEQ ID NO:11 | IGHV1-67*01 | cacagtgtgaaaactcatatcctgagagtgtcagtaaccctgagggaggaagcagctgtcccagttttcaggatatgacaggatttatgggtttaatgttgtttagaaaat aggttata |
| SEQ ID NO:12 | IGHV1/OR21-1*01 | cacaatgtgaaaacccacatcttgagagtttcagaaactgcagggaggaggcagctgtgttcctgcagaggagatgacagggaagatgaggtttaaagttgtttagaaaa tgggtcaagt |
| SEQ ID NO:13 | IGHV1/OR15-3*03 | gacacagagtgaaaacccacatcctgagagtgtcagaaaccccaaggaggagcagctgtactggagctgaggaaatggacaaagattattcagattgaagactttctac gaaaatgactt |
| SEQ ID NO:14 | IGHV1-3*02 | cacagtgtgaaaacccacatcctgagagtgtcagaaaccccaggggggaagcagctgtgctggcatggaggaaatgacaaagattattagattgaagactttctcagaa aatgatattaa |
| SEQ ID NO:15 | IGHV1-17*01 | gacacagtgcgaaaacccacatcctgagagtgtcagaaaccccaggaaggaggcacctgtgctgacacagagggagatgacaaagattattagattaacgattttctta ga |
| SEQ ID NO:16 | IGHV1-17*02 | cacagtgcgaaaacccacatcctgagagtgtcagaaaccccaggaaggaggcacctgtgctgacacagaggagatgacaaagattattagattaaagattttcttagaa aatgacactaa |
| SEQ ID NO:17 | IGHV1-38-4*01 | cacagtgtgaaaacccacatcctgagagtgtcagaaagcctgaggaaggaggcagctgtgctggggctgaggagatgacagggattacttgattgaagactttcttagaa aacgaggtta |
| SEQ ID NO:18 | IGHV5-51*01 | cacagtgtgaaaacccacatcctgagagtgtcagaaagcctgaggaaggaggcagctgtgctggggctgaggagatgacagggattacttgattgaagactttcttagaa aacgaggtta cacagtgagagaaaccagccccgagcccgtctaaaaccctccacaccgcaggtgcagaatgagctgctagagactcactccccaggggcctctctattcatctgggagg aaacactggc |
| SEQ ID NO:19 | IGHV5-78*01 | gaccatctaaaccttccgcggtgcaggtgcagagtgagctgccagacacaccctccccaggggcctctctattcatccggggaggaaacactggctgtttgtgtcctcagg agcaaaaa |
| SEQ ID NO:20 | IGHV3-50*01 | gcgaataatggagaacttgagatatggagtgtgagtggatatgagtgaaaaacagtgattctgtgtggcaggttctgactcagatgtctctgtgcttgtaggtgtctagtgt ggggtgc |
| SEQ ID NO:21 | IGHV(III)-76-1*01 | cacaggagatatccgtgtggcaacctaacacaggggacacctgtatttgtgtctgagcccagacacaaacctccctgcagggagacaggaggggaccgtgtgacagaca ctgctcagaac |
| SEQ ID NO:22 | IGHV3-30-22*01 | ccaagtgagagctgaggacatggctgtgcatggctgtacataaggtcccaagtgagcaaacatcggtgtgagtccagacacaacacttcctgcaaaaacaagaaaggag tctgggccgaa |
| SEQ ID NO:23 | IGHV(III)-22-2 | acaagagtcagaaaagtgtgcaggaggccgggtgaggctgtagacactgtcagcccactatgccaatcccaccacgagtgctggagaaggtgggagtctgatgaagctt actaacaaacc |
| SEQ ID NO:24 | IGHV(II)-44-1D*01 | gccgagattgcgccactgcactcagcctgggcgacagagcgagacttcgtctcaaaaaacaaaaaaaaaatcaatcattggaatactgttgttcattacaattaatga acgtttgata |
| SEQ ID NO:25 | IGLV(IV)-64*01 | cacaggtggggaagtgggacaaaatctcagcctgctcagagtcttgttctctgatgaaatttagatcttaaaataacttatatcacttgtgtgggatgagtgagatatcccga gctcaca |
| SEQ ID NO:26 | IGHV(II)-23-2*01 | cacagcgaggggaagccattgtgcgctcagaacactctacaaattttcctccctagtgttttaccaaaactggtatatatttcagatactgaaatatttacaa |
| SEQ ID NO:27 | IGLV(VI)-22-1*01 | agtaagaccaaaaccctcctgagattcctggcttgtgtcctgacactggggctgttgggattcctgtctttccttcaagattgttcaaataagcaccgacaatcacttccatgt gagata |
| SEQ ID NO:28 | IGLV(V)-58*01 | [?] aaggcaaagtgaccccagtgaatgaggaagcaggacaaaaactgttttctgctccactatgaaggctgccacgtggccctgagaaacagtgcctgttttccttactactc aagaaaga |
| SEQ ID NO:29 | IGLV(V)-66*01 | ccgtttgggtaaagcacagataaatggggaaatgaggcaaaaactgtttttctactctgctaccaaggttgaaaaatggctctcagaaccagtgtctgctgacctgcatacc caaatatg |
| SEQ ID NO:30 | IGHV(II)-20-3*01 | taaaataaaataaaatgtaaaaaatgatcaataaatgaaattactatcagttgaaactcattaaatttaaagacattttctactcaagtaactataagaacatgaatgtca agtttcaga |
| SEQ ID NO:31 | IGLV(IV)-59*01 | cacaggcagatgagaaagtgagacgaaactcagcctactaagaatggaactatggctctttttccaattgtcaaataattttcacatacacaaactattttggaagtagcta ctgattca |
| SEQ ID NO:32 | IGLV7-46*02 | cacagtgacagacccatgagaggaaccaagacataaacctccctcggcccttgtgatgtggagatcacatgatcagacatgccagatcccaagatagcctacatgtggac cagccataga |
| SEQ ID NO:33 | IGLV8-61*01 | cacagtgatttaaacctatgaggaagtgcaactaaaacctctttatatactgagaacagttcagcccttacagacaggagggaaagtgagagggtggaaatggtcaacac ggtgagtgag |
| SEQ ID NO:34 | IG LV8/O RS-1*01 | tttaaacccatgaggaggtgcaactaaaacctctttacatactcagaaagattcagcccttagaagcaagagagaagttgagagggtggaatgtcaacaccatgagctg ggaacctcct |
| SEQ ID NO:35 | IGLV(I)-56*01 | ttctctgattatctggatgctctgtgactccttctgtgcatctctgggatcatcattcagactcacctgcaccctgagcagtaacatcaatgttgtttgctatgacatttactgga aaca |
| SEQ ID NO:36 | IGKV3-31*01 | cacagtgattccacaggaaaccaaacctccacaagacagctggtgtttttcctcaagccttctgtttacttatgggaagctactatggtggctgcttagttattgagagaaa acaatgg |
| SEQ ID NO:37 | IGKV3-34*01 | cacagtaattcaacatgaaacaaaaactttcacaaaaccattgatttttttttctaaaaccagcagctttatgggctgcagctatgatggctgctcagttttagcaactgtgc ctctat |
| SEQ ID NO:38 | IGKV3D-25*01 | catactgattcaacatgcaacaaaaacctccaggagacctaaggtgtttatttgattataccacctgcttcctttttagtcatctgatgtggtgctgctcagttttagcatctctg cttt |
| SEQ ID NO:39 | IGKV3-11*01 | cacagtgattccacatgaaacaaaaccccaacaagaccatcagtgtttactagattattataccagctgcttcctttacagacagctagtggggtggccactcagtgttag catctcag |
| SEQ ID NO:40 | IGHV2-70*13 | cacagagacacagcccagggcgcttcctgtacaagaacccaggtgtttttcagtggtgctccctccccacttctgcagaacaggatagtgtggctgagatgccatttcctgc ccagggcg |
| SEQ ID NO:41 | IGHV2-70D*04 | cacagagacacagcccagggcgcctcctgtacaagaacccaggctgcttctcagtggtgctccctccccacctctgcagaacaggatagtgtggctgagatgccatttcctg ccagggcc |
| SEQ ID NO:42 | IGLV(VI)-25-1*01 | agtaagaccaaaaccctcctgagattcctgacttgtgtcctgacaccaggtctgttcttccctcccctagaataaaacatctcttaagcacaaggctgaagaaatgtggcctc ctcccttt |
| SEQ ID NO:43 | IGHV(II)-22-1*01 | cacagcgaggggaagccattgtgcgctcagaacactctacaaattttcctccctagtgttttaccaaaactggtatatatttcagatactgaaatatttacaacctacgttatt atgcta |
| SEQ ID NO:44 | IGHV(II)-30-31*01 | caaacaaaacgacacaaaaaattccaaagttgtgcaccctctaaaagcatatgtacttaattctcatttttaatttattaaacagctctaataagttcaatgttcctgccttct cagttg |
| SEQ ID NO:45 | IGKV2D-36*01 | aaaacttgaacttccatcaatgataaatattccttttgcctcaagcacatatttgaggaattttccattgagtagatctaccgataaggtcacatttttctgtctgttttaatctg aata |
| SEQ ID NO:46 | IGHV1-12*02 | tagttatttgagagatttttcatacaacatttattctgtaagcaaatttcagggattgttgaatgaatcatattaacaaatctgacacagaacttcctctgaatcaatctttgta aacat |
| SEQ ID NO:47 | IGHV(II)-44-2D*01 | tgcctggccgtaagttaccatgtgctttttaaaaaatcatagcaaaggggtgtcttctggaaatgacattttgaaatggtgttattagaccacccctggaagggacacagt aaccacac |
| SEQ ID NO:48 | IGHV(II)-74-1 | agtgatggtgggggtcctactagcctgtggcaaatggaagcatctctttttatcagactgaataatattgtagtgttttcttataccacatttacttcatccctttgtgcattaa cact |
| SEQ ID NO:49 | IGHV(II)-46-1*01 | aaaatccattgctagtggtggtgggagtccatttgtcttgtggaaaatggcagcatttccttattttataaggcataataatgctatgttgtgtacacataccacattgtcttta tccat |
| SEQ ID NO:50 | IGHV(II)-67-1*01 | aaaatgcatggctagtgctgctggaaacccattcctactgtggcaaatggcagcatctcttttaaaaggctaaataatattctattctgtatacataccacattgccattatcc tttttg |
| SEQ ID NO:51 | IGHV(11)-23-1*01 | atagatggataaactaacctaggcctttgaaaataaacccttatctgagagtgaaaagataagccatagatttggagagtttgcttgcaaatcaaatatttggaaaaggac ttttattac |
| SEQ ID NO:52 | IGHV(II)-40-1*01 | taggcactggatggaaagcacaggagtgggtcaggtgcatacgtgatgagtggaggatgaattccagcccacttatcatgaattcagacaagcccacatgttcccacatg cactatatct |
| SEQ ID NO:53 | IGHV(IV)-44-1*01 | cactgtgactcgaatccagagtgaactcagacacaaacctgccctgcaggggttcttgggaccacaaggggaaggatcaggtcaccagggtgtacttaggaaccactgaa ctgggtcagg |
| SEQ ID NO:54 | IGHV(II)-28-1*02 | cgcaatgaagggccttcattgtgagcctagacacaaccctccctgcaggggtgaataggagcagcaggggggcattcggggcagtatgggggcttaggatgattgttaggg gtcaggatga |
| SEQ ID NO:55 | IGHV(II)-30-41*01 | cattgtgagcctagacacaaccctccctgcaggggtgaataggagcagcagggggcattcggggcagtatgggggcttaggatgattgttaggggtcaggatgagcagg atcaaggcttc |
| SEQ ID NO:56 | IGHV(II)-65-1*01 | cacaacgaggggaagtcattgtgagcccagatacaaacctccctgcaggggagctcagaaagagcaggaggcactcaggacaccagggaacactctggacacatcaag gcaggtgcaatg |
| SEQ ID NO:57 | IGHV(II)-51-2*01 | aacagaagagatgtcagtgtgatcccagacacaaacttccctggagaggggcccaggaccaccaaagagcactcaggcccatgaaaacagggcccaagctggagaac gggtttcctgtca |
| SEQ ID NO:58 | GHV(II)-15-1*01 | cacagaaggggaggtcattgtgaggccagacacaaacctccctgcagggaagctcaggacaccaggggggtgctcagacaccaaggctctcaggacacatcaaggcag gtgcaagagggg |
| SEQ ID NO:59 | IGHV6-1*01 | cacagtgaggggaagtcagtgtgagcccagacacaaacctccctgcagggatgctcaggaccccagaaggcacccagcactaccagcgcagggcccagaccaggagc aggtgtggagtta |
| SEQ ID NO:60 | IGHV(II)-60-1*01 | cagagtgaggggaccacggtgcgagctcacacccaaaccttcctggaggggtgcaggacagcaggagtcccgatgatggaagggggtggtctggattccaggtcact ctcaagatcat |
| SEQ ID NO:61 | IGHV(II)-53-1*01 | cacagtaaggtaaccacagtgggaactcacacccaaacctccctgtgggggtgcacaggacagccacagttactcaggaccccaggattcctcaggacaccaaggggca ctcaaggccat |
| SEQ ID NO:62 | IGHV(II)-20-1*01 | cacagtgaggggacatcagtgtgagcccagacacaaacctccctatgcgggttcacaggacagcatggggtgctgaggacagaggtgggcactcaggaaccagcaggg aaacccaggggg |
| SEQ ID NO:63 | IGHV3-41*01 | agtgagaggaagtccgtgtgagcccagacacaaacctccctgcaggggcacgcggggccaccagagggtgcccaggatcccctgaagacagggacagcccaaaggca ggtgcagatggat |
| SEQ ID NO:64 | IGHV3-52*01 | cacagtgaggggaggtcagtgtgagcccagacacaaacctcctgcaggggcatctggagccacaaggggcgctcaggatacacagaggacaggggcagccccaggg caggtgcaggtgg |
| SEQ ID NO:65 | IGHV3-73*02 | cacagtgaggggaggtcagtgtgagcccggacacaaacctccctgcaggggcgcgcggggctaccaggggggcgctcgggactcactgaggcgggacaggtcccagga acaggtgcagcg |
| SEQ ID NO:66 | IGHV3-42*03 | cagtgagggggaggttaacgtaggcccatacacaaatctccctgcaggggcgcgcagggccaactgggggcgctcgggacccactgaggatgggacaggtcccagggg cgggtgcagggg |
| SEQ ID NO:67 | IGHV3-6*01 | tacggtaaggagaagtcagtgtgagcccagacacaaacctcccttcagggtacctgggacaaccagggaaagcctgggacactgtgcactgtgctgaccccaggggcaa gtgcaggtgct |
| SEQ ID NO:68 | IGHV3/OR16-9*01 | cacagagtgaggggaagtcagtgagagcccaggcacaaacctccctgaaggggtcccagaaacgactaggggcgccaggacactgtgcacggggctgtctccagggc aggtgcaggtgc |
| SEQ ID NO:69 | IGHV(II)-44-2*01 | aacagtgagaggaagtcaatgtgagtccagacataaaccttcctgctgagaacaatggaaagcttttcttctaagataaggaataagaaaagaatgcccagtcttaataa ttctaatcag |
| SEQ ID NO:70 | IGHV3-25*02 | cacagtgaggggaggtcagtgtgagcccagacacaaacctccctgcagggccatgcgggtggtttcctttctcagctgcaggaggcgggcttattgttgcaggactctgga gacttatta |
| SEQ ID NO:71 | IGHV(II)-26-2*01 | ctcagtgaggaggtgtccttatgagccctgacacaaacctgtcagggcacttaggacctccaggaagactcaagaccaccaaggggactcacgaccactggggaagggc aggttgcagta |
| SEQ ID NO:72 | IGHV(III)-67-3*01 | cacagcgagggacatttctgtgagtccagacagaaacctccctgcagggagacaagagaggactttgtgataaatggtgcttaggacaccaggggcactcaggacagc agagggtgctc |
| SEQ ID NO:73 | IGHV(III)-47-1 | cacggtgaggggacatctgtgtgagctcagacacaaacctgcctgcagggagacacaaacctccctgcatggtagatgcttctcagaaccaccaggggggtgcacaggaa accagaaggtg |
| SEQ ID NO:74 | IGHV(III)-82*01 | cataggagcaggaacatctgcgtgagcccagacacaaaatcctctgcagggagacaggagggaatcgcatggtagatgctgattggaactaccatgtgtcgctcagaac taccaggaggt |
| SEQ ID NO:75 | IGHV(III)-67-4*01 | cacaggagagagattatctgcacaagcccagacacaaaatctgcagggagacaggagggaactgcatggtagatgctgctcagaagcaccagggggcactcaacac aagggggcgctca |
| SEQ ID NO:76 | IGHV(III)-16-1*01 | agacacaggagagggaatatctgcgtgagcccagacagaaaatctctgcaggaagacaggagggagctgcatggtagatgctcctcagaaccaccagggcaccttgg ggacaacctggg |
| SEQ ID NO:77 | IGHV3-57*02 | cacaggagagggaatatctgtgtgagcccagacacaaaaatctctgcagagagacaggagggaactgcatggtagatgctcctcataaccacaaaggggcagtcagga ccatcaggagga |
| SEQ ID NO:78 | IGHV(III)-5-1*01 | cacatgaggaaaggccggtgtgagacacaaacctccaggaacacctgggctaatgagctgcagggggcgctcaggacccactgatcagtcaaccacagaggggagtgc aaaggttaggac |
| SEQ ID NO:79 | IGHV3-63*01 | ccaagtgaggaaacatcggtgtgagtccagacacaaaatttcctgcagaaagaagaaaggattctgggccgaaggggacactcagcactcacaaaacaggtggagccc cagggcaggtac |
| SEQ ID NO:80 | IGHV3 54*01 | gtcaccaggtaagaagacatcagtgtgatcacagacacagaatttcctgaaataagggaggagtctgggctaaagggcactcaggacccacagaaaacagcggaagc tctagggc |
| SEQ ID NO:81 | IGHV3-54*04 | caccaggtaagaagacatcagtgtgaacacagacacagaatttcctgaaataagggaggagtctgggctaaaagggcactcaggacccacagaaaacaggggaagct ctagggcaggtgc |
| SEQ ID NO:82 | IGHV3-79*01 | agaagacatcagtgtgaacacagacacagaggttcctgtaatgataagggaggaggctgggataaagggagcactcaagacccacagaaaacaggggaagctctagg gcaggtgcagacg |
| SEQ ID NO:83 | IGHV3-30-33*01 | caccaggtaagaagacatcagtgtgaacacagacacagagtttcctgcaatgataagggaggaggctgggctaaaggggcactcaggacccactgaaaacgggcagc tctagggcaggt |
| SEQ ID NO:84 | IGHV3-30-2*01 | ccaggtaagaagacatcagtgtgaacacagacacagtttcctgcaatgataagggaggaggctgggctaaaggggcactcaggacccactgaaaacgggcagctcta gggcaggtacag |
| SEQ ID NO:85 | IGHV3-9*01 | cacagtgaggggaagtcagcgagagcccagacaaaaacctcctgcaggaagacaggaggggcctgggctgcagagggcactcaagacacactgaaaacacggttaac actgggacaagtt |
| SEQ ID NO:86 | IGHV(III)-51-1*01 | catcgtgatgggaagtccacgtgggctcagagacagactgccatgcaggacacaggggggtggcttggctgaagggggcactcagcacccacagaagacaggagcagcc cagggcaggggc |
| SEQ ID NO:87 | IGHV3 62*01 | cgcagtgagaagtcagtgtgagcccagacacaaacctcctgcagggtacctgggacaatcagggaaagcctgggacactgtatactggctgtccccaggggcaagtcc aggtgatataa |
| SEQ ID NO:88 | IGHV3-19*01 | cactgtgagaggacggaagtgtgagcccagacacaaacctcctgcaggaacgttgggggaaatcagctgcaggggcgctcaagacccactcatcagagtcaacccca gagcaggtgcac |
| SEQ ID NO:89 | IGHV3-76*01 | cacagtgaggagaagtcagtgtgagcccagtcacaaacctcctacaggaacgctgggaggaaaatcagctacagggctcactcaaggcccactgatcagagtccactcc agagggaggtt |
| SEQ ID NO:90 | IGHV3-37*01 | catggtgaggggaaatcagtatgagcccagccagaaacctccctgcaggaaccctggggtgggggaaatcagctgcagggggcactcaggacccactgatcagaatc aaccccagaagg |
| SEQ ID NO:91 | IGHV3-23D*01 | cacagtgaggggaagtcattgtgagcccagacacaaacctccctgcaggaacgatgggggtgaaatcagcggcagggggcgctcaggacccgctgatcagagtcatccg cagaggcaggt |
| SEQ ID NO:92 | IGHV3-53*02 | cacagtgaggggaggccattgtgcgcccagacacaaacctccctgcaggaacgctggggaaatcagcggcagggggcgctcaggagccactgatcagagtcagccccg gaggcaggtgca |
| SEQ ID NO:93 | IGHV4-39*07 | cacagtgaggggaggtgagtgtgagcccagacaaaacctccctgcagggaggctgagggcgcggtcgcaggtgcagctcagggccagcagggggcgcgcggagctc acggaatacaagg |
| SEQ ID NO:94 | IGHV4-55*02 | tacacagtgaggggaggtgagtgtgagcccagacacaaacctccctacagataggcagagggggcgggcacaggtgctgctcaggaccaacagggggcgcgcgaggc acagagcccgagg |
| SEQ ID NO:95 | IGLV11-55*01 | cacagtgagacagatgaggaagtcggacaaaaaccaaggttttaagcttgtcatttttactgaactggttaagaacttcagtggttaataaaatcacattaaatacaggatt gttgttaa |
| SEQ ID NO:96 | IGLV(IV)-53*01 | cactgtgctctaggccaatgggaaaatcccctctgcttgtgctgcctgggctcccactaggcccctgctgtttgtgacaacagccagcactggtggtgacgcttcagccatgt atgccct |
| SEQ ID NO:97 | IGKV6D-41*01 | cactgtgctacaacccaaaacaaaaattagctcagcctggcggaacagagaaactgaacaataccccgtttttatgatccttgcaggtgcagttggggaaataatttacca aataccatc |
| SEQ ID NO:98 | IGKV7-3*01 | cacagtgctttaggtctaaacaaaaacctccccaggcagctgctccctgaggctcaaatccctcagatgtggctttttatgcaggtccatcagcctgctgtcataggcttgttt gaacaa |
| SEQ ID NO:99 | IGKV2-23*01 | cacaatggttcagcaccaaacaaaagcctcctgcttggattgtcccagctgcccaaattagttccttcactgaggagtagacagggtatatgctctaaatctatgtaacagg aagatgtt |
| SEQ ID NO:100 | IGKV2-18*01 | cacagtggtacaaccctgaacagaaacctcccttcttgctgtggttcagctgcccaaatgtgttgtttatctggaaagcagacactgtctattatcttgggagagtaaagaga ggaagat |
| SEQ ID NO:101 | IGKV2-4*01 | cacagtggtaaaccctgaacacaaacctccctacttgggatggcccagccatccacaagtgtttgcacgtggactgtctgcatggcagattctgagttggcttcacaggta gatgttag |
| SEQ ID NO:102 | IGKV2/OR22-4*01 | cacagtgctacatcctcgaacagaaacctccctgctggttgacccagctcgcgcatgggctgcttgtctgagggaacagctgagcagagtctttgagtctgcagaggagaa ggctgttgg |
| SEQ ID N0:103 | IGKV4-1*01 | cacagtgcttcagcctcgaacacaaacctcctccccatacgctgggccagtaggtctttgctgcagcagctgcttcctctgcacacagcccccaacatgcatgcttcctctgt gtgttgg |
| SEQ ID NO:104 | IGHV4-80*01 | gggaggcggagggggcgggcgcaggtgccgctcaggaccagcagggggcgcggggcccacagagcaggaggccgggtcaggagcaggtgcagggagggcgggg cttcctcatctgctc |
| SEQ ID NO:105 | IGLV2-11*01 | ctcagcctcctcactcagggcacaggtgacacctccagggaaagggtcacaggggtctctgggctgatccttggtctcctgctcctcaggctcacctgggcccagcactgac tcactaga |
| SEQ ID NO:106 | IGLV(I)-70*01 | tgcccttggcctgtcccgaggctgatcactccatacttgcctatgacaaacaaagagggtgcctgtggctgatcgtacagtttaagcaagggaggaagtgagactcagcca caggcccct |
| SEQ ID NO:107 | IGLV(IV)-66-1*01 | cactgtgctccagacttacggggaagtgagattagaacctcccctgcattctctgccttgtgcaggcaacaatacactgtctgggaccgagtgtggctcatcagtagcag ctttgttg |
| SEQ ID NO:108 | IGLV5-52*01 | cacagtgctccagacccatgaggaagtaagacaaaaccctcccctctactctcctggtctagtgaaatcacccctgctggtggctctgaccaaatctagctcaggggtgac atctgttg |
| SEQ ID NO:109 | IGLV1-62*01 | tacagtgctccaggcttgcaggggagtgagacaagaaccccttcctcctttcccaggagggtgagtgcccagcagctactgcacaggcctggcctgtggcttctgcagttg ctgtttcc |
| SEQ ID NO:110 | IGLV6-57*01 | cacagtgctccagacccatggggaagtgagacagaaactccccagagcatctctacctgggccagtctcagcctgtctccaccagagagggtagctctcccatctctcctgt ctaagtgc |
| SEQ ID NO:111 | IGLV(I)-20*01 | caccgtggtccaagttcatggggaattgagacccaaacctgccctgggctctcagcctctcttgttctgaagatgcttcctcaccctgtgcaaggggcttcttgcagcactg ccttga |
| SEQ ID NO:112 | IGLV8/OR8-1*02 | tccacagtgatttaaacccatgaggaggtgcaactaaaacctctttacatactcagaaagattcagcccttagaagcaagagagaagttgagagggtgggaatgtcaaca ccatgagctg |
| SEQ ID NO:113 | IGLV3-17*01 | cacagtgacacagacagattggaaagtgagatctaaagaccttcactgtctgtatcaccctctttctccagccatagcaggactgagcagggctggcccgggtcacctgga tcgaagccc |
| SEQ ID NO:114 | IGLV3-26*01 | cactcatgggacagcagtgctactcacctcacaatgacacagacagattgggaagtgagatctaaagaccttcactgtctgtgtcaccctcttcctccagccatagcaggac tgtggaga |
| SEQ ID NO:115 | IGLV3-29*01 | cacagtgacagaggcagacaaggaagtaagacacagaccccttccccatctgtgctgctgtcgtcctccagcccggcaacactgtggacaaagccatgagcatgcatgac ccagttcacc |
| SEQ ID NO:116 | IGLV4-60*02 | cacagtgatacaggcagatgaggaagtgggacaaaatcctcaacctgctgaggctattgttcagtgacaatttttaattttaaaacattttctgtatgtaaaaaatctatctg gatgcat |
| SEQ ID NO:117 | IGLV10-54*02 | cacagtgcctcaggccagtggggaagtgagataaaaactcaagagctccctcggcctcactgaacaggcctcacagagcactgtttaaactggaccacccaaaagacaa gggatgcattc |
| SEQ ID NO:118 | IGLV10-67*01 | cacagcgcctcaggggaagtgagacgaaaactcaggagctcccctagcttcactcggtatgcgggggcgtcatagagcactgtttaaactaaaccaaaaatgacaaggg ctggtttccac |
| SEQ ID NO:119 | IGLV(I)-42*01 | aacagtgctgcagtctgggaaagtgagatgagaacacgccaggtctcctaggagcatgaccttccaatggcaccacccacaaccaggacacgctggtcttgttttaccatt tgtgtggat |
| SEQ ID NO:120 | IGLV2-28*01 | cacagtggacataagattgattctcaggctccaagtctggccagtgagcttctttgagactccctgggatcccagcagtgacactgatcactattgctgtcccacacatccca agtgatg |
| SEQ ID NO:121 | IGLV(IV)-65*01 | cagcactccagacccactgggaggttacaaaacctcttctgatctcctggcctggtgtagtcactcctgctggtggctctaataaagtctatctcactgggtgacttatat tttaga |
| SEQ ID NO:122 | IGLV(I)-63*01 | cacagtgctccgggttgaagtaagtcagaccaaaacacacagtgtgcccagccatgaagctctcccatgcacccccctactctgcagctaagtcaatgtgttctctcacttgtt tgtccta |
| SEQ ID NO:123 | IGLV(I)-68*01 | ggcagtacttcaggccagtggggaagtgggagaaaaagctgctgcccatccagcaatggagcttctctgtgcagccccccacttcttgggcaagtcagctgattaacgttgc ttttcattt |
| SEQ ID NO:124 | IGHV(II)-28-2*01 | acacctggcctcttcgtttttattcatatattccttcagcagccactatgtcttcccactgatttcttcagtttctgccttttcctttgaataaggctgttactcctgagggaagat gg |
| SEQ ID NO:125 | IGHV(II)-44-3*01 | ggcaggccaccaagtccagctaatttttgtatttttagtagacactgggtttcacaatattggtctggctggtctcaaactcctgatctcagcctcccaaagtgctgggattaa agccgt |
| SEQ ID NO:126 | IGHV3-36*01 | attgtgtgcatcccttgtttaggtacatgcagagatgctgctttggtgtgttcaggggctcctgttttggggacaccaattttggagtttgcagtatccttgagtccagtacgttc atgg |
| SEQ ID NO:127 | IGHV(III)-25-1*01 | atggtctcactgatatctttacttcttttatcacttttgttatgtaaatcacaatgaatagtgtattcctcatctattatacatttgttaagtctttttggtgtctttaaaaaaactg a |
| SEQ ID NO:128 | IGHV(III)-25-1*02 | cacaatgaatagtgtattcctcatctattatacatttgttaagtctttttggtgtctttaaaaaaactgataactttatagtatgtaatatccttaagtcctgaaagtgtttttg atg |
| SEQ ID NO:129 | IGHV(III)-11-1*01 | cttcatctattatacacttgttaagtctttttggcatcttttaaaaactggtaactttatcctgtgtaatatccctgttaagtcctaaaagtctttttgatgtctattttttcttaa |
| SEQ ID NO:130 | IGHV(III)-20-2*01 | tacctaaatgtgtgtgggggaagcagggggtgttattctgttgttctgtgttctctgagatgcatggattcaccatttactctgcctccattttggggaacacagttagaaaaa atgtca |
| SEQ ID NO:131 | IGHV(III)-44D*01 | tggttttcagcagttttaataagattcacctaaatgtgtgtgtgtgtcgaggggtgttatgctattgttctgtgttctctgagatgcatggattcaccgtttactctgtctccat ttt |
| SEQ ID NO:132 | TRGV1*01 | cacagtgattcagacactgaaaatctgcctgtggttgcttctggtacacaagatagaccagccaactctcatttcctgccctgaatttactgtattctgtacaaagagaaaca cagctta |
| SEQ ID NO:133 | TRGV4*01 | cacagtgattcagatccgccctacaccacactgaaaacctgccttgtggctgcttctggtacacaagatagagctgccccctctcatttcctgccaccaaatttaccgtgtgct gaacaa |
| SEQ ID NO:134 | TRGV9*01 | cacagcagcagacagtttgagccatcccattcaataaatgtttattgagtctttgtttataattacgaattgggaagccacagttaccaccagtgtgcttgtaaacagtttttaagataa |
| SEQ ID NO:135 | TRGVA*01 | cgcagccttgcatgctgccccagccctacacaaaggactcttcctcccgatccaacaaggccttgggcattttcacttactcttggtcccttgggtttccctgtggcatagaa gaaaaa |
| SEQ ID NO:136 | TRBV8-1*01 | caataatggcaatgtggcagtttccatacatatgtttgtgctagctttttattattatatagtaaacttctttgcctctttttatagttattgtcttgaaatatattttatctgatat a |
| SEQ ID NO:137 | TRBV22-1*01 | cacaatggaagcacaaccattgtctctgtgcggaaatgtgtcctcaccctacagcccccaccacatcctctagcttaatttttcatttttaatattttcttgagattttactat gtc |
| SEQ ID NO:138 | TRAV1-1*01 | cacagtgactatgaggcctccttaactgtgccaaaattcaaaagacaatcagtggagtacaggtgggcttgagaagttctagaacttcctgagtgtatctttgcttaccgtct aatttta |
| SEQ ID NO:139 | TRAV1-2*01 | cacggtgactatgaggcctctttagctgcaccaaaattcaaaggcaaccacagcagcgagaagctgtatttcctgagtgtatgcctgctgtgagttaagactggggacttt ggaaccag |
| SEQ ID NO:140 | TRAV8-5*01 | tcaggaccctgtgataattgtgttaactgcacaaattatagagcatgtgtgttcaaacaatatgaaatctgggcaccttgaaaaaagaacaggataacagcaatgttcagg gaataagag |
| SEQ ID NO:141 | TRBV21/OR9-2*01 | cacagtgccgaatgttagcccttcttagaacacaaactcattatggacccagctcaggaaataagtgtatgtcaggttggtacacactataataacagaaagccaacttga aagacaata |
| SEQ ID NO:142 | TRBV16*01 | cacaatgttaaatattagctaatcttaggacacagactcatcacggactcagctcaggaagcaggtggtatactaggttggaaggaaataacagaaactagagctagctt aagccaaagg |
| SEQ ID NO:143 | TRBV23-1*01 | cacagcactgaaatgtcagttcctcttagcacacaaacttgtcacagacccagctcaggaagcaggtgatgtattaggctggaagggagtaacagaaaataactggagcc agcttaagcc |
| SEQ ID NO:144 | TRAV40*01 | cactgtgttaaaagcacagtgggagctatacaaaaacctcaaaggctcagaggaagtatgtagtgaggctggaaaacccaggttgtagagccctgttctctctttcacaga cagtcctgt |
| SEQ ID NO:145 | TRDV3*01 | cactatgatgcaggtgcccaggaagtcataacacaaactcctggggcacagctcagcagagctgcctcttagggcaggtcatgtctgggacttggcatccttctcttagcca ttttgggt |
| SEQ ID NO:146 | TRAV2*01 | cacagaggcagggaacccatgaagagctgaacagaaacagagatcacagcctttgcaggaggcaaaacagagatgagcaataactttttcctccttaattcagtattacc caagcttttt |
| SEQ ID NO:147 | TRAV16*01 | cacagtagctggttttgcaaggaagcagaacacaaaccctttaaatacaggaaatatttctttgcaaactctctgtatggccacagcagggcattctttctccagaaattaat attgagt |
| SEQ ID NO:148 | TRAV8-7*01 | gactgtgcctgggactgcaggaggagctgaacacaaacttcctgagacactgaggttttcaggaactcaagggcacagcctgacctatttgtagcaaggtctctcatttgat gaaagtga |
| SEQ ID NO:149 | TRAV8-6*01 | cacagtgcctgagactgcaggagagctgaacacaaacctcctgagatgctgagactttctgtgactcaagaactcaacctgtggagctttcaagagggtcccttttttctgt gcccgttt |
| SEQ ID NO:150 | TRAV3*01 | cacactgataggggctgcagggggagcagaacacaaactcttgagtctggtaaagcccattttcttgaagtctttgttccttcacatgagaacggtgtgcttccaggatatgt cacttat |
| SEQ ID NO:151 | TRAV8-1*01 | cacagtgtctgggactgcaaagggagctgaacacaaacttcctaaggtgctagggagaataactgcctctgaaagattttggattctgtcacagtagaaaccatgatgtta gtattttta |
| SEQ ID NO:152 | TRAV18*01 | cagagtgggagggactgcagcgagagcccagcacaaaccctggggaacgcaggtggggcctgggtgtgagccgctttgggagatgaatgaatatggactcttgttcgct gggaccccaaa |
| SEQ ID NO:153 | TRAV9-1*01 | cacagtgacagggactgcaggggaagctgagcacaaactctgagcagcacgaggggcctggctgctgagtgtaagccactgtgatcccctctggttagggaccaggaac tactctactat |
| SEQ ID NO:154 | TRAV31*01 | cactgtgaagaacatgttagaagagccttacaaaaagatcggaactcaacctgaggcaattgcctattcccacattctcaggaaaaactcacaaaccttacccaggcattt gttagcagc |
| SEQ ID NO:155 | TRAV38-1*01 | cacaatgagatgagcagcagggagaggcttacagaaacctcagacctcagcatctgtgcaaaggtcacagggtgagagggaagtggtagggtaataggtatagaaaat cattgacttctc |
| SEQ ID NO:156 | TRAV19*01 | cacagtgagatgggtgcctgtgggagccctacaaaaacctcaacaagaggcagggctcctggggagagactctgtcacagacaggaagaagcaaggagggtctgtgtc agcacaggtggt |
| SEQ ID NO:157 | TRAV14/DV4 *01 | cacagtgacagaactgtcggagggaggtgtacaaaagccctggggacctgcttgagacctccacctgctggagaaccaaggcgggaaatcaacatcacagacaggaag tggcta |
| SEQ ID NO:158 | TRAV33*01 | cacagaagtagaaatgacagtggaagataaacaaaaaccttagcactccataaaggaagccacctgctcaggagcttagggaaaatacatgaagcacagacaggaag aaggcacattagt |
| SEQ ID NO:159 | TRDV1*01 | cacagtgtttgaagtgatagtaaaagcaaaacaaaaaccctagggctcaataagagaacccctctactccccatcctttgctacaggagccaatctgaaatgcacacctgc agatctcag |
| SEQ ID NO:160 | TRDV2*03 | caccctgctgcagctctacttctgagcagctcaaaaccactgaccaggcgcggtggctcacacctgtaatcccagcactttgggaggccgaggtgggtggatcacgaggt caggagatc |
| SEQ ID NO:161 | TRBV30*01 | cacactgagctgggtggggcagacatctgtgcaaaaaccccaccctctcctgagccctaaccatactccccaggggccttcacttagggactgggtggaggatatttgtaa gtaggtttc |
| SEQ ID NO:162 | TRBV20-1*01 | cacagcgccaggaggggatcagacaccgcggcaagaacccctgcagctgccctccgccccagcgggccccctgagtgctgagaggggaagcgtggagaatggaaaacc acagctttcctg |
| SEQ ID NO:163 | TRBV29/OR9-2*01 | cacagtgcagggcacagatcaaagatctaagcaagaacctcagctcccttctacccagctcccctcacatgaacctgagggccctgtcaaggtgggacagaagaggaaa ccacagctctt |
| SEQ ID NO:164 | TRBVB*01 | gccacacacactcaagatgccccagacaccctgcactccgatcttactcgttcctttactgttttcatcctaattgccctcttacacatttgaccacacatttttggtcttggtgg ttgt |
| SEQ ID NO:165 | TRGV10*02 | accatactagaactgttgaaacaacatgcacaaaatcccctcccagggtctgtgcccaccacatccttcccaacaggggcaaccacagccagtccccagctgggctccca gactcaggct |
| SEQ ID NO:166 | TRGVB*01 | [?] cacagcatcagtgccacactgtcccacacaacaacctctgttgggtctctgcccaaccacatccttcccatgggagcaaactctatggactcctagctgggctcccaccctca gccttgc |
| SEQ ID NO:167 | TRGV11*02 | cacagtgttagagttgtcaagataacctacacagaaactatctccgagtctgtgcctgtccacatccttctccatgtgggcaaccacagcggtttgctcagctgggtgcccag ccggagc |
| SEQ ID NO:168 | TRAV4*01 | ctcgtgggtgacacacagtgagacagatggcctgcacctgtgccgttttcctctgtggggtgggagtcacagcctagaaagaagtccaaaagtgctttctaaaattttatt ttcaaaa |
| SEQ ID NO:169 | TRAV26-1*01 | cacactgggacagatggggctgcacctgtgcaatatctccctggtggcaagtgaggaggagggtagcattcacctagagcaaaatgtcgataggagtcaaaaagtaaca agaaaagagga |
| SEQ ID NO:170 | TRAV26-2*01 | cacagtgggacagatggggctgcagctgtgcaatatctccctggtgatgaaagggaaggcatctaacgaggccactgcacaagaaggagcagaagtttaatagaggaa gaagaaaattta |
| SEQ ID NO:171 | TRBV10-1*01 | cacagtgctgcacagctgcctcctctgcacataaagggcagttagaatgactgaggttgcctgtgctcccaagtcccagccttcacaggagtcggagagccctggctagc ctgggggc |
| SEQ ID NO:172 | TRBV10-2*01 | cacagtgctgcacagctgcctcctctgcacggaaacggcagttagaaaaactgaggttgcctgtgcacccaagtctgggccccaccctgggacgtctcagccccccatag gagtcacag |
| SEQ ID NO:173 | TRBV10-3*01 | cacagtgctgcatggctgcctcctctgcacgtaaacagcagttagaaagactgaggttgctctgtgtctatccccacccttggaagtccaggcctccatagaagtcagag ggccctgg |
| SEQ ID NO:174 | TRBV28*01 | cacagcgcagcacagctgcatcctctctgcacaaaaagagcggacgtaagagagaaggggccctaactcagggctggtgctggctccgatggcacattcgtgctaaata gaaaaaaagcg |
| SEQ ID NO:175 | TRBV6-2*01 | cacagtgctgcacggctgtctcctctctgcacagaaaggcaagggaaggtgctgccctcctccgcagcacagattcagcgatgcccttggtcctagcaccgaaaactttgg agccccaat |
| SEQ ID NO:176 | TRBV6-4*01 | cacagtgctgcacagccatctcctctctgtacataaatgcaggggaggctctgccctcctccccgaccccagactcaaccatgtccttggcagagttctcagcactgggaat cttggaag |
| SEQ ID NO:177 | TRBV6-9*01 | cacagcgctgcaagcctgtctcctctgcacataaaggcacagaggctctgccctcctcccacccaagactcaaggatgccctgggcagagttctctgcaccaggaacctt ggaaccca |
| SEQ ID NO:178 | TRBV6-7*01 | cacagcgctgcaaggctgtctcctctctgcacataaaggcaagggaaggtgctgccctcctcccccacccaagactcaaggatgccctgtgcagagatctctgcaccagga accttggaa |
| SEQ ID NO:179 | TRBV6-5*01 | cacagcgctacaaggccgtctcctctctgcacataaaggcagggaggttctgccctcctcccccacccaagactcagggatgccctgggcagagatctctgcgccaggaac cttggaacc |
| SEQ ID NO:180 | TRBV19*02 | gccagtagtatagacacagtgaagcacggatgtcgcctctctgtgcataaatgtgcccagtcctgcttccccgaccaggtggcagggctcctctgcactctatgatggcagg |
| SEQ ID NO:181 | TRBV19*01 | cacagtgaagcacggatgtcgcctctctgtgcataaatgtgcccagtcctgcttccccgaccaggtgacagggctcctctgcactctatgatggcaggaaacgccactcagc cactaagc |
| SEQ ID NO:182 | TRBVA*01 | cacagcactgcacaggcatgtgctcacctcacaaaatggcagtctcaaagggaggagtgcccacccacaagaggctccaccctattctgagaaagaacttctttcagagg aggagagaat |
| SEQ ID NO:183 | TRBV26/OR9-2*01 | cacagcactgcatagctgccacatcctctccacataaaaaaggtgcataccaaagaggaaaagcctgccctcaaaattcctcaccgcaaataagagaagttacctcaca ggtattgaca |
| SEQ ID NO:184 | TRBV25/OR9-2*01 | cacagtgctacatagataccgacactctgcacagaaagggtcgcctctaaggtgaggacatcttgccttcagaaaccttatcttaaactacagaaacccctgcaaatcttcc cagactcc |
| SEQ ID NO:185 | TRBV27*01 | cacagtgttgcacagccagctgctctctgcacaaaaacagagggtagctgcaagaacaaggagactcctccttcaggagacccctcaccgaccaacaggataaacttcct ccatcatccc |
| SEQ ID NO:186 | TRBV8-2*01 | cgcagccctgcacagccagctgccctctgcacaaaaagggcagtcacaggctggaggtgggcactccttatggaagcccgtgtctcaaccagaagaaaaagctgcccttt ctgaagctct |
| SEQ ID NO:187 | TRBV24-1*01 | cacagtgcttcttggccacctgctctctacacagaaagacagacacatgggtgagttgtttgctctgaagggtacctggatgtgggttgtgggatgtggggtgtttagagctt tcagtgg |
| SEQ ID NO:188 | TRBV2*01 | cacagccttgcaaagacaactccagcctgtgcaaaatccctcacagagctgcctccctcccagccgccagctcccacttcctgcctaagaaaaggaagtctctggttgggtt tgttcttg |
| SEQ ID NO:189 | TRBV11-1*01 | cacagcgttgcagagactttctctcctgtgcacaaaactccagggctctctccgctctactcagctcacagcagcctttccttattcctcatcctctcagggaagaagtgagtt ttcaga |
| SEQ ID NO:190 | TRBV11-2*01 | cacagtgtagcagagacacttccctcctgtgcagaaaaccagaaaaccgcaggactctctcctctctactcagctcacagcagcctttccttattcctcatcctcccaaggaa gaagtga |
| SEQ ID NO:191 | TRBV11-3*01 | cacagtgtagcagagacacttccctcctgtgcagaaaaccgcaggactctctcctctctactcagctcacagcagcctttccttattcctcatcctcccaggaaagaagtgag ttttcag |
| SEQ ID NO:192 | TRBV15*01 | cacagagctgcagtgcttcctgctctctgttcataaacctcattgtttcccagatccaggtgctttctctaggacttctccctcaccacctcttacaacaataggaagtgggttg |
| | | gtggc |
| SEQ ID NO:193 | TRBV12-5*01 | cacagcgctgcagaatcacctgctccctgtgcagaaaccctggtgcttcctcttcctccagtacccagcagctctcagcagcctttcttgctcctcccctagcacaggaagt acatag |
| SEQ ID NO:194 | TRBV12-1*01 | cacagcactgcagaatctccccatctctgtgcagaaaccctggtgcttcctcttctccccacagctctcagcagtcgtcagcaaagtctttcctgctctctgctcaccatggctc acgcc |
| SEQ ID NO:195 | TRBV7-3*01 | cacagcatgacacaatcgcctccttcctgctcataaacctcctcctctctctccttgcttccttatgatactattttgcaccagggggatcctcatctcacaccactccactgcctc ttcc |
| SEQ ID NO:196 | TRBV7-9*01 | cacagcatggcacagtcgcctccttcctgctcacaaaccctcaggcacttacttctccttccagctctcagaagccctgaacaaaggagctgccctgctctttcctcagcaag gagaatg |
| SEQ ID NO:197 | TRBV7-2*01 | cacagcatggcacagtcgcctccttcctgctcataaacctcatccttctctctccttgcagctcctagacacccttaacagaggcttctctttgcttctccctccccatgggaaa caagt |
| SEQ ID NO:198 | TRBV7-6*01 | cacagtgtggcatagtcgcctccttcctgttcacaaacctcatccttctctctccttgcacctcctagagacccttaacagaggcctctctttgctcctcacttttgatgggaaag aagt |
| SEQ ID NO:199 | TRBV17*01 | cacagcatggctgagtcagttccctccagggtgcaaaccctctggctgctcttctcccagttgaactccaagaaaacatttgaaaaagcctcttccttatcttcctaccccag aagaaag |
| SEQ ID NO:200 | TRBV5-7*01 | cacagcccagcagagtcactgacattctgtatataaacttccgccttagctttgacttgagaactgcaggccccacccaggtttcactccttcaagggaagcttttagttgttt ggaagg |
| SEQ ID NO:201 | TRBV5-6*01 | cacagcccatcagagtcactgacgttctgtatataaacttcctgccttagctttgccttgagagctgcaggccccacccagatttcactccttcaagggaagcttttagttgttt ggaag |
| SEQ ID NO:202 | TRBV5-1*01 | cacagccctacaaagccaaccacattctgtgcacaaacctccctggcccaatgtggagcaacctcagccctgacatatctgtgagaacctggggactgcagggagaaaga aaggcaattt |
| SEQ ID NO:203 | TRBV5-3*01 | cacagccctgcagagtcactggaactctgtgcactaatctctctgcttccgtgtacagcagtctcagaccagacagctgtgagaacctggggccttcaggggggaaagataa acaatttca |
| SEQ ID NO:204 | TRBV5-2*01 | atgcaggcctgcagagccaagaacattctgtgtacaaacatccctgccccagtgtggagaacttcagccctaacatatctgtgagaacttgaggactgtagtgggaaaga aaagcagttt |
| SEQ ID NO:205 | TRBV4-1*01 | cacagccttgcagagtcaccgctttcctgtgcagaaaccttcggggcctgccaggaagccgtggggccacggagggctcggtgaacatttcctccaagagccccgaag aagcttcaga |
| SEQ ID NO:206 | TRBV1*01 | cacagccctgcagagtcaccgcctccctgtgcacaaacctcctggatctaatcagaaaaccgtgggggcaacgcatccagctgagcctcagcactcggttcagcattctgt aagacctca |
| SEQ ID NO:207 | TRBV3-2*02 | acacagccttacagagccactgcatccctgtgcacaaacctcccggctcagccaggaagctgtgggccgtgtgtgcacctgcacccaaggctccagtctccattccctgatg gcctctga |
| SEQ ID NO:208 | TRBV18*01 | cacattgatgcagagccacatcctctcagtccacaaacatcctccagacctgccttggaaacagcggtgggccaggaagggaaacgcgttacctgtacagtgaacaggtc |
| | | agctctacgg |
| SEQ ID NO:209 | TRBV9*01 | cacagccctgcatgagcatcagccttctgtgcaataacattcctgccccactcaggaagtgacggtgaggggagggctgccagccagaggggctcaggccctggagagtg gacaggcctt |
| SEQ ID NO:210 | TRBV13*01 | cacagaccctggagaattactggctttctgtacccaaaccctcctatctcacttgaggatgtaatagggagaaggaggtgggggctgccacacaactttagccaagcccca gagatgctt |
| SEQ ID NO:211 | TRAV34*01 | cacagcgatcttcaggcctctatcagctgtctccaaacctgcagctgggccacatatgctcttctgacatggggctcctgagatgtggctgggacctttgccaagacatgaa gtctcaga |
| SEQ ID NO:212 | TRAV30*01 | cacagtgatacccaggcctccaagacctgtactcaaacctaaagctgagccgcagatgctcccctagcacagatgcccaccacaggagtatggggaacttaccagaaggt tcatccatga |
| SEQ ID NO:213 | TRAV7*01 | cacagtactccctaggcacctgcaacctgtatccaaacatgcagctgggtagaagtaccataacagaagcatcagcaataggggccctgagcctgagtagacgtgaaga actaaggcatg |
| SEQ ID NO:214 | TRAV22*01 | cacagtgctccccaggcacctgcggcctgtacacaaaccctcatccgggctcggttcctctaccagtaacaaccacatcacgaggccaccgcagcagcattttgcacagctt aatattcc |
| SEQ ID NO:215 | TRAV6*01 | cacagtagtgccctggcagctgcttcctgcacccaaactctgctaactctcacaatcagagctcatggctgtgctgtctcccaaaggctaatcacagctcctgacagaatgg gggggtgt |
| SEQ ID NO:216 | TRAV27*01 | cacagtgctcttgaggcacctgctgcctgcacccaaaccctgctgccagccccagtcacgaggctgccacatgcctccagctccgcctcgcacagcttatggcatgaataga gagaacaa |
| SEQ ID NO:217 | TRAV20*01 | [?] cacagcgttccccaggcacctgcaacttgtatcaaaaccctgcagctgaggatctgaaatgatggcagaggtatctctgctgttcttcctcttgaaggagtatttatttaatgc ccagga |
| SEQ ID NO:218 | TRAV36/DV7 *01 | cacagtgctccctagtcacctgcagcctgtactcaaattctacagctgaggctctgcaactgtaagatggggaacttgctacattgagcaagccctcaaaaataaactatac ggaaaagc |
| SEQ ID NO:219 | TRAV21*01 | cacagtgcacaacaggcacctgcaaccaatacccaaactctatagctggggctctaactgcatgttttatcttgagactgagcaatgtttttgcattaagaggacttctaaat tgacact |
| SEQ ID NO:220 | TRAV41*01 | cacagtgctccccaggcacctggagcccgtacctaaactctaaagttgaggcatcatttcttactcctgtctttcagacttgtctgtctctatccttggtcagatgatgtaaaat gttta |
| SEQ ID NO:221 | TRAV37*01 | cacagtgcccacagtcacctgcacccggtacctaaagcttgctgaggggcctgggcacacctccttttataagggccctggggcactgactataactctgctgcatacaaag ggaaatat |
| SEQ ID NO:222 | TRAV11*01 | -- agtagtgtctccccagcacctgcagcctgtaccataacctgcagccgggacccttgacacaggctagccttgcaggtgggagtgaagatttttttttttttttgtatagaggg aacttt |
| SEQ ID NO:223 | TRAV15*01 | cacagggtccccaagcacctgcagcctgtaccacaacctgcatccgggacccttgacacagccttgccttgcaggtgggagtgaaggtgttgtctttatatgtagagagaa cttctttat |
| SEQ ID NO:224 | TRAV17*01 | cacagtgttccccaggaacctgcagcctctacgcaaaccctgccaaagcagcttcttagaagccctaatagtgggtagaattagtggttatgtctttcagtcaagaagagtc |
| | | tacaaaca |
| SEQ ID NO:225 | TRAV10*01 | cactgtgctccacaggcacttgaagccagtatgcaaacctgcacctggaggttatcaaggaggcataggagttagagtagaccgttatttttatgcagaatatgatttcac tagtgaat |
| SEQ ID NO:226 | TRBV7-1*01 | cacagcactactgctccagtgtcagcttggttccctaggaaatggggtttctagaacctgaatgctgacaaataagagttgtatatgtgtataccatgcaacctgcgtttaa aatgtat |
| SEQ ID NO:227 | TRAV24*01 | cacagtgctgttcaggcacctgcagcccatacgcaaacctgtgtctggtgttgcactgttaccagcattgacaaagaaccatgagtaggatggaaaagacaagttcgttga attacagtt |
| SEQ ID NO:228 | TRAV39*01 | cacagtgctcccctgacgccaccagtctgtacccaaacctgcagctggtgggcccactcctcctgcaggaactatgactgtgaggcttcgttcactgtctgtacatttctttct gcaagg |
| SEQ ID NO:229 | TRAV35*01 | cacagtgctccccaaacacctgcagcctgtactcaaacttgcagctggaactctagtctctatgctgccttcagctcttagtcctcttggcatgaaatgtgattatgcatgcca cctttg |
| SEQ ID NO:230 | TRAV12-2*01 | cacagtgctccccagacacctgcagtctgtacccaaacctgccatgccccaggaatgcctgatgtagagcttagactgcagggtagtgaaactccccttgctctctagtttca agtggaa |
| SEQ ID NO:231 | TRAV29/DV5 *01 | cacagtgctctccaggcacctgcagcccgtactcaaacctgctttggggactcagactgggagacacatagactcgcttccatttacacatgccaatatgagagattatgct ttgaagta |
| SEQ ID NO:232 | TRAV23/DV6 *01 | cacagtgctccccaggcacctgaagcctgtacccaaacctgcagttgaggttccagccaaaccccacagtgggagcttacgtaggcagagatgtagcctagttttcatctg catatgcaa |
| SEQ ID NO:233 | TRAV28*01 | cactgtgctcttcagacacctgcagcctatacatgaaaccatagctgaaggcctaacccatccccgagagtggcagtaggtcccgatgtgattagcattgcattcccactgc ctacatct |
| SEQ ID NO:234 | TRAV25*01 | cacagtgctccccaagcacctgctgcctgtctccaaatcttgccctgggtcttcaggagcagatcatcctactctccccaaagagcgggcgccagagaaagccaaagtcac aatgtctgt |
| SEQ ID NO:235 | TRAV32*01 | cacagaactcttcaggcacctgcaacctgtactcaaacctgcaactgggagtccagtcacattctttgtctttgaacgggttttgggttagaatggtttaccataatgtgcttg tttcta |
| SEQ ID NO:236 | TRAV5*01 | cacattgcttctcaggcacctgtatcctgtacccaaacctgcacctgggactaaagccacactctatttcctttacctttaagtcagggattttgctgtaaggtatttttaatgta cgga |
| SEQ ID NO:237 | TRAV13-2*01 | cacattgctttccaggcatctgtaaccatcacccaaacctgagatgggaggtgaagcagcatccctttcctttgcaataaatttagttatagcacttgtcattttgttgttcat aagt |
| SEQ ID NO:238 | TRAV13-1*01 | gcagcaagtacacattgcttcccaggcacctgctacccgtacacaaacctgagactggagctgaagctgcacccccttttcctttgtcatagatcgtcaattatagcatttgtc atattgt |

**TableB1**

| **SEQ ID NO** | **Name** | **Sequence** |
|---|---|---|
| SEQ ID NO:239 | IGHV(II)-1-1*01 | CACACTTCAGCCCAGCCTTTCTGGGCCAACTCTCCATCTGTAGAGACACATCCAAGGCCCAGTTATCCCTGCAGCTGAGCTCCGTGAT GGCCAAGGGCAGGGCCGCACATTCCCGTGGGA |
| SEQ ID NO:240 | IGHV(II)-20-1*01_IGHV(II )-20-1*02 | GCTTGTTGCTCATGTAGCTCAGCCATAGGAAGAGCTGCCCCGGCGGACATAGATCTGGAGGTGGCGACTGGACTCTTGAGGAGTG GGTTGGAATTTTTGCTGCCTTCATGACCTGTGCAC |
| SEQ ID NO:241 | IGHV(II)-22-1*01_IGHV(II )-23-2*01 | AATCCAACCCACTCCTCAAGAGTCCAGTCACCATCTCCAGATCCACATCCAAAAAACAGTTTCTCCTACAGCTGAGCTACCTTAACAA GGAGTACACAACCATGATTTTTATACAAAAGA |
| SEQ ID NO:242 | IGHV(II)-26-2*01 | CATCATGCACCCTCCACCCAGGTCCATGTCCCCATCAACAGTGACTCAACCAAGAGCCAGTTCTCTGTGAAGCTCAGCTCCATGACCA CCTAGGACACGGCTGAGTATTACTGTGAAAGA |
| SEQ ID NO:243 | IGHV(II)-28-1*02_IGHV(II )-28-1*03 | GTGAAGGGAGCACAAATTACAACCCACTGCTCAAGAGTCCATATCCAGATCCAAGAAACAGTTCTTACAGCTGAGCTCTGTGCCCA GTGAACACACAACTACGCATTTTTAAGCAAAAGA |
| SEQ ID NO:244 | IGHV(II)-30-1*01_IGHV(II )-30-1*02_IGHV(II )-30-32*01_IGH V(II)-30-51*01 | TTACTCCCCTCTTCTCAAGAGTCCAGTCACCATCTCCAGATCCATGTCCAAAAAGTAGTTCTTCTTACAGCTGAACTATGTGAGGAAC AAACACATAGCCATGTATTTTAGAGCAAAAGA |
| SEQ ID NO:245 | IGHV(II)-30-21*01 | TTACAACCCACTTCTCAAGAGTCCATATCCGGATCCAAGAAACAGTTCTTACAGCTGAGCTCTGTGCCCAGTGAACACACAACTACG CATTTTGAAGCAAAAGATGCAATGAAGGGCCTT |
| SEQ ID NO:246 | IGHV(II)-30-41*01 | TTACAACCCACTGCTCAAGAGTCCATATCCAGATCCAAGAAACAGTTCTTACAGCTGAGCTCTGTGCCCAGTGAACACACAACTACG CATTTTTAAGCAAAAGACGCAATGAAGGGCCTT |
| SEQ ID NO:247 | IGHV(II)-30-51*02_IGHV( II)-33-1*01 | TTACTCCCCTCTTCTCAAGAGTCCAGTCACCATCTCCAGATCCATGTCCAAAAAGTACTTCTTCTTACAGGTGAACTATGTGAGCAACA AACACATAGCCATGTATTTTAGAGCAAAAGA |
| SEQ ID NO:248 | IGHV(II)-31-1*01 | TTACATCCCACTTCTCAAGAGTCCATATCCAGATCCAAGAAACAGTTCTTACAGCTGAGCTCTGTGCCCAGTGAACACACAACTACAC |
| | | ATTTTGAAGCAAAAGACGCAATGAAGGGCCTT |
| SEQ ID NO:249 | IGHV(II)-40-1*01 | AGCCTGGTGAAGCCCTTGCAAACCCCCTCACTCACCTGTGCTGCCTCTGGATTCTCTGTCACAATCAGTGCTTCCTG |
| SEQ ID NO:250 | IGHV(II)-43-1*01 | CATGAAGGGAGCACAAATTCTAACCCACTCCTCAAGAGTCCAGTCACCACCTCCAGATCTATGTCCAAAAACAGCTCTTCGTATGGC TGAGTGACATTAGCAACAAGCACACAGCCATGT |
| SEQ ID NO:251 | IGHV(II)-43-1D*01 | CATGAAGGGAGCACAAATTCTAACCCACTCCTCAAGAGTCCAGTCACCACCTCCAGATCTATGTCCAAAAACAGCTCTTCGTATGGC TGAGTGACATTAGCAACAAGCACACAACCATGT |
| SEQ ID NO:252 | IGHV(II)-44-2*01 | ACGATGATCCATCTCTGCAGAGCCAACTCTCCTTCTCCAGAGATTCATCCAAGAAACAATTTTGACTATACCTGAGCTCTGTGACATC TGAGGACATGGTTTGTATTACTGTGCAAGACA |
| SEQ ID NO:253 | IGHV(II)-46-1*01 | GACCTGAATAGCACACACTTACCCTCTGCCTCACCTACACTGTTACTGGCCACTCCGTCACAACCAGTCCTTACTAGTGGACCTGGAT CTGCCGGCTCTCAGGGAGGGGCTGCAATGGAT |
| SEQ ID NO:254 | IGHV(II)-49-1*01 | ACGCAACCCACGCCTCAAGAGTCCAGTCACCATCTCCAGATCCACATCCAAAACACAGTTTCTTCTACAGCTGAGCTACCTGAGCAAC GAGTACACAACCATGAATTTTTACACAAAAGA |
| SEQ ID NO:255 | IGHV(II)-51-2*01 | AATTCTAACCCACTCCTCATGAGCTCAGTCACCATCTCCAGATCCACGTCCAAGAACCAAATTTTCTTTTAGCTGAGTTCTGTGACCAA CAATGCCACAACCTTGTATTACTGTGAGAGG |
| SEQ ID NO:256 | IGHV(II)-53-1*01 | ATTCCAACCCACTCCTCAAGAGTCCAGTCACCATCTCCAGATCCATGTCCAAAAAGCAGTTCTTCCTACAGCCGAGCTAAGTGAGTCA CAAGCACACAGCCATGTATTTTTAACAAAAGA |
| SEQ ID NO:257 | IGHV(II)-60-1*01 | AAATTCCCACCCACTCCTTATGAATCCAGTCACCATCTCCAAATTCGGGTCCAAAAACACTTGTTTTTACAGTGGAGCTATGTGAGC AACAAGCTCACAGCCATGTTTTAAAGAAGAGA |
| SEQ ID NO:258 | IGHV(II)-62-1*01 | ATTACTCCCCTTTCCTCAAGAGTCCAGTCACCATCCCCAGATCCATGTCCAAAAACAGTTCTTCCTACAGCTGAGCTACATGAGCAAC AATCACATAGCCATATATTTTTCAGCAATAGA |
| SEQ ID NO:259 | IGHV(IIj-65-1*01 | TTCCAACCCACTCCTCAAGAGTCCAGTCACTATCTCCAGATCCACATCCAAAAAACAGTGTTTCCTGTAGCTGAGCTACCTGAGCAAC AAGTACACAACCATGAATTTTAATACAAAAGA |
| SEQ ID NO:260 | IGHV(II)-67-1*01 | ATGCCTAGGTGTGAAGATCACACACTGACCTCACCCATGCTGTCTCTGGCCACTTCATCACAACCAATGCTTAATATTGGACGTGGAT CTGCCAGTCCCCGGGGAATGGGTTGAATGGAT |
| SEQ ID NO:261 | IGHV(III)-11-1*01 | GGCAGCAACAGGGAGAAATTCAAGAGGAAGTTCTTACATGCACCCTTACGTGCACGGTCTCACTGAGATCTTTACTTCCTTTATCAC GTTTGTTCTGTAAATCACAACGAATGGTGCATT |
| SEQ ID NO:262 | IGHV(III)-13-1*01 | TGGGACTCTCCTTGAGTAAAAGATGATTAACAATCCTCAAATACACTCAGTTCAGGAGATTCTCTTTTAAGATGATTAACCTGAGA GCTCAGGAAAAGTCCGTGTATTACTTTGAGGGA |
| SEQ ID NO:263 | IGHV(III)-16-1*01 | TCAGAGTTACTCTCCATGAGTACAAATAAATTAACAGTCCCAAGCGACACCTTTTCATGTGCAGTCTACCTTAAAGGGACCAAACTG AAAGTCAAGGACAAGGCCTTGTAATACTGTGAG |
| SEQ ID NO:264 | IGHV(III)-20-2*01 | ACCAGAAGAATGCTATCATCATCTTTTCTGTTCTTTGGAAGGAATGCCCCCTCTACTCACCTCCACTTGCCTGCATATATTTCTATTTG |
| | | TCTTTGCTTTTCAGCAGTTTTAATAAGATT |
| SEQ ID NO:265 | IGHV(III)-2-1*01 | GGGTTACTTTCCATGAGTACAAATAAATTAACAATCTCAAGCAACACCCTTTTAAGTGCAGTCTGCCTTACAATGACCAATCTGAAAG CCAAGGACAAGGTCATGTATTACTGTGAGTGA |
| SEQ ID NO:266 | IGHV(III)-25-1*01 | GCAAGCTCCAGGACCAGGGTTGATGTGGGCAGCAACAGGGAGAAATTGAAGAGGAAGCTCTCAGTGGTGCCCTCCATGAATACAA AGAATCTTCACAGTCCCCAGGACACCCTTACGTGC |
| SEQ ID NO:267 | IGHV(III)-25-1*02 | AGAGGAAGCTCTCAGTGGTGCCCTCCATGAATACAAAGAATCTTCACAGTCCCCAGGACACCCTTACGTGCATGGTCTCACTGATAT CTTTACTTCCTTTATCACTTTTGTTATGTAAAT |
| SEQ ID NO:268 | IGHV(III)-26-1*01_IGHV(II I)-26-1*02 | GGGTTACTCTCCATGAGTACAGATAAATCAACATTCCCAAGTGACACCCTTTCAAGTGCAGTCTACCTTACAAGGACCAACCTGAAA GCCAAGGGCAAGGCCGTATATTACAGTGAGGGA |
| SEQ ID NO:269 | IGHV(III)-38-1*01 | AATGGGACTCGCCTTCAGTACAAAGAAGATTAACAGTCCTCAGAGACACTGTTCAGAAGATTCTCTTTTAAGATAATAAAACTGAGA GCCCAAGACAAGTCTGTGTATTACTGTGAGGGA |
| SEQ ID NO:270 | IGHV(III)-38-1*02 | AATGGGACTCGCCTTCAGTACAAAGAAGATTAACAGTCCTCAGAGACACTGTTCAGAAGATTCTCTTTTAAGATAATAAAACCGAGA GCCCAAGACAAGTCTGTGTATTACTGTGAGGGA |
| SEQ ID NO:271 | IGHV(III)-38-1D*01 | AGTGGGACTCTCCTTCAGTACAAAGAAGATTAACAGTCCTCAGAGACACTGTTCAGAAGATTCTCTTTTAAGATAATTAAACCAAGA GCCCAGGACAAGTCTGTGTATTACTGTGAGGGA |
| SEQ ID NO:272 | IGHV(III)-44*01 | TTTAGGAAGAATGCCCCCTCAACTCATCTCCACTTGTCTGCATGTATTTCTATTTGTCTTGGACGTTCCCAACAGCCTCNCGAACACTC ACCTCACCCTACAATGCTGCTCGAGGGGGTC |
| SEQ ID NO:273 | IGHV(III)-44D*01 | ATTTTCCTCTTGCTTATAAGGTTTTAACCAGAAGAATGCTGTCATCATCTTTCCTGTTCTTTTAGAAGGAATGCCCCCTCAACTCATCTC CACTTGTCTGCATGTATTTCTATTTGTCTT |
| SEQ ID NO:274 | IGHV(III)-5-1*01 | GATTTATCATCTCAAGAGACAATGTCAAGAAGATGCTGTTTCTGCAAATGGGCAATCTGCAAACCAAGGACACGTCACTACATTACT GTGCAAGAGAAG |
| SEQ ID NO:275 | IGHV(III)-51-1*01 | CAATGCAGACTATGTTAGGGGCAGACTCACCACTTCCAGAGACAACACCAAGTACATGCTGTACATGCAAATGAACAGCCTGAGAA CCCAGAACATGGCAGCATTTAACTGTGCAGGAAA |

**Table B2**

| **SEQ ID NO** | | | | **Name** | **Sequence** |
|---|---|---|---|---|---|
| SEQ ID NO:276 | | 89161040 | 89161073 | IGKJxxx-Z11891 | TACACTTTTGGCCAGGGGACCAAGCTGGAAATCAGACGTAAGTACTTTTTTCCACT GATTCTTCACTGTTGCTAATTAGTTTACTTTGTGTTCCTTTGTGTGGATTTTCATTAG TCGG |
| SEQ ID NO:277 | | 89160080 | 89160117 | IGKJ5*01-X67858 | GATCACCTTCGGCCAAGGGACACGACTGGAGATTAAACGTAAGTAATTTTTCACTA TTGTCTTCTGAAATTTGGGTCTGATGGCCAGTATTGACTTTTAGAGGCTTAAATAG GAGTTTGG |
| SEQ ID NO:278 | 2 | 89160398 | 89160435 | IGKJ4*01-X67858 | GCTCACTTTCGGCGGAGGGACCAAGGTGGAGATCAAACGTAAGTGCACTTTCCTA ATGCTTTTTCTTATAAGGTTTTAAATTTGGAGCGTTTTTGTGTTTGAGATATTAGCT CAGGTCAA |
| SEQ ID NO:279 | 2 | 89160733 | 89160770 | IGKJ3*01-X67858 | ATTCACTTTCGGCCCTGGGACCAAAGTGGATATCAAACGTAAGTACATCTGTCTCA ATTATTCGTGAGATTTTAGTGCCATTGTATCATTTGTGCAAGTTTTGTGATATTTTG GTTGAAT |
| SEQ ID NO:280 | 2 | 89161037 | 89161075 | IGKJ2*01-X67858 | TGTACACTTTTGGCCAGGGGACCAAGCTGGAGATCAAACGTAAGTACTTTTTTCCA CTGATTCTTCACTGTTGCTAATTAGTTTACTTTGTGTTCCTTTGTGTGGATTTTCATT AGTCGG |
| SEQ ID NO:281 | 2 | 89161398 | 89161435 | IGKJ1*01-X67858 | GTGGACGTTCGGCCAAGGGACCAAGGTGGAAATCAAACGTGAGTAGAATTTAAA CTTTGCTTCCTCAGTTGTCTGTGTCTTCTGTTCCCTGTGTCTATGAAGTGATCTATAA GGTGACTC |
| SEQ ID NO:282 | 2 | 89161398 | 89161433 | IGKJ1*01-X63370 | GGACGTTCGGCCAAGGGACCAAGGTGGAAATCAAACGTGAGTAGAATTTAAACT TTGCTTCCTCAGTTGTCTGTGTCTTCTGTTCCCTGTGTCTATGAAGTGATCTATAAG GTGACTCTG |
| SEQ ID NO:283 | 2 | 23235961 | 23235998 | IGLJ1*01-X51755 | GGCTCCTGCTCCAGCCCAGCCCCCAGAGAGCAGACCCCAGGTGCTGGCCCCGGGG GTTTTGGTCTGAGCCTCAGTCACTGTGTTATGTCTTCGGAACTGGGACCAAGGTCA CCGTCCTAG |
| SEQ ID NO:284 | 2 | 23235961 | 23235998 | IGU1*01-X51755(2) | TTATGTCTTCGGAACTGGGACCAAGGTCACCGTCCTAGGTAAGTGGCTCTCAACCT TTCCCAGCCTGTCTCACCCTCTGCTGTCCCTGGAAAATCTGTTTTCTCTCTCTGGGG CTTCCTC |
| SEQ ID NO:285 | 22 | 23241798 | 23241835 | IGLJ2*01-X51755 | CAGCTTCCTCCTTCACAGCTGCAGTGGGGGCTGGGGCTGGGGCATCCCAGGGAG GGTTTTTGTATGAGCCTGTGTCACAGTGTGTGGTATTCGGCGGAGGGACCAAGCT GACCGTCCTAG |
| SEQ ID NO:286 | 22 | 23241798 | 23241835 | IGLJ2*01-X51755(2) | TGTGGTATTCGGCGGAGGGACCAAGCTGACCGTCCTAGGTGAGTCTCTTCTCCCCT CTCCTTCCCCACTCTTGGGACAATTTCTGCTGTTTTTGTTTGTTTCTGTATCTTGTCT CAACTT |
| SEQ ID NO:287 | 22 | 23241801 | 23241835 | IGLJ3*02-D87023 | CAGCTTCCTCCTTCACAGCTGCAGTGGGGGCTGGGCTGGGGCATCCCAGGGAG GGTTTTTGTATGAGCCTGTGTCACAGTGTTGGGTGTTCGGCGGAGGGACCAAGCT GACCGTCCTAG |
| SEQ ID NO:288 | 22 | 23241801 | 23241835 | IGU3*02-D87023(2) | TTGGGTGTTCGGCGGAGGGACCAAGCTGACCGTCCTAGGTGAGTCTCTTCTCCCC TCTCCTTCCCCGCTCTTGGGACAATTTCTGCTGTTTTTGTTTGTTTCTGTATCTTGTC TCAACTT |
| SEQ ID NO:289 | 22 | 23247168 | 23247205 | IGU3*02-D87023 | AGCTTCCTCCTTCACAGCTGCAGTGGGGGCTGGGGCTAGGGGCATCCCAGGGAG GGTTTTTGTATGAGCCTGTGTCACAGTGTTGGGTGTTCGGCGGAGGGACCAAGCT GACCGTCCTAG |
| SEQ ID NO:290 | 22 | 23247168 | 23247205 | IGU3*02-D87023(2) | TTGGGTGTTCGGCGGAGGGACCAAGCTGACCGTCCTAGGTGAGTCTCTTCTCCCC TCTCCTTCCCCGCTCTTGGGACAATTTCTGCTGTTTTTGTTTGTTTCTGTATCTTGTC TCAACTT |
| SEQ ID NO:291 | 22 | 23247171 | 23247205 | IGU3*01-X51755 | AGCTTCCTCCTTCACAGCTGCAGTGGGGGCTGGGGCTAGGGGCATCCCAGGGAG GGTTTTTGTATGAGCCTGTGTCACAGTGTGTGGTATTCGGCGGAGGGACCAAGCT GACCGTCCTAG |
| SEQ ID NO:292 | 22 | 23247171 | 23247205 | IGU3*01-X51755(2) | TGTGGTATTCGGCGGAGGGACCAAGCTGACCGTCCTAGGTGAGTCTCTTCTCCCCT CTCCTTCCCCGCTCTTGGGACAATTTCTGCTGTTTTTGTTTGTTTCTGTATCTTGTCT CAACTT |
| SEQ ID NO:293 | 22 | 23252740 | 23252777 | IGLJ4*01-X51755 | GTATTTGGTGGAGGAACCCAGCTGATCATTTTAGATGAGTCTCTTCTTCCCTTTCTT TCCCTGCCAAGTTGGTGACAATTTTATTCTGATTTCGATCTTTGTCTGTGACTTGCC ACAGCC |
| SEQ ID NO:294 | 22 | 23252740 | 23252777 | IGLJ4*01-X51755(2) | TTTTGTATTTGGTGGAGGAACCCAGCTGATCATTTTAGATGAGTCTCTTCTTCCCTT TCTTTCCCTGCCAAGTTGGTGACAATTTTATTCTGATTTCGATCTTTGTCTGTGACTT GCCAC |
| SEQ ID NO:295 | 22 | 23256443 | 23256480 | IGU5*02-D87017 | CAGAGAGGGTTTTTGTATGAGCCTGTGTCACAGCACTGGGTGTTTGGTGAGGGGA CGGAGCTGACCGTCCTAGATGAGTCTTTTCCCCCTCCTTCCCTGGTCTCCCCAAGGT ACTGGGAA |
| SEQ ID NO:296 | 22 | 23256443 | 23256480 | IGU5*02-D87017(2) | CTGGGTGTTTGGTGAGGGGACGGAGCTGACCGTCCTAGGATGAGTCTTTTCCCCC TCCTTCCCTGGTCTCCCCAAGGTACTGGGAAATTTTCTGCTGCTTTTGTTCTTTTCTG TATCTTG |
| SEQ ID NO:297 | 22 | 23260336 | 23260373 | IGU6*01-X58181 | GGAGGGTTTGTGTGCAGGGTTATATCACAGTGTAATGTGTTCGGCAGTGGCACCA AGGTGACCGTCCTCGGTGAGTCCCCTTTTCTATTCTTTTGGGTCTAGGGTGAGATC TGGGGAGAC |
| SEQ ID NO:298 | 22 | 23260336 | 23260373 | IGU6*01-X58181(2) | TAATGTGTTCGGCAGTGGCACCAAGGTGACCGTCCTCGGTGAGTCCCCTTTTCTAT TCTTTTGGGTCTAGGGTGAGATCTGGGGAGACTTTTCTGTCCTTTCTGTTCTCTCTA GGGTAGA |
| SEQ ID NO:299 | 22 | 23263570 | 23263607 | IGU7*01-X57808 | TCACTGTGTGCTGTGTTCGGAGGAGGCACCCAGCTGACCGTCCTCGGTAAGTCTC CCCGCTTCTCTCCTCTTTGAGATCCCAAGTTAAACACGGGGAGTTTTTCCCTTTCCT GTCTGTCG |
| SEQ ID NO:300 | 22 | 23263570 | 23263607 | IGU7*01-X57808(2) | TGCTGTGTTCGGAGGAGGCACCCAGCTGACCGTCCTCGGTAAGTCTCCCCGCTTCT CTCCTCTTTGAGATCCCAAGTTAAACACGGGGAGTTTTTCCCTTTCCTGTCTGTCGA AGGCTAA |
| SEQ ID NO:301 | 22 | 23263570 | 23263607 | IGLJ7*02-D87017 | TCACTGTGTGCTGTGTTCGGAGGAGGCACCCAGCTGACCGCCCTCGGTAAGTCTC CCCGCTTCTCTCCTCTTTGAGATCCCAAGTTAAACACGGGGAGTTTTTCCCTTTCCT GTCTGTCG |
| SEQ ID NO:302 | 22 | 23263570 | 23263607 | IGU7*02-D87017(2) | [?] TGCTGTGTTCGGAGGAGGCACCCAGCTGACCGCCCTCGGTAAGTCTCCCCGCTTCT CTCCTCTTTGAGATCCCAAGTTAAACACGGGGAGTTTTTCCCTTTCCTGTCTGTCGA AGGCTAA |
| SEQ ID NO:303 | 22 | 106329408 | 1.06E+08 | IGHJ6*03-M63030 | TACTACTACTACTACTACATGGACGTCTGGGGCAAAGGGACCACGGTCACCGTCTC CTCAGGTAAGAATGGCCACTCTAGGGCCTTTGTTTTCTGCTACTGCCTGTGGGGTT TCCTGAGC |
| SEQ ID NO:304 | 22 | 106329408 | 1.06E+08 | IGHJ6*03-M63030(2) | ATTACTACTACTACTACTACATGGACGTCTGGGGCAAAGGGACCACGGTCACCGT CTCCTCAGGTAAGAATGGCCACTCTAGGGCCTTTGTTTTCTGCTACTGCCTGTGGG GAATTC |
| SEQ ID NO:305 | 14 | 106329408 | 1.06E+08 | IGHJ6*04-AJ879487 | ATTACTACTACTACTACGGTATGGACGTCTGGGGCAAAGGGACCACGGTCACCGT CTCCTCAGGTAAGAATGGCCACTCTAGGGCCTTTGTTTTCTGCTACTGCCTGTGGG GTTTCCTGA |
| SEQ ID NO:306 | 14 | 106329409 | 1.06E+08 | IGHJ6*03-X86359 | TGATGCTTTTGATATCTGGGGCCAAGGGACAATGGTCACCGTCTCTTCAGGTAAG ATGGCTTTCCTTCTGCCTCCTTTCTCTGGGCCCAGCGTCCTCTGTCCTGGAGCTGGG AGATAATG |
| SEQ ID NO:307 | 14 | 106329626 | 1.06E+08 | IGHJ3P*02-X97051 | CTTGCAGTTGGACTTCCCAGGCCGACAGTGGTCTGGCTTCTGAGGGGTCAGGCCA GAATGTGGGGTACGTGGGAGGCCAGCAGAGGGTTCCATGAGAAGGGCAGGACA GGGCCACGGACA |
| SEQ ID NO:308 | 14 | 106330024 | 1.06E+08 | IGHJ4-U42590 | GACTATTGGGGCCAGGGAACCCTGGTCACCGTCTCCTCAGGTGAGTCCTCACAAG CTCTCTCCTACTTTAACTCAGAAGACTCTCACTGCATTTTTGGGGGGAGATAAGGG TGCTGGGTC |
| SEQ ID NO:309 | 14 | 106330024 | 1.06E+08 | IGHJ4*02-X97051 | ACTACTTTGACTACTGGGGCCAGGGAACCCTGGTCACCGTCTCCTCAGGTGAGTCC TCACAACCTCTCTCCTGCTTTAACTCTGAAGGGTTTTGCTGCATTTTTGGGGGGAA ATAAGGGT |
| SEQ ID NO:310 | 14 | 106330024 | 1.06E+08 | IGHJ4-U42588 | AACTGGTTCGACCCCTGGGGCCAGGGAACCCTGGTCACCGTCTCCTCAGGTGAGT CCTCACCACCCCCTCTCTGAGTCCACTTAGGGAGACTCAGCTTGCCAGGGTCTCAG GGTCAGAGT |
| SEQ ID NO:311 | 14 | 106330024 | 1.06E+08 | IGHJ5-M18810 | CAGTGCTTCGACCCCTGGGGCCAGGGAACCCTGGTCACCGTCTCCTCAGGAGATT CCTCACCACCCCCTCTCTGAGTCCTCTTAGTGAGACTCAGTTTGCCGGACTCTCAGG GTCAGAGT |
| SEQ ID NO:312 | 14 | 106330024 | 1.06E+08 | IGHJ5*02-X97051 | ACAACTGGTTCGACCCCTGGGGCCAGGGAACCCTGGTCACCGTCTCCTCAGGTGA GTCCTCACCACCCCCTCTCTGAGTCCACTTAGGGAGACTCAGCTTGCCAGGGTCTC AGGGTCAGA |
| SEQ ID NO:313 | 14 | 106330425 | 1.06E+08 | IGHJ5*02-X97051 | TACTTTGACTACTGGGGCCAGGGAACCCTGGTCACCGTCTCCTCAGGTGAGTCCTC ACAACCTCTCTCCTGCTTTAACTCTGAAGGGTTTTGCTGCATTTCTGGGGGGAAAT AAGGGTGC |
| SEQ ID NO:314 | 14 | 106330425 | 1.06E+08 | IGHJ4-U42588 | TTTGACTGCTGGGGCCAGGGAACCCTGGTCACCGTCTCCTCAGGTGAGCCCTCAC AACCTCTCTCCTGGGTTAACTCTGAAGGGTTTTGCTGCATTTTTGGGGGGAAATAA GGGTGCTGG |
| SEQ ID NO:315 | 14 | 106330797 | 1.06E+08 | IGHJ3*01-M 25625 | TGATGCTTTTGATGTCTGGGGCCAAGGGACAATGGTCACCGTCTCTTCAGGTAAG ATGGGCTTTCCTTCTGCCTCCTTTCTCTGGCCCCAGCGTCCTCTGTCCTGGAGCTGG GAGATAAT |
| SEQ ID NO:316 | 14 | 106330797 | 1.06E+08 | IGHJ3*02-X97051 | TGATGCTTTTGATATCTGGGGCCAAGGGACAATGGTCACCGTCTCTTCAGGTAAG ATGGCTTTCCTTCTGCCTCCTTTCTCTGGGCCCAGCGTCCTCTGTCCTGGAGCTGGG AGATAATG |
| SEQ ID NO:317 | 14 | 106330797 | 1.06E+08 | IGHJ3*02-X97051(2) | GATGCTTTTGATATCTGGGGCCAAGGGACAATGGTCACCGTCTCTTCAGGTAAGA TGGCTTTCCTTCTGCCTCCTTTCTCTGGGCCCAGCGTCCTCTGTCCTGGAGCTGGGA GATAATGT |
| SEQ ID NO:318 | 14 | 106331001 | 1.06E+08 | IGHJ2P*01-X97051 | GCTACAAGTGCTTGGAGCACTGGGGCCAGGGCAGCCCGGCCACCGTCTCCCTGG GAACGTCACCCCTCCCTGCCTGGGTCTCAGCCCGGGGGTCTGTGTGGCTGGGGAC AGGGACGCCGG |
| SEQ ID NO:319 | 14 | 106331409 | 1.06E+08 | IGHJ2*01-X97051 | CTACTGGTACTTCGATCTCTGGGGCCGTGGCACCCTGGTCACTGTCTCCTCAGGTG AGTCCCACTGCAGCCCCCTCCCAGTCTTCTCTGTCCAGGCACCAGGCCAGGTATCT GGGGTCTG |
| SEQ ID NO:320 | 14 | 106331617 | 1.06E+08 | IGHJ1*01-X97051 | GCTGAATACTTCCAGCACTGGGGCCAGGGCACCCTGGTCACCGTCTCCTCAGGTG AGTCTGCTGTCTGGGGATAGCGGGGAGCCAGGTGTACTGGGCCAGGCAAGGGCT TTGGCTTCAGA |
| SEQ ID NO:321 | 14 | 106331834 | 1.06E+08 | IGHJ1P*01-X97051 | AAAGGTGCTGGGGGCCCCTGGACCCGACCCGCCCTGGAGACCGCAGCCACATCA AGCCCCCAGCCCCACAGGCCCCCTACCAGCCGCAGGGTTTTGGCTGAGCTGAGAA CCACTGTGCTA |

**Table 2.1**

| **SEQ ID NO** | **Name** | **Sequence** |
|---|---|---|
| SEQ ID NO: 322 | TRAV1-1*01-5' | ggacaaagccttgagcagccctctgaagtgacagctgtggaaggagccattgtccagataaactgcacgtaccagacatctgggttttatgggctgtcct |
| SEQ ID NO: 323 | TRAV1-1*01-3' | gtttttcttcattccttagtcgctctgatagttatggttacctccttctacaggagctccagatgaaagactctgcctcttacttctgcgctgtgagaga |
| SEQ ID NO: 324 | TRAV1-1*02-5' | ggacaaagccttgagcagccctctgaagtgacagctgtggaaggagccattgtccagataaactgcacgtaccagacatctgggttttatgggctgtcct |
| SEQ ID NO: 325 | TRAV1-1*02-3' | caggtcgtttttcttcattccttagtcgctctgatagttatggttacctccttctacaggagctccagatgaaagactctgcctcttacttctgcgctgt |
| SEQ ID NO: 326 | TRAV1-2*01-5' | ggacaaaacattgaccagcccactgagatgacagctacggaaggtgccattgtccagatcaactgcacgtaccagacatctgggttcaacgggctgttct |
| SEQ ID NO: 327 | TRAV1-2*01-3' | gtttttcttcattccttagtcggtctaaagggtacagttacctccttttgaaggagctccagatgaaagactctgcctcttacctctgtgctgtgagaga |
| SEQ ID NO: 328 | TRAV 1-2 *02-5' | ggacaaaacattgaccagcccactgagatgacagctacggaaggtgccattgtccagatcaactgcacgtaccagacatctgggttcaacgggctgttct |
| SEQ ID NO: 325 | TRAV1-2*02-3' | catctgggttcaacgggctgttctggtaccagcaacatgctggcgaagcacccacatttctgtcttacaatgttctggatggtctggaggagaaaggtcg |
| SEQ ID NO: 33C | TRAV10*01-5' | aaaaaccaagtggagcagagtcctcagtccctgatcatcctggagggaaagaactgcactcttcaatgcaattatacagtgagccccttcagcaacttaa |
| SEQ ID NO: 331 | TRAV10*01-3' | agatatacagcaactctggatgcagacacaaagcaaagctctctgcacatcacagcctcccagctcagcgattcagcctcctacatctgtgtggtgagcg |
| SEQ ID NO: 332 | TRAV11*01-5' | ctacatacactggagcagagtccttcattcctgaatattcaggagggaatgcatgccgttcttaattgtacttatcaggagagaacactcttcaatttcc |
| SEQ ID NO: 333 | TRAV11*01-3' | caaatattttaaagaactgcttggaaaagaaaaattttatagtgtttggaatatcgcagcctctcatctgggagattcagccacctacttctgtgctttg |
| SEQ ID NO: 334 | TRAV12-1*01-5' | cggaaggaggtggagcaggatcctggacccttcaatgttccagagggagccactgtcgctttcaactgtacttacagcaacagtgcttctcagtctttct |
| SEQ ID NO: 335 | TRAV12-1*01-3' | aggtttacagcacagctcaatagagccagccagtatatttccctgctcatcagagactccaagctcagtgattcagccacctacctctgtgtggtgaaca |
| SEQ ID NO: 336 | TRAV12-1*02-5' | cggaaggaggtggagcaggatcctggacccttcaatgttccagagggagccactgtcgctttcaactgtacttacagcaacagtgcttctcagtctttct |
| SEQ ID NO: 337 | TRAV12-1*02-3' | acagcacacgtcaatagagccagccagtatatttccctgctcatcagagactccaagctcagtgattcagccacctacctctgtgtggtgaacattcgcc |
| SEQ ID NO: 338 | TRAV12-2*01-5' | cagaaggaggtggagcagaattctggacccctcagtgttccagagggagccattgcctctctcaactgcacttacagtgaccgaggttcccagtccttct |
| SEQ ID NO: 339 | TRAV12-2*01-3' | aggtttacagcacagctcaataaagccagccagtatgtttctctgctcatcagagactcccagcccagtgattcagccacctacctctgtgccgtgaaca |
| SEQ ID NO: 340 | TRAV12-2*02-5' | cagaaggaggtggagcagaattctggacccctcagtgttccagagggagccattgcctctctcaactgcacttacagtgaccgaggttcccagtccttct |
| SEQ ID NO: 341 | TRAV12-2*02-3' | gtttacagcacagctcaataaagccagccagtatgtttctctgctcatcagagactcccagcccagtgattcagccacctacctctgtgccgtgtaccac |
| SEQ ID NO: 342 | TRAV12-2*03-5' | ggacccctcagtgttccagagggagccattgcctctctcaactgcacttacagtgaccgagtttcccagtccttcttctggtacagacaatattctggga |
| SEQ ID NO: 343 | TRAV12-2*03-3' | aaggtttacagcacagctcaataaagccagccagtatgtttctctgctcatcagagactcccagcccagtgattcagccacctacctctgtgccgtgaac |
| SEQ ID NO: 344 | TRAV12-3*01-5' | cagaaggaggtggagcaggatcctggaccactcagtgttccagagggagccattgtttctctcaactgcacttacagcaacagtgcttttcaatacttca |
| SEQ ID NO: 345 | TRAV12-3*01-3' | aggtttacagcacaggtcgataaatccagcaagtatatctccttgttcatcagagactcacagcccagtgattcagccacctacctctgtgcaatgagcg |
| SEQ ID NO: 346 | TRAV12-3*02-5' | cagaaggaggtggagcaggatcctggaccactcagtgttccagagggagccattgtttctctcaactgcacttacagcaacagtgcttttcaatacttca |
| SEQ ID NO: 347 | TRAV12-3*02-3' | aggtttacagcacaggtcgataaatccagcaagtatatctccttgttcatcagagactcacagcccagtgattcagccacctacctctgtgcaatgagcg |
| SEQ ID NO: 348 | TRAV13-1*01-5' | ggagagaatgtggagcagcatccttcaaccctgagtgtccaggagggagacagcgctgttatcaagtgtacttattcagacagtgcctcaaactacttcc |
| SEQ ID NO: 345 | TRAV13-1*01-3' | cgaattgctgttacattgaacaagacagccaaacatttctccctgcacatcacagagacccaacctgaagactcggctgtctacttctgtgcagcaagta |
| SEQ ID NO: 35C | TRAV13-1*02-5' | ggagagaatgtggagcagcatccttcaaccctgagtgtccaggagggagacagcgctgttatcaagtgtacttattcagacagtgcctcaaactacttcc |
| SEQ ID NO: 351 | TRAV13-1*02-3' | tgttacattgaacaagacagccaaacatttctccctgcacatcacagagacccaacctgaagactcggctgtctacttctgtgcagcaagtaggaaggac |
| SEQ ID NO: 352 | TRAV13-1*03-5' | ggagagaatgtggagcagcatccttca a ccctgagtgtccaggagggaga cagcgctgttatca agtgta cttattcagacagtgcct ca aacta cttcc |
| SEQ ID NO: 353 | TRAV13-1*03-3' | gcttattatagacattcgttcaaatgtgggcgaaaagaaagaccaacgaattgctgttacattgaacaagacagccaaacatttctccctgcagatcaca |
| SEQ ID NO: 354 | TRAV13-2*01-5' | ggagagagtgtggggctgcatcttcctaccctgagtgtccaggagggtgacaactctattatcaactgtgcttattcaaacagcgcctcagactacttca |
| SEQ ID NO: 355 | TRAV13-2*01-3' | agagtcaccgttttattgaataagacagtgaaacatctctctctgcaaattgcagctactcaacctggagactcagctgtctacttttgtgcagagaata |
| SEQ ID NO: 356 | TRAV13-2*02-5' | ggagagagtgtggggctgcatcttcctaccctgagtgtccaggagggtgacaactctattatcaactgtgcttattcaaacagcgcctcagactacttca |
| SEQ ID NO: 357 | TRAV13-2*02-3' | caaagagtcaccgttttattgaataagacagtgaaacatctctctctgcaaattgcagctactcaacctggagactcagctgtctacttttgtgcagaga |
| SEQ ID NO: 358 | TRAV14/DV4*01-5' | gcccagaagataactcaaacccaaccaggaatgttcgtgcaggaaaaggaggctgtgactctggactgcacatatgacaccagtgatccaagttatggtc |
| SEQ ID NO: 359 | TRAV14/DV4*01-3' | actcattgaatttccagaaggcaagaaaatccgccaaccttgtcatctccgcttcacaactgggggactcagcaatgtacttctgtgcaatgagagaggg |
| SEQ ID NO: 36C | TRAV14/DV4*02-5' | gcccagaagataactcaaacccaaccaggaatgttcgtgcaggaaaaggaggctgtgactctggactgcacatatgacaccagtgatcaaagttatggtc |
| SEQ ID NO: 361 | TRAV14/DV4*02-3' | actcattgaatttccagaaggcaagaaaatccgccaaccttgtcatctccgcttcacaactgggggactcagcaatgtatttctgtgcaatgagagaggg |
| SEQ ID NO: 362 | TRAV14/DV4*03-5' | gcccagaagataactcaaacccaaccaggaatgttcgtgcaggaaaaggaggctgtgactctggactgcacatatgacaccagtgatccaagttatggtc |
| SEQ ID NO: 363 | TRAV14/DV4*03-3' | aggtcgctactcattgaatttccagaaggcaagaaaatccgccaaccttgtcatctccgcttcacaactgggggactcagcaatgtatttctgtgcaatg |
| SEQ ID NO: 364 | TRAV14/DV4*04-5' | cagaagataactcaaacccaaccaggaatgttcgtgcaggaaaaggaggctgtgactctggactgcacatatgacaccagtgatcaaagttatggtctct |
| SEQ ID NO: 365 | TRAV14/DV4*04-3' | gcaacagaaggtcgctactcattgaatttccagaaggcaagaaaatccgccaaccttgtcatctccgcttcacaactgggggactcagcaatgtacttct |
| SEQ ID NO: 366 | TRAV15*01-5' | ctccatattctggagtagagtccttcattcattcctgagtatccgggagggaatgcacaacattcttaattgcacttatgaggagagaacgttctcttaa |
| SEQ ID NO: 367 | TRAV15*01-3' | acattttaaagaagcgcttggaaaagagaagttttatagtgttttgaatatgctggtctctcatcctggagattcaggcacctacttctgtgctttgagg |
| SEQ ID NO: 368 | TRAV16*01-5' | gcccagagagtgactcagcccgagaagctcctctctgtctttaaaggggccccagtggagctgaagtgcaactattcctattctgggagtcctgaactct |
| SEQ ID NO: 369 | TRAV16*01-3' | gcttcactgctgaccttaacaaaggcgagacatctttccacctgaagaaaccatttgctcaagaggaagactcagccatgtattactgtgctctaagtgg |
| SEQ ID NO: 370 | TRAV17*01-5' | agtcaacagggagaagaggatcctcaggccttgagcatccaggagggtgaaaatgccaccatgaactgcagttacaaaactagtataaacaatttacagt |
| SEQ ID NO: 371 | TRAV17*01-3' | agattaagagtcacgcttgacacttccaagaaaagcagttccttgttgatcacggcttcccgggcagcagacactgcttcttacttctgtgctacggacg |
| SEQ ID NO: 372 | TRAV18*01-5' | ggagactcggttacccagacagaaggcccagttaccctccctgagagggcagctctgacattaaactgcacttatcagtccagctattcaacttttctat |
| SEQ ID NO: 373 | TRAV18*01-3' | gttttcaggccagtcctatcaagagtgacagttccttccacctggagaagccctcggtgcagctgtcggactctgccgtgtactactgcgctctgagaga |
| SEQ ID NO: 374 | TRAV19*01-5' | gctcagaaggtaactcaagcgcagactgaaatttctgtggtggagaaggaggatgtgaccttggactgtgtgtatgaaacccgtgatactacttattact |
| SEQ ID NO: 375 | TRAV19*01-3' | attcttggaacttccagaaatccaccagttccttcaacttcaccatcacagcctcacaagtcgtggactcagcagtatacttctgtgctctgagtgaggc |
| SEQ ID NO: 376 | TRAV2*01-5' | aaggaccaagtgtttcagccttccacagtggcatcttcagagggagctgtggtggaaatcttctgtaatcactctgtgtccaatgcttacaacttcttct |
| SEQ ID NO: 377 | TRAV2*01-3' | agggacgatacaacatgacctatgaacggttctcttcatcgctgctcatcctccaggtgcgggaggcagatgctgctgtttactactgtgctgtggagga |
| SEQ ID NO: 378 | TRAV2*02-5' | aaggaccaagtgtttcagccttccacagtggcatcttcagagggagctgtggtggaaatcttctgtaatcactctgtgtccaatgcttacaacttcttct |
| SEQ ID NO: 379 | TRAV2*02-3' | gggacgatacaacatgacctatgaacggttctcttcatcgctgctcatcctccaggtgcgggaggcagatgctgctgtttactactgtgctgtggcctgg |
| SEQ ID NO: 38C | TRAV20*01-5' | gaagaccaggtgacgcagagtcccgaggccctgagactccaggagggagagagtagcagtcttaactgcagttacacagtcagcggtttaagagggctgt |
| SEQ ID NO: 381 | TRAV20*01-3' | aaagaaaggctaaaagccacattaacaaagaaggaaagctttctgcacatcacagcccctaaacctgaagactcagccacttatctctgtgctgtgcagg |
| SEQ ID NO: 382 | TRAV20*02-5' | gaagaccaggtgacgcagagtcccgaggccctgagactccaggagggagagagtagcagtctcaactgcagttacacagtcagcggtttaagagggctgt |
| SEQ ID NO: 383 | TRAV20*02-3' | aaaggagaaagaaaggctaaaagccacattaacaaagaaggaaagctttctgcacatcacagcccctaaacctgaagactcagccacttatctctgtgct |
| SEQ ID NO: 384 | TRAV20*03-5' | gaagaccaggtgacgcagagtcccgaggccctgagactccaggagggagagagtcgcagtctcaactgcagttacacagtcagcggtttaagagggctgt |
| SEQ ID NO: 385 | TRAV20*03-3' | agaaaaggagaaagaaaggctaaaagccacattaacaaagaaggaaagctttctgcacatcacagcccctaaacctgaagactcagccacttatctctgt |
| SEQ ID NO: 386 | TRAV20*04-5' | gaagaccaggtgacgcagagtcccgaggccctgagactccaggagggagagagtagcagtctcaactgcagttgcacagtcagcggtttaagagggctgt |
| SEQ ID NO: 387 | TRAV20*04-3' | aaaggagaaagaaaggctaaaagccacattaacaaagaaggaaagctttctgcacatcacagcccctaaacctgaagactcagccacttatctctgtgct |
| SEQ ID NO: 388 | TRAV21*01-5' | aaacaggaggtgacgcagattcctgcagctctgagtgtcccagaaggagaaaacttggttctcaactgcagtttcactgatagcgctatttacaacctcc |
| SEQ ID NO: 389 | TRAV21*01-3' | aagacttaatgcctcgctggataaatcatcaggacgtagtactttatacattgcagcttctcagcctggtgactcagccacctacctctgtgctgtgagg |
| SEQ ID NO: 390 | TRAV21*02-5' | aaacaggaggtgacacagattcctgcagctctgagtgtcccagaaggagaaaacttggttctcaactgcagtttcactgatagcgctatttacaacctcc |
| SEQ ID NO: 391 | TRAV21*02-3' | aagtggaagacttaatgcctcgctggataaatcatcaggacgtagtactttatacattgcagcttctcagcctggtgactcagccacctacctctgtgct |
| SEQ ID NO: 392 | TRAV22*01-5' | ggaatacaagtggagcagagtcctccagacctgattctccaggagggagccaattccacgctgcggtgcaatttttctgactctgtgaacaatttgcagt |
| SEQ ID NO: 393 | TRAV22*01-3' | agattaagcgccacgactgtcgctacggaacgctacagcttattgtacatttcctcttcccagaccacagactcaggcgtttatttctgtgctgtggagc |
| SEQ ID NO: 394 | TRAV23/DV6*01-5' | cagcagcaggtgaaacaaagtcctcaatctttgatagtccagaaaggagggatttcaattataaactgtgcttatgagaacactgcgtttgactactttc |
| SEQ ID NO: 395 | TRAV23/DV6*01-5 | agattcacaatctccttcaataaaagtgccaagcagttctcattgcatatcatggattcccagcctggagactcagccacctacttctgtgcagcaagca |
| SEQ ID NO: 396 | TRAV23/DV6*02-5' | cagcagcaggtgaaacaaagtcctcaatctttgatagtccagaaaggagggattccaattataaactgtgcttatgagaacactgcgtttgactactttc |
| SEQ ID NO: 397 | TRAV23/DV6*02-3' | agattcacaatctccttcaataaaagtgccaagcagttctcattgcatatcatggattcccagcctggagactcagccacctacttctgtgcagcaagcg |
| SEQ ID NO: 398 | TRAV23/DV6*03-5' | cagcagcaggtgaaacaaagtcctcaatctttgatagtccagaaaggagggatttcaattataaactgtgcttatgagaacactgcgtttgactactttc |
| SEQ ID NO. 399 | TRAV23/DV6*03-3' | agattcacaatctccttcaataaaagtgccaagcagttctcattgcatatcatggattcccagcctggagactcagccacctacttctgtgcagcaagca |
| SEQ ID NO: 400 | TRAV23/DV6*04-5' | cagcaggtgaaacaaagtcctcaatctttgatagtccagaaaggagggatttcaattataaactgtgcttatgagaacactgcgtttgactactttccat |
| SEQ ID NO: 401 | TRAV23/DV6*04-3' | gaaagaaggaagattcacaatctccttcaataaaagtgccaagcagttctcattgcatatcatggattcccagcctggagactcagccacctacttctgt |
| SEQ ID NO: 402 | TRAV24*01-5' | aggacgaataagtgccactcttaataccaaggagggttacagctatttgtacatcaaaggatcccagcctgaagactcagccacatacctctgtgccttt |
| SEQ ID NO: 403 | TRAV24*01-3' | ggacgaataagtgccactcttaataccaaggagggttacagctatttgtacatcaaaggatcccagcctgaagactcagccacatacctctgtgccttta |
| SEQ ID NO: 404 | TRAV24*02-5' | atactgaacgtggaacaaggtcctcagtcactgcatgttcaggagggagacagcaccaatttcacctgcagcttcccttccagcaatttttatgccttac |
| SEQ ID NO: 405 | TRAV24*02-3' | ggacgaataagtgccactcttaataccaaggagggttacagctatttgtacatcaaaggatcccagcctgaagattcagccacatacctctgtgccttta |
| SEQ ID NO: 406 | TRAV25*01-5' | ggacaacaggtaatgcaaattcctcagtaccagcatgtacaagaaggagaggacttcaccacgtactgcaattcctcaactactttaagcaatatacagt |
| SEQ ID NO: 407 | TRAV25*01-3' | gaaaagactgacatttcagtttggagaagcaaaaaagaacagctccctgcacatcacagccacccagactacagatgtaggaacctacttctgtgcaggg |
| SEQ ID NO: 408 | TRAV26-1*01-5' | gatgctaagaccacccagcccccctccatggattgcgctgaaggaagagctgcaaacctgccttgtaatcactctaccatcagtggaaatgagtatgtgt |
| SEQ ID NO: 409 | TRAV26-1*01-3' | gcctctctgatcatcacagaagacagaaagtccagcaccttgatcctgccccacgctacgctgagagacactgctgtgtactattgcatcgtcagagtcg |
| SEQ ID NO: 410 | TRAV26-1*02-5' | gatgctaagaccacccagcccacctccatggattgcgctgaaggaagagctgcaaacctgccttgtaatcactctaccatcagtggaaatgagtatgtgt |
| SEQ ID NO: 411 | TRAV26-1*02-3' | ctctgatcatcacagaagacagaaagtccagcaccttgatcctgccccacgctacgctgagagacactgctgtgtactattgcatcgtcagagattgggt |
| SEQ ID NO: 412 | TRAV26-1*03-5' | gatgctaagaccacccagcccccctccatggattgcgctgaaggaagagctgcaaacctgccttgtaatcactctaccatcagtggaaagtatgtgt |
| SEQ ID NO: 413 | TRAV26-1*03-3' | caatgaaatggcctctctgatcatcacagaagacagaaagtccagcaccttgatcctgccccacgctacgctgagagacactgctgtgtactattgcatc |
| SEQ ID NO:414 | TRAV26-2*01-5' | gatgctaagaccacacagccaaattcaatggagagtaacgaagaagagcctgttcacttgccttgtaaccactccacaatcagtggaactgattacatac |
| SEQ ID NO: 415 | TRAV26-2*01-3' | ggcctctctggcaatcgctgaagacagaaagtccagtaccttgatcctgcaccgtgctaccttgagagatgctgctgtgtactactgcatcctgagagac |
| SEQ ID NO: 416 | TRAV26-2*02-5' | gatgctaagaccacacagccaaattcaatggagagtaacgaagaagagcctgttcacttgccttgtaaccactccacaatcagtggaactgattacatac |
| SEQ ID NO: 417 | TRAV26-2*02-3' | ccctcccagggtccagagtacgtgattcatggtcttacaagcaatgtgaacaacagaatggcctgtgtggcaatcgctgaagacagaaagtccagtacct |
| SEQ ID NO: 418 | TRAV27*01-5' | acccagctgctggagcagagccctcagtttctaagcatccaagagggagaaaatctcactgtgtactgcaactcctcaagtgttttttccagcttacaat |
| SEQ ID NO: 419 | TRAV27*01-3' | aagagactaacctttcagtttggtgatgcaagaaaggacagttctctccacatcactgcagcccagcctggtgatacaggcctctacctctgtgcaggag |
| SEQ ID NO: 420 | TRAV27*02-5' | acccagctgctggagcagagccctcagtttctaagcatccaagagggagaaaatctcactgtgtactgcaactcctcaagtgttttttccagcnacaat |
| SEQ ID NO: 421 | TRAV27*02-3' | tgaagagactaacctttcagtttggtgatgcaagaaaggacagttctctccacatcactgcggcccagcctggtgatacaggccactacctctgtgcagg |
| SEQ ID NO: 422 | TRAV27*03-5' | acccagctgctggagcagagccctcagtttctaagcatccaagagggagaaaatctcactgtgtactgcaactcctcaagtgttttttccagcttacaat |
| SEQ ID NO: 423 | TRAV27*03-3' | gctgaagagactaacctttcagtttggtgatgcaagaaaggacagttctctccacatcactgcagcccagactggtgatacaggcctctacctctgtgca |
| SEQ ID NO: 424 | TRAV28*01-5' | aaagtggagcagagtcctcaggtcctgatcctccaagagggaagaaattcattcctggtgtgcagttgttctatttacatgatccgtgtgcagtggtttc |
| SEQ ID NO: 425 | TRAV28*01-3' | gaagactaaaatccgcagtcaaagctgaggaactttatggccacctatacatcagattcccagcctgaggactcagctatttacttctgtgctgtgggga |
| SEQ ID NO: 426 | TRAV29/DV5*01-5' | gaccagcaagttaagcaaaattcaccatccctgagcgtccaggaaggaagaatttctattctgaactgtgactatactaacagcatgtttgattatttcc |
| SEQ ID NO: 427 | TRAV29/DV5*01-3' | agattcactgtcttcttaaacaaaagtgccaagcacctctctctgcacattgtgccctcccagcctggagactctgcagtgtacttctgtgcagcaagcg |
| SEQ ID NO: 428 | TRAV29/DV5*02-5' | gaccagcaagttaagcaaaattcaccatccctgagcgtccaggaaggaagaatttctattctgaactgtgactatactaacagcatgtttgattatttcc |
| SEQ ID NO: 429 | TRAV29/DV5*02-3' | aagattcactgttttcttaaacaaaagtgccaagcacctctctctcgacattgtgccctcccagcctggagactctgcagtgtacttctgtgcagcaagc |
| SEQ ID NO: 43C | TRAV29/DV5*03-5' | gaccagcaagttaagcaaaattcaccatccctgagcgtccaggaaggaagaatttctattctgaactgtgactatactaacagcatgtttgattatttcc |
| SEQ ID NO: 431 | TRAV29/DV5*03-3' | agattcactgttttcttaaacaaaagtgccaagcacctctctctgcacattgtgccctcccagcctggagactctgcagtgtacttctgtgcagcaagcg |
| SEQ ID NO: 432 | TRAV3*01-5' | gctcagtcagtggctcagccggaagatcaggtcaacgttgctgaagggaatcctctgactgtgaaatgcacctattcagtctctggaaacccttatcttt |
| SEQ ID NO: 433 | TRAV3*01-3' | tttgaagctgaatttaacaagagccaaacctccttccacctgaagaaaccatctgcccttgtgagcgactccgctttgtacttctgtgctgtgagagaca |
| SEQ ID NO: 434 | TRAV3*02-5' | gctcagtcagtggctcagcggaagatcaggtcaacgttgctgaagggaatcctctgactgtgaaatgcacctattcagtctctggaaacccttatctttt |
| SEQ ID NO: 435 | TRAV3*02-3' | ctttgaagctgaatttaacaagagccaaacctccttccacctgaagaaaccatctgcccttgtgagcgactccgctttgtacttctgtgctgtgagaccc |
| SEQ ID NO: 436 | TRAV30*01-5' | caacaaccagtgcagagtcctcaagccgtgatcctccgagaaggggaagatgctgtcatcaactgcagttcctccaaggctttatattctgtacactggt |
| SEQ ID NO: 437 | TRAV30*01-3' | aaaatatctgcttcatttaatgaaaaaaagcagcaaagctccctgtaccttacggcctcccagctcagttactcaggaacctacttctgcggcacagaga |
| SEQ ID NO: 438 | TRAV30*02-5' | caacaaccagtgcagagtcctcaagccgtgatcctccgagaaggggaagatgctgtcaccaactgcagttcctccaaggctttatattctgtacactggt |
| SEQ ID NO: 439 | TRAV30*02-3' | tcgtgaaaaaatatctgcttcatttaatgaaaaaaagcagcaaagctccctgtaccttacggcctcccagctcagttactcaggaacctacttctgcggg |
| SEQ ID NO: 440 | TRAV30*03-5' | caacaaccagtgcagagtcctcaagccgtgatcctccgagaaggggaagatgctgtcatcaactgcagttcctccaaggctttatattctgtacactggt |
| SEQ ID NO: 441 | TRAV30*03-3' | tcatgaaaaaatatctgcttcatttaatgaaaaaaagcggcaaagctccctgtaccttacggcctcccagctcagttactcaggaacctacttctgcggc |
| SEQ ID NO: 442 | TRAV30*04-5' | caacaaccagtgcagagtcctcaagccgtgatcctccgagaaggggaagatgctgtcatcaactgcagttcctccaaggctttatattctgtacactggt |
| SEQ ID NO: 443 | TRAV30*04-3' | tcctgatgatattactgaagggtggagaacagaagcgtcatgaaaaaatatctgcttcatttaatgaaaaaaagcagcaaagctccctgtaccttacggc |
| SEQ ID NO: 444 | TRAV31*01-5' | cagagggtcattcaatcccaaccagcaatatctacgcaggagggtgagaccgtgaaactggactgtgcatacaaaactaatattgtatattacatattgt |
| SEQ ID NO: 445 | TRAV31*01-3' | tattctgtgagcttccagaaaacaactaaaactattcagcttatcatatcatcatcacagccagaagacctgcaacatatttctgttgtctcaaagagcc |
| SEQ ID NO: 44C | TRAV32*01-5' | aaggatgtgatacagagttattcaaatctaaatgtctaggagagagaaatggccgttattaatgacagttatacagatggagctttgaattatttctgtt |
| SEQ ID NO: 447 | TRAV32*01-3' | aggctcactgtactgttgaataaaaatgctaaacatgtctccctgcatattacagccacccaaccaggagactcattcctgtacttctgtgcagtgagaa |
| SEQ ID NO: 448 | TRAV33*01-5' | gctcagaaagtaacccaagttcagaccacagtaactaggcagaaaggagtagctgtgaccttggactgcatgtttgaaaccagatagaattcgtacactt |
| SEQ ID NO: 449 | TRAV33*01-3' | gcaaagcctgtgaactttgaaaaaaagaaaaagttcatcaacctcaccatcaattccttaaaactgactcagccaagtacttctgtgctctcaggaatcc |
| SEQ ID NO: 45C. | TRAV34*01-5' | agccaagaactggagcagagtcctcagtccttgatcgtccaagagggaaagaatctcaccataaactgcacgtcatcaaagacgttatatggcttatact |
| SEQ ID NO: 451 | TRAV34*01-3' | aagataactgccaagttggatgagaaaaagcagcaaagttccctgcatatcacagcctcccagcccagccatgcaggcatctacctctgtggagcagaca |
| SEQ ID NO: 452 | TRAV35*01-5' | ggtcaacagctgaatcagagtcctcaatctatgtttatccaggaaggagaagatgtctccatgaactgcacttcttcaagcatatttaacacctggctat |
| SEQ ID NO: 453 | TRAV35*01-3' | aagactgactgctcagtttggtataaccagaaaggacagcttcctgaatatctcagcatccatacctagtgatgtaggcatctacttctgtgctgggcag |
| SEQ ID NO: 454 | TRAV35*02-5' | ggtcaacagctgaatcagagtcctcaatctatgtttatccaggaaggagaagatgtctccatgaactgcacttcttcaagcatatttaacacctggctat |
| SEQ ID NO: 455 | TRAV35*02-3' | aaatggaagactgactgctcagtttggtataaccagaaaggacagcttcctgaatatctcagcatccatacctagtgatgtaggcatctacttctgtgct |
| SEQ ID NO: 456 | TRAV36/DV7*01-5' | gaagacaaggtggtacaaagccctctatctctggttgtccacgagggagacaccgtaactctcaattgcagttatgaagtgactaactttcgaagcctac |
| SEQ ID NO: 457 | TRAV36/DV7*01-3' | agactaagtagcatattagataagaaagaactttccagcatcctgaacatcacagccacccagaccggagactcggccatctacctctgtgctgtggagg |
| SEQ ID NO: 458 | TRAV36/DV7*02-5' | gaagacaaggtggtacaaagccctcaatctctggttgtccacgagggagacactgtaactctcaattgcagttatgaaatgactaactttcgaagcctac |
| SEQ ID NO: 455 | TRAV36/DV7*02-3' | ggaagactaagtagcatattagataagaaagaacttttcagcatcctgaacatcacagccacccagaccggagactcggccgtctacctctgtgctgtgg |
| SEQ ID NO: 460 | TRAV36/DV7*03-5' | gaagacaaggtggtacaaagccctctatctctggttgtccacgagggagacactgtaactcccaattgcagttatgaagtgactaactttcgaagcctac |
| SEQ ID NO: 461 | TRAV36/DV7*03-3' | gtcaggaagactaagtagcatattagataagaaagaacttttcagcatcctgaacatcacagccacccagaccggagactcggccgtctacctctgtgct |
| SEQ ID NO: 462 | TRAV36/DV7*04-5' | gaagacaaggtggtacaaagccctctatctctggttgtccacgagggagacactgtaactctcaattgcagttatgaagtgactaactttcgaagcctac |
| SEQ ID NO: 463 | TRAV36/DV7*04-3' | tcaggaagactaagtagcatattagataagaaagaacttttcagcatcctgaacatcacagccacccagaccggagactcggccgtctacctctgtgctg |
| SEQ ID NO: 464 | TRAV37*01-5' | caactgccagtggaacagaatgctccttccctgaaagtcaaggaaggtgacagcgtcacactgaactgcagttacagagacagcccttcagatttcttca |
| SEQ ID NO: 465 | TRAV37*01-3' | agattcacagccaggcttaaaaaaggagaccagcacatttccctgcacatacaggattcccagctccatgactcaaccacattcttctgcgcagcaagca |
| SEQ ID NO: 466 | TRAV38-1*01-5' | gcccagacagtcactcagtctcaaccagagatgtctgtgcaggaggcagagactgtgaccctgagttgcacatatgacaccagtgagaataattattatt |
| SEQ ID NO: 467 | TRAV38-1*01-3' | tctctgtgaacttccagaaagcagccaaatccttcagtctcaagatctcagactcacagctgggggacactgcgatgtatttctgtgctttcatgaagca |
| SEQ ID NO: 468 | TRAV38-1*02-5' | gcccagacagtcactcagtctcaaccagagatgtctgtgcaggaggcagagactgtgaccctgagttgcacatatgacaccagtgagaatgattattatt |
| SEQ ID NO: 469 | TRAV38-1*02-3' | gagaatcgtttctctgtgaacttccagaaagcagccaaatccttcagtctcaagatctcagactcacagctgggggacactgcgatgtatttctgtgctt |
| SEQ ID NO: 47C | TRAV38-1*03-5' | gcccagacagtcactcagtctcaaccagagatgtctgtgcaggaggcagagactgtgaccctgagttgcacatatgacaccagtgagagtaattattatt |
| SEQ ID NO: 471 | TRAV38-1*03-3' | aatcgtttctctgtgaacttccagaaagcagccaaatccttcagtctcaagatctcagactcacagctgggggacactgcgatgtatttctgtgctttca |
| SEQ ID NO: 472 | TRAV38-1*04-5' | gcccagacagtcactcagtcccagccagagatgtctgtgcaggaggcagagactgtgaccctgagttgcacatatgacaccagtgagaataattattatt |
| SEQ ID NO: 473 | TRAV38-1*04-3' | ggagaatcgtttctctgtgaacttccagaaagcagccaaatccttcagtctcaagatctcagactcacagctgggggacactgcgatgtatttctgtgca |
| SEQ ID NO: 474 | TRAV38-2/DV8*01-5' | gctcagacagtcactcagtctcaaccagagatgtctgtgcaggaggcagagaccgtgaccctgagctgcacatatgacaccagtgagagtgattattatt |
| SEQ ID NO: 475 | TRAV38-2/DV8*01-3' | ttctctgtgaacttccagaaagcagccaaatccttcagtctcaagatctcagactcacagctgggggatgccgcgatgtatttctgtgcttataggagcg |
| SEQ ID NO: 476 | TRAV39*01-5' | gagctgaaagtggaacaaaaccctctgttcctgagcatgcaggagggaaaaaactataccatctactgcaattattcaaccacttcagacagactgtatt |
| SEQ ID NO: 477 | TRAV39*01-3' | cgattaatggcctcacttgataccaaagcccgtctcagcaccctccacatcacagctgccgtgcatgacctctctgccacctacttctgtgccgtggaca |
| SEQ ID NO: 478 | TRAV4*01-5' | cttgctaagaccacccagcccatctccatggactcatatgaaggacaagaagtgaacataacctgtagccacaacaacattgctacaaatgattatatca |
| SEQ ID NO: 479 | TRAV4*01-3' | gcctccctgtttatccctgccgacagaaagtccagcactctgagcctgccccgggtttccctgagcgacactgctgtgtactactgcctcgtgggtgaca |
| SEQ ID NO: 480 | TRAV40*01-5' | agcaattcagtcaagcagacgggccaaataaccgtctcggagggagcatctgtgactatgaactgcacatacacatccacggggtaccctacccttttct |
| SEQ ID NO: 481 | TRAV40*01-3' | aaaacttcggaggcggaaatattaaagacaaaaactcccccattgtgaaatattcagtccaggtatcagactcagccgtgtactactgtcttctgggaga |
| SEQ ID NO: 482 | TRAV41*01-5' | aaaaatgaagtggagcagagtcctcagaacctgactgcccaggaaggagaatttatcacaatcaactgcagttactcggtaggaataagtgccttacact |
| SEQ ID NO: 483 | TRAV41*01-3' | aagattaattgccacaataaacatacaggaaaagcacagctccctgcacatcacagcctcccatcccagagactctgccgtctacatctgtgctgtcaga |
| SEQ ID NO: 484 | TRAV5*01-5' | ggagaggatgtggagcagagtcttttcctgagtgtccgagagggagacagctccgttataaactgcacttacacagacagctcctccacctacttatact |
| SEQ ID NO: 485 | TRAV5*01-3' | agactcactgttctattgaataaaaaggataaacatctgtctctgcgcattgcagacacccagactggggactcagctatctacttctgtgcagagagta |
| SEQ ID NO: 486 | TRAV6*01-5' | agccaaaagatagaacagaattccgaggccctgaacattcaggagggtaaaacggccaccctgacctgcaactatacaaactattccccagcatacttac |
| SEQ ID NO: 487 | TRAV6*01-3' | agactgaaggtcacctttgataccacccttaaacagagtttgtttcatatcacagcctcccagcctgcagactcagctacctacctctgtgctctagaca |
| SEQ ID NO: 488 | TRAV6*02-5' | agccaaaagatagaacagaattccgaggccctgaacattcaggagggtaaaacggccaccctgacctgcaactatacaaactattctccagcatacttac |
| SEQ ID NO: 489 | TRAV6*02-3' | gaaagaaagactgaaggtcacctttgataccacccttaaacagagtttgtttcatatcacagcctcccagcctgcagactcagctacctacctctgtgct |
| SEQ ID NO: 490 | TRAV6*03-5' | gaggccctgaacattcaggagggtaaaacggccaccctgacctgcaactatacaaactattctccagcatacttacagtggtaccgacaagatccaggaa |
| SEQ ID NO: 491 | TRAV6*03-3' | gaaagaaagactgaaggtcacctttgataccacccttaaacagagtttgtttcatatcacagcctcccagcctgcagactcagctacctacctctgtgct |
| SEQ ID NO: 492 | TRAV6*04-5' | gaggccctgaacattcaggagggtaaaacggccaccctgacctgcaactatacaaactattctccagcatacttacagtggtaccgacaagatccaggaa |
| SEQ ID NO: 493 | TRAV6*04-3' | gaaagaaagactgaaggtcacctttgataccacccttaaacagagtttgtttcatgtcacagcctcccagcctgcagactcagctacctacctctgtgct |
| SEQ ID NO: 494 | TRAV6*05-5' | gaggccctgaacattcaggagggtaaaacggccaccctgacctgcaactatacgaactattctccagcatacttacagtggtaccgacaagatccaggaa |
| SEQ ID NO: 495 | TRAV6*05-3' | gaaagaaagactgaaggtcacctttgataccacccttaaacagagtttgtttcatatcacagcctcccagcctgcagactcagctacctacctctgtgct |
| SEQ ID NO: 496 | TRAV6*06-5' | agccaaaagatagaacagaattccgaggccctgaacattcaggagggtaaaacggccaccctgacctgcaactatacaaactattctccagcatacttac |
| SEQ ID NO: 497 | TRAV6*06-3' | ccaggaagaggccctgttttcttgctactcatacgtgaaaatgagaaagaaaaaaggaaagaaagactgaaggtcacctttgataccacccttaaccaga |
| SEQ ID NO: 498 | TRAV7*01-5' | gaaaaccaggtggagcacagccctcattttctgggaccccagcagggagacgttgcctccatgagctgcacgtactctgtcagtcgttttaacaatttgc |
| SEQ ID NO: 499 | TRAV7*01-3' | aaaggaagactaaatgctacattactgaagaatggaagcagcttgtacattacagccgtgcagcctgaagattcagccacctatttctgtgctgtagatg |
| SEQ ID NO: 50C | TRAV8-1*01-5' | gcccagtctgtgagccagcataaccaccacgtaattctctctgaagcagcctcactggagttgggatgcaactattcctatggtggaactgttaatctct |
| SEQ ID NO: 501 | TRAV8-1*01-3' | gctttgaggctgaatttataaagagtaaattctcctttaatctgaggaaaccctctgtgcagtggagtgacacagctgagtacttctgtgccgtgaatgc |
| SEQ ID NO: 502 | TRAV8-1*02-5' | gcccagtctgtgagccagcataaccaccacgtaattctctctgaagcagcctcactggagttgggatgcaactattcctatggtggaactgttaatctct |
| SEQ ID NO: 503 | TRAV8-1*02-3' | ttttcaggggatccactggttaaaggcatcaagggcgttgaggctgaatttataaagagtaaattctcctttaatctgaggaaaccctctgtgcagtgga |
| SEQ ID NO: 504 | TRAV8-2*01-5' | gcccagtcggtgacccagcttgacagccacgtctctgtctctgaaggaaccccggtgctgctgaggtgcaactactcatcttcttattcaccatctctct |
| SEQ ID NO: 505 | TRAV8-2*01-3' | gttttgaggctgaatttaagaagagtgaaacctccttccacctgacgaaaccctcagcccatatgagcgacgcggctgagtacttctgtgttgtgagtga |
| SEQ ID NO: 506 | TRAV8-2*02-5' | gcccagtcggtgacccagcttagcagccacgtctctgtctctgaaggaaccccggtgctgctgaggtgcaactactcatcttcttattcaccatctctct |
| SEQ ID NO: 507 | TRAV8-2*02-3' | tttaagaagagtgaaacctccttccacctgacgaaaccctcagcccatatgagcgacgcggctgagtacttctgtgttgtgacccgtcacgagctttcag |
| SEQ ID NO: 508 | TRAV8-3*01-5' | gcccagtcagtgacccagcctgacatccacatcactgtctctgaaggagcctcactggagttgagatgtaactattcctatggggcaacaccttatctct |
| SEQ ID NO: 509 | TRAV8-3*01-3' | gctttgaggctgaatttaagaggagtcaatcttccttcaatctgaggaaaccctctgtgcattggagtgatgctgctgagtacttctgtgctgtgggtgc |
| SEQ ID NO: 510 | TRAV8-3*02-5' | gcccagtcagtgacccagcctgacatccacatcactgtctctgaaggagcctcactggagttgagatgtaactattcctatggggcaacaccttatctct |
| SEQ ID NO: 511 | TRAV8-3*02-3' | aggctttgaggctgaatttaagaggagtcaatcttccttcaacctgaggaaaccctctgtgcattggagtgatgctgctgagtacttctgtgctgtggtt |
| SEQ ID NO: 512 | TRAV8-3*03-5' | gcccagtcagtgacccagcctgacatccacatcactgtctctgaaggagcctcactggagttgagatgtaactattcctatggggcaacaccttatctct |
| SEQ ID NO: 513 | TRAV8-3*03-3' | tattaaaggctttgaggctgaatttaagaggagtcaatcttccttcaatctgaggaaaccctctgtgcattggagtgatgcgtctgagtacttctgtgct |
| SEQ ID NO: 514 | TRAV8-4*01-5' | gcccagtcggtgacccagcttggcagccacgtctctgtctctgaaggagccctggttctgctgaggtgcaactactcatcgtctgttccaccatatctct |
| SEQ ID NO: 515 | TRAV8-4*01-3' | gttttgaggctgaatttaagaagagtgaaacctccttccacctgacgaaaccctcagcccatatgagcgacgcggctgagtacttctgtgctgtgagtga |
| SEQ ID NO: 516 | TRAV8-4*02-5' | gcccagtcggtgacccagcttggcagccacgtctctgtctctgaaggagccctggttctgctgaggtgcaactactcatcgtctgttccaccatatctct |
| SEQ ID NO: 517 | TRAV8-4*02-3' | gaatttaagaagagtgaaacctccttccacctgacaaaaccctcagcccatatgagcgacgcggctgagtacttctgtgctgtgagtgatctcgaaccga |
| SEQ ID NO: 518 | TRAV8-4*03-5' | gcccagtcggtgacccagcttggcagccacgtctctgtctctgagggagccctggttctgctgaggtgcaactactcatcgtctgttccaccatatctct |
| SEQ ID NO: 519 | TRAV8-4*03-3' | catcaacggttttgaggctgaatttaagaagagtgaaacctccttccacctgacgaaaccctcagcccatatgagcgacgcggctgagtacttctgtgct |
| SEQ ID NO: 520 | TRAV8-4*04-5' | gcccagtcggtgacccagcttggcagccacgtctctgtctctgaacgagccctggttctgctgaggtgcaactactcatcgtctgttccaccatatctct |
| SEQ ID NO: 521 | TRAV8-4*04-3' | aggcatcaacggttttgaggctgaatttaagaagagtgaaacctccttccacctgacgaaaccctcagcccatatgagcgacgcggctgagtacttctgt |
| SEQ ID NO: 522 | TRAV8-4*05-5' | gcccagtcggtgacccagcttggcagccacgtctctgtctctgaaggagccctggttctgctgaggtgcaactactcatcgtctgttccaccatatctct |
| SEQ ID NO: 523 | TRAV8-4*05-3' | ggctgaatttaagaagagtgaaacctccttccacctgacgaaaccctcagcccatatgagcgacgcggctgagtacttctgtgctgtgagtgagtctcca |
| SEQ ID NO: 524 | TRAV8-4*06-5' | ctcttctggtatgtgcaataccccaaccaggactccagcttctcctgaagtacacatcagcggccaccctggttaaaggcatcaacggttttgaggctg |
| SEQ ID NO: 525 | TRAV8-4*06-3' | gaatttaagaagagtgaaacctccttccacctgacgaaacccgcagcccatatgagcgacgcggctgagtacttctgtcctgtgagtgatctcgaaccga |
| SEQ ID NO: 526 | TRAV8-4*07-5' | gttgaaccatatctcttctggtatgtgcaataccccaaccaaggactccagcttctcctgaagtacacaacaggggccaccctggttaaaggcatcaacg |
| SEQ ID NO: 527 | TRAV8-4*07-3' | acggttttgaggctgaatttaaaaagagtgaaacctccttccacctgacgaaaccctcagcccatatgaccgacccggctgagtacttctgtgctgtgag |
| SEQ ID NO: 528 | TRAV8-5*01-5' | gcccagtcagtgacccagcctgacatccgcatcactgtctctgaaggagcctcactggagttgagatgtaactattcctatggggcgatgttgtgggaag |
| SEQ ID NO: 529 | TRAV8-5*01-3' | tggacacttatcacttccccaatcaatacccctgtgatttcctatgcctgtctttactttaatctcttaatcctgtcagctgaggaggatgtatgtcacc |
| SEQ ID NO: 53C | TRAV8-6*01-5' | gcccagtctgtgacccagcttgacagccaagtccctgtctttgaagaagcccctgtggagctgaggtgcaactactcatcgtctgtttcagtgtatctct |
| SEQ ID NO: 531 | TRAV8-6*01-3' | gttttgaggctgaatttaacaagagtcaaacttccttccacttgaggaaaccctcagtccatataagcgacacggctgagtacttctgtgctgtgagtga |
| SEQ ID NO: 532 | TRAV8-6*02-5' | gcccagtctgtgacccagcttgacagccaagtccctgtctttgaagaagcccctgtggagctgaggtgcaactactcatcgtctgtttcagtgtatctct |
| SEQ ID NO: 533 | TRAV8-6*02-3' | gttttgaggctgaatttaacaagagtcaaacttccttccacttgaggaaaccctcagtccatataagcgacacggctgagtacttctgtgctgtgagtga |
| SEQ ID NO: 534 | TRAV8-7*01-5' | acccagtcggtgacccagcttgatggccacatcactgtctctgaagaagcccctctggaactgaagtgcaactattcctatagtggagttccttctctct |
| SEQ ID NO: 535 | TRAV8-7*01-3' | aggctgaatttaagaagagcgaaacctccttctacctgaggaaaccatcaacccatgtgagtgatgctgctgagtacttctgtgctgtgggtgacaggag |
| SEQ ID NO: 536 | TRAV9-1*01-5' | ggagattcagtggtccagacagaaggccaagtgctcccctctgaaggggattccctgattgtgaactgctcctatgaaaccacacagtacccttcccttt |
| SEQ ID NO: 537 | TRAV9-1*01-3' | gttttgaagccatgtaccgtaaagaaaccacttctttccacttggagaaagactcagttcaagagtcagactccgctgtgtacttctgtgctctgagtga |
| SEQ ID NO: 538 | TRAV9-2*01-5' | ggaaattcagtgacccagatggaagggccagtgactctctcagaagaggccttcctgactataaactgcacgtacacagccacaggatacccttcccttt |
| SEQ ID NO: 539 | TRAV9-2*01-3' | gttttgaagccacataccgtaaagaaaccacttctttccacttggagaaaggctcagttcaagtgtcagactcagcggtgtacttctgtgctctgagtga |
| SEQ ID NO: 54C | TRAV9-2*02-5' | ggagattcagtgacccagatggaagggccagtgactctctcagaagaggccttcctgactataaactgcacgtacacagccacaggatacccttcccttt |
| SEQ ID NO: 541 | TRAV9-2*02-3' | caacaaaggttttgaagccacataccgtaaagaaaccacttctttccacttggagaaaggctcagttcaagtgtcagactcagcggtgtacttctgtgct |
| SEQ ID NO: 542 | TRAV9-2*03-5' | ggagattcagtgacccagatggaagggccagtgactctctcagaagaggccttcctgactataaactgcacgtacacagccacaggatacccttcccttt |
| SEQ ID NO: 543 | TRAV9-2*03-3' | caacaaaggttttgaagccacataccgtaaggaaaccacttctttccacttggagaaaggctcagttcaagtgtcagactcagcggtgtacttctgtgct |
| SEQ ID NO: 544 | TRAV9-2*04-5' | ggaaattcagtgacccagatggaagggccagtgactctctcagaagaggccttcctgactataaactgcacgtacacagccacaggatacccttcccttt |
| SEQ ID NO: 545 | TRAV9-2*04-3' | caacaaaggttttgaagccacataccgtaaggaaaccacttctttccacttggagaaaggctcagttcaagtgtcagactcagcggtgtacttctgtgct |
| SEQ ID NO: 546 | TRBV1*01-5' | gatactggaattacccagacaccaaaatacctggtcacagcaatggggagtaaaaggacaatgaaacgtgagcatctgggacatgattctatgtattggt |
| SEQ ID NO: 547 | TRBV1*01-3' | acttcacacctgaatgccctgacagctctcgcttataccttcatgtggtcgcactgcagcaagaagactcagctgcgtatctctgcaccagcagccaaga |
| SEQ ID NO: 548 | TRBV10-1*01-5' | gatgctgaaatcacccagagcccaagacacaagatcacagagacaggaaggcaggtgaccttggcgtgtcaccagacttggaaccacaacaatatgttct |
| SEQ ID NO: 549 | TRBV10-1*01-3' | gctacagtgtctctagatcaaacacagaggacctccccctcactctggagtctgctgcctcctcccagacatctgtatatttctgcgccagcagtgagtc |
| SEQ ID NO: 550 | TRBV10-1*02-5' | gatgctgaaatcacccagagcccaagacacaagatcacagagacaggaaggcaggtgaccttggcgtgtcaccagacttggaaccacaacaatatgttct |
| SEQ ID NO: 551 | TRBV10-1*02-3' | agatggctacagtgtctctagatcaaacacagaggacctccccctcactctggagtctgctgcctcctcccagacatctgtatatttctgcgccagcagt |
| SEQ ID NO: 552 | TRBV10-1*03-5' | aggcaggtgaccttggcgtgtcaccagacttggaaccacaacaatatgttctggtatcgacaagacctgggacatgggctgaggctgatccattactcat |
| SEQ ID NO: 553 | TRBV10-1*03-3' | ctaacaaaggagaagtctcagatggctacagtgtctctagatcaaacacagaggacctccccctcactctgtagtctgctgcctcctcccagacatctgt |
| SEQ ID NO: 554 | TRBV10-2*01-5' | gatgctggaatcacccagagcccaagatacaagatcacagagacaggaaggcaggtgaccttgatgtgtcaccagacttggagccacagctatatgttct |
| SEQ ID NO: 555 | TRBV10-2*01-3' | gctatgttgtctccagatccaagacagagaatttccccctcactctggagtcagctacccgctcccagacatctgtgtatttctgcgccagcagtgagtc |
| SEQ ID NO: 556 | TRBV10-2*02-5' | aaggcaggtgaccttgatgtgtcaccagacttggagccacagctatatgttctggtatcgacaagacctgggacatgggctgaggctgatctattactca |
| SEQ ID NO: 557 | TRBV10-2*02-3' | agataaaggagaagtccccgatggctacgttgtctccagatccaagacagagaatttccccctcactctggagtcagctacccgctcccagacatctgtg |
| SEQ ID NO: 558 | TRBV10-3*01-5' | gatgctggaatcacccagagcccaagacacaaggtcacagagacaggaacaccagtgactctgagatgtcaccagactgagaaccaccgctatatgtact |
| SEQ ID NO: 559 | TRBV10-3*01-3' | gctatagtgtctctagatcaaagacagaggatttcctcctcactctggagtccgctaccagctcccagacatctgtgtacttctgtgccatcagtgagtc |
| SEQ ID NO: 56C | TRBV10-3*02-5' | gatgctggaatcacccagagcccaagacacaaggtcacagagacaggaacaccagtgactctgagatgtcatcagactgagaaccaccgctatatgtact |
| SEQ ID NO: 561 | TRBV10-3*02-3' | gctatagtgtctctagatcaaagacagaggatttcctcctcactctggagtccgctaccagctcccagacatctgtgtacttctgtgccatcagtgagtc |
| SEQ ID NO: 562 | TRBV10-3*03-5' | gatgctggaatcacccagagcccaagacacaaggtcacagagacaggaacaccagtgactctgagatgtcaccagactgagaaccaccgctacatgtact |
| SEQ ID NO: 563 | TRBV10-3*03-3' | agaagtctcagatggctatagtgtctctagatcaaagacagaggatttcctcctcactctggagtccgctaccagctcccagacatctgtgtacttctgt |
| SEQ ID NO: 564 | TRBV10-3*04-5' | gatgctggaatcacccagagcccaagacacaaggtcacagagacaggaacaccagtgactctgagatgtcaccagactgagaaccaccgctacatgtact |
| SEQ ID NO: 565 | TRBV10-3*04-3' | agaagtctcagatggctatagtgtctctagatcaaagacagaggatttcctcctcactctggagtccgctaccagctcccagacatctgtgtacttctgt |
| SEQ ID NO: 566 | TRBV11-1*01-S' | gaagctgaagttgcccagtcccccagatataagattacagagaaaagccaggctgtggctttttggtgtgatcctatttctggccatgctaccctttact |
| SEQ ID NO: 567 | TRBV11-1*01-3' | gattttctgcagagaggctcaaaggagtagactccactctcaagatccagcctgcagagcttggggactcggccatgtatctctgtgccagcagcttagc |
| SEQ ID NO: 568 | TRBV11-2*01-5' | gaagctggagttgcccagtctcccagatataagattatagagaaaaggcagagtgtggctttttggtgcaatcctatatctggccatgctaccctttact |
| SEQ ID NO: 569 | TRBV11-2*01-3' | gattttctgcagagaggctcaaaggagtagactccactctcaagatccagcctgcaaagcttgaggactcggccgtgtatctctgtgccagcagcttaga |
| SEQ ID NO: 570 | TRBV11-2"02-5' | gaagctggagttgcccagtctcccagatataagattatagagaaaaggcagagtgtggctttttggtgcaatcctatatctggccatgctaccctttact |
| SEQ ID NO: 571 | TRBV11-2*02-3' | ggatcgattttctgcagagaggctcaaaggagtagactccactctcaagatccagcctgcaaagcttgagaactcggccgtgtatctctgtgccagcagt |
| SEQ ID NO: 572 | TRBV11-2*03-5' | gaagctggagttgcccagtctcccagatataagattatagagaaaaggcagagtgtggctttttggtgcaatcctatatctggccatgctaccctttact |
| SEQ ID NO: 573 | TRBV11-2*03-3' | ggatcgattttctgcagagaggctcaaaggagtagactccactctcaagatccaacctgcaaagcttgaggactcggccgtgtatctctgtgccagcagc |
| SEQ ID NO: 574 | TRBV11-3*01-S' | gaagctggagtggttcagtctcccagatataagattatagagaaaaaacagcctgtggctttttggtgcaatcctatttctggccacaataccctttact |
| SEQ ID NO: 575 | TRBV11-3*01-3' | gattttctgcagagaggctcaaaggagtagactccactctcaagatccagcctgcagagcttggggactcggccgtgtatctctgtgccagcagcttaga |
| SEQ ID NO: 576 | TRBV11-3*02-5' | gaagctggagtggttcagtctcccagatataagattatagagaaaaagcagcctgtggctttttggtgcaatcctatttctggccacaataccctttact |
| SEQ ID NO: 577 | TRBV11-3*02-3' | ggatcgattttctgcagagaggctcaaaggagtagactccactctcaagatccagcctgcagagcttggggactcggccgtgtatctctgtgccagcagc |
| SEQ ID NO: 578 | TRBV11-3*03-5' | ggtctcccagatataagattatagagaagaaacagcctgtggctttttggtgcaatccaatttctggccacaataccctttactggtacctgcagaactt |
| SEQ ID NO: 579 | TRBV11-3*03-3' | ggatcgattttctgcagagaggctcaaaggagtagactccactctcaagatccagccagcagagcttggggactcggccatgtatctctgtgccagcagc |
| SEQ ID NO: 580 | TRBV12-1*01-5' | gatgctggtgttatccagtcacccaggcacaaagtgacagagatgggacaatcagtaactctgagatgcgaaccaatttcaggccacaatgatcttctct |
| SEQ ID NO: 581 | TRBV12-1*01-3' | gattctcagcacagatgcctgatgtatcattctccactctgaggatccagcccatggaacccagggacttgggcctatatttctgtgccagcagctttgc |
| SEQ ID NO: 582 | TRBV12-2*01-5' | gatgctggcattatccagtcacccaagcatgaggtgacagaaatgggacaaacagtgactctgagatgtgagccaatttttggccacaatttccttttct |
| SEQ ID NO: 583 | TRBV12-2*01-3' | gattctcagctgagaggcctgatggatcattctctactctgaagatccagcctgcagagcagggggactcggccgtgtatgtctgtgcaagtcgcttagc |
| SEQ ID NO: 584 | TRBV12-3*01-5' | gatgctggagttatccagtcaccccgccatgaggtgacagagatgggacaagaagtgactctgagatgtaaaccaatttcaggccacaactcccttttct |
| SEQ ID NO: 585 | TRBV12-3*01-3' | gattctcagctaagatgcctaatgcatcattctccactctgaagatccagccctcagaacccagggactcagctgtgtacttctgtgccagcagtttagc |
| SEQ ID NO: 58f | TRBV12-4*01-5' | gatgctggagttatccagtcaccccggcacgaggtgacagagatgggacaagaagtgactctgagatgtaaaccaatttcaggacacgactaccttttct |
| SEQ ID NO: 587 | TRBV12-4*01-3' | gattctcagctaagatgcctaatgcatcattctccactctgaagatccagccctcagaacccagggactcagctgtgtacttctgtgccagcagtttagc |
| SEQ ID NO: 588 | TRBV12-4*02-5' | gatgctggagttatccagtcaccccggcacgaggtgacagagatgggacaagaagtgactctgagatgtaaaccaatttcaggacatgactaccttttct |
| SEQ ID NO: 589 | TRBV12-4*02-3' | tcgattctcagctaagatgcctaatgcatcattctccactctgaggatccagccctcagaacccagggactcagctgtgtacttctgtgccagcagttta |
| SEQ ID NO: 59f | TRBV12-5*01-5' | gatgctagagtcacccagacaccaaggcacaaggtgacagagatgggacaagaagtaacaatgagatgtcagccaattttaggccacaatactgttttct |
| SEQ ID NO: 591 | TRBV12-5*01-3' | gattctcagcagagatgcctgatgcaactttagccactctgaagatccagccctcagaacccagggactcagctgtgtatttttgtgctagtggtttggt |
| SEQ ID NO: 592 | TRBV13*01-5' | gctgctggagtcatccagtccccaagacatctgatcaaagaaaagagggaaacagccactctgaaatgctatcctatccctagacacgacactgtctact |
| SEQ ID NO: 593 | TRBV13*01-3' | gattctcagctcaacagttcagtgactatcattctgaactgaacatgagctccttggagctgggggactcagccctgtacttctgtgccagcagcttagg |
| SEQ ID NO: 594 | TRBV13*02-5' | gctgctggagtcatccagtccccaagacatctgatcagagaaaagagggaaacagccactctgaaatgctatcctatccctagacacgacactgtctact |
| SEQ ID NO: 595 | TRBV13*02-3' | tgatcgattctcagctcaacagttcagtgactatcattctgaactgaacatgagctccttggagctgggggactcagccctgtacttctgtgccagcagc |
| SEQ ID NO: 59F | TRBV14*01-5' | gaagctggagttactcagttccccagccacagcgtaatagagaagggccagactgtgactctgagatgtgacccaatttctggacatgataatctttatt |
| SEQ ID NO: 597 | TRBV14*01-3' | gattcttagctgaaaggactggagggacgtattctactctgaaggtgcagcctgcagaactggaggattctggagtttatttctgtgccagcagccaaga |
| SEQ ID NO: 598 | TRBV14*02-5' | gaagctggagttactcagttccccagccacagcgtaatagagaagggccagactgtgactctgagatgtgacccaatttctggacatgataatctttatt |
| SEQ ID NO: 595 | TRBV14*02-3' | caatcgattcttagctgaaaggactggagggacgtattctactctgaaggtgcagcctgcagaactggaggattctggagtttatttctgtgccagcagc |
| SEQ ID NO: 600 | TRBV15*01-5' | gatgccatggtcatccagaa cccaagataccaggtta cccagtttggaa agccagtgaccctgagttgttctcaga ctttgaaccataa cgtcatgtact |
| SEQ ID NO: 601 | TRBV15*01-3' | acttccaatccaggaggccgaacacttctttctgctttcttgacatccgctcaccaggcctgggggacacagccatgtacctgtgtgccaccagcagaga |
| SEQ ID NO: 602 | TRBV15*02-5' | gatgccatggtcatccagaacccaagataccaggttacccagtttggaaagccagtgaccctgagttgttctcagactttgaaccataacgtcatgtact |
| SEQ ID NO: 603 | TRBV15*02-3' | tgataacttccaatccaggaggccgaacacttctttctgctttcttgacatccgctcaccaggcctgggggacgcagccatgtacctgtgtgccaccagc |
| SEQ ID NO: 604 | TRBV15*03-5' | gatgccatggtcatccagaacccaagataccgggttacccagtttggaaagccagtgaccctgagttgttctcagactttgaaccataacgtcatgtact |
| SEQ ID NO: 605 | TRBV15*03-3' | tgataacttccaatccaggaggccgaacacttctttctgctttctagacatccgctcaccaggcctgggggacgcagccatgtaccagtgtgccaccagc |
| SEQ ID NO: 606 | TRBV16*01-5' | ggtgaagaagtcgcccagactccaaaacatcttgtcagaggggaaggacagaaagcaaaattatattgtgccccaataaaaggacacagttatgtttttt |
| SEQ ID NO: 607 | TRBV16*01-3' | gattttcagctaagtgcctcccaaattcaccctgtagccttgagatccaggctacgaagcttgaggattcagcagtgtatttttgtgccagcagccaatc |
| SEQ ID NO: 608 | TRBV16*02-5' | ggtgaagaagtcgcccagactccaaaacatcttgtcagaggggaaggacagaaagcaaaattatattgtgccccaataaaaggacacagttaggtttttt |
| SEQ ID NO: 609 | TRBV16*02-3' | gattttcagctaagtgcctcccaaattcaccctgtagccttgagatccaggctacgaagcttgaggattcagcagtgtatttttgtgccagcagccaatc |
| SEQ ID NO: 610 | TRBV16*03-5' | ggtgaagaagtcgcccagactccaaaacatcttgtcagaggggaaggacagaaagcaaaattatattgtgccccaataaaaggacacagttatgtttttt |
| SEQ ID NO: 611 | TRBV16*03-3' | ggaaagattttcagctaagtgcctcccaaattcaccctgtagccttgagatccaggctacgaagcttgaggattcagcagtgtatttttgtgccagcagc |
| SEQ ID NO: 612 | TRBV17*01-5' | gagcctggagtcagccagacccccagacacaaggtcaccaacatgggacaggaggtgattctgaggtgcgatccatcttctggtcacatgtttgttcact |
| SEQ ID NO: 613 | TRBV17*01-3' | aacgattcacagctgaaagacctaacggaacgtcttccacgctgaagatccatcccgcagagccgagggactcagccgtgtatctctacagtagcggtgg |
| SEQ ID NO: 614 | TRBV18*01-5' | aatgccggcgtcatgcagaacccaagacacctggtcaggaggaggggacaggaggcaagactgagatgcagcccaatgaaaggacacagtcatgtttact |
| SEQ ID NO: 615 | TRBV18*01-3' | gattttctgctgaatttcccaaagagggccccagcatcctgaggatccagcaggtagtgcgaggagattcggcagcttatttctgtgccagctcaccacc |
| SEQ ID NO: 616 | TRBV19*01-5' | gatggtggaatcactcagtccccaaagtacctgttcagaaaggaaggacagaatgtgaccctgagttgtgaacagaatttgaaccacgatgccatgtact |
| SEQ ID NO: 617 | TRBV19*01-3' | ggtacagcgtctctcgggagaagaaggaatcctttcctctcactgtgacatcggcccaaaagaacccgacagctttctatctctgtgccagtagtataga |
| SEQ ID NO: 618 | TRBV19*02-5' | gatggtggaatcactcagtccccaaagtacctgttcagaaaggaaggacagaatgtgaccctgagttgtgaacagaatttgaaccacgatgccatgtact |
| SEQ ID NO: 619 | TRBV19*02-3' | ggtacagcgtctctcgggagaagaaggaatcctttcctctcactgtgacatcggcccaaaagaacccgacagctttctatctctgtgccagtagtataga |
| SEQ ID NO: 620 | TRBV19*03-5' | gatggtggaatcactcagtccccaaagtacctgttcagaaaggaaggacagaatgtgaccctgagttgtgaacagaatttgaaccacgatgccatgtact |
| SEQ ID NO: 621 | TRBV19*03-3' | tgaagggtacagcgtctctcgggagaagaaggaatcctttcctctcactgtgacatcggcccaaaagaacccgacagctttctatctctgtgccagtagc |
| SEQ ID NO: 622 | TRBV2*01-5' | gaacctgaagtcacccagactcccagccatcaggtcacacagatgggacaggaagtgatcttgcgctgtgtccccatctctaatcacttatacttctatt |
| SEQ ID NO: 623 | TRBV2*01-3' | aattctcagttgaaaggcctgatggatcaaatttcactctgaagatccggtccacaaagctggaggactcagccatgtacttctgtgccagcagtgaagc |
| SEQ ID NO: 624 | TRBV2*02-5' | gaacctgaagtcacccagactcccagccatcaggtcacacagatgggacaggaagtgatcttgcactgtgtccccatctctaatcacttatacttctatt |
| SEQ ID NO: 625 | TRBV2*02-3' | tgatcaattctcagttgaaaggcctgatggatcaaatttcactctgaagatccggtccacaaagctggaggactcagccatgtacttctgtgccagcagt |
| SEQ ID NO: 626 | TRBV2*03-5' | gaacctgaagtcacccagactcccagccatcaggtcacacagatgggacaggaagtgatcttgcgctgtgtccccatctctaatcacttatacttctatt |
| SEQ ID NO: 627 | TRBV2*03-3' | tcaattctcagttgagaggcctgatggatcaaatttcactctgaagatccggtccacaaagctggaggactcagccatgtacttctgtgccagcagtgaa |
| SEQ ID NO: 628 | TRBV20-1*01-5' | ggtgctgtcgtctctcaacatccgagctgggttatctgtaagagtggaacctctgtgaagatcgagtgccgttccctggactttcaggccacaactatgt |
| SEQ ID NO: 629 | TRBV20-1*01-3' | acaagtttctcatcaaccatgcaagcctgaccttgtccactctgacagtgaccagtgcccatcctgaagacagcagcttctacatctgcagtgctagaga |
| SEQ ID NO: 630 | TRBV20-1*02-5' | ggtgctgtcgtctctcaacatccgagcagggttatctgtaagagtggaacctctgtgaagatcgagtgccgttccctggactttcaggccacaactatgt |
| SEQ ID NO: 631 | TRBV20-1*02-3' | gaaggacaagtttctcatcaaccatgcaagcctgaccttgtccactctgacagtgaccagtgcccatcctgaagacagcagcttctacatctgcagtgct |
| SEQ ID NO: 632 | TRBV20-1*03-5' | ggtgctgtcgtctctcaacatccgagctgggttatctgtaagagtggaacctctgtgaagatcgagtgccgttccctggactttcaggccacaactatgt |
| SEQ ID NO: 633 | TRBV20-1*03-3' | gaaggacaagtttctcatcaaccatgcaagcctgaccttgtccactctgacagtgaccagtgcccatcctgaagacagcagcttctacatctgcagtgct |
| SEQ ID NO: 634 | TRBV20-1*04-5' | ggtgctgtcgtctctcaacatccgagcagggttatctgtaagagtggaacctctgtgaagatcgagtgccgttccttggactttcaggccacaactatgt |
| SEQ ID NO: 635 | TRBV20-1*04-3' | ggacaagtttctcatcaaccatgcaagcctgaccttgtccactctgacagtgaccagtgcccatcctgaagacagcagcttctacatctgcagtgctagt |
| SEQ ID NO: 636 | TRBV20-1*05-5' | ggtgctgtcgtctctcaacatccgagcagggttatctgtaagagtggaacctctgtgaagatcgagtgccgttccctggactttcaggccacaactatgt |
| SEQ ID NO: 637 | TRBV20-1*05-3' | ggacaagtttctcatcaaccatgcaagcctgaccttgtccactctgacagtgaccagtgcccatcctgaagacagcagcttctacatctgcagtgctaga |
| SEQ ID NO: 638 | TRBV20-1*06-5' | ggtgctgtcgtctctcaacatccgagtagggttatctgtaagagtggaacctctgtgaagatcgagtgccgttccctggactttcaggccacaactatgt |
| SEQ ID NO: 634 | TRBV20-1*06-3' | gaaggacaagtttctcatcaaccatgcaagcctgaccttgtccactctgacagtgaccagtgcccatcctgaagacagcagcttctacatctgcagtgct |
| SEQ ID NO: 64C | TRBV20-1*07-5' | ggtgctgtcgtctctcaacatccgagcagggttatctgtaagagtggaacctctgtgaagatcgagtgccgttccctggactttcaggccacaactatgt |
| SEQ ID NO: 641. | TRBV20-1*07-3' | ggacaagtttctcatcaaccatgcaagcctgaccttgtccactctgacagtgaccagtgcccatcctgaagacagcagcttctacatctgcagtgctaga |
| SEQ ID NO: 642 | TRBV20/OR9-2*01-5' | agtgctgtcgtctctcaacatccgagcagggttatctgtaagagtggaacctctgtgaacatcgagtgccgttccctggactttcaggccacaactatgt |
| SEQ ID NO: 643 | TRBV20/OR9-2*01-3' | acaagtttcccatcaaccatccaaacctgaccttctccgctctgacagtgaccagtgcccatcctgaagacagcagcttctacatctgcagtgctagaga |
| SEQ ID NO: 64; | TRBV20/OR9-2*02-5' | ggtgctgtcgtctctcaacatccgagcagggttatctgtaagagtggaacctctgtgaacatcgagtgccgttccctggactttcaggccacaactatgt |
| SEQ ID NO: 645 | TRBV20/OR9-2*02-3' | gaaggacaagtttcccatcaaccatccaaacctgaccttctccgctctgacagtgacctgtgcccatcctgaagacagcagcttctacatctgcagtgct |
| SEQ ID NO: 646 | TRBV20/OR9-2*03-5' | agtgctgtcgtctctcaacatccgagcagggttatctgtaagagtggaacctctgtgaacatcgagtgccgttccctggactttcaggccacaactatgt |
| SEQ ID NO: 647 | TRBV20/OR9-2*03-3' | acaagtttcccatcaaccatccaaacctgaccttctccgctctgacagtgaccagtgcccatcctgaagacagcagcttctacatctgcagtgctagaga |
| SEQ ID NO: 648 | TRBV21-1*01-5' | gacaccaaggtcacccagagacctagacttctggtcaaagcaagtgaacagaaagcaaagatggattgtgttcctataaaagcacatagttatgtttact |
| SEQ ID NO: 649 | TRBV21-1*01-3' | gatttttagcccaatgctccaaaaactcatcctgtaccttggagatccagtccacggagtcaggggacacagcactgtatttctgtgccagcagcaaagc |
| SEQ ID NO: 65C | TRBV21/OR9-2*01-5' | gacaccaaggtcacccagagacctagatttctggtcaaagcaaatgaacagaaagcaaagatggactgtgttcctataaaaagacatagttatgtttact |
| SEQ ID NO: 651 | TRBV21/OR9-2*01-3' | gattttcagcccaatgcccccaaaactcaccctgtaccttggagatccagtccacggagtcaggagacacagcacggtatttctgtgccaacagcaaagc |
| SEQ ID NO: 652 | TRBV22-1*01-5' | gatgctgacatctatcagatgccattccagctcactggggctggatgggatgtgactctggagtggaaacggaatttgagacacaatgacatgtactgct |
| SEQ ID NO: 653 | TRBV22-1*01-3' | aggctacgtgtctgccaagaggagaaggggctatttcttctcagggtgaagttggcccacaccagccaaacagctttgtacttctgtcctgggagcgcac |
| SEQ ID NO: 654 | TRBV22/OR9-2*01-5' | gatgctgacatctatcagacgccattccagctcactggggctggatgggatgtgaccctggagtagaaacaatttgagacacaatgacatgtactggtac |
| SEQ ID NO: 655 | TRBV22/OR9-2*01-3' | ggctacggtgtctcccgagaggagaaggggctgtttcttctcatggtgaagctggcccacaccagccaaacagctctgtacttctgtcctgggagtgcac |
| SEQ ID NO: 656 | TRBV23-1*01-5' | catgccaaagtcacacagactccaggacatttggtcaaaggaaaaggacagaaaacaaagatggattgtacccccgaaaaaggacatacttttgtttatt |
| SEQ ID NO: 657 | TRBV23-1*01-3' | gattctcatctcaatgccccaagaacgcaccctgcagcctggcaatcctgtcctcagaaccgggagacacggcactgtatctctgcgccagcagtcaatc |
| SEQ ID NO: 658 | TRBV23/OR9-2*01-5' | catgccaaagtcacacagactccaggatatttggtcaaaggaaaaggaaggaaaacaaagatgtattgtacccccaaaaacggacatacttttgtttgtt |
| SEQ ID NO: 659 | TRBV23/OR9-2*01-3' | gatgcacaagaagcgattctcatctcaatgccccaagaacccaccctgcagcctggcaatcctgtcctcggaaccgggagacaccgcactgtatctctgt |
| SEQ ID NO: 66C | TRBV23/OR9-2*02-5' | catgccaaagtcacacagactccaggatatttggtcaaaggaaaaggaaggaaaacaaagatgtattgtacccccaaaaacggacatacttttgtttgtt |
| SEQ ID NO: 661 | TRBV23/OR9-2*02-3' | gtttttgatttcctttcagaatgaacaagttcttcaagaaatggagatgcacaagaagcgattctcatctcaatgccccaagaacgcaccctgcagcctg |
| SEQ ID NO: 662 | TRBV24-1*01-5' | gatgctgatgttacccagaccccaaggaataggatcacaaagacaggaaagaggattatgctggaatgttctcagactaagggtcatgatagaatgtact |
| SEQ ID NO: 663 | TRBV24-1*01-3' | atacagtgtctctcgacaggcacaggctaaattctccctgtccctagagtctgccatccccaaccagacagctctttacttctgtgccaccagtgatttg |
| SEQ ID NO: 664 | TRBV24/OR9-2*01-5' | gatgctgatgttacccagaccccaaggaataggatcacaaagacaggaaagaggattatgctggaatgttctcagactaagggtcatgatagaatgtact |
| SEQ ID NO: 665 | TRBV24/OR9-2*01-3' | atacagtgtctctcgacaggcacaggctaaattctccctgtccctagagtctgccatccccaaccagacagctctttacttctgtgccaccagtgatttg |
| SEQ ID NO: 666 | TRBV24/OR9-2*02-5' | gatgctgatgttatccagaccccaaggaataggatcacaaagacaggaaagaggattatgctggcatgttctcagactaagggtcatgatggaatgtact |
| SEQ ID NO: 667 | TRBV24/OR9-2*02-3' | cagttgatctattgctcctttgatgtcaaaatatataaacaaaagagagatctctgatggatacagtgtctcttgacaggaacaggctaaattctccctg |
| SEQ ID NO: 668 | TRBV24/OR9-2*03-5' | gatgctgatgttatccagaccccaaggaataggatcacaaagacaggaaagaggattatgctggaatgttctcagactaagggtcatgatggaatgtact |
| SEQ ID NO: 669 | TRBV24/OR9-2*03-3' | agtgtctcttgacaggaacaggctaaattctccctgtccctagagcctgccacccccaaccagacagcttctaggttacttcagtgccaccagtgatttc |
| SEQ ID NO: 670 | TRBV25-1*01-5' | gaagctgacatctaccagaccccaagataccttgttatagggacaggaaagaagatcactctggaatgttctcaaaccatgggccatgacaaaatgtact |
| SEQ ID NO: 671 | TRBV25-1*01-3' | agtcaacagtctccagaataaggacggagcattttcccctgaccctggagtctgccaggccctcacatacctctcagtacctctgtgccagcagtgaata |
| SEQ ID NO: 672 | TRBV25/OR9-2*01-5' | gaagctgaaatctaccagaccccaagacaccgtgttataggggcaggaaagaagatcactctggaatgttctcaaaccatgggccatgacaaaatgtact |
| SEQ ID NO: 673 | TRBV25/OR9-2*01-3' | agtcaacagtctccagaataaggatagagcgttttcccctgaccctggagtctgccagcccctcacatacctctcagtacctctgtgccagcagtgaata |
| SEQ ID NO: 674 | TRBV25/OR9-2*02-5' | gaagctgaaatctaccagaccccaagacaccgtgttataggggcaggaaagaagatcactctggaatgttctcaaaccatgggccatgacaaaatgtact |
| SEQ ID NO: 675 | TRBV25/OR9-2*02-3' | gagttaattccacagagaagggagatctttgctctgagtcaacagtctccagaataaggatagagcgttttcccctgaccctggagtctgccagcccctc |
| SEQ ID NO: 676 | TRBV26*01-5' | gatgctgtagttacacaattcccaagacacagaatcattgggacaggaaaggaattcattctacagtgttcccagaatatgaatcatgttacaatgtact |
| SEQ ID NO: 677 | TRBV26*01-3' | ggtatcatgtttcttgaaatactatagcatcttttcccctgaccctgaagtctgccagcaccaaccagacatctgtgtatctctatgccagcagttcatc |
| SEQ ID NO: 678 | TRBV26/OR9-2*01-5' | gatgctgtagttacacaattctcaagacacagaatcattgggacaggaaaggaattcattctactgtgtccccagaatatgaatcatgttgcaatgtact |
| SEQ ID NO: 679 | TRBV26/OR9-2*01-3' | ggtatcatgtttcttgaaatactatagcatcttttctcctgaccctgaagtctgctagcaccaaccagacatgtgtgtatctctgcgccagcagttcatc |
| SEQ ID NO: 680 | TRBV26/OR9-2*02-5' | gatgctgtagttacacaattcccaagacacagaatcattgggacaggaaaggaattcattctactgtgtccccagaatatgaatcatgttgcaatgtact |
| SEQ ID NO: 681 | TRBV26/OR9-2*02-3' | ggtatcatgtttcttgaaatactatagcatcttttctcctgaccctgaagtctgctagcaccaaccagacatgtgtgtatctctgcgccagcagttcatc |
| SEQ ID NO: 682 | TRBV27*01-5' | gaagcccaagtgacccagaacccaagatacctcatcacagtgactggaaagaagttaacagtgacttgttctcagaatatgaaccatgagtatatgtcct |
| SEQ ID NO: 683 | TRBV27*01-3' | ggtacaaagtctctcgaaaagagaagaggaatttccccctgatcctggagtcgcccagccccaaccagacctctctgtacttctgtgccagcagtttatc |
| SEQ ID NO: 684 | TRBV28*01-5' | gatgtgaaagtaacccagagctcgagatatctagtcaaaaggacgggagagaaagtttttctggaatgtgtccaggatatggaccatgaaaatatgttct |
| SEQ ID NO: 685 | TRBV28*01-3' | ggtacagtgtctctagagagaagaaggagcgcttctccctgattctggagtccgccagcaccaaccagacatctatgtacctctgtgccagcagtttatg |
| SEQ ID NO: 686 | TRBV29-1*01-5' | agtgctgtcatctctcaaaagccaagcagggatatctgtcaacgtggaacctccctgacgatccagtgtcaagtcgatagccaagtcaccatgatgttct |
| SEQ ID NO: 687 | TRBV29-1*01-3' | acaagtttcccatcagccgcccaaacctaacattctcaactctgactgtgagcaacatgagccctgaagacagcagcatatatctctgcagcgttgaaga |
| SEQ ID NO: 688 | TRBV29-1*02-5' | agtgctgtcatctctcaaaagccaagcagggatatctgtcaacgtggaacctccctgacgatccagtgtcaagtcgatagccaagtcaccatgatgttc |
| SEQ ID NO: 689 | TRBV29-1*02-3' | tgacaagtttcccatcagccgcccaaacctaacattctcaagtctgactgtgagcaacatgagccctgaagacagcagcatatatctctgcagcgttgaa |
| SEQ ID NO: 69t | TRBV29-1*03-5' | acgatccagtgtcaagtcgatagccaagtcaccatgatattctggtaccgtcagcaacctggacagagcctgacactgatcgcaactgcaaatcagggct |
| SEQ ID NO: 691 | TRBV29-1*03-3' | tgacaagtttcccatcagccgcccaaacctaacattctcaactctgactgtgagcaacatgagccctgaagacagcagcatatatctctgcagcgcgggc |
| SEQ ID NO: 692 | TRBV29/OR9-2*01-5' | agtgctgtcatctctcaaaagccaagcagggatatctgtcaacgtggaacctccatgatgatccagtgtcaagtcgacagccaagtcaccatgatgttct |
| SEQ ID NO: 693 | TRBV29/OR9-2*01-3' | acaagtttcccatcagccgcccaaacctaacattctcaactctgactgtgagcaacaggagacctgaagacagcagcatatacctctgcagcgttgaaga |
| SEQ ID NO: 694 | TRBV29/OR9-2*02-5' | agtgctgtcatctctcaaaagccaagcagggatatctgtcaacgtggaacctccatgatgatccagtgtcaagtcgacagccaagtcaccatgatgttct |
| SEQ ID NO: 695 | TRBV29/OR9-2*02-3' | acaagtttcccatcagccgcccaaacctaacattctcaactctgactgtgagcaacaggagacctgaagacagcagcatatacctctgcagcgttgaaga |
| SEQ ID NO: 696 | TRBV3-1*01-5' | gacacagctgtttcccagactccaaaatacctggtcacacagatgggaaacgacaagtccattaaatgtgaacaaaatctgggccatgatactatgtatt |
| SEQ ID NO: 697 | TRBV3-1*01-3' | gcttctcacctaaatctccagacaaagctcacttaaatcttcacatcaattccctggagcttggtgactctgctgtgtatttctgtgccagcagccaaga |
| SEQ ID NO: 698 | TRBV3-1*02-5' | gacacagctgtttcccagactccaaaatacctggtcacacagatgggaaacgacaagtccattaaatgtgaacaaaatctgggccatgatactatgtatt |
| SEQ ID NO: 699 | TRBV3-1*02-3' | tccaaatcgattctcacctaaatctccagacaaagctaaattaaatcttcacatcaattccctggagcttggtgactctgctgtgtatttctgtgccagc |
| SEQ ID NO: 700 | TRBV3-2*01-5' | gacacagccgtttcccagactccaaaatacctggtcacacagatgggaaaaaaggagtctcttaaatgagaacaaaatctgggccataatgctatgtatt |
| SEQ ID NO: 701 | TRBV3-2*01-3' | gcttctcacctgactctccagacaaagctcatttaaatcttcacatcaattccctggagcttggtgactctgctgtgtatttctgtgccagcagccaaga |
| SEQ ID NO: 702 | TRBV3-2*02-5' | gacacagccgtttcccagactccaaaatacctggtcacacagatgggaaaaaaggagtctcttaaatgagaacaaaatctgggccataatgctatgtatt |
| SEQ ID NO: 703 | TRBV3-2*02-3' | gcttctcacctgactctccagacaaagttcatttaaatcttcacatcaattccctggagcttggtgactctgctgtgtatttctgtgccagcagccaaga |
| SEQ ID NO: 704 | TRBV3-2*03-5' | gacacagccgtttcccagactccaaaatacctggtcacacagacgggaaaaaaggagtctcttaaatgagaacaaaatctgggccataatgctatgtatt |
| SEQ ID NO: 705 | TRBV3-2*03-3' | tcgcttctcacctgactctccagacaaagttcatttaaatcttcacatcaattccctggagcttggtgactctgctgtgtatttctgtgccagcagccaa |
| SEQ ID NO: 706 | TRBV30*01-5' | tctcagactattcatcaatggccagcgaccctggtgcagcctgtgggcagcccgctctctctggagtgcactgtggagggaacatcaaaccccaacctat |
| SEQ ID NO: 707 | TRBV30*01-3' | agaatctctcagcctccagaccccaggaccggcagttcatcctgagttctaagaagctccttctcagtgactctggcttctatctctgtgcctggagtgt |
| SEQ ID NO: 708 | TRBV30*02-5' | tctcagactattcatcaatggccagcgaccctggtgcagcctgtgggcagcccgctctctctggagtgcactgtggagggaacatcaaaccccaacctat |
| SEQ ID NO: 709 | TRBV30*02-3' | agaatctctcagcctccagaccccaggaccggcagttcatcctgagttctaagaagctcctcctcagtgactctggcttctatctctgtgcctggagtgt |
| SEQ ID NO: 710 | TRBV30*04-5' | actattcatcaatggccagcgaccctggtgcagcctgtgggcagcccgctctctctggagtgcactgtggagggaacatcaaaccccaacctatactggt |
| SEQ ID NO: 711 | TRBV30*04-3' | ccagaatctctcagcctccagaccccaggaccggcagttcattctgagttctaagaagctcctcctcagtgactctggcttctatctctgtgcctggagt |
| SEQ ID NO: 712 | TRBV30*05-5' | tctcagactattcatcaatggccagcgaccctggtgcagcctgtgggcagcccgctctccctggagtgcactgtggagggaacatcaaaccccaacctat |
| SEQ ID NO: 712 | TRBV30*05-3' | ccagaatctctcagcctccagaccccaggaccggcagttcatcctgagttctaagaagctccttctcagtgactctggcttctatctctgtgcctgggga |
| SEQ ID NO: 714 | TRBV4-1*01-5' | gacactgaagttacccagacaccaaaacacctggtcatgggaatgacaaataagaagtctttgaaatgtgaacaacatatggggcacagggctatgtatt |
| SEQ ID NO: 715 | TRBV4-1*01-3' | gcttctcacctgaatgccccaacagctctctcttaaaccttcacctacacgccctgcagccagaagactcagccctgtatctctgcgccagcagccaaga |
| SEQ ID NO: 716 | TRBV4-1*02-5' | cacctggtcatgggaatgacaaataagaagtctttgaaatgtgaacaacatatggggcacagggcaatgtattggtacaagcagaaagctaagaagccac |
| SEQ ID NO: 717 | TRBV4-1*02-3' | tcgcttctcacctgaatgccccaacagctctctcttaaaccttcacctacacgccctgcagccagaagactcagccctgtatctctgcgccagcagccaa |
| SEQ ID NO: 718 | TRBV4-2*01-5' | gaaacgggagttacgcagacaccaagacacctggtcatgggaatgacaaataagaagtctttgaaatgtgaacaacatctggggcataacgctatgtatt |
| SEQ ID NO: 719 | TRBV4-2*01-3' | gcttctcacctgaatgccccaacagctctcacttattccttcacctacacaccctgcagccagaagactcggccctgtatctctgtgccagcagccaaga |
| SEQ ID NO: 720 | TRBV4-2*02-5' | gaaacgggagttacgcagacaccaagacacctggtcatgggaatgacaaataagaagtctttgaaatgtgaacaacatctggggcataacgctatgtatt |
| SEQ ID NO: 721 | TRBV4-2*02-3' | aagtcgcttctcacctgaatgccccaacagctctcacttatgccttcacctacacaccctgcagccagaagactcggccctgtatctctgtgccagcacc |
| SEQ ID NO: 722 | TRBV4-3*01-5' | gaaacgggagttacgcagacaccaagacacctggtcatgggaatgacaaataagaagtctttgaaatgtgaacaacatctgggtcataacgctatgtatt |
| SEQ ID NO: 723 | TRBV4-3*01-3' | gcttctcacctgaatgccccaacagctctcacttattccttcacctacacaccctgcagccagaagactcggccctgtatctctgcgccagcagccaaga |
| SEQ ID NO: 724 | TRBV4-3*02-5' | gaaacgggagttacgcagacaccaagacacctggtcatgggaatgacaaataagaagtctttgaaatgtgaacaacatctgggtcataacgctatgtatt |
| SEQ ID NO: 725 | TRBV4-3*02-3' | aagtcgcttctcacctgaatgccccaacagctctcacttatcccttcacctacacaccctgcagccagaagactcggccctgtatctctgcgccagcagc |
| SEQ ID NO: 726 | TRBV4-3*03-5' | gaaacgggagttacgcagacaccaagacacctggtcatgggaatgacaaataagaagtctttgaaatgtgaacaacatctgggtcataacgctatgtatt |
| SEQ ID NO: 727 | TRBV4-3*03-3' | aagtcgcttctcacctgaatgccccaacagctctcacttattccttcacctacacaccctgcagccagaagactcggccctgtatctctgcgccagcagc |
| SEQ ID NO: 728 | TRBV4-3*04-5' | aagaagtctttgaaatgtgaacaacatctggggcataacgctatgtattggtacaagcaaagtgctaagaagccactggagctcatgtttgtctacagtc |
| SEQ ID NO: 725 | TRBV4-3*04-3' | aagtcgcttctcacctgaatgccccaacagctctcacttattccttcacctacacaccctgcagccagaagactcggccctgtatctctgcgccagcagc |
| SEQ ID NO: 730 | TRBV5-1*01-5' | aaggctggagtcactcaaactccaagatatctgatcaaaacgagaggacagcaagtgacactgagctgctcccctatctctgggcataggagtgtatcct |
| SEQ ID NO: 731 | TRBV5-1*01-3' | cgattctcagggcgccagttctctaactctcgctctgagatgaatgtgagcaccttggagctgggggactcggccctttatctttgcgccagcagcttgg |
| SEQ ID NO: 732 | TRBV5-1*02-5' | agggctggggtcactcaaactccaagacatctgatcaaaacgagaggacagcaagtgacactgggctgctcccctatctctgggcataggagtgtatcct |
| SEQ ID NO: 733 | TRBV5-1*02-3' | tcgattctcagggcgccagttctctaactctcgctctgagatgaatgtgagcaccttggagctgggggactcggccctttatctttgcgccagcgcttgc |
| SEQ ID NO: 734 | TRBV5-2*01-5' | gaggctggaatcacccaagctccaagacacctgatcaaaacaagagaccagcaagtgacactgagatgctcccctgcctctgggcataactgtgtgtcct |
| SEQ ID NO: 735 | TRBV5-2*01-3' | aacttgcctaattgattctcagctcaccacgtccataactattactgagtcaaacacggagctaggggactcagccctgtatctctgtgccagcaacttg |
| SEQ ID NO: 736 | TRBV5-3*01-5' | gaggctggagtcacccaaagtcccacacacctgatcaaaacgagaggacagcaagtgactctgagatgctctcctatctctgggcacagcagtgtgtcct |
| SEQ ID NO: 737 | TRBV5-3*01-3' | cgattctcagggcgccagttccatgactgttgctctgagatgaatgtgagtgccttggagctgggggactcggccctgtatctctgtgccagaagcttgg |
| SEQ ID NO: 738 | TRBV5-3*02-5' | gaggctggagtcacccaaagtcccacacacctgatcaaaacgagaggacagcaagtgactctgagatgctctcctatctctgggcacagcagtgtgtcct |
| SEQ ID NO: 739 | TRBV5-3*02-3' | cgattctcagggcgccagttccatgactattgctctgagatgaatgtgagtgccttggagctgggggactcggccctgtatctctgtgccagaagcttgg |
| SEQ ID NO: 740 | TRBV5-4*01-5' | gagactggagtcacccaaagtcccacacacctgatcaaaacgagaggacagcaagtgactctgagatgctcttctcagtctgggcacaacactgtgtcct |
| SEQ ID NO: 741 | TRBVS-4*01-3' | agattctcaggtctccagttccctaattatagctctgagctgaatgtgaacgccttggagctggacgactcggccctgtatctctgtgccagcagcttgg |
| SEQ ID NO: 742 | TRBV5-4*02-5' | gagactggagtcacccaaagtcccacacacctgatcaaaacgagaggacagcaagtgactctgagatgctcttctcagtctgggcacaacactgtgtcct |
| SEQ ID NO: 743 | TRBVS-4*02-3' | tcctagattctcaggtctccagttccctaattataactctgagctgaatgtgaacgccttggagctggacgactcggccctgtatctctgtgccagcagc |
| SEQ ID NO: 744 | TRBV5-4*03-5' | cagcaagtgacactgagatgctcttctcagtctgggcacaacactgtgtcctggtaccaacaggccctgggtcaggggccccagtttatctttcagtatt |
| SEQ ID NO: 745 | TRBVS-4*03-3' | tcctagattctcaggtctccagttccctaattatagctctgagctgaatgtgaacgccttggagctggacgactcggccctgtatctctgtgccagcagc |
| SEQ ID NO: 746 | TRBVS-4*04-5' | actgtgtcctggtaccaacaggccctgggtcaggggccccagtttatctttcagtattatagggaggaagagaatggcagaggaaactcccctcctagat |
| SEQ ID NO: 747 | TRBV5-4*04-3' | tcctagattctcaggtctccagttccctaattatagctctgagctgaatgtgaacgccttggagctggacgactcggccctgtatctctgtgccagcagc |
| SEQ ID NO: 748 | TRBV5-5*01-5' | gacgctggagtcacccaaagtcccacacacctgatcaaaacgagaggacagcaagtgactctgagatgctctcctatctctgggcacaagagtgtgtcct |
| SEQ ID NO: 749 | TRBV5-5*01-3' | cgattctcagctcgccagttccctaactatagctctgagctgaatgtgaacgccttgttgctgggggactcggccctgtatctctgtgccagcagcttgg |
| SEQ ID NO: 75C | TRBVS-5*02-5' | gacgctggagtcacccaaagtcccacacacctgatcaaaacgagaggacagcacgtgactctgagatgctctcctatctctgggcacaagagtgtgtcct |
| SEQ ID NO: 751 | TRBV5-5*02-3' | tgatcgattctcagctcgccagttccctaactatagctctgagctgaatgtgaacgccttgttgctgggggactcggccctgtatctctgtgccagcagc |
| SEQ ID NO: 752 | TRBV5-5*03-5' | gacgctggagtcacccaaagtcccacacacctgatcaaaacgagaggacagcaagtgactctgagatgctctcctatctctgagcacaagagtgtgtcct |
| SEQ ID NO: 753 | TRBV5-5*03-3' | tgatcgattctcagctcgccagttccctaactatagctctgagctgaatgtgaacgccttgttgctgggggactcggccctgtatctctgtgccagcagc |
| SEQ ID NO: 754 | TRBV5-6*01-5' | gacgctggagtcacccaaagtcccacacacctgatcaaaacgagaggacagcaagtgactctgagatgctctcctaagtctgggcatgacactgtgtcct |
| SEQ ID NO: 755 | TRBV5-6*01-3' | cgattctcaggtcaccagttccctaactatagctctgagctgaatgtgaacgccttgttgctgggggactcggccctctatctctgtgccagcagcttgg |
| SEQ ID NO: 756 | TRBV5-7*01-5' | gacgctggagtcacccaaagtcccacacacctgatcaaaacgagaggacagcacgtgactctgagatgctctcctatctctgggcacaccagtgtgtcct |
| SEQ ID NO: 757 | TRBVS-7*01-3' | caattctcaggtcaccagttccctaactatagctctgagctgaatgtgaacgccttgttgctaggggactcggccctctatctctgtgccagcagcttgg |
| SEQ ID NO: 758 | TRBV5-8*01-5' | gaggctggagtcacacaaagtcccacacacctgatcaaaacgagaggacagcaagcgactctgagatgctctcctatctctgggcacaccagtgtgtact |
| SEQ ID NO: 759 | TRBV5-8*01-3' | agattttcaggtcgccagttccctaattatagctctgagctgaatgtgaacgccttggagctggaggactcggccctgtatctctgtgccagcagcttgg |
| SEQ ID NO: 76C | TRBV5-8*02-5' | aggacagcaagcgactctgagatgctctcctatctctgggcacaccagtgtgtactggtaccaacaggccctgggtctgggcctccagctcctcctttgg |
| SEQ ID NO: 761 | TRBV5-8*02-3' | tcctagattttcaggtcgccagttccctaattatagctctgagctgaatgtgaacgccttggagctggaggactcggccctgtatctctgtgccagcagc |
| SEQ ID NO: 762 | TRBV6-1*01-5' | aatgctggtgtcactcagaccccaaaattccaggtcctgaagacaggacagagcatgacactgcagtgtgcccaggatatgaaccataactccatgtact |
| SEQ ID NO: 763 | TRBV6-1*01-3' | gctacaatgtctccagattaaacaaacgggagttctcgctcaggctggagtcggctgctccctcccagacatctgtgtacttctgtgccagcagtgaagc |
| SEQ ID NO: 764 | TRBV6-2*01-5' | aatgctggtgtcactcagaccccaaaattccgggtcctgaagacaggacagagcatgacactgctgtgtgcccaggatatgaaccatgaatacatgtact |
| SEQ ID NO: 765 | TRBV6-2*01-3' | gctacaatgtctccagattaaaaaaacagaatttcctgctggggttggagtcggctgctccctcccaaacatctgtgtacttctgtgccagcagttactc |
| SEQ ID NO: 766 | TRBV6-2*02-5' | aatgctggtgtcactcagaccccaaaattccgggtcctgaagacaggacagagcatgacactgctgtgtgcccaggatatgaaccatgaatacatgtact |
| SEQ ID NO: 767 | TRBV6-2*02-3' | tggctacaatgtctccagattaaaaaaacagaatttcctgctggggttggagtcggctgctccctcccaaacatctgtgtacttctgtgccagcagccct |
| SEQ ID NO: 768 | TRBV6-3*01-5' | aatgctggtgtcactcagaccccaaaattccgggtcctgaagacaggacagagcatgacactgctgtgtgcccaggatatgaaccatgaatacatgtact |
| SEQ ID NO: 769 | TRBV6-3*01-3' | gctacaatgtctccagattaaaaaaacagaatttcctgctggggttggagtcggctgctccctcccaaacatctgtgtacttctgtgccagcagttactc |
| SEQ ID NO: 77C | TRBV6-4*01-5' | attgctgggatcacccaggcaccaacatctcagatcctggcagcaggacggcgcatgacactgagatgtacccaggatatgagacataatgccatgtact |
| SEQ ID NO: 771 | TRBV6-4*01-3' | gttatagtgtctccagagcaaacacagatgatttccccctcacgttggcgtctgctgtaccctctcagacatctgtgtacttctgtgccagcagtgactc |
| SEQ ID NO: 772 | TRBV6-4*02-5' | actgctgggatcacccaggcaccaacatctcagatcctggcagcaggacggagcatgacactgagatgtacccaggatatgagacataatgccatgtact |
| SEQ ID NO: 773 | TRBV6-4*02-3' | gttatagtgtctccagagcaaacacagatgatttccccctcacgttggcgtctgctgtaccctctcagacatctgtgtacttctgtgccagcagtgactc |
| SEQ ID NO: 774 | TRBV6-5*01-5' | aatgctggtgtcactcagaccccaaaattccaggtcctgaagacaggacagagcatgacactgcagtgtgcccaggatatgaaccatgaatacatgtcct |
| SEQ ID NO: 775 | TRBV6-5*01-3' | gctacaatgtctccagatcaaccacagaggatttcccgctcaggctgctgtcggctgctccctcccagacatctgtgtacttctgtgccagcagttactc |
| SEQ ID NO: 776 | TRBV6-6*01-5' | aatgctggtgtcactcagaccccaaaattccgcatcctgaagataggacagagcatgacactgcagtgtacccaggatatgaaccataactacatgtact |
| SEQ ID NO: 777 | TRBV6-6*01-3' | gctacaacgtctccagatcaaccacagaggatttcccgctcaggctggagttggctgctccctcccagacatctgtgtacttctgtgccagcagttactc |
| SEQ ID NO: 778 | TRBV6-6*02-5' | aatgctggtgtcactcagaccccaaaattccgcatcctgaagataggacagagcatgacactgcagtgtgcccaggatatgaaccataactacatgtact |
| SEQ ID NO: 779 | TRBV6-6*02-3' | gaatggctacaacgtctccagatcaaccacagaggatttcccgctcaggctggagttggctgctccctcccagacatctgtgtacttctgtgccagcagt |
| SEQ ID NO: 78C | TRBV6-6*03-5' | aatgctggtgtcactcagaccccaaaattccgcatcctgaagataggacagagcatgacactgcagtgtgcccaggatatgaaccataactacatgtact |
| SEQ ID NO: 781 | TRBV6-6*03-3' | gaatggctacaacgtctccagatcaaccacagaggatttcccgctcaggctggagttggctgctccctcccagacatctgtgtacttctgtgccagcagt |
| SEQ ID NO: 782 | TRBV6-6*04-5' | aatgctggtgtcactcagaccccaaaattccgcatcctgaagataggacagagcatgacactgcagtgtacccaggatatgaaccatgaatacatgtact |
| SEQ ID NO: 783 | TRBV6-6*04-3' | tggctacaatgtctccagatcaaccacagaggatttcccgctcaggctggagttggctgctccctcccagacatctgtgtacttctgtgccagcagtcga |
| SEQ ID NO: 784 | TRBV6-6*05-5' | aatgctggtgtcactcagaccccaaaattccgcatcctgaagataggacagagcatgacactgcagtgtgcccaggatatgaaccataactacatgtact |
| SEQ ID NO: 785 | TRBV6-6*05-3' | gaatggctacaacgtctccagatcaaccacagaggatttcccgctcaggctggagttggctgctgcctcccagacatctgtgtacttctgtgccagcagc |
| SEQ ID NO: 786 | TRBV6-7*01-5' | aatgctggtgtcactcagaccccaaaattccacgtcctgaagacaggacagagcatgactctgctgtgtgcccaggatatgaaccatgaatacatgtatc |
| SEQ ID NO: 787 | TRBV6-7*01-3' | gctacaatgtctccagatcaaacacagaggatttccccctcaagctggagtcagctgctccctctcagacttctgtttacttctgtgccagcagttactc |
| SEQ ID NO: 788 | TRBV6-8*01-5' | aatgctggtgtcactcagaccccaaaattccacatcctgaagacaggacagagcatgacactgcagtgtgcccaggatatgaaccatggatacatgtcct |
| SEQ ID NO: 785 | TRBV6-8*01-3' | gctacaatgtctctagattaaacacagaggatttcccactcaggctggtgtcggctgctccctcccagacatctgtgtacttgtgtgccagcagttactc |
| SEQ ID NO: 790 | TRBV6-9*01-5' | aatgctggtgtcactcagaccccaaaattccacatcctgaagacaggacagagcatgacactgcagtgtgcccaggatatgaaccatggatacttgtcct |
| SEQ ID NO: 791 | TRBV6-9*01-3' | gctacaatgtatccagatcaaacacagaggatttcccgctcaggctggagtcagctgctccctcccagacatctgtatacttctgtgccagcagttattc |
| SEQ ID NO: 792 | TRBV7-1*01-5' | ggtgctggagtctcccagtccεtgagacacaaggtagcaaagaagggaaaggatgtagctctcagatatgatccaatttcaggtcataatgccctttatt |
| SEQ ID NO: 793 | TRBV7-1*01-3' | ggttctctgcacagaggtctgagggatccatctccactctgaagttccagcgcacacagcagggggacttggctgtgtatctctgtgccagcagctcagc |
| SEQ ID NO: 794 | TRBV7-2*01-5' | ggagctggagtctcccagtcccccagtaacaaggtcacagagaagggaaaggatgtagagctcaggtgtgatccaatttcaggtcatactgccctttact |
| SEQ ID NO: 795 | TRBV7-2*01-3' | gcttctctgcagagaggactgggggatccgtctccactctgacgatccagcgcacacagcaggaggactcggccgtgtatctctgtgccagcagcttagc |
| SEQ ID NO: 796 | TRBV7-2*02-5' | ggagctggagtctcccagtcccccagtaacaaggtcacagagaagggaaaggatgtagagctcaggtgtgatccaatttcaggtcatactgccctttact |
| SEQ ID NO: 797 | TRBV7-2*02-3' | gcttctctgcagagaggactggggaatccgtctccactctgacgatccagcgcacacagcaggaggactcggccgtgtatctctgtgccagcagcttagc |
| SEQ ID NO: 798 | TRBV7-2*03-5' | ggagctggagtctcccagtcccccagtaacaaggtcacagagaagggaaaggatgtagagctcaggtgtgatccaatttcaggtcatactgccctttact |
| SEQ ID NO: 799 | TRBV7-2*03-3' | gcttctctgcagagaggactggggaatccgtctccactctgacgatccagcgcacacagcaggaggactcggccgtgtatctctgtaccagcagcttagc |
| SEQ ID NO: 800 | TRBV7-2*04-5' | ggagctggagtttcccagtcccccagtaacaaggtcacagagaagggaaaggatgtagagctcaggtgtgatccaatttcaggtcatactgccctttact |
| SEQ ID NO: 801 | TRBV7-2*04-3' | tcgcttctctgcagagaggactgggggatccgtctccactctgacgatccagcgcacacagcaggaggactcggccgtgtatctctgtgccagcagctta |
| SEQ ID NO: 802 | TRBV7-3*01-5' | ggtgctggagtctcccagacccccagtaacaaggtcacagagaagggaaaatatgtagagctcaggtgtgatccaatttcaggtcatactgccctttact |
| SEQ ID NO: 803 | TRBV7-3*01-3' | ggttctttgcagtcaggcctgagggatccgtctctactctgaagatccagcgcacagagcggggggactcagccgtgtatctctgtgccagcagcttaac |
| SEQ ID NO: 804 | TRBV7-3*02-5' | ggtgctggagtctcccagacccccagtaacaaggtcacagagaagggaaaagatgtagagctcaggtgtgatccaatttcaggtcatactgccctttact |
| SEQ ID NO: 805 | TRBV7-3*02-3' | ggttctttgcagtcaggcctgagggatccgtctctactctgaagatccagcgcacagagcagggggactcagccgtgtatctccgtgccagcagcttaac |
| SEQ ID NO: 806 | TRBV7-3*03-5' | ggtgctggagtctcccagacccccagtaacaaggtcacagagaagggaaaagatgtagagctcaggtgtgatccaatttcaggtcatactgccctttact |
| SEQ ID NO: 807 | TRBV7-3*03-3' | ggttctttgcagtcaggcctgagggatccgtctctactctgaagatccagcgcacagagcagggggactcagccgcgtatctccgtgccagcagcttaac |
| SEQ ID NO: 808 | TRBV7-3*04-5' | ggtgctggagtctcccagacccccagtaacaaggtcacagagaagggaaaatatgtagagctcaggtgtgatccaatttcaggtcatactgccctttact |
| SEQ ID NO: 809 | TRBV7-3*04-3' | cgatcggttctttgcagtcaggcctgagggatccgtctctactctgaagatccagcgcacagagcggggggactctgccgtgtatctctgtgccagcagc |
| SEQ ID NO: 810 | TRBV7-3*05-5' | tgggagctcaggtgtgatccaatttcaggtcatactgccctttactggtaccgacaaagcctggggcagggcccagagcttctaatttacttccaaggca |
| SEQ ID NO: 811 | TRBV7-3*05-3' | cgatcggttctttgcagtcaggcctgagggatccgtctctactctgaagatccagcgcacagagcggggggactcagccgtgtatctctgtgccagcagc |
| SEQ ID NO: 812 | TRBV7-4*01-5' | ggtgctggagtctcccagtccccaaggtacaaagtcgcaaagaggggacgggatgtagctctcaggtgtgattcaatttcgggtcatgtaaccctttatt |
| SEQ ID NO: 813 | TRBV7-4*01-3' | ggttctctgcagagaggcctgagagatccgtctccactctgaagatccagcgcacagagcagggggactcagctgtgtatctctgtgccagcagcttagc |
| SEQ ID NO: 814 | TRBV7-4*02-5' | ggtgctggagtctcccagtccccaaggtacaaagtcgcaaagaggggacgggatgtagctctcaggtgtgattcaatttcgggtcatgtaaccctttatt |
| SEQ ID NO: 815 | TRBV7-4*02-3' | aacgagacaaatcagggcggcccagtggtcggttctctgcagagaggcctgagagatcgtctccactccgaagatccagcgcacagagcagggggactca |
| SEQ ID NO: 816 | TRBV7-5*01-5' | ggtgctggagtctcccagtccccaaggtacgaagtcacacagaggggacaggatgtagctcccaggtgtgatccaatttcgggtcaggtaaccctttatt |
| SEQ ID NO: 817 | TRBV7-5*01-3' | tcaattctccacagagaggtctgaggatctttctccacctgaagatccagcgcacagagcaagggcgactcggctgtgtatctctgtgccagaagcttag |
| SEQ ID NO: 818 | TRBV7-5*02-5' | ggtgctggagtctcccagtccccaaggtacgaagtcacacagaggggacaggatgtagctcccaggtgtgatccaatttcgggtcaggtaaccctttatt |
| SEQ ID NO: 819 | TRBV7-5*02-3' | caattctccacagagaggtctgaggatctttctccacctgaagatccagcgcacagagcaagggcgactcggctgtgtatctctgtgtcagaagcttagc |
| SEQ ID NO: 820 | TRBV7-6*01-5' | ggtgctggagtctcccagtctcccaggtacaaagtcacaaagaggggacaggatgtagctctcaggtgtgatccaatttcgggtcatgtatccctttatt |
| SEQ ID NO: 821 | TRBV7-6*01-3' | ggttctctgcagagaggcctgagggatccatctccactctgacgatccagcgcacagagcagcgggactcggccatgtatcgctgtgccagcagcttagc |
| SEQ ID NO: 822 | TR8V7-6*02-5' | ggtgctggagtctcccagtctcccaggtacaaagtcacaaagaggggacaggatgtagctctcaggtgtgatccaatctcgggtcatgtatccctttatt |
| SEQ ID NO: 823 | TRBV7-6*02-3' | tgatcggttctctgcagagaggcctgagggatccatctccactctgacgatccagcgcacagagcagcgggactcggccatgtatcgctgtgccagcagc |
| SEQ ID NO: 824 | TRBV7-7*01-5' | ggtgctggagtctcccagtctcccaggtacaaagtcacaaagaggggacaggatgtaactctcaggtgtgatccaatttcgagtcatgcaaccctttatt |
| SEQ ID NO: 825 | TRBV7-7*01-3' | ggttctctgcagagaggcctgagggatccatctccactctgacgattcagcgcacagagcagcgggactcagccatgtatcgctgtgccagcagcttagc |
| SEQ ID NO: 826 | TRBV7-7*02-5' | ggtgctggagtctcccagtctcccaggtacaaagtcacaaagaggggacaggatgtaactctcaggtgtgatccaatttcgagtcatgtaaccctttatt |
| SEQ ID NO: 827 | TRBV7-7*02-3' | tgatcggttctctgcagagaggcctgagggatccatctccactctgacgattcagcgcacagagcagcgggactcagccatgtatcgctgtgccagcagc |
| SEQ ID NO: 828 | TRBV7-8*01-5' | ggtgctggagtctcccagtcccctaggtacaaagtcgcaaagagaggacaggatgtagctctcaggtgtgatccaatttcgggtcatgtatccctttttt |
| SEQ ID NO: 829 | TRBV7-8*01-3' | gcttctttgcagaaaggcctgagggatccgtctccactctgaagatccagcgcacacagcaggaggactccgccgtgtatctctgtgccagcagcttagc |
| SEQ ID NO: 830 | TRBV7-8*02-5' | ggtgctggagtctcccagtcccctaggtacaaagtcgcaaagagaggacaggatgtagctctcaggtgtgatccaatttcgggtcatgtatccctttttt |
| SEQ ID NO: 831 | TRBV7-8*02-3' | gcttctttgcagaaaggcctgagggatccgtctccactctgaagatccagcgcacacagaaggaggactccgccgtgtatctctgtgccagcagcttagc |
| SEQ ID NO: 832 | TRBV7-8*03-5' | ggtgctggagtctcccagtcccctaggtacaaagtcgcaaagagaggacaggatgtagctctcaggtgtgatccaatttcgggtcatgtatccctttttt |
| SEQ ID NO: 833 | TRBV7-8*03-3' | tcgcttctttgcagaaaggcctgagggatccgtctccactctgaagatccagcgcacacagcaggaggactccgccgtgtatctctgtgccagcagccga |
| SEQ ID NO: 834 | TRBV7-9*01-5' | gatactggagtctcccagaaccccagacacaagatcacaaagaggggacagaatgtaactttcaggtgtgatccaatttctgaacacaaccgcctttatt |
| SEQ ID NO: 835 | TRBV7-9*01-3' | ggttctctgcagagaggcctaagggatctttctccaccttggagatccagcgcacagagcagggggactcggccatgtatctctgtgccagcagcttagc |
| SEQ ID NO: 836 | TRBV7-9*02-5' | gatactggagtctcccagaaccccagacacaacatcacaaagaggggacagaatgtaactttcaggtgtgatccaatttctgaacacaaccgcctttatt |
| SEQ ID NO: 837 | TRBV7-9*02-3' | tcggttctctgcagagaggcctaagggatctttctccaccttggagatccagcgcacagagcagggggactcggccatgtatctctgtgccagcagctta |
| SEQ ID NO: 838 | TRBV7-9*03-5' | gatactggagtctcccaggaccccagacacaagatcacaaagaggggacagaatgtaactttcaggtgtgatccaatttctgaacacaaccgcctttatt |
| SEQ ID NO: 839 | TRBV7-9*03-3' | tgatcggttctctgcagagaggcctaagggatctttctccacctiggagatccagcgcacagagcagggggactcggccatgtatctrtgtgccagcagc |
| SEQ ID NO: 840 | TR8V7-9*04-5' | atatctggagtctcccacaaccccagacacaagatcacaaagaggggacagaatgtaactttcaggtgtgatccaatttctgaacacaaccgcctttatt |
| SEQ ID NO: 841 | TRBV7-9*04-3' | tcggatctctgcagagaggcctaagggatctttctccaccttggagatccagcgcacagagcagggggactcggccatgtatctctgtgccagcagctct |
| SEQ ID NO: 842 | TRBV7-9*05-5' | gatactggagtctcccagaaccccagacacaagatcacaaagaggggacagaatgtaactttcaggtgtgatccaatttctgaacacaaccgcctttatt |
| SEQ ID NO: 843 | TRBV7-9*05-3' | tcggttctctgcagagaggcctaagggatctctctccaccttggagatccagcgcacagagcagggggactcggccatgtatctctgtgccagcaccaaa |
| SEQ ID NO: 844 | TRBV7-9*06-S' | gatactggagtctcccagaaccccagacacaagatcacaaagaggggacagaatgtaactttcaggtgtgatccaatttctgaacacaaccgcctttatt |
| SEQ ID NO: 845 | TRBV7-9*06-3' | tcggttctctgcagagaggcctaagggatctctttccaccttggagatccagcgcacagagcagggggactcggccatgtatctctgtgccagcacgttg |
| SEQ ID NO: 846 | TRBV7-9*07-5' | cacaaccgctttattggtaccgacagaccctggggcagggcccagagtttctgacttacttccagaatgaagctcaactagaaaaatcaaggctgctca |
| SEQ ID NO: 847 | TRBV7-9*07-3' | gttctctgcagagaggcctaagggatctttctccaccttggagatccagcgcacagaggagggggactcggccatgtatctctgtgccagcagcagcagt |
| SEQ ID NO: 848 | TRBV8-1*01-5' | gaggcagggatcagccagataccaagatatcacagacacacagggaaaaagatcatcctgaaatatgctcagattaggaaccattattcagtgttctgtt |
| SEQ ID NO: 849 | TRBV8-1*01-3' | ggaagggtacaatgtctctggaaacaagctcaagcattttccctcaaccctggagtctactagcaccagccagacctctgtacctctgtggcagtgcatc |
| SEQ ID NO: 850 | TRBV8-2*01-5' | gatgctgggatcacccagatgccaagatatcacattgtacagaagaaagagatgatcctggaatgtgctcaggttaggaacagtgttctgatatcgacag |
| SEQ ID NO: 851 | TRBV8-2*01-3' | agaggggtactgtgtttcttgaaacaagcttgagcatttccccaatcctggcatccaccagcaccagccagacctatctgtaccactgtggcagcacatc |
| SEQ ID NO: 852 | TRBV9*01-5' | gattctggagtcacacaaaccccaaagcacctgatcacagcaactggacagcgagtgacgctgagatgctcccctaggtctggagacctctctgtgtact |
| SEQ ID NO: 853 | TRBV9*01-3' | cgattctccgcacaacagttccctgacttgcactctgaactaaacctgagctctctggagctgggggactcagctttgtatttctgtgccagcagcgtag |
| SEQ ID NO: 854 | TRBV9*02-5' | gattctggagtcacacaaaccccaaagcacctgatcacagcaactggacagcgagtgacgctgagatgctcccctaggtctggagacctctctgtgtact |
| SEQ ID NO: 855 | TRBV9*02-3' | cgattctccgcacaacagttccctgacttgcactctgaactaaacctgagctctctggagctgggggactcagctttgtatttctgtgccagcagcgtag |
| SEQ ID NO: 856 | TRBV9*03-5' | gattctggagtcacacaaaccccaaagcacctgatcacagcaactggacagcgagtgacgctgagatgctcccctaggtctggagacctctctgtgtact |
| SEQ ID NO: 857 | TRBV9*03-3' | tgaacgattctccgcacaacagttccctgacttgcactctgaactaaacctgagctctctggagctgggggactcagctttgtatttctgtgccagcagc |
| SEQ ID NO: 858 | TRBVA*01-5' | gaagctgaagccacctagactctaagacacctgattgcagagacaggaaaggagttctcaagataagtgccaagatttcatactggttttcacaagaatc |
| SEQ ID NO: 859 | TRBVA*01-3' | tccctattgaaaatatttcctggcaaaaaatagaagttctctttggctctgaaatctgcaactccctttcaggtgtccctgtgtccttgtaccgtcactc |
| SEQ ID NO: 860 | TRBVA/OR9-2*01-5' | gaagctgaagtcacctagactccaagacacctgattgtagagacaggaaaggagttctcaggatatgtgccataatttcatactggtttctacaagaatc |
| SEQ ID NO: 861 | TRBVA/OR9-2*01-3' | tccctgttgaaaatatttcccggcaaaaaacagaagttccctttggctctgaaatctgcaaagccctttcagatgtccctgtgtccttgtgccgtcactc |
| SEQ ID NO: 862 | TRBVB*01-5' | aatgtcaaagtaacacagaccctgagatgaggcaggaaagttgtatcggaatgttttcagactatcaaccagaccaaacgttctggaatccataagatcc |
| SEQ ID NO: 863 | TRBVB*01-3' | gactctgagaccctctgcagcagcagcctatcagtgcagccacatcctctctgagcggatatgacaaaccccagggttgaagcgacctaacctatgagcc |
| SEQ ID NO: 864 | TRBVC*01-5' | agtgacttctaaattggtctatgaaggagaatctcccccattcctggagtcgcccagtccagacctctctgtacatttgcaccagcagtttatccacagt |
| SEQ ID NO: 865 | TRDV1*01-5' | gcccagaaggttactcaagcccagtcatcagtatccatgccagtgaggaaagcagtcaccctgaactgcctgtatgaaacaagttggtggtcatattata |
| SEQ ID NO: 866 | TRDV1*01-3' | attctgtcaacttcaagaaagcaeceaaatccetceccttaaccatttcagccttacaectagaagattcagcaaagtacttttgtgctcttggggaact |
| SEQ ID NO: 867 | TRDV2*01-5' | gccattgagttggtgcctgaacaccaaacagtgcctgtgtcaataggggtccctgccaccctcaggtgctccatgaaaggagaagcgatcggtaactact |
| SEQ ID NO: 868 | TRDV2*01-3' | tttccaaggtgacattgatattgcaaagaacctggctgtacttaagatacttgcaccatcagagagagatgaagggtcttactactgtgcctgtgacacc |
| SEQ ID NO: 869 | TRDV2*02-5' | attgagttggtgcctgaacaccaaacagtgcctgtgtcaatagggatccctgccaccctcaggtgctccatgaaaggagaagcgatcggtaactactata |
| SEQ ID NO: 870 | TRDV2*02-3' | aatttccaaggtgacattgatattgcaaagaacctggctgtacttaagatacttgcaccatcagagagagatgaagggtcttactactgtgcctgtgaca |
| SEQ ID NO: 871 | TRDV2*03-5' | gccattgagttggtgcctgaacaccaaacagtgcctgtgtcaataggggtccctgccaccctcaggtgctccatgaaaggagaagcgatcggtaactact |
| SEQ ID NO: 872 | TRDV2*03-3' | tttccaaggtgacattgatattgcaaagaacctggctgtacttaagatacttgcaccatcagagagagatgaagggtcttactactgtgcctgtgacacc |
| SEQ ID NO: 873 | TRDV3*01-5' | tgtgacaaagtaacccagagttccccggaccagacggtggcgagtggcagtgaggtggtactgctctgcacttacgacactgtatattcaaatccagatt |
| SEQ ID NO: 874 | TRDV3*01-3' | gacggttttctgtgaaacacattctgacccagaaagcctttcacttggtgatctctccagtaaggactgaagacagtgccacttactactgtgcctttag |
| SEQ ID NO: 875 | TRDV3*02-5' | tgtgacaaagtaacccagagttccccggaccagacggtggcgagtggcagtgaggtggtactgctctgcacttacgacactgtatattcaaatccagatt |
| SEQ ID NO: 87F | TRDV3*02-3' | gacggttttctgtgaaacacattctgacccagaaagcctttcacttggtgatctctccagtaaggactgaagacagtgccacttactactgtgcctttag |
| SEQ ID NO: 877 | TRGV1*01-5' | tcttccaacttggaagggagaacgaagtcagtcaccaggctgactgggtcatctgctgaaatcacctgtgatcttcctggagcaagtaccttatacatcc |
| SEQ ID NO: 878 | TRGV1*01-3' | aaagtatgacactggaagcacaaggagcaattggaatttgagactgcaaaatctaattaaaaatgattctgggttctattactgtgccacctgggacagg |
| SEQ ID NO: 879 | TRGV10*01-5' | ttatcaaaagtggagcagttccagctatccatttccacggaagtcaagaaaagtattgacataccttgcaagatatcgagcacaaggtttgaaacagatg |
| SEQ ID NO: 880 | TRGV10*01-3' | aggcaagaaagaattctcaaactctcacttcaatccttaccatcaagtccgtagagaaagaagacatggccgtttactactgtgctgcgtggtgggtggc |
| SEQ ID NO: 881 | TRGV10*02-5' | ttatcaaaagtggagcagttccagctatccatttccacggaagtcaagaaaagtattgacataccttgcaagatatcgagcacaaggtttgaaacagatg |
| SEQ ID NO: 882 | TRGV10*02-3' | tggaggcaagaaagaattctcaaactctcacttcaatccttaccatcaagtccgtagagaaagaagacatggccgtttactactgtgctgcgtgggatta |
| SEQ ID NO: 883 | TRGV11*01-5' | cttgggcagttggaacaacctgaaatatctatttccagaccagcaaataagagtgcccacatatcttggaaggcatccatccaaggctttagcagtaaaa |
| SEQ ID NO: 884 | TRGV11*01-3' | ggtaagtaaaaatgctcacacttccacttccactttgaaaataaagttcttagagaaagaagatgaggtggtgtaccactgtgcctgctggattaggcac |
| SEQ ID NO: 885 | TRGV11*02-5' | cttgggcagttggaacaacctgaaatatctatttccagaccagcaaataagagtgcccacatatcttggaaggcatccatccaaggctttagcagtaaaa |
| SEQ ID NO: 886 | TRGV11*02-3' | gataagtaaaaatgctcacacttccacttccactttgaaaataaagttcttagagaaagaagatgaggtggtgtaccactgtgcctgctggattaggcac |
| SEQ ID NO: 887 | TRGV2*01-5' | tcttccaacttggaagggagaacgaagtcagtcatcaggcagactgggtcatctgctgaaatcacttgtgatcttgctgaaggaagtaacggctacatcc |
| SEQ ID NO: 888 | TRGV2*01-3' | gtattatacttacgcaagcacaaggaacaacttgagattgatactgcgaaatctaattgaaaatgactctggggtctattactgtgccacctgggacggg |
| SEQ ID NO: 889 | TRGV2*02-5' | tcttccaacttggaagggagaacgaagtcagtcatcaggcagactgggtcatctgctgaaatcacttgtgatcttgctgaaggaagtaacggctacatcc |
| SEQ ID NO: 890 | TRGV2*02-3' | gaagtattatacttacgcaagcacaaggaacaacttgagattgatactgcaaaatctaattgaaaatgactctggggtctattactgtgccacctgggac |
| SEQ ID NO: 891 | TRGV3*01-5' | tcttccaacttggaagggagaacgaagtcagtcaccaggcagactgggtcatctgctgaaatcacttgcgatcttactgtaacaaataccttctacatcc |
| SEQ ID NO: 892 | TRGV3*01-3' | gtattatactcatacacccaggaggtggagctggatattgagactgcaaaatctaattgaaaatgattctggggtctgttactgtgccacctgggacagg |
| SEQ ID NO: 893 | TRGV3*02-5' | tcttccaacttggaagggagaacgaagtcagtcaccaggcagactgggtcatctgctgaaatcacttgcgatcttactgtaacaaataccttctacatcc |
| SEQ ID NO: 894 | TRGV3*02-3' | agtattatactcatacacccaggaggtggagctggatattgagactgcaaaatctaattgaaaatgattctggggtctattactgtgccacctgggacag |
| SEQ ID NO: 895 | TRGV4*01-5' | tcttccaacttggaagggagaacgaagtcagtcatcaggcagactgggtcatctgctgaaatcacttgtgatcttgctgaaggaagtaccggctacatcc |
| SEQ ID NO: 896 | TRGV4*01-3' | gtatgatacttatggaagcacaaggaagaacttgagaatgatactgcgaaatcttattgaaaatgactctggagtctattactgtgccacctgggatggg |
| SEQ ID NO: 897 | TRGV4*02-5' | tcttccaacttggaagggagaacgaagtcagtcatcaggcagactgggtcatctgctgaaatcacttgtgatcttgctgaaggaagtaccggctacatcc |
| SEQ ID NO: 898 | TRGV4*02-3' | gtatgatacttacggaagcacaaggaagaacttgagaatgatactgcgaaatcttattgaaaatgactctggagtctattactgtgccacctgggatggg |
| SEQ ID NO: 899 | TRGV5*01-5' | tcttccaacttggaagggggaacgaagtcagtcacgaggccgactaggtcatctgctgaaatcacttgtgaccttactgtaataaatgccttctacatcc |
| SEQ ID NO: 900 | TRGV5*01-3' | gtattatactcatacacccaggaggtggagctggatattgatactacgaaatctaattgaaaatgattctggggtctattactgtgccacctgggacagg |
| SEQ ID NO: 901 | TRGV5P*01-5' | tcttccaacttggaagggagaatgaagtcagtcaccaggccgactgggtcatctgctgaaatcacttgtgaccttactgtaataaatgccgtctacatcc |
| SEQ ID NO: 902 | TRGV5P*01-3' | gtattatactcatacaccgaggaggtggagctggaatttgagactgcaaaatctaattgaaaatgattctggggtctattactgtgccacctggggcagg |
| SEQ ID NO: 903 | TRGVSP*02-5' | tcttccaacttggaagggagaatgaagtcagtcaccaggccgactgggtcatctgctgaaatcacttgtgaccttactgtaataaatgccgtctacatcc |
| SEQ ID NO: 904 | TRGV5P*02-3' | gtattatactcatacaccgaggaggtggagctggaatttgagactgcaaaatctaattgaaaatgattctggggtctattactgtgccacctggggcagg |
| SEQ ID NO: 905 | TRGV6*01-5' | tctactaacttggaagcgaaaataaagtcaggcaccaggcagatggggtcatctgctgtaatcacctgtgatcttcctgtagaaaatgccttctacatcc |
| SEQ ID NO: 906 | TRGV6*01-3' | gcatgatacttatggaagtagaaggataagctggaaatttatacctccaaaactaaatgaaaatgcctctggggtctattactgtgccacctaggacagg |
| SEQ ID NO: 907 | TRGV6*02-5' | tctactaacttggaagcgaaaataaagtcaggcaccaggcagatggggtcatctgctgtaatcacctgtgatcttcctgtagaaaatgccttctacatcc |
| SEQ ID NO: 908 | TRGV6*02-3' | gcatgatacttatggaagtagaaggataagctggaaatttatacctccaaaactaaatgaaaatgcctctggggtctattactgtgccacctaggacagg |
| SEQ ID NO: 909 | TRGV7*01-5' | tcttccaacttgcaagggagaaggaagtcagtcaccaggccagctgggtcatctgctgtaatcacttgtgatcttactgtaataaataccttctacatcc |
| SEQ ID NO: 910 | TRGV7*01-3' | agtattttacttatgcaagcatgaggaggagctggaaattgatactgcaaaatctaattgaaaatgattctggatctattactgtgccacctgggacagg |
| SEQ ID NO: 911 | TRGV8*01-5' | tcttccaacttggaagggagaacaaagtcagtcaccaggccaactgggtcatcagctgtaatcacttgtgatcttcctgtagaaaatgccgtctacaccc |
| SEQ ID NO: 912 | TRGV8*01-3' | gtatcatacttatgcaagcacagggaagagccttaaatttatactggaaaatctaattgaacgtgactctggggtctattactgtgccacctgggatagg |
| SEQ ID NO: 913 | TRGV9*01-5' | gcaggtcacctagagcaacctcaaatttccagtactaaaacgctgtcaaaaacagcccgcctggaatgtgtggtgtctggaataacaatttctgcaacat |
| SEQ ID NO: 914 | TRGV9*01-3' | tgaggtggataggatacctgaaacgtctacatccactctcaccattcacaatgtagagaaacaggacatagctacctactactgtgccttgtgggaggtg |
| SEQ ID NO: 915 | TRGV9*02-5' | gcaggtcacctagagcaacctcaaatttccagtactaaaacgctgtcaaaaacagcccgcctggaatgtgtggtgtctggaataaaaatttctgcaacat |
| SEQ ID NO: 916 | TRGV9*02-3' | tgaggtggataggatacctgaaacgtctacatccactctcaccattcacaatgtagagaaacaggacatagctacctactactgtgccttgtgggaggtg |
| SEQ ID NO: 917 | TRGVA*01-5' | ctcatcaggccggagcagctggcccatgtcctggggcactagggaagcttggtcatcctgcagtgcgtggtccgcaccaggatcagctacacccactggt |
| SEQ ID NO: 918 | TRGVA*01-3' | agataaaatcatagccaaggatggcagcagctctatcttggcagtactgaagttggagacaggcatcgagggcatgaactactgcacaacctgggccctg |
| SEQ ID NO: 919 | TRGVB*01-5' | tttaaagcaataaaaaatgtcaactacatttttgtcaacagagcaacagataaaagtgtctaggtatcttgtgtggtgtccactgaagactttgtaaata |
| SEQ ID NO: 920 | TRGVB*01-3' | cttgaggcaagaacaaattttcaaatgtctacttcagtctttaccataaacttcataggaaaggaagatgaggccatttactactgcactgcttaggacc |
| SEQ ID NO: 921 | TRAJ1*01 | aatagagacacggggcatggtatgaaagtattacctcccagttgcaatttggcaaaggaaccagagtttccacttctccccgtacgtctgcccatgccca |
| SEQ ID NO: 922 | TRAJ10*01 | gaggcatcaaacactgtgatactcacgggaggaggaaacaaactcacctttgggacaggcactcagctaaaagtggaactcagtaagtatgagattctat |
| SEQ ID NO: 923 | TRAJ11*01 | tatggggatttgctatagtgtgaattcaggatacagcaccctcacctttgggaaggggactatgcttctagtctctccaggtacatgttgaccccatccc |
| SEQ ID NO: 924 | TRAJ12*01 | actgactaagaaacactgtgggatggatagcagctataaattgatcttcgggagtgggaccagactgctggtcaggcctggtaagtaaggtgtcagagag |
| SEQ ID NO: 925 | TRAJ13*01 | aaggcaggcattacagtgtgaattctgggggttaccagaaagttacctttggaattggaacaaagctccaagtcatcccaagtgagtccaatttcctatg |
| SEQ ID NO: 926 | TRAJ13*02 | aaaggcaggcattacagtgtgaattctgggggttaccagaaagttacctttggaactggaacaaagctccaagtcatcccaagtgagtccaatttcctat |
| SEQ ID NO: 927 | TRAJ14*01 | tttgtcaggcagcacagtgctgtgatttatagcacattcatctttgggagtgggacaagattatcagtaaaacctggtaagtaggcaatatgtcactaaa |
| SEQ ID NO: 928 | TRAJ15*01 | cagggcctcatttcactgtgccaaccaggcaggaactgctctgatctttgggaagggaaccaccttatcagtgagttccagtaagtacctgataattatt |
| SEQ ID NO: 929 | TRAJ15*02 | cagggcctcatttcactgtgccaaccaggcaggaactgctctgatctttgggaagggaacccacctatcagtgagttccagtaagtacctgataattatt |
| SEQ ID NO: 930 | TRAJ16*01 | tggtacaatagatcactgtgggttttcagatggccagaagctgctctttgcaaggggaaccatgttaaaggtggatcttagtaagtattattactaatga |
| SEQ ID NO: 931 | TRAJ17*01 | cctgtggtttttgctgggccttaaatcattgtgtgatcaaagctgcaggcaacaagctaacttttggaggaggaaccagggtgctagttaaaccaagtga |
| SEQ ID NO: 932 | TRAJ18*01 | aggggaccagcattgtgccgacagaggctcaaccctggggaggctatactttggaagaggaactcagttgactgtctggcctggtgagtgagtcgctttc |
| SEQ ID NO: 933 | TRAJ19*01 | ttttgcagaggacagatgtggctatcaaagattttacaatttcacctttggaaagggatccaaacataatgtcactccaagtaagtgagcagccttttgt |
| SEQ ID NO: 934 | TRAJ2*01 | tggtgtcacctacggtatgaatactggaggaacaattgataaactcacatttgggaaagggacccatgtattcattatatctggtgagtcatcccaggtg |
| SEQ ID NO: 935 | TRAJ20*01 | tgtaggcgacctcgcactgtggttctaacgactacaagctcagctttggagccggaaccacagtaactgtaagagcaagtaagtaagaaagaaaagtcca |
| SEQ ID NO: 936 | TRAJ21*01 | tgtaatgccaataaacatggtgtacaacttcaacaaattttactttggatctgggaccaaactcaatgtaaaaccaagtaagttatagttgcctagaaga |
| SEQ ID NO: 937 | TRAJ22*01 | gttgagcaaatcatagtgtttcttctggttctgcaaggcaactgacctttggatctgggacacaattgactgttttacctggtaggctgcctcaattaaa |
| SEQ ID NO: 938 | TRAJ23*01 | aggatatgtaacacagtgtgatttataaccagggaggaaagcttatcttcggacagggaacggagttatctgtgaaacccagtaagtataaaattgtatc |
| SEQ ID NO: 939 | TRAJ23*02 | gactggatgtgtttttgacaggatatgtaacacagtgtgatttataaccagggaggaaagcttatcttcggacagggaacggagctatctgtgaaaccca |
| SEQ ID NO: 940 | TRAJ24*01 | gaggtgtttgtcacagtgtgacaactgacagctgggggaaattcgagtttggagcagggacccaggttgtggtcaccccaggtaagcccattcctggagc |
| SEQ ID NO: 941 | TRAJ24*02 | gaggtgtttgtcacagtgtgacaactgacagctgggggaaattgcagtttggagcagggacccaggttgtggtcaccccaggtaagccccattccctgga |
| SEQ ID NO: 942 | TRAJ25*01 | atgctgagataatcactatgcagaaggacaaggcttctcctttatctttgggaaggggacaaggctgcttgtcaagccaagtaagtgacatataatttat |
| SEQ ID NO: 943 | TRAJ26*01 | ctgagcccagaaacactgtggggataactatggtcagaattttgtctttggtcccggaaccagattgtccgtgctgccctgtaagtacagttaagtggag |
| SEQ ID NO: 944 | TRAJ27*01 | caatagcactaaagactgtgtaacaccaatgcaggcaaatcaacctttggggatgggactacgctcactgtgaagccaagtaagttgtgttcttctttgc |
| SEQ ID NO: 945 | TRAJ28*01 | agaaaggaaactctgtgcatactctggggctgggagttaccaactcactttcgggaaggggaccaaactctcggtcataccaagtaagttcttctttctg |
| SEQ ID NO: 946 | TRAJ29*01 | ttatggaggaaatcactgtgggaattcaggaaacacacctcttgtctttggaaagggcacaagactttctgtgattgcaagtaagtgtttctagccatcc |
| SEQ ID NO: 947 | TRAJ3*01 | aaagaccttacccacagtgggggtacagcagtgcttccaagataatctttggatcagggaccagactcagcatccggccaagtaagtagaatgaagcagg |
| SEQ ID NO: 948 | TRAJ30*01 | gttatggtcccaatcacagtgtgaacagagatgacaagatcatctttggaaaagggacacgacttcatattctccccagtaagtgctgtttatgtgattt |
| SEQ ID NO: 949 | TRAJ31*01 | agtaaaggcaggaagtgctgtggaataacaatgccagactcatgtttggagatggaactcagctggtggtgaagcccagtaagtggccatgttttattga |
| SEQ ID NO: 950 | TRAJ32*01 | ggctctgaaggactgtgtgaattatggcggtgctacaaacaagctcatctttggaactggcactctgcttgctgtccagccaagtacgtaagtagtggca |
| SEQ ID NO: 951 | TRAJ32*02 | gtgattcagccacctacctctgtgccgatggtggtgctacaaacaagctcatctttggaactggcactctgcttgctgtccagccaaatatccagaaccc |
| SEQ ID NO: 952 | TRAJ33*01 | gttaaggtttttgtgtctgtgtggatagcaactatcagttaatctggggcgctgggaccaagctaattataaagccaggtaagtctcagagatgtgactg |
| SEQ ID NO: 953 | TRAJ34*01 | aggtttttgtagatctcagtatcactgtgtcttataacaccgacaagctcatctttgggactgggaccagattacaagtctttccaagt |
| SEQ ID NO: 954 | TRAJ35*01 | taaaagaatgagccattgtggataggctttgggaatgtgctgcattgcgggtccggcactcaagtgattgttttaccacgtaagtatatcttttctcatt |
| SEQ ID NO: 955 | TRAJ36*01 | tactgggcagaaacactgtgtcaaactggggcaaacaacctcttctttgggactggaacgagactcaccgttattccctgtaagtccttacctcttgaca |
| SEQ ID NO: 956 | TRAJ37*01 | aaagtacagcattagagtgtggctctggcaacacaggcaaactaatctttgggcaagggacaactttacaagtaaaaccaggtaggtctggatgtttcca |
| SEQ ID NO: 957 | TRAJ37*02 | ctcagcggtgtacttctgtgctcttcatggctctagcaacacaggcaaactaatctttgggcaagggacaactttacaagtaaaaccagatatccagaac |
| SEQ ID NO: 958 | TRAJ38*01 | aaagctttctatgactgtgtaatgctggcaacaaccgtaagctgatttggggattgggaacaagcctggcagtaaatccgagtgagtcttcgtgttaact |
| SEQ ID NO: 959 | TRAJ39*01 | cagccgaagatcactgtgtgaataataatgcaggcaacatgctcacctttggagggggaacaaggttaatggtcaaaccccgtgagtatctctgctgaat |
| SEQ ID NO: 960 | TRAJ4*01 | aagcaccatctgattgtgtgttttctggtggctacaataagctgatttttggagcagggaccaggctggctgtacacccatgtgagtatgaccctgcaag |
| SEQ ID NO: 961 | TRAJ40*01 | tatgttggtttatgtagagacacataacactgtgactacctcaggaacctacaaatacatctttggaacaggcaccaggctgaaggttttagcaagt |
| SEQ ID NO: 962 | TRAJ41*01 | ttagggagaacgcactgtggaactcaaattccgggtatgcactcaacttcggcaaaggcacctcgctgttggtcacaccccgtgagtttttgtggtttac |
| SEQ ID NO: 963 | TRAJ42*01 | agccccataggactgtgtgaattatggaggaagccaaggaaatctcatctttggaaaaggcactaaactctctgttaaaccaagtaagtgttggggattc |
| SEQ ID NO: 964 | TRAJ43*01 | ttgttagagcatgtattactgtgacaataacaatgacatgcgctttggagcagggaccagactgacagtaaaaccaagtaagttgggggaatgggtcaat |
| SEQ ID NO: 965 | TRAJ44*01 | aggtttctgttatgaagcatctcacagtgtaaataccggcactgccagtaaactcacctttgggactggaacaagacttcaggtcacgctcggt |
| SEQ ID NO: 966 | TRAJ45*01 | agggttggcccagagtgtgtattcaggaggaggtgctgacggactcacctttggcaaagggactcatctaatcatccagccctgtaagtgcttttgcctg |
| SEQ ID NO: 967 | TRAJ46*01 | aagctgctgacagccgtgagaagaaaagcagcggagacaagctgacttttgggaccgggactcgtttagcagttaggcccagtaagtctgagcagaaagt |
| SEQ ID NO: 968 | TRAJ47*01 | gtagaggagtttgacgctgtgtggaatatggaaacaaactggtctttggcgcaggaaccattctgagagtcaagtcctgtgagtataaaacacactcaag |
| SEQ ID NO: 965 | TRAJ47*02 | gtgtactattgcatctcggccctggaatatggaaacaagctggtctttggcgcaggaaccattctgagagtcaagtcctatatccagaaccctgaccctg |
| SEQ ID NO: 970 | TRAJ48*01 | atgacttagaacactgtgtatctaactttggaaatgagaaattaacctttgggactggaacaagactcaccatcatacccagtaagttcttcatccttgg |
| SEQ ID NO: 971 | TRAJ49*01 | tgttgagcttcctatcacagtggaacaccggtaaccagttctattttgggacagggacaagtttgacggtcattcεaagtaagtcaaagaaaattttcca |
| SEQ ID NO: 972 | TRAJ5*01 | tactgtgatgtaccagggtgtggacacgggcaggagagcacttacttttgggagtggaacaagactccaagtgcaaccaagtaagtacccaaacttaggc |
| SEQ ID NO: 973 | TRAJ50*01 | taaaggtttggatggctgtgtgaaaacctcctacgacaaggtgatatttgggccagggacaagcttatcagtcattccaagtaagtgtccctggggtgct |
| SEQ ID NO: 974 | TRAJ51*01 | aaactccctgaagcagggagatgcgtgacagctatgagaagctgatatttggaaaggagacatgactaactgtgaagccaagcaagctggaaagacctaa |
| SEQ ID NO: 975 | TRAJ52*01 | gcctccagtgcagtgctaatgctggtggtactagctatggaaagctgacatttggacaagggaccatcttgactgtccatccaagtaagtgtaacaagac |
| SEQ ID NO: 976 | TRAJ53*01 | agccttctgtggctgtgagaatagtggaggtagcaactataaactgacatttggaaaaggaactctcttaaccgtgaatccaagtaagtttgaagggagt |
| SEQ ID NO: 977 | TRAJ54*01 | taaagcctcgtgctgtggtgtaattcagggagcccagaagctggtatttggccaaggaaccaggctgactatcaacccaagtaagtatgacagggtgaag |
| SEQ ID NO: 978 | TRAJ55*01 | gaggatggatccctgttagtgacaagtgctggtaatgctcctgttggggaaaggggatgagtacaaaaataaatccaagtaagtgtggagggacaagaag |
| SEQ ID NO: 979 | TRAJ56*01 | agatcctcgtgtcattgtgttatactggagccaatagtaagctgacatttggaaaaggaataactctgagtgttagaccaggtatgttttaatgaatgtt |
| SEQ ID NO: 980 | TRAJ57*01 | aagcagtctgtgggggtgtaactcagggcggatctgaaaagctggtctttggaaagggaacgaaactgacagtaaacccatgtaagtctgaataatgctt |
| SEQ ID NO: 981 | TRAJ58*01 | aagcccctcagcacagtgtttaagaaaccagtggctctaggttgacctttggggaaggaacacagctcacagtgaatcctggtaagtggaggggagcatt |
| SEQ ID NO: 982 | TRAJ59*01 | atgtaaaggcagcagctcctgtgggaaggaaggaaacaggaaatttacatttggaatggggacgcaagtgagagtgaagctatctttaaaccaaaggtgt |
| SEQ ID NO: 983 | TRAJ6*01 | caggttttatcaaaggctgtcctcactgtgtgcatcaggaggaagctacatacctacatttggaagaggaaccagccttattgttcatccgtgtaagt |
| SEQ ID NO: 984 | TRAJ60*01 | gtaaagggcctgggcactatgtgaagatcacctagatgctcaactttgggaaggggactgagttaattgtgagcctgggtgagtacctcaactccagagg |
| SEQ ID NO: 985 | TRAJ61*01 | taaaggtgcccactcctgtgggtaccgggttaataggaaactgacatttggagccaacactagaggaatcatgaaactcagcaagtaatatttggcagaa |
| SEQ ID NO: 986 | TRAJ7*01 | tgtaatacacttacacagtgtgactatgggaacaacagactcgcttttgggaaggggaaccaagtggtggtcataccaagtaagtgagctgggatcctcc |
| SEQ ID NO: 987 | TRAJ8*01 | tacagagttatgtcagagtgtgaacacaggctttcagaaacttgtatttggaactggcacccgacttctggtcagtccaagtaagtcaaatctgcagaaa |
| SEQ ID NO: 988 | TRAJ9*01 | cgcagtgcaaatcactgtgggaaatactggaggcttcaaaactatctttggagcaggaacaagactatttgttaaagcaagtaagttccatgaaataacc |
| SEQ ID NO: 989 | TRBJ1-1*01 | ttttcaccttgacccctgtcactgtgtgaacactgaagctttctttggacaaggcaccagactcacagttgtaggtaagacatttttcaggttcttttgc |
| SEQ ID NO: 99C | TRBJ1-2*01 | ttttagagtggctatattcttatgtgctaactatggctacaccttcggttcggggaccaggttaaccgttgtaggtaaggctgggggtctctaggagggg |
| SEQ ID NO: 991 | TRBJ1-3*01 | tttgaagtggccctgggaggctgtgctctggaaacaccatatattttggagagggaagttggctcactgttgtaggtgagtaagtcaaggctggacagct |
| SEQ ID NO: 992 | TRBJ1-4*01 | ttccttccagtctttaatgttgtgcaactaatgaaaaactgttttttggcagtggaacccagctctctgtcttgggtatgtaaaagacttctttcgggat |
| SEQ ID NO: 993 | TRBJ1-5*01 | tttgccacactcatgatgcactgtgtagcaatcagccccagcattttggtgatgggactcgactctccatcctaggtaagttggcagaatcagggtggta |
| SEQ ID NO: 994 | TRBJ1-6*01 | ttatctaagcctctgcagctgtgctcctataattcacccctccactttgggaatgggaccaggctcactgtgacaggtatgggggctccactcttgactc |
| SEQ ID NO: 995 | TRBJ1-6*02 | ttatctaagcctctgcagctgtgctcctataattcacccctccactttgggaacgggaccaggctcactgtgacaggtatgggggctccactcttgactc |
| SEQ ID NO: 996 | TRBJ2-1*01 | ttctgggcagccccttcccactgtgctcctacaatgagcagttcttcgggccagggacacggctcaccgtgctaggtaagaagggggctccaggtgggag |
| SEQ ID NO: 997 | TRBJ2-2*01 | tgcgagggtccccagggctgtgcgaacaccggggagctgttttttggagaaggctctaggctgaccgtactgggtaaggaggcggctggggctccgga |
| SEQ ID NO: 998 | TRBJ2-2P*01 | agctgccccactctgagaggggctgtgctgagaggcgctgctgggcgtctgggcggaggactcctggttctgggtgctgggagagcgatggggctctcag |
| SEQ ID NO: 999 | TRBJ2-3*01 | ttttgtcctgggcctccaggctgtgagcacagatacgcagtattttggcccaggcacccggctgacagtgctcggtaagcgggggctcccgctgaagccc |
| SEQ ID NO: 100 | TRBJ2-4*01 | ttctgtgccgcgtctcggggctgtgagccaaaaacattcagtacttcggcgccgggacccggctctcagtgctgggtaagctggggccgccgggggaccg |
| SEQ ID NO: 100 | TRBJ2-5*01 | tttttgtgcgrggctcgggggccgtgaccaagagacccagtacttcgggccaggcacgcggctcctggtgctcggtgagcgcgggctgctggggcgcggg |
| SEQ ID NO: 100 | TRBJ2-6*01 | ttgcggggagtccccgggctgtgctctggggccaacgtcctgactttcggggccggcagcaggctgaccgtgctgggtgagttttcgcgggaccacccgg |
| SEQ ID NO: 100 | TRBJ2-7*01 | tttgcatgcgggggtgcacctccgtgctcctacgagcagtacttcgggccgggcaccaggctcacggtcacaggtgagattcgggcgtctccccaccttc |
| SEQ ID NO: 100 | TRBJ2-7*02 | tttgcatgcggggatgcacctccgtgctcctacgagcagtacgtcgggccgggcaccaggctcacggtcacaggtgagattcgggcgtctccccaccttc |
| SEQ ID NO: 100 | TRDJ1*01 | ttttggaacgtcctcaagtgctgtgacaccgataaactcatctttggaaaaggaacccgtgtgactgtggaaccaagtaagtaactcattatttatctga |
| SEQ ID NO: 100 | TRDJ2*01 | tttttcgtaatgacgcctgtggtagtgctttgacagcacaactcttctttggaaagggaacacaactcatcgtggaaccaggtaagttatgcattttact |
| SEQ ID NO: 100 | TRDJ3*01 | tgaggcactgtcataatgtgctcctgggacacccgacagatgtttttcggaactggcatcaaactcttcgtggagccccgtgagttgatctttttcctat |
| SEQ ID NO: 100 | TRDJ4*01 | atgagacatacaaaaaggtaatgccgccccagacccctgatctttggcaaaggaacctatctggaggtacaacaac |
| SEQ ID NO: 100 | TRGJ1*01 | ttttgatatggactgaatcactgtggaattattataagaaactctttggcagtggaacaacactggttgtcacaggtaagtatcggaagaatacaacatt |
| SEQ ID NO: 101 | TRGJ1*02 | tactgtgccttgtgggaggtgcttattataagaaactctttggcagtggaacaacacttgttgtcacaggt |
| SEQ ID NO: 101 | TRGJ2*01 | ttttgatatggactgaatcactgtggaattattataagaaactctttggcagtggaacaacacttgttgtcacaggtaagtatcggaagaatacaacatt |
| SEQ ID NO: 101 | TRGJP*01 | ataaaggcttctcaggtggtgggcaagagttgggcaaaaaaatcaaggtatttggtcccggaacaaagcttatcattacaggtaagttttctttaaattt |
| SEQ ID NO: 101 | TRGJP1*01 | gatttttctagaagcttagaccggtgtgataccactggttggttcaagatatttgctgaagggactaagctcatagtaacttcacctggtaagt |
| SEQ ID NO: 101 | TRGJP2*01 | gatttttgtagaagcttagaccagtgtgatagtagtgattggatcaagacgtttgcaaaagggactaggctcatagtaacttcgcctggtaagt |

**Table 4**

| **SEQ ID NO** | **Name** | **Sequence** |
|---|---|---|
| SEQ ID NO: 1015 | TRAV1-1*02-RIGHT | caggtcgtttttcttcattccttagtcgctctgatagttatggttacctccttctacaggagctccagatgaaagactctgcctcttacttctgcgct gt |
| SEQ ID NO: 1016 | TRAV1-2*02-RIGHT | catctgggttcaacgggctgttctggtaccagcaacatgctggcgaagcacccacatttctgtcttacaatgttctggatggtctggaggagaa aggtcg |
| SEQ ID NO: 1017 | TRAV12-1*02-RIGHT | acagcacacgtcaatagagccagccagtatatttccctgctcatcagagactccaagctcagtgattcagccacctacctctgtgtggtgaaca ttcgcc |
| SEQ ID NO: 1018 | TRAV12-2*02-RIGHT | gtttacagcacagctcaataaagccagccagtatgtttctctgctcatcagagactcccagcccagtgattcagccacctacctctgtgccgtgt accac |
| SEQ ID NO: 1019 | TRAV12-2*03-RIGHT | aaggtttacagcacagctcaataaagccagccagtatgtttctctgctcatcagagactcccagcccagtgattcagccacctacctctgtgcc gtgaac |
| SEQ ID NO: 1020 | TRAV12-3*02-RIGHT | aggtttacagcacaggtcgataaatccagcaagtatatctccttgttcatcagagactcacagcccagtgattcagccacctacctctgtgcaat gagcg |
| SEQ ID NO: 1021 | TRAV13-1*02-RIGHT | tgttacattgaacaagacagccaaacatttctccctgcacatcacagagacccaacctgaagactcggctgtctacttctgtgcagcaagtagg aaggac |
| SEQ ID NO: 1022 | TRAV13-1*03-RIGHT | gcttattatagacattcgttcaaatgtgggcgaaaagaaagaccaacgaattgctgttacattgaacaagacagccaaacatttctccctgcag atcaca |
| SEQ ID NO: 1023 | TRAV13-2*02-RIGHT | caaagagtcaccgttttattgaataagacagtgaaacatctctctgcaaattgcagctactcaacctggagactcagctgtctactttgtgc agaga |
| SEQ ID NO: 1024 | TRAV14/DV4*03-RIGHT | aggtcgctactcattgaatttccagaaggcaagaaaatccgccaaccttgtcatctccgcttcacaactgggggactcagcaatgtatttctgtg caatg |
| SEQ ID NO: 1025 | TRAV14/DV4*04-RIGHT | gcaacagaaggtcgctactcattgaatttccagaaggcaagaaaatccgccaaccttgtcatctccgcttcacaactggggactcagcaatg tacttct |
| SEQ ID NO: 1026 | TRAV2*02-RIGHT | gggacgatacaacatgacctatgaacggttctcttcatcgctgctcatcctccaggtgcgggaggcagatgctgctgtttactactgtgctgtgg cctgg |
| SEQ ID NO: 1027 | TRAV20*02-RIGHT | aaaggagaaagaaaggctaaaagccacattaacaaagaaggaaagctttctgcacatcacagcccctaaacctgaagactcagccacttat ctctgtgct |
| SEQ ID NO: 1028 | TRAV20*03-RIGHT | agaaaaggagaaagaaaggctaaaagccacattaacaaagaaggaaagctttctgcacatcacagcccctaaacctgaagactcagccac ttatctctgt |
| SEQ ID NO: 1029 | TRAV20*04-RIGHT | aaaggagaaagaaaggctaaaagccacattaacaaagaaggaaagctttctgcacatcacagcccctaaacctgaagactcagccacttat ctctgtgct |
| SEQ ID NO: 1030 | TRAV21*02-RIGHT | aagtggaagacttaatgcctcgctggataaatcatcaggacgtagtactttatacattgcagcttctcagcctggtgactcagccacctacctct gtgct |
| SEQ ID NO: 1031 | TRAV23/DV6*02-RIGHT | agattcacaatctccttcaataaaagtgccaagcagttctcattgcatatcatggattcccagcctggagactcagccacctacttctgtgcagc aagcg |
| SEQ ID NO: 1032 | TRAV23/DV6*03-RIGHT | agattcacaatctccttcaataaaagtgccaagcagttctcattgcatatcatggattcccagcctggagactcagccacctacttctgtgcagc aagca |
| SEQ ID NO: 1033 | TRAV23/DV6*04-RIGHT | gaaagaaggaagattcacaatctccttcaataaaagtgccaagcagttctcattgcatatcatggattcccagcctggagactcagccaccta cttctgt |
| SEQ ID NO: 1034 | TRAV24*02-RIGHT | ggacgaataagtgccactcttaataccaaggagggttacagctatttgtacatcaaaggatcccagcctgaagattcagccacatacctctgtg cctttta |
| SEQ ID NO: 1035 | TRAV26-1*02-RIGHT | ctctgatcatcacagaagacagaaagtccagcaccttgatcctgccccacgctacgctgagagacactgctgtgtactattgcatcgtcagaga ttgggt |
| SEQ ID NO: 1036 | TRAV26-1*03-RIGHT | caatgaaatggcctctctgatcatcacagaagacagaaagtccagcaccttgatcctgccccacgctacgctgagagacactgctgtgtactat tgcatc |
| SEQ ID NO: 1037 | TRAV26-2*02-RIGHT | ccctcccagggtccagagtacgtgattcatggtcttacaagcaatgtgaacaacagaatggcctgtgtggcaatcgctgaagacagaaagtcc agtacct |
| SEQ ID NO: 1038 | TRAV27*02-RIGHT | tgaagagactaacctttcagtttggtgatgcaagaaaggacagttctctccacatcactgcggcccagcctggtgatacaggccactacctctg tgcagg |
| SEQ ID NO: 1039 | TRAV27*03-RIGHT | gctgaagagactaacctttcagtttggtgatgcaagaaaggacagttctctccacatcactgcagcccagactggtgatacaggcctctacctc tgtgca |
| SEQ ID NO: 1040 | TRAV29/DV5*02-RIGHT | aagattcactgttttcttaaacaaaagtgccaagcacctctctctcgacattgtgccctcccagcctggagactctgcagtgtacttctgtgcagc aagc |
| SEQ ID NO: 1041 | TRAV29/DV5*03-RIGHT | agattcactgttttcttaaacaaaagtgccaagcacctctctgcacattgtgccctcccagcctggagactctgcagtgtacttctgtgcagca agcg |
| SEQ ID NO: 1042 | TRAV3*02-RIGHT | ctttgaagctgaatttaacaagagccaaacctccttccacctgaagaaaccatctgcccttgtgagcgactccgctttgtacttctgtgctgtgag accc |
| SEQ ID NO: 1043 | TRAV30*02-RIGHT | tcgtgaaaaaatatctgcttcatttaatgaaaaaaagcagcaaagctccctgtaccttacggcctcccagctcagttactcaggaacctacttct gcggg |
| SEQ ID NO: 1044 | TRAV30*03-RIGHT | tcatgaaaaatatctgcttcatttaatgaaaaaagcggcaaagctccctgtaccttacggcctcccagctcagttactcaggaacctacttct gcggc |
| SEQ ID NO: 1045 | TRAV30*04-RIGHT | tcctgatgatattactgaagggtggagaacagaagcgtcatgaaaaaatatctgcttcatttaatgaaaaaaagcagcaaagctccctgtacc ttacggc |
| SEQ ID NO: 1046 | TRAV35*02-RIGHT | aaatggaagactgactgctcagtttggtataaccagaaaggacagcttcctgaatatctcagcatccatacctagtgatgtaggcatctacttct gtgct |
| SEQ ID NO: 1047 | TRAV36/DV7*02-RIGHT | ggaagactaagtagcatattagataagaaagaacttttcagcatcctgaacatcacagccacccagaccggagactcggccgtctacctctgt gctgtgg |
| SEQ ID NO: 1048 | TRAV36/DV7*03-RIGHT | gtcaggaagactaagtagcatattagataagaaagaacttttcagcatcctgaacatcacagccacccagaccggagactcggccgtctacct ctgtgct |
| SEQ ID NO: 1049 | TRAV36/DV7*04-RIGHT | tcaggaagactaagtagcatattagataagaaagaacttttcagcatcctgaacatcacagccacccagaccggagactcggccgtctacctc tgtgctg |
| SEQ ID NO: 1050 | TRAV38-1*02-RIGHT | gagaatcgtttctctgtgaacttccagaaagcagccaaatccttcagtctcaagatctcagactcacagctgggggacactgcgatgtatttctg tgctt |
| SEQ ID NO: 1051 | TRAV38-1*03-RIGHT | aatcgtttctctgtgaacttccagaaagcagccaaatccttcagtctcaagatctcagactcacagctgggggacactgcgatgtatttctgtgc tttca |
| SEQ ID NO: 1052 | TRAV38-1*04-RIGHT | ggagaatcgtttctctgtgaacttccagaaagcagccaaatccttcagtctcaagatctcagactcacagctgggggacactgcgatgtatttct gtgca |
| SEQ ID NO: 1053 | TRAV6*02-RIGHT | gaaagaaagactgaaggtcacctttgataccacccttaaacagagtttgtttcatatcacagcctcccagcctgcagactcagctacctacctct gtgct |
| SEQ ID NO: 1054 | TRAV6*03-RIGHT | gaaagaaagactgaaggtcacctttgataccacccttaaacagagtttgtttcatatcacagcctcccagcctgcagactcagctacctacctct gtgct |
| SEQ ID NO: 1055 | TRAV6*04-RIGHT | gaaagaaagactgaaggtcacctttgataccacccttaaacagagtttgtttcatgtcacagcctcccagcctgcagactcagctacctacctct gtgct |
| SEQ ID NO: 1056 | TRAV6*05-RIGHT | gaaagaaagactgaaggtcacctttgataccacccttaaacagagtttgtttcatatcacagcctcccagcctgcagactcagctacctacctct gtgct |
| SEQ ID NO: 1057 | TRAV6*06-RIGHT | ccaggaagaggccctgttttcttgctactcatacgtgaaaatgagaaagaaaaaggaaagaaagactgaaggtcacctttgataccacccctt aaccaga |
| SEQ ID NO: 1058 | TRAV8-1*02-RIGHT | ttttcaggggatccactggttaaaggcatcaagggcgttgaggctgaatttataaagagtaaattctcctttaatctgaggaaaccctctgtgca gtgga |
| SEQ ID NO: 1059 | TRAV8-2*02-RIGHT | tttaagaagagtgaaacctccttccacctgacgaaaccctcagcccatatgagcgacgcggctgagtacttctgtgttgtgacccgtcacgagc tttcag |
| SEQ ID NO: 1060 | TRAV8-3*02-RIGHT | aggctttgaggctgaatttaagaggagtcaatcttccttcaacctgaggaaaccctctgtgcattggagtgatgctgctgagtacttctgtgctgt ggtt |
| SEQ ID NO: 1061 | TRAV8-3*03-RIGHT | tattaaaggctttgaggctgaatttaagaggagtcaatcttccttcaatctgaggaaaccctctgtgcattggagtgatgcgtctgagtacttctg tgct |
| SEQ ID NO: 1062 | TRAV8-4*02-RIGHT | gaatttaagaagagtgaaacctccttccacctgacaaaaccctcagcccatatgagcgacgcggctgagtacttctgtgctgtgagtgatctcg aaccga |
| SEQ ID NO: 1063 | TRAV8-4*03-RIGHT | catcaacggttttgaggctgaatttaagaagagtgaaacctccttccacctgacgaaaccctcagcccatatgagcgacgcggctgagtacttc tgtgct |
| SEQ ID NO: 1064 | TRAV8-4*04-RIGHT | aggcatcaacggttttgaggctgaatttaagaagagtgaaacctccttccacctgacgaaaccctcagcccatatgagcgacgcggctgagta cttctgt |
| SEQ ID NO: 1065 | TRAV8-4*05-RIGHT | ggctgaatttaagaagagtgaaacctccttccacctgacgaaaccctcagcccatatgagcgacgcggctgagtacttctgtgctgtgagtga gtctcca |
| SEQ ID NO: 1066 | TRAV8-4*06-RIGHT | gaatttaagaagagtgaaacctccttccacctgacgaaacccgcagcccatatgagcgacgcggctgagtacttctgtgctgtgagtgatctcg aaccga |
| SEQ ID NO: 1067 | TRAV8-4*07-RIGHT | acggttttgaggctgaatttaaaaagagtgaaacctccttccacctgacgaaaccctcagcccatatgaccgacccggctgagtacttctgtgc tgtgag |
| SEQ ID NO: 1068 | TRAV9-2*02-RIGHT | caacaaaggttttgaagccacataccgtaaagaaaccacttctttccacttggagaaaggctcagttcaagtgtcagactcagcggtgtacttc tgtgct |
| SEQ ID NO: 1069 | TRAV9-2*03-RIGHT | caacaaaggttttgaagccacataccgtaaggaaaccacttctttccacttggagaaaggctcagttcaagtgtcagactcagcggtgtacttc tgtgct |
| SEQ ID NO: 1070 | TRAV9-2*04-RIGHT | caacaaaggttttgaagccacataccgtaaggaaaccacttctttccacttggagaaaggctcagttcaagtgtcagactcagcggtgtacttc tgtgct |
| SEQ ID NO: 1071 | TRBV10-1*03-RIGHT | ctaacaaaggagaagtctcagatggctacagtgtctctagatcaaacacagaggacctccccctcactctgtagtctgctgcctcctcccagac atctgt |
| SEQ ID NO: 1072 | TRBV10-2*02-RIGHT | agataaaggagaagtccccgatggctacgttgtctccagatccaagacagagaatttccccctcactctggagtcagctacccgctcccagac atctgtg |
| SEQ ID NO: 1073 | TRBV10-3*03-RIGHT | agaagtctcagatggctatagtgtctctagatcaaagacagaggatttcctcctcactctggagtccgctaccagctcccagacatctgtgtact tctgt |
| SEQ ID NO: 1074 | TRBV10-3 *04-RIGHT | agaagtctcagatggctatagtgtctctagatcaaagacagaggatttcctcctcactctggagtccgctaccagctcccagacatctgtgtact tctgt |
| SEQ ID NO: 1075 | TRBV11-2*02-RIGHT | ggatcgattttctgcagagaggctcaaaggagtagactccactctcaagatccagcctgcaaagcttgagaactcggccgtgtatctctgtgcc agcagt |
| SEQ ID NO: 1076 | TRBV11-2*03-RIGHT | ggatcgattttctgcagagaggctcaaaggagtagactccactctcaagatccaacctgcaaagcttgaggactcggccgtgtatctctgtgcc agcagc |
| SEQ ID NO: 1077 | TRBV11-3*02-RIGHT | ggatcgattttctgcagagaggctcaaaggagtagactccactctcaagatccagcctgcagagcttggggactcggccgtgtatctctgtgcc agcagc |
| SEQ ID NO: 1078 | TRBV11-3*03-RIGHT | ggatcgattttctgcagagaggctcaaaggagtagactccactctcaagatccagccagcagagcttggggactcggccatgtatctctgtgcc agcagc |
| SEQ ID NO: 1079 | TRBV12-4*02-RIGHT | tcgattctcagctaagatgcctaatgcatcattctccactctgaggatccagccctcagaacccagggactcagctgtgtacttctgtgccagca gttta |
| SEQ ID NO: 1080 | TRBV13*02-RIGHT | tgatcgattctcagctcaacagttcagtgactatcattctgaactgaacatgagctccttggagctggggactcagccctgtacttctgtgcca gcagc |
| SEQ ID NO: 1081 | TRBV14*02-RIGHT | caatcgattcttagctgaaaggactggagggacgtattctactctgaaggtgcagcctgcagaactggaggattctggagtttatttctgtgcca gcagc |
| SEQ ID NO: 1082 | TRBV15*02-RIGHT | tgataacttccaatccaggaggccgaacacttctttctgctttcttgacatccgctcaccaggcctgggggacgcagccatgtacctgtgtgcca ccagc |
| SEQ ID NO: 1083 | TRBV15*03-RIGHT | tgataacttccaatccaggaggccgaacacttctttctgctttctagacatccgctcaccaggcctggggacgcagccatgtaccagtgtgcc accagc |
| SEQ ID NO: 1084 | TRBV16*03-RIGHT | ggaaagattttcagctaagtgcctcccaaattcaccctgtagccttgagatccaggctacgaagcttgaggattcagcagtgtatttttgtgcca gcagc |
| SEQ ID NO: 1085 | TRBV19*03-RIGHT | tgaagggtacagcgtctctcgggagaagaaggaatcctttcctctcactgtgacatcggcccaaaagaacccgacagctttctatctctgtgcc agtagc |
| SEQ ID NO: 1086 | TRBV2*02-RIGHT | tgatcaattctcagttgaaaggcctgatggatcaaatttcactctgaagatccggtccacaaagctggaggactcagccatgtacttctgtgcca gcagt |
| SEQ ID NO: 1087 | TRBV2*03-RIGHT | tcaattctcagttgagaggcctgatggatcaaatttcactctgaagatccggtccacaaagctggaggactcagccatgtacttctgtgccagc agtgaa |
| SEQ ID NO: 1088 | TRBV20-1*02-RIGHT | gaaggacaagtttctcatcaaccatgcaagcctgaccttgtccactctgacagtgaccagtgcccatcctgaagacagcagcttctacatctgc agtgct |
| SEQ ID NO: 1089 | TRBV20-1*04-RIGHT | ggacaagtttctcatcaaccatgcaagcctgaccttgtccactctgacagtgaccagtgcccatcctgaagacagcagcttctacatctgcagt gctagt |
| SEQ ID NO: 1090 | TRBV20-1*05-RIGHT | ggacaagtttctcatcaaccatgcaagcctgaccttgtccactctgacagtgaccagtgcccatcctgaagacagcagcttctacatctgcagt gctaga |
| SEQ ID NO: 1091 | TRBV20-1*06-RIGHT | gaaggacaagtttctcatcaaccatgcaagcctgaccttgtccactctgacagtgaccagtgcccatcctgaagacagcagcttctacatctgc agtgct |
| SEQ ID NO: 1092 | TRBV20-1*07-RIGHT | ggacaagtttctcatcaaccatgcaagcctgaccttgtccactctgacagtgaccagtgcccatcctgaagacagcagcttctacatctgcagt gctaga |
| SEQ ID NO: 1093 | TRBV20/OR9-2*02-RIGHT | gaaggacaagtttcccatcaaccatccaaacctgaccttctccgctctgacagtgacctgtgcccatcctgaagacagcagcttctacatctgc agtgct |
| SEQ ID NO: 1094 | TRBV23/OR9-2*02-RIGHT | gtttttgatttcctttcagaatgaacaagttcttcaagaaatggagatgcacaagaagcgattctcatctcaatgccccaagaacgcaccctgc agcctg |
| SEQ ID NO: 1095 | TRBV24/OR9-2*02-RIGHT | cagttgatctattgctcctttgatgtcaaaatatataaacaaaagagagatctctgatggatacagtgtctcttgacaggaacaggctaaattct ccctg |
| SEQ ID NO: 1096 | TRBV25/OR9-2*02-RIGHT | gagttaattccacagagaagggagatctttgctctgagtcaacagtctccagaataaggatagagcgttttcccctgaccctggagtctgccag cccctc |
| SEQ ID NO: 1097 | TRBV29-1*02-RIGHT | tgacaagtttcccatcagccgcccaaacctaacattctcaagtctgactgtgagcaacatgagccctgaagacagcagcatatatctctgcagc gttgaa |
| SEQ ID NO: 1098 | TRBV29-1*03-RIGHT | tgacaagtttcccatcagccgcccaaacctaacattctcaactctgactgtgagcaacatgagccctgaagacagcagcatatatctctgcagc gcgggc |
| SEQ ID NO: 1099 | TRBV3-1*02-RIGHT | tccaaatcgattctcacctaaatctccagacaaagctaaattaaatcttcacatcaattccctggagcttggtgactctgctgtgtatttctgtgcc agc |
| SEQ ID NO: 1100 | TRBV3-2*03-RIGHT | tcgcttctcacctgactctccagacaaagttcatttaaatcttcacatcaattccctggagcttggtgactctgctgtgtatttctgtgccagcagc caa |
| SEQ ID NO: 1101 | TRBV30*02-RIGHT | agaatctctcagcctccagaccccaggaccggcagttcatcctgagttctaagaagctcctcctcagtgactctggcttctatctctgtgcctgga gtgt |
| SEQ ID NO: 1102 | TRBV30*04-RIGHT | ccagaatctctcagcctccagaccccaggaccggcagttcattctgagttctaagaagctcctcctcagtgactctggcttctatctctgtgcctg gagt |
| SEQ ID NO: 1103 | TRBV30*05-RIGHT | ccagaatctctcagcctccagaccccaggaccggcagttcatcctgagttctaagaagctccttctcagtgactctggcttctatctctgtgcctg ggga |
| SEQ ID NO: 1104 | TRBV4-1*02-RIGHT | tcgcttctcacctgaatgccccaacagctctctcttaaaccttcacctacacgccctgcagccagaagactcagccctgtatctctgcgccagca gccaa |
| SEQ ID NO: 1105 | TRBV4-2*02-RIGHT | aagtcgcttctcacctgaatgccccaacagctctcacttatgccttcacctacacaccctgcagccagaagactcggccctgtatctctgtgcca gcacc |
| SEQ ID NO: 1106 | TRBV4-3*02-RIGHT | aagtcgcttctcacctgaatgccccaacagctctcacttatcccttcacctacacaccctgcagccagaagactcggccctgtatctctgcgcca gcagc |
| SEQ ID NO: 1107 | TRBV4-3*03-RIGHT | aagtcgcttctcacctgaatgccccaacagctctcacttattccttcacctacacaccctgcagccagaagactcggccctgtatctctgcgcca gcagc |
| SEQ ID NO: 1108 | TRBV4-3*04-RIGHT | aagtcgcttctcacctgaatgccccaacagctctcacttattccttcacctacacaccctgcagccagaagactcggccctgtatctctgcgcca gcagc |
| SEQ ID NO: 1109 | TRBV5-1*02-RIGHT | tcgattctcagggcgccagttctctaactctcgctctgagatgaatgtgagcaccttggagctggggactcggccctttatctttgcgccagcgc ttgc |
| SEQ ID NO: 1110 | TRBV5-4*02-RIGHT | tcctagattctcaggtctccagttccctaattataactctgagctgaatgtgaacgccttggagctggacgactcggccctgtatctctgtgccag cagc |
| SEQ ID NO: 1111 | TRBV5-4*03-RIGHT | tcctagattctcaggtctccagttccctaattatagctctgagctgaatgtgaacgccttggagctggacgactcggccctgtatctctgtgccag cagc |
| SEQ ID NO: 1112 | TRBV5-4*04-RIGHT | tcctagattctcaggtctccagttccctaattatagctctgagctgaatgtgaacgccttggagctggacgactcggccctgtatctctgtgccag cagc |
| SEQ ID NO: 1113 | TRBV5-5*02-RIGHT | tgatcgattctcagctcgccagttccctaactatagctctgagctgaatgtgaacgccttgttgctgggggactcggccctgtatctctgtgccag cagc |
| SEQ ID NO: 1114 | TRBV5-5*03-RIGHT | tgatcgattctcagctcgccagttccctaactatagctctgagctgaatgtgaacgccttgttgctgggggactcggccctgtatctctgtgccag cagc |
| SEQ ID NO: 1115 | TRBV5-8*02-RIGHT | tcctagattttcaggtcgccagttccctaattatagctctgagctgaatgtgaacgccttggagctggaggactcggccctgtatctctgtgccag cagc |
| SEQ ID NO: 1116 | TRBV6-2*02-RIGHT | tggctacaatgtctccagattaaaaaaacagaatttcctgctggggttggagtcggctgctccctcccaaacatctgtgtacttctgtgccagca gccct |
| SEQ ID NO: 1117 | TRBV6-6*03-RIGHT | . gaatggctacaacgtctccagatcaaccacagaggatttcccgctcaggctggagttggctgctccctcccagacatctgtgtacttctgtgcca gcagt |
| SEQ ID NO: 1118 | TRBV6-6*04-RIGHT | tggctacaatgtctccagatcaaccacagaggatttcccgctcaggctggagttggctgctccctcccagacatctgtgtacttctgtgccagca gtcga |
| SEQ ID NO: 1119 | TRBV6-6*05-RIGHT | gaatggctacaacgtctccagatcaaccacagaggatttcccgctcaggctggagttggctgctgcctcccagacatctgtgtacttctgtgcca gcagc |
| SEQ ID NO: 1120 | TRBV7-2*03-RIGHT | :gcttctctgcagagaggactggggaatccgtctccactctgacgatccagcgcacacagcaggaggactcggccgtgtatctctgtaccagca gcttagc |
| SEQ ID NO: 1121 | TRBV7-2*04-RIGHT | ⁝ tcgcttctctgcagagaggactggggatccgtctccactctgacgatccagcgcacacagcaggaggactcggccgtgtatctctgtgccagc agctta |
| SEQ ID NO: 1122 | TRBV7-3*04-RIGHT | cgatcggttctttgcagtcaggcctgagggatccgtctctactctgaagatccagcgcacagagcgggggactctgccgtgtatctctgtgcca gcagc |
| SEQ ID NO: 1123 | TRBV7-3*05-RIGHT | cgatcggttctttgcagtcaggcctgagggatccgtctctactctgaagatccagcgcacagagcgggggactcagccgtgtatctctgtgcc agcagc |
| SEQ ID NO: 1124 | TRBV7-4*02-RIGHT | aacgagacaaatcagggcggcccagtggtcggttctctgcagagaggcctgagagatcgtctccactccgaagatccagcgcacagagcag ggggactca |
| SEQ ID NO: 1125 | TRBV7-6*02-RIGHT | tgatcggttctctgcagagaggcctgagggatccatctccactctgacgatccagcgcacagagcagcgggactcggccatgtatcgctgtgcc agcagc |
| SEQ ID NO: 1126 | TRBV7-7*02-RIGHT | tgatcggttctctgcagagaggcctgagggatccatctccactctgacgattcagcgcacagagcagcgggactcagccatgtatcgctgtgcc agcagc |
| SEQ ID NO: 1127 | TRBV7-8*03-RIGHT | tcgcttctttgcagaaaggcctgagggatccgtctccactctgaagatccagcgcacacagcaggaggactccgccgtgtatctctgtgccagc agccga |
| SEQ ID NO: 1128 | TRBV7-9*02-RIGHT | tcggttctctgcagagaggcctaagggatctttctccaccttggagatccagcgcacagagcaggggggactcggccatgtatctctgtgccagc agctta |
| SEQ ID NO: 1129 | TRBV7-9*04-RIGHT | tcggatctctgcagagaggcctaagggatctttctccaccttggagatccagcgcacagagcaggggggactcggccatgtatctctgtgccagc agctct |
| SEQ ID NO: 1130 | TRBV7-9*05-RIGHT | tcggttctctgcagagaggcctaagggatctctctccaccttggagatccagcgcacagagcaggggggactcggccatgtatctctgtgccagc accaaa |
| SEQ ID NO: 1131 | TRBV7-9*06-RIGHT | tcggttctctgcagagaggcctaagggatctctttccaccttggagatccagcgcacagagcaggggggactcggccatgtatctctgtgccagc acgttg |
| SEQ ID NO: 1132 | TRBV7-9*07-RIGHT | gttctctgcagagaggcctaagggatctttctccaccttggagatccagcgcacagaggagggggactcggccatgtatctctgtgccagcagc agcagt |
| SEQ ID NO: 1133 | TRBV9*03-RIGHT | tgaacgattctccgcacaacagttccctgacttgcactctgaactaaacctgagctctctggagctggggactcagctttgtatttctgtgccag cagc |
| SEQ ID NO: 1134 | TRGV2*02-RIGHT | gaagtattatacttacgcaagcacaaggaacaacttgagattgatactgcaaaatctaattgaaaatgactctggggtctattactgtgccacc tgggac |
| SEQ ID NO: 1135 | TRBV20-1*03-RIGHT | gaaggacaagtttctcatcaaccatgcaagcctgaccttgtccactctgacagtgaccagtgcccatcctgaagacagcagcttctacatctgc agtgct |
| SEQ ID NO: 1136 | TRGV6*01-RIGHT | gcatgatacttatggaagtagaaggataagctggaaatttatacctccaaaactaaatgaaaatgcctctggggtctattactgtgccacctag gacagg |
| SEQ ID NO: 1137 | TRGV4*02-RIGHT | gtatgatacttacggaagcacaaggaagaacttgagaatgatactgcgaaatcttattgaaaatgactctggagtctattactgtgccacctgg gatggg |
| SEQ ID NO: 1138 | TRGV5P*01-RIGHT | gtattatactcatacaccgaggaggtggagctggaatttgagactgcaaaatctaattgaaaatgattctggggtctattactgtgccacctgg ggcagg |
| SEQ ID NO: 1139 | TRBV10-3*02-RIGHT | gctatagtgtctctagatcaaagacagaggatttcctcctcactctggagtccgctaccagctcccagacatctgtgtacttctgtgccatcagtg agtc |
| SEQ ID NO: 1140 | TRBV24/OR9-2*01-RIGHT | atacagtgtctctcgacaggcacaggctaaattctccctgtccctagagtctgccatccccaaccagacagctctttacttctgtgccaccagtg atttg |
| SEQ ID NO: 1141 | TRBV20/OR9-2*01-RIGHT | acaagtttcccatcaaccatccaaacctgaccttctccgctctgacagtgaccagtgcccatcctgaagacagcagcttctacatctgcagtgct agaga |
| SEQ ID NO: 1142 | TRGV11*01-RIGHT | ggtaagtaaaaatgctcacacttccacttccactttgaaaataaagttcttagagaaagaagatgaggtggtgtaccactgtgcctgctggatt aggcac |
| SEQ ID NO: 1143 | TRBV7-8*02-RIGHT | gcttctttgcagaaaggcctgagggatccgtctccactctgaagatccagcgcacacagaaggaggactccgccgtgtatctctgtgccagca gcttagc |
| SEQ ID NO: 1144 | TRBV7-3*02-RIGHT | ggttctttgcagtcaggcctgagggatccgtctctactctgaagatccagcgcacagagcaggggactcagccgtgtatctccgtgccagcag cttaac |
| SEQ ID NO: 1145 | TRGV10*01-RIGHT | aggcaagaaagaattctcaaactctcacttcaatccttaccatcaagtccgtagagaaagaagacatggccgtttactactgtgctgcgtggtg ggtggc |
| SEQ ID NO: 1146 | TRGV9*02-RIGHT | tgaggtggataggatacctgaaacgtctacatccactctcaccattcacaatgtagagaaacaggacatagctacctactactgtgccttgtgg gaggtg |
| SEQ ID NO: 1147 | TRDV3*02-RIGHT | gacggttttctgtgaaacacattctgacccagaaagcctttcacttggtgatctctccagtaaggactgaagacagtgccacttactactgtgcc tttag |
| SEQ ID NO: 1148 | TRDV2*02-RIGHT | aatttccaaggtgacattgatattgcaaagaacctggctgtacttaagatacttgcaccatcagagagagatgaagggtcttactactgtgcct gtgaca |
| SEQ ID NO: 1149 | TRGV3*02-RIGHT | agtattatactcatacacccaggaggtggagctggatattgagactgcaaaatctaattgaaaatgattctggggtctattactgtgccacctg ggacag |
| SEQ ID NO: 1150 | TRDV2*01-RIGHT | tttccaaggtgacattgatattgcaaagaacctggctgtacttaagatacttgcaccatcagagagagatgaagggtcttactactgtgcctgtg acacc |
| SEQ ID NO: 1151 | TRBV19*02-RIGHT | ggtacagcgtctctcgggagaagaaggaatcctttcctctcactgtgacatcggcccaaaagaacccgacagctttctatctctgtgccagtag tataga |
| SEQ ID NO: 1152 | TRAV14/DV4*01-RIGHT | actcattgaatttccagaaggcaagaaaatccgccaaccttgtcatctccgcttcacaactgggggactcagcaatgtacttctgtgcaatgag agaggg |
| SEQ ID NO: 1153 | TRBV3-2*02-RIGHT | gcttctcacctgactctccagacaaagttcatttaaatcttcacatcaattccctggagcttggtgactctgctgtgtatttctgtgccagcagcca aga |
| SEQ ID NO: 1154 | TRGV10*02-RIGHT | tggaggcaagaaagaattctcaaactctcacttcaatccttaccatcaagtccgtagagaaagaagacatggccgtttactactgtgctgcgtg ggatta |
| SEQ ID NO: 1155 | TRAV11*01-RIGHT | caaatattttaaagaactgcttggaaaagaaaaattttatagtgtttggaatatcgcagcctctcatctgggagattcagccacctacttctgtg ctttg |
| SEQ ID NO: 1156 | TRBV5-2*01-RIGHT | aacttgcctaattgattctcagctcaccacgtccataactattactgagtcaaacacggagctaggggactcagccctgtatctctgtgccagca acttg |
| SEQ ID NO: 1157 | TRBV8-1*01-RIGHT | ggaagggtacaatgtctctggaaacaagctcaagcattttccctcaaccctggagtctactagcaccagccagacctctgtacctctgtggcag tgcatc |
| SEQ ID NO: 1158 | TRAV38-1*01-RIGHT | tctctgtgaacttccagaaagcagccaaatccttcagtctcaagatctcagactcacagctgggggacactgcgatgtatttctgtgctttcatg aagca |
| SEQ ID NO: 1159 | TRBV22-1*01-RIGHT | aggctacgtgtctgccaagaggagaaggggctatttcttctcagggtgaagttggcccacaccagccaaacagctttgtacttctgtcctggga gcgcac |
| SEQ ID NO: 1160 | TRBV16*01-RIGHT | gattttcagctaagtgcctcccaaattcaccctgtagccttgagatccaggctacgaagcttgaggattcagcagtgtatttttgtgccagcagc caatc |
| SEQ ID NO: 1161 | TRBV30*01-RIGHT | agaatctctcagcctccagaccccaggaccggcagttcatcctgagttctaagaagctccttctcagtgactctggcttctatctctgtgcctgga gtgt |
| SEQ ID NO: 1162 | TRAV3*01-RIGHT | tttgaagctgaatttaacaagagccaaacctccttccacctgaagaaaccatctgcccttgtgagcgactccgctttgtacttctgtgctgtgag agaca |
| SEQ ID NO: 1163 | TRAV26-1*01-RIGHT | gcctctctgatcatcacagaagacagaaagtccagcaccttgatcctgccccacgctacgctgagagacactgctgtgtactattgcatcgtca gagtcg |
| SEQ ID NO: 1164 | TRAV32*01-RIGHT | aggctcactgtactgttgaataaaaatgctaaacatgtctccctgcatattacagccacccaaccaggagactcattcctgtacttctgtgcagt gagaa |
| SEQ ID NO: 1165 | TRAV33*01-RIGHT | gcaaagcctgtgaactttgaaaaaagaaaaagttcatcaacctcaccatcaattccttaaaactgactcagccaagtacttctgtgctctcag gaatcc |
| SEQ ID NO: 1166 | TRBV13*01-RIGHT | gattctcagctcaacagttcagtgactatcattctgaactgaacatgagctccttggagctgggggactcagccctgtacttctgtgccagcagc ttagg |
| SEQ ID NO: 1167 | TRBV15*01-RIGHT | acttccaatccaggaggccgaacacttctttctgctttcttgacatccgctcaccaggcctgggggacacagccatgtacctgtgtgccaccagc agaga |
| SEQ ID NO: 1168 | TRAV2*01-RIGHT | agggacgatacaacatgacctatgaacggttctcttcatcgctgctcatcctccaggtgcgggaggcagatgctgctgtttactactgtgctgtg gagga |
| SEQ ID NO: 1169 | TRBV7-1*01-RIGHT | ggttctctgcacagaggtctgagggatccatctccactctgaagttccagcgcacacagcaggggacttggctgtgtatctctgtgccagcag ctcagc |
| SEQ ID NO: 1170 | TRBV23-1*01-RIGHT | gattctcatctcaatgccccaagaacgcaccctgcagcctggcaatcctgtcctcagaaccgggagacacggcactgtatctctgcgccagca gtcaatc |
| SEQ ID NO: 1171 | TRBV23/OR9-2*01-RIGHT | gatgcacaagaagcgattctcatctcaatgccccaagaacccaccctgcagcctggcaatcctgtcctcggaaccgggagacaccgcactgta tctctgt |
| SEQ ID NO: 1172 | TRBVA*01-RIGHT | tccctattgaaaatatttcctggcaaaaaatagaagttctctttggctctgaaatctgcaactccctttcaggtgtccctgtgtccttgtaccgtca ctc |
| SEQ ID NO: 1173 | TRBVA/OR9-2*01-RIGHT | tccctgttgaaaatatttcccggcaaaaaacagaagttccctttggctctgaaatctgcaaagccctttcagatgtccctgtgtccttgtgccgtc actc |
| SEQ ID NO: 1174 | TRBV12-1*01-RIGHT | gattctcagcacagatgcctgatgtatcattctccactctgaggatccagcccatggaacccagggacttgggcctatatttctgtgccagcagc tttgc |
| SEQ ID NO: 1175 | TRBV26/OR9-2*01-RIGHT | ggtatcatgtttcttgaaatactatagcatcttttctcctgaccctgaagtctgctagcaccaaccagacatgtgtgtatctctgcgccagcagttc atc |
| SEQ ID NO: 1176 | TRGV9*01-RIGHT | tgaggtggataggatacctgaaacgtctacatccactctcaccattcacaatgtagagaaacaggacatagctacctactactgtgccttgtgg gaggtg |
| SEQ ID NO: 1177 | TRGVB*01-RIGHT | cttgaggcaagaacaaattttcaaatgtctacttcagtctttaccataaacttcataggaaaggaagatgaggccatttactactgcactgctta ggacc |
| SEQ ID NO: 1178 | TRBV7-3*01-RIGHT | ggttctttgcagtcaggcctgagggatccgtctctactctgaagatccagcgcacagagcgggggactcagccgtgtatctctgtgccagcag cttaac |
| SEQ ID NO: 1179 | TRBV7-9*01-RIGHT | ggttctctgcagagaggcctaagggatctttctccaccttggagatccagcgcacagagcaggggggactcggccatgtatctctgtgccagcag cttagc |
| SEQ ID NO: 1180 | TRBV7-2*01-RIGHT | gcttctctgcagagaggactggggatccgtctccactctgacgatccagcgcacacagcaggaggactcggccgtgtatctctgtgccagca gcttagc |
| SEQ ID NO: 1181 | TRBV7-2*02-RIGHT | gcttctctgcagagaggactggggaatccgtctccactctgacgatccagcgcacacagcaggaggactcggccgtgtatctctgtgccagca gcttagc |
| SEQ ID NO: 1182 | TRBV7-7*01-RIGHT | ggttctctgcagagaggcctgagggatccatctccactctgacgattcagcgcacagagcagcgggactcagccatgtatcgctgtgccagca gcttagc |
| SEQ ID NO: 1183 | TRBV7-8*01-RIGHT | gcttctttgcagaaaggcctgagggatccgtctccactctgaagatccagcgcacacagcaggaggactccgccgtgtatctctgtgccagcag cttagc |
| SEQ ID NO: 1184 | TRBV17*01-RIGHT | aacgattcacagctgaaagacctaacggaacgtcttccacgctgaagatccatcccgcagagccgagggactcagccgtgtatctctacagta gcggtgg |
| SEQ ID NO: 1185 | TRBV5-8*01-RIGHT | agattttcaggtcgccagttccctaattatagctctgagctgaatgtgaacgccttggagctggaggactcggccctgtatctctgtgccagcag cttgg |
| SEQ ID NO: 1186 | TRBV5-7*01-RIGHT | caattctcaggtcaccagttccctaactatagctctgagctgaatgtgaacgccttgttgctaggggactcggccctctatctctgtgccagcagc ttgg |
| SEQ ID NO: 1187 | TRBV5-6*01-RIGHT | cgattctcaggtcaccagttccctaactatagctctgagctgaatgtgaacgccttgttgctgggggactcggccctctatctctgtgccagcagc ttgg |
| SEQ ID NO: 1188 | TRBV5-5*01-RIGHT | cgattctcagctcgccagttccctaactatagctctgagctgaatgtgaacgccttgttgctgggggactcggccctgtatctctgtgccagcagc ttgg |
| SEQ ID NO: 1189 | TRBV5-4*01-RIGHT | agattctcaggtctccagttccctaattatagctctgagctgaatgtgaacgccttggagctggacgactcggccctgtatctctgtgccagcag cttgg |
| SEQ ID NO: 1190 | TRBV5-1*01-RIGHT | cgattctcagggcgccagttctctaactctcgctctgagatgaatgtgagcaccttggagctggggactcggccctttatctttgcgccagcag cttgg |
| SEQ ID NO: 1191 | TRBV3-1*01-RIGHT | gcttctcacctaaatctccagacaaagctcacttaaatcttcacatcaattccctggagcttggtgactctgctgtgtatttctgtgccagcagcc aaga |
| SEQ ID NO: 1192 | TRBV1*01-RIGHT | acttcacacctgaatgccctgacagctctcgcttataccttcatgtggtcgcactgcagcaagaagactcagctgcgtatctctgcaccagcagc caaga |
| SEQ ID NO: 1193 | TRBV5-3*01-RIGHT | cgattctcagggcgccagttccatgactgttgctctgagatgaatgtgagtgccttggagctgggggactcggccctgtatctctgtgccagaag cttgg |
| SEQ ID NO: 1194 | TRBV5-3*02-RIGHT | cgattctcagggcgccagttccatgactattgctctgagatgaatgtgagtgccttggagctgggggactcggccctgtatctctgtgccagaag cttgg |
| SEQ ID NO: 1195 | TRBV9*01-RIGHT | cgattctccgcacaacagttccctgacttgcactctgaactaaacctgagctctctggagctgggggactcagctttgtatttctgtgccagcagc gtag |
| SEQ ID NO: 1196 | TRBV3-2*01-RIGHT | gcttctcacctgactctccagacaaagctcatttaaatcttcacatcaattccctggagcttggtgactctgctgtgtatttctgtgccagcagcca aga |
| SEQ ID NO: 1197 | TRBV2*01-RIGHT | aattctcagttgaaaggcctgatggatcaaatttcactctgaagatccggtccacaaagctggaggactcagccatgtacttctgtgccagcag tgaagc |
| SEQ ID NO: 1198 | TRBV4-3*01-RIGHT | gcttctcacctgaatgccccaacagctctcacttattccttcacctacacaccctgcagccagaagactcggccctgtatctctgcgccagcagc caaga |
| SEQ ID NO: 1199 | TRBV4-1*01-RIGHT | gcttctcacctgaatgccccaacagctctctcttaaaccttcacctacacgccctgcagccagaagactcagccctgtatctctgcgccagcagc caaga |
| SEQ ID NO: 1200 | TRBV4-2*01-RIGHT | gcttctcacctgaatgccccaacagctctcacttattccttcacctacacaccctgcagccagaagactcggccctgtatctctgtgccagcagc caaga |
| SEQ ID NO: 1201 | TRAV34*01-RIGHT | aagataactgccaagttggatgagaaaaagcagcaaagttccctgcatatcacagcctcccagcccagccatgcaggcatctacctctgtgga gcagaca |
| SEQ ID NO: 1202 | TRBV28*01-RIGHT | ggtacagtgtctctagagagaagaaggagcgcttctccctgattctggagtccgccagcaccaaccagacatctatgtacctctgtgccagcag tttatg |
| SEQ ID NO: 1203 | TRBV20-1*01-RIGHT | acaagtttctcatcaaccatgcaagcctgaccttgtccactctgacagtgaccagtgcccatcctgaagacagcagcttctacatctgcagtgct agaga |
| SEQ ID NO: 1204 | TRBV20/OR9-2*03-RIGHT | acaagtttcccatcaaccatccaaacctgaccttctccgctctgacagtgaccagtgcccatcctgaagacagcagcttctacatctgcagtgct agaga |
| SEQ ID NO: 1205 | TRBV6-6*02-RIGHT | gaatggctacaacgtctccagatcaaccacagaggatttcccgctcaggctggagttggctgctccctcccagacatctgtgtacttctgtgcca gcagt |
| SEQ ID NO: 1206 | TRBV6-6*01-RIGHT | gctacaacgtctccagatcaaccacagaggatttcccgctcaggctggagttggctgctccctcccagacatctgtgtacttctgtgccagcagt tactc |
| SEQ ID NO: 1207 | TRBV6-5*01-RIGHT | gctacaatgtctccagatcaaccacagaggatttcccgctcaggctgctgtcggctgctccctcccagacatctgtgtacttctgtgccagcagtt actc |
| SEQ ID NO: 1208 | TRBV6-8*01-RIGHT | gctacaatgtctctagattaaacacagaggatttcccactcaggctggtgtcggctgctccctcccagacatctgtgtacttgtgtgccagcagtt actc |
| SEQ ID NO: 1209 | TRBV6-9*01-RIGHT | gctacaatgtatccagatcaaacacagaggatttcccgctcaggctggagtcagctgctccctcccagacatctgtatacttctgtgccagcagt tattc |
| SEQ ID NO: 1210 | TRBV6-7*01-RIGHT | gctacaatgtctccagatcaaacacagaggatttccccctcaagctggagtcagctgctccctctcagacttctgtttacttctgtgccagcagtt actc |
| SEQ ID NO: 1211 | TRBV12-3*01-RIGHT | gattctcagctaagatgcctaatgcatcattctccactctgaagatccagccctcagaacccagggactcagctgtgtacttctgtgccagcagt ttagc |
| SEQ ID NO: 1212 | TRBV12-4*01-RIGHT | gattctcagctaagatgcctaatgcatcattctccactctgaagatccagccctcagaacccagggactcagctgtgtacttctgtgccagcagt ttagc |
| SEQ ID NO: 1213 | TRBV12-5*01-RIGHT | gattctcagcagagatgcctgatgcaactttagccactctgaagatccagccctcagaacccagggactcagctgtgtatttttgtgctagtggt ttggt |
| SEQ ID NO: 1214 | TRBV12-2*01-RIGHT | gattctcagctgagaggcctgatggatcattctctactctgaagatccagcctgcagagcaggggactcggccgtgtatgtctgtgcaagtcg cttagc |
| SEQ ID NO: 1215 | TRBV6-1*01-RIGHT | gctacaatgtctccagattaaacaaacgggagttctcgctcaggctggagtcggctgctccctcccagacatctgtgtacttctgtgccagcagt gaagc |
| SEQ ID NO: 1216 | TRBV7-4*01-RIGHT | ggttctctgcagagaggcctgagagatccgtctccactctgaagatccagcgcacagagcaggggactcagctgtgtatctctgtgccagca gcttagc |
| SEQ ID NO: 1217 | TRBV7-5*01-RIGHT | tcaattctccacagagaggtctgaggatctttctccacctgaagatccagcgcacagagcaagggcgactcggctgtgtatctctgtgccagaa gcttag |
| SEQ ID NO: 1218 | TRAV20*01-RIGHT | aaagaaaggctaaaagccacattaacaaagaaggaaagctttctgcacatcacagcccctaaacctgaagactcagccacttatctctgtgct gtgcagg |
| SEQ ID NO: 1219 | TRBV11-1*01-RIGHT | gattttctgcagagaggctcaaaggagtagactccactctcaagatccagcctgcagagcttggggactcggccatgtatctctgtgccagcag cttagc |
| SEQ ID NO: 1220 | TRAV15*01-RIGHT | acatttaaagaagcgcttggaaaagagaagttttatagtgttttgaatatgctggtctctcatcctggagattcaggcacctacttctgtgcttt gagg |
| SEQ ID NO: 1221 | TRAV7*01-RIGHT | aaaggaagactaaatgctacattactgaagaatggaagcagcttgtacattacagccgtgcagcctgaagattcagccacctatttctgtgctg tagatg |
| SEQ ID NO: 1222 | TRAV16*01-RIGHT | gcttcactgctgaccttaacaaaggcgagacatctttccacctgaagaaaccatttgctcaagaggaagactcagccatgtattactgtgctct aagtgg |
| SEQ ID NO: 1223 | TRAV6*01-RIGHT | agactgaaggtcacctttgataccacccttaaacagagtttgtttcatatcacagcctcccagcctgcagactcagctacctacctctgtgctcta gaca |
| SEQ ID NO: 1224 | TRBV19*01-RIGHT | ggtacagcgtctctcgggagaagaaggaatcctttcctctcactgtgacatcggcccaaaagaacccgacagctttctatctctgtgccagtag tataga |
| SEQ ID NO: 1225 | TRAV14/DV4*02-RIGHT | actcattgaatttccagaaggcaagaaaatccgccaaccttgtcatctccgcttcacaactggggactcagcaatgtatttctgtgcaatgag agaggg |
| SEQ ID NO: 1226 | TRAV9-1*01-RIGHT | gttttgaagccatgtaccgtaaagaaaccacttctttccacttggagaaagactcagttcaagagtcagactccgctgtgtacttctgtgctctg agtga |
| SEQ ID NO: 1227 | TRAV9-2*01-RIGHT | gttttgaagccacataccgtaaagaaaccacttctttccacttggagaaaggctcagttcaagtgtcagactcagcggtgtacttctgtgctctg agtga |
| SEQ ID NO: 1228 | TRAV1-1*01-RIGHT | gtttttcttcattccttagtcgctctgatagttatggttacctccttctacaggagctccagatgaaagactctgcctcttacttctgcgctgtgaga ga |
| SEQ ID NO: 1229 | TRAV38-8*01-RIGHT | ttctctgtgaacttccagaaagcagccaaatccttcagtctcaagatctcagactcacagctgggggatgccgcgatgtatttctgtgcttatag gagcg |
| SEQ ID NO: 1230 | TRAV19*01-RIGHT | attcttggaacttccagaaatccaccagttccttcaacttcaccatcacagcctcacaagtcgtggactcagcagtatacttctgtgctctgagtg aggc |
| SEQ ID NO: 1231 | TRAV30*01-RIGHT | aaaatatctgcttcatttaatgaaaaaaagcagcaaagctccctgtaccttacggcctcccagctcagttactcaggaacctacttctgcggca cagaga |
| SEQ ID NO: 1232 | TRGV7*01-RIGHT | agtattttacttatgcaagcatgaggaggagctggaaattgatactgcaaaatctaattgaaaatgattctggatctattactgtgccacctggg acagg |
| SEQ ID NO: 1233 | TRGV1*01-RIGHT | aaagtatgacactggaagcacaaggagcaattggaatttgagactgcaaaatctaattaaaaatgattctgggttctattactgtgccacctgg gacagg |
| SEQ ID NO: 1234 | TRGV3*01-RIGHT | gtattatactcatacacccaggaggtggagctggatattgagactgcaaaatctaattgaaaatgattctggggtctattactgtgccacctgg gacagg |
| SEQ ID NO: 1235 | TRGV5*01-RIGHT | gtattatactcatacacccaggaggtggagctggatattgatactacgaaatctaattgaaaatgattctggggtctattactgtgccacctggg acagg |
| SEQ ID NO: 1236 | TRGV8*01-RIGHT | gtatcatacttatgcaagcacagggaagagccttaaatttatactggaaaatctaattgaacgtgactctggggtctattactgtgccacctggg atagg |
| SEQ ID NO: 1237 | TRGV4*01-RIGHT | gtatgatacttatggaagcacaaggaagaacttgagaatgatactgcgaaatcttattgaaaatgactctggagtctattactgtgccacctgg gatggg |
| SEQ ID NO: 1238 | TRGV2*01-RIGHT | gtattatacttacgcaagcacaaggaacaacttgagattgatactgcgaaatctaattgaaaatgactctggggtctattactgtgccacctgg gacggg |
| SEQ ID NO: 1239 | TRGV5P*02-RIGHT | gtattatactcatacaccgaggaggtggagctggaatttgagactgcaaaatctaattgaaaatgattctggggtctattactgtgccacctgg ggcagg |
| SEQ ID NO: 1240 | TRAV21*01-RIGHT | aagacttaatgcctcgctggataaatcatcaggacgtagtactttatacattgcagcttctcagcctggtgactcagccacctacctctgtgctgt gagg |
| SEQ ID NO: 1241 | TRBV29/OR9-2*01-RIGHT | acaagtttcccatcagccgcccaaacctaacattctcaactctgactgtgagcaacaggagacctgaagacagcagcatatacctctgcagcg ttgaaga |
| SEQ ID NO: 1242 | TRAV37*01-RIGHT | agattcacagccaggcttaaaaaggagaccagcacatttccctgcacatacaggattcccagctccatgactcaaccacattcttctgcgca gcaagca |
| SEQ ID NO: 1243 | TRBV21/OR9-2*01-RIGHT | gattttcagcccaatgcccccaaaactcaccctgtaccttggagatccagtccacggagtcaggagacacagcacggtatttctgtgccaacag caaagc |
| SEQ ID NO: 1244 | TRBV21-1*01-RIGHT | gatttttagcccaatgctccaaaaactcatcctgtaccttggagatccagtccacggagtcaggggacacagcactgtatttctgtgccagcag caaagc |
| SEQ ID NO: 1245 | TRAV8-6*01-RIGHT | gttttgaggctgaatttaacaagagtcaaacttccttccacttgaggaaaccctcagtccatataagcgacacggctgagtacttctgtgctgtg agtga |
| SEQ ID NO: 1246 | TRAV8-3*01-RIGHT | gctttgaggctgaatttaagaggagtcaatcttccttcaatctgaggaaaccctctgtgcattggagtgatgctgctgagtacttctgtgctgtgg gtgc |
| SEQ ID NO: 1247 | TRBV29-1*01-RIGHT | acaagtttcccatcagccgcccaaacctaacattctcaactctgactgtgagcaacatgagccctgaagacagcagcatatatctctgcagcgt tgaaga |
| SEQ ID NO: 1248 | TRBV25/OR9-2*01-RIGHT | agtcaacagtctccagaataaggatagagcgttttcccctgaccctggagtctgccagcccctcacatacctctcagtacctctgtgccagcagt gaata |
| SEQ ID NO: 1249 | TRBV25-1*01-RIGHT | agtcaacagtctccagaataaggacggagcatttttcccctgaccctggagtctgccaggccctcacatacctctcagtacctctgtgccagcag tgaata |
| SEQ ID NO: 1250 | TRAV35*01-RIGHT | aagactgactgctcagtttggtataaccagaaaggacagcttcctgaatatctcagcatccatacctagtgatgtaggcatctacttctgtgctg ggcag |
| SEQ ID NO: 1251 | TRAV25*01-RIGHT | gaaaagactgacatttcagtttggagaagcaaaaaagaacagctccctgcacatcacagccacccagactacagatgtaggaacctacttct gtgcaggg |
| SEQ ID NO: 1252 | TRAV12-2*01-RIGHT | aggtttacagcacagctcaataaagccagccagtatgtttctgctcatcagagactcccagcccagtgattcagccacctacctctgtgccgt gaaca |
| SEQ ID NO: 1253 | TRAV12-1*01-RIGHT | aggtttacagcacagctcaatagagccagccagtatatttccctgctcatcagagactccaagctcagtgattcagccacctacctctgtgtggt gaaca |
| SEQ ID NO: 1254 | TRAV12-3*01-RIGHT | aggtttacagcacaggtcgataaatccagcaagtatatctccttgttcatcagagactcacagcccagtgattcagccacctacctctgtgcaat gagcg |
| SEQ ID NO: 1255 | TRAV23/DV6*01-RIGHT | agattcacaatctccttcaataaaagtgccaagcagttctcattgcatatcatggattcccagcctggagactcagccacctacttctgtgcagc aagca |
| SEQ ID NO: 1256 | TRAV22*01-RIGHT | agattaagcgccacgactgtcgctacggaacgctacagcttattgtacatttcctcttcccagaccacagactcaggcgtttatttctgtgctgtg gagc |
| SEQ ID NO: 1257 | TRAV41*01-RIGHT | aagattaattgccacaataaacatacaggaaaagcacagctccctgcacatcacagcctcccatcccagagactctgccgtctacatctgtgct gtcaga |
| SEQ ID NO: 1258 | TRAV39*01-RIGHT | cgattaatggcctcacttgataccaaagcccgtctcagcaccctccacatcacagctgccgtgcatgacctctctgccacctacttctgtgccgt ggaca |
| SEQ ID NO: 1259 | TRAV36/DV7*01-RIGHT | agactaagtagcatattagataagaaagaactttccagcatcctgaacatcacagccacccagaccggagactcggccatctacctctgtgct gtggagg |
| SEQ ID NO: 1260 | TRAV29/DV5*01-RIGHT | agattcactgtcttcttaaacaaaagtgccaagcacctctctgcacattgtgccctcccagcctggagactctgcagtgtacttctgtgcagca agcg |
| SEQ ID NO: 1261 | TRAV27*01-RIGHT | aagagactaacctttcagtttggtgatgcaagaaaggacagttctctccacatcactgcagcccagcctggtgatacaggcctctacctctgtg caggag |
| SEQ ID NO: 1262 | TRBV6-4*01-RIGHT | gttatagtgtctccagagcaaacacagatgatttccccctcacgttggcgtctgctgtaccctctcagacatctgtgtacttctgtgccagcagtg actc |
| SEQ ID NO: 1263 | TRBV10-1*01-RIGHT | gctacagtgtctctagatcaaacacagaggacctccccctcactctggagtctgctgcctcctcccagacatctgtatatttctgcgccagcagt gagtc |
| SEQ ID NO: 1264 | TRBV10-2*01-RIGHT | gctatgttgtctccagatccaagacagagaatttccccctcactctggagtcagctacccgctcccagacatctgtgtatttctgcgccagcagt gagtc |
| SEQ ID NO: 1265 | TRBV6-2*01-RIGHT | gctacaatgtctccagattaaaaaacagaatttcctgctggggttggagtcggctgctccctcccaaacatctgtgtacttctgtgccagcagt tactc |
| SEQ ID NO: 1266 | TRBV10-3*01-RIGHT | gctatagtgtctctagatcaaagacagaggatttcctcctcactctggagtccgctaccagctcccagacatctgtgtacttctgtgccatcagtg agtc |
| SEQ ID NO: 1267 | TRAV24*01-RIGHT | ggacgaataagtgccactcttaataccaaggagggttacagctatttgtacatcaaaggatcccagcctgaagactcagccacatacctctgt gccttta |
| SEQ ID NO: 1268 | TRBV14*01-RIGHT | gattcttagctgaaaggactggagggacgtattctactctgaaggtgcagcctgcagaactggaggattctggagtttatttctgtgccagcag ccaaga |
| SEQ ID NO: 1269 | TRBV24-1*01-RIGHT | atacagtgtctctcgacaggcacaggctaaattctccctgtccctagagtctgccatccccaaccagacagctctttacttctgtgccaccagtg atttg |
| SEQ ID NO: 1270 | TRBV24/OR9-2*03-RIGHT | agtgtctcttgacaggaacaggctaaattctccctgtccctagagcctgccacccccaaccagacagcttctaggttacttcagtgccaccagtg atttc |
| SEQ ID NO: 1271 | TRAV8-2*01-RIGHT | gttttgaggctgaatttaagaagagtgaaacctccttccacctgacgaaacccctcagcccatatgagcgacgcggctgagtacttctgtgttgtg agtga |
| SEQ ID NO: 1272 | TRAV8-4*01-RIGHT | gttttgaggctgaatttaagaagagtgaaacctccttccacctgacgaaaccctcagcccatatgagcgacgcggctgagtacttctgtgctgt gagtga |
| SEQ ID NO: 1273 | TRBV22/OR9-2*01-RIGHT | ggctacggtgtctcccgagaggagaaggggctgtttcttctcatggtgaagctggcccacaccagccaaacagctctgtacttctgtcctggga gtgcac |
| SEQ ID NO: 1274 | TRAV26-2*01-RIGHT | ggcctctctggcaatcgctgaagacagaaagtccagtaccttgatcctgcaccgtgctaccttgagagatgctgctgtgtactactgcatcctga gagac |
| SEQ ID NO: 1275 | TRBV11-2*01-RIGHT | gattttctgcagagaggctcaaaggagtagactccactctcaagatccagcctgcaaagcttgaggactcggccgtgtatctctgtgccagcag cttaga |
| SEQ ID NO: 1276 | TRBV11-3*01-RIGHT | gattttctgcagagaggctcaaaggagtagactccactctcaagatccagcctgcagagcttggggactcggccgtgtatctctgtgccagcag cttaga |
| SEQ ID NO: 1277 | TRAV8-1*01-RIGHT | gctttgaggctgaatttataaagagtaaattctcctttaatctgaggaaaccctctgtgcagtggagtgacacagctgagtacttctgtgccgtg aatgc |
| SEQ ID NO: 1278 | TRBV7-5*02-RIGHT | caattctccacagagaggtctgaggatctttctccacctgaagatccagcgcacagagcaagggcgactcggctgtgtatctctgtgtcagaag cttagc |
| SEQ ID NO: 1279 | TRBV7-6*01-RIGHT | ggttctctgcagagaggcctgagggatccatctccactctgacgatccagcgcacagagcagcgggactcggccatgtatcgctgtgccagca gcttagc |
| SEQ ID NO: 1280 | TRGV11*02-RIGHT | gataagtaaaaatgctcacacttccacttccactttgaaaataaagttcttagagaaagaagatgaggtggtgtaccactgtgcctgctggatt aggcac |
| SEQ ID NO: 1281 | TRAV17*01-RIGHT | agattaagagtcacgcttgacacttccaagaaaagcagttccttgttgatcacggcttcccgggcagcagacactgcttcttacttctgtgctac ggacg |
| SEQ ID NO: 1282 | TRBV27*01-RIGHT | ggtacaaagtctctcgaaaagagaagaggaatttccccctgatcctggagtcgcccagccccaaccagacctctctgtacttctgtgccagcag tttatc |
| SEQ ID NO: 1283 | TRDV1*01-RIGHT | attctgtcaacttcaagaaagcagcgaaatccgtcgccttaaccatttcagccttacagctagaagattcagcaaagtacttttgtgctcttggg gaact |
| SEQ ID NO: 1284 | TRBV18*01-RIGHT | gattttctgctgaatttcccaaagagggccccagcatcctgaggatccagcaggtagtgcgaggagattcggcagcttatttctgtgccagctc accacc |
| SEQ ID NO: 1285 | TRAV5*01-RIGHT | agactcactgttctattgaataaaaggataaacatctgtctctgcgcattgcagacacccagactggggactcagctatctacttctgtgcag agagta |
| SEQ ID NO: 1286 | TRAV13-2*01-RIGHT | agagtcaccgttttattgaataagacagtgaaacatctctctgcaaattgcagctactcaacctggagactcagctgtctacttttgtgcaga gaata |
| SEQ ID NO: 1287 | TRDV2*03-RIGHT | tttccaaggtgacattgatattgcaaagaacctggctgtacttaagatacttgcaccatcagagagagatgaagggtcttactactgtgcctgtg acacc |
| SEQ ID NO: 1288 | TRAV1-2*01-RIGHT | gtttttcttcattccttagtcggtctaaagggtacagttacctccttttgaaggagctccagatgaaagactctgcctcttacctctgtgctgtgag aga |
| SEQ ID NO: 1289 | TRDV3*01-RIGHT | gacggttttctgtgaaacacattctgacccagaaagcctttcacttggtgatctctccagtaaggactgaagacagtgccacttactactgtgcc tttag |
| SEQ ID NO: 1290 | TRAV31*01-RIGHT | tattctgtgagcttccagaaaacaactaaaactattcagcttatcatatcatcatcacagccagaagacctgcaacatatttctgttgtctcaaa gagcc |
| SEQ ID NO: 1291 | TRAV10*01-RIGHT | agatatacagcaactctggatgcagacacaaagcaaagctctctgcacatcacagcctcccagctcagcgattcagcctcctacatctgtgtg gtgagcg |
| SEQ ID NO: 1292 | TRAV28*01-RIGHT | gaagactaaaatccgcagtcaaagctgaggaactttatggccacctatacatcagattcccagcctgaggactcagctatttacttctgtgctgt gggga |
| SEQ ID NO: 1293 | TRAV40*01-RIGHT | aaaacttcggaggcggaaatattaaagacaaaaactcccccattgtgaaatattcagtccaggtatcagactcagccgtgtactactgtcttct gggaga |
| SEQ ID NO: 1294 | TRGV6*02-RIGHT | gcatgatacttatggaagtagaaggataagctggaaatttatacctccaaaactaaatgaaaatgcctctggggtctattactgtgccacctag gacagg |
| SEQ ID NO: 1295 | TRAV18*01-RIGHT | gttttcaggccagtcctatcaagagtgacagttccttccacctggagaagccctcggtgcagctgtcggactctgccgtgtactactgcgctctg agaga |
| SEQ ID NO: 1296 | TRBV26*01-RIGHT | ggtatcatgtttcttgaaatactatagcatcttttcccctgaccctgaagtctgccagcaccaaccagacatctgtgtatctctatgccagcagttc atc |
| SEQ ID NO: 1297 | TRBV8-2*01-RIGHT | agaggggtactgtgtttcttgaaacaagcttgagcatttccccaatcctggcatccaccagcaccagccagacctatctgtaccactgtggcag cacatc |
| SEQ ID NO: 1298 | TRGVA*01-RIGHT | agataaaatcatagccaaggatggcagcagctctatcttggcagtactgaagttggagacaggcatcgagggcatgaactactgcacaacct gggccctg |
| SEQ ID NO: 1299 | TRAV4*01-RIGHT | gcctccctgtttatccctgccgacagaaagtccagcactctgagcctgccccgggtttccctgagcgacactgctgtgtactactgcctcgtgggt gaca |
| SEQ ID NO: 1300 | TRAV8-7*01-RIGHT | aggctgaatttaagaagagcgaaacctccttctacctgaggaaaccatcaacccatgtgagtgatgctgctgagtacttctgtgctgtgggtga caggag |
| SEQ ID NO: 1301 | TRAV13-1*01-RIGHT | cgaattgctgttacattgaacaagacagccaaacatttctccctgcacatcacagagacccaacctgaagactcggctgtctacttctgtgcag caagta |
| SEQ ID NO: 1302 | TRBVB*01-RIGHT | gactctgagaccctctgcagcagcagcctatcagtgcagccacatcctctctgagcggatatgacaaaccccagggttgaagcgacctaacct atgagcc |
| SEQ ID NO: 1303 | TRAV8-5*01-RIGHT | tggacacttatcacttccccaatcaataccccctgtgatttcctatgcctgtctttactttaatctcttaatcctgtcagctgaggaggatgtatgtca cc |
| SEQ ID NO: 1304 | TRBV16*02-RIGHT | gattttcagctaagtgcctcccaaattcaccctgtagccttgagatccaggctacgaagcttgaggattcagcagtgtatttttgtgccagcagc caatc |
| SEQ ID NO: 1305 | TRBV26/OR9-2*02-RIGHT | ggtatcatgtttcttgaaatactatagcatcttttctcctgaccctgaagtctgctagcaccaaccagacatgtgtgtatctctgcgccagcagttc atc |
| SEQ ID NO: 1306 | TRBV7-3*03-RIGHT | ggttctttgcagtcaggcctgagggatccgtctctactctgaagatccagcgcacagagcaggggactcagccgcgtatctccgtgccagca gcttaac |
| SEQ ID NO: 1307 | TRBV7-9*03-RIGHT | a tgatcggttctctgcagagaggcctaagggatctttctcca ccttggagatccag cgcacagagcagggggactcggccatgtatctctgtgcc agcagc |
| SEQ ID NO: 1308 | TRBV9*02-RIGHT | cgattctccgcacaacagttccctgacttgcactctgaactaaacctgagctctctggagctgggggactcagctttgtatttctgtgccagcagc gtag |
| SEQ ID NO: 1309 | TRBV29/OR9-2*02-RIGHT | acaagtttcccatcagccgcccaaacctaacattctcaactctgactgtgagcaacaggagacctgaagacagcagcatatacctctgcagcg ttgaaga |
| SEQ ID NO: 1310 | TRAV8-6*02-RIGHT | gttttgaggctgaatttaacaagagtcaaacttccttccacttgaggaaaccctcagtccatataagcgacacggctgagtacttctgtgctgtg agtga |
| SEQ ID NO: 1311 | TRBV6-4*02-RIGHT | gttatagtgtctccagagcaaacacagatgatttccccctcacgttggcgtctgctgtaccctctcagacatctgtgtacttctgtgccagcagtg actc |
| SEQ ID NO: 1312 | TRBV10-1*02-RIGHT | agatggctacagtgtctctagatcaaacacagaggacctccccctcactctggagtctgctgcctcctcccagacatctgtatatttctgcgcca gcagt |
| SEQ ID NO: 1313 | TRBV6-3*01-RIGHT | gctacaatgtctccagattaaaaaacagaatttcctgctggggttggagtcggctgctccctcccaaacatctgtgtacttctgtgccagcagt tactc |
| SEQ ID NO: 1314 | TRAJ1*01 | aatagagacacggggcatggtatgaaagtattacctcccagttgcaatttggcaaaggaaccagagtttccacttctccccgtacgtctgccca tgccca |
| SEQ ID NO: 1315 | TRAJ10*01 | gaggcatcaaacactgtgatactcacgggaggaggaaacaaactcacctttgggacaggcactcagctaaaagtggaactcagtaagtatg agattctat |
| SEQ ID NO: 1316 | TRAJ11*01 | tatggggatttgctatagtgtgaattcaggatacagcaccctcacctttgggaaggggactatgcttctagtctctccaggtacatgttgacccc atccc |
| SEQ ID NO: 1317 | TRAJ12*01 | actgactaagaaacactgtgggatggatagcagctataaattgatcttcgggagtgggaccagactgctggtcaggcctggtaagtaaggtgt cagagag |
| SEQ ID NO: 1318 | TRAJ13*01 | aaggcaggcattacagtgtgaattctgggggttaccagaaagttacctttggaattggaacaaagctccaagtcatcccaagtgagtccaattt cctatg |
| SEQ ID NO: 1319 | TRAJ13*02 | aaaggcaggcattacagtgtgaattctgggggttaccagaaagttacctttggaactggaacaaagctccaagtcatcccaagtgagtccaat ttcctat |
| SEQ ID NO: 1320 | TRAJ14*01 | tttgtcaggcagcacagtgctgtgatttatagcacattcatctttgggagtgggacaagattatcagtaaaacctggtaagtaggcaatatgtca ctaaa |
| SEQ ID NO: 1321 | TRAJ15*01 | cagggcctcatttcactgtgccaaccaggcaggaactgctctgatctttgggaagggaaccaccttatcagtgagttccagtaagtacctgata attatt |
| SEQ ID NO: 1322 | TRAJ15*02 | caggcctcatttcactgtgccaaccaggcaggaactgctctgatctttgggaagggaacccacctatcagtgagttccagtaagtacctgata attatt |
| SEQ ID NO: 1323 | TRAJ16*01 | tggtacaatagatcactgtgggttttcagatggccagaagctgctctttgcaaggggaaccatgttaaaggtggatcttagtaagtattattact aatga |
| SEQ ID NO: 1324 | TRAJ17*01 | cctgtggtttttgctgggccttaaatcattgtgtgatcaaagctgcaggcaacaagctaacttttggaggaggaaccagggtgctagttaaacc aagtga |
| SEQ ID NO: 1325 | TRAJ18*01 | aggggaccagcattgtgccgacagaggctcaaccctggggaggctatactttggaagaggaactcagttgactgtctggcctggtgagtgagt cgctttc |
| SEQ ID NO: 1326 | TRAJ19*01 | ttttgcagaggacagatgtggctatcaaagattttacaatttcacctttggaaagggatccaaacataatgtcactccaagtaagtgagcagcc ttttgt |
| SEQ ID NO: 1327 | TRAJ2*01 | tggtgtcacctacggtatgaatactggaggaacaattgataaactcacatttggaaagggacccatgtattcattatatctggtgagtcatccc aggtg |
| SEQ ID NO: 1328 | TRAJ20*01 | tgtaggcgacctcgcactgtggttctaacgactacaagctcagctttggagccggaaccacagtaactgtaagagcaagtaagtaagaaaga aaagtcca |
| SEQ ID NO: 1329 | TRAJ21*01 | tgtaatgccaataaacatggtgtacaacttcaacaaattttactttggatctgggaccaaactcaatgtaaaaccaagtaagttatagttgccta gaaga |
| SEQ ID NO: 1330 | TRAJ22*01 | gttgagcaaatcatagtgtttcttctggttctgcaaggcaactgacctttggatctgggacacaattgactgttttacctggtaggctgcctcaatt aaa |
| SEQ ID NO: 1331 | TRAJ23*01 | aggatatgtaacacagtgtgatttataaccagggaggaaagcttatcttcggacagggaacggagttatctgtgaaacccagtaagtataaa attgtatc |
| SEQ ID NO: 1332 | TRAJ23*02 | gactggatgtgtttttgacaggatatgtaacacagtgtgatttataaccagggaggaaagcttatcttcggacagggaacggagctatctgtga aaccca |
| SEQ ID NO: 1333 | TRAJ24*01 | gaggtgtttgtcacagtgtgacaactgacagctgggggaaattcgagtttggagcagggacccaggttgtggtcaccccaggtaagcccattc ctggagc |
| SEQ ID NO: 1334 | TRAJ24*02 | gaggtgtttgtcacagtgtgacaactgacagctgggggaaattgcagtttggagcagggacccaggttgtggtcaccccaggtaagccccatt ccctgga |
| SEQ ID NO: 1335 | TRAJ25*01 | atgctgagataatcactatgcagaaggacaaggcttctcctttatctttggaaggggacaaggctgcttgtcaagccaagtaagtgacatata atttat |
| SEQ ID NO: 1336 | TRAJ26*01 | ctgagcccagaaacactgtggggataactatggtcagaattttgtctttggtcccggaaccagattgtccgtgctgccctgtaagtacagttaag tggag |
| SEQ ID NO: 1337 | TRAJ27*01 | caatagcactaaagactgtgtaacaccaatgcaggcaaatcaacctttggggatgggactacgctcactgtgaagccaagtaagttgtgttctt ctttgc |
| SEQ ID NO: 1338 | TRAJ28*01 | agaaaggaaactctgtgcatactctggggctgggagttaccaactcactttcgggaaggggaccaaactctcggtcataccaagtaagttcttc tttctg |
| SEQ ID NO: 1339 | TRAJ29*01 | ttatggaggaaatcactgtgggaattcaggaaacacacctcttgtctttggaaagggcacaagactttctgtgattgcaagtaagtgtttctagc catcc |
| SEQ ID NO: 1340 | TRAJ3*01 | aaagaccttacccacagtgggggtacagcagtgcttccaagataatctttggatcagggaccagactcagcatccggccaagtaagtagaat gaagcagg |
| SEQ ID NO: 1341 | TRAJ30*01 | gttatggtcccaatcacagtgtgaacagagatgacaagatcatctttggaaaagggacacgacttcatattctccccagtaagtgctgtttatgt gattt |
| SEQ ID NO: 1342 | TRAJ31*01 | agtaaaggcaggaagtgctgtggaataacaatgccagactcatgtttggagatggaactcagctggtggtgaagcccagtaagtggccatgtt ttattga |
| SEQ ID NO: 1343 | TRAJ32*01 | ggctctgaaggactgtgtgaattatggcggtgctacaaacaagctcatctttggaactggcactctgcttgctgtccagccaagtacgtaagta gtggca |
| SEQ ID NO: 1344 | TRAJ32*02 | gtgattcagccacctacctctgtgccgatggtggtgctacaaacaagctcatctttggaactggcactctgcttgctgtccagccaaatatccag aaccc |
| SEQ ID NO: 1345 | TRAJ33*01 | gttaaggtttttgtgtctgtgtggatagcaactatcagttaatctggggcgctgggaccaagctaattataaagccaggtaagtctcagagatgt gactg |
| SEQ ID NO: 1346 | TRAJ34*01 | aggtttttgtagatctcagtatcactgtgtcttataacaccgacaagctcatctttgggactgggaccagattacaagtctttccaagt |
| SEQ ID NO: 1347 | TRAJ35*01 | taaaagaatgagccattgtggataggctttgggaatgtgctgcattgcgggtccggcactcaagtgattgttttaccacgtaagtatatcttttct catt |
| SEQ ID NO: 1348 | TRAJ36*01 | tactgggcagaaacactgtgtcaaactggggcaaacaacctcttctttgggactggaacgagactcaccgttattccctgtaagtccttacctct tgaca |
| SEQ ID NO: 1349 | TRAJ37*01 | aaagtacagcattagagtgtggctctggcaacacaggcaaactaatctttgggcaagggacaactttacaagtaaaaccaggtaggtctgga tgtttcca |
| SEQ ID NO: 1350 | TRAJ37*02 | ctcagcggtgtacttctgtgctcttcatggctctagcaacacaggcaaactaatctttgggcaagggacaactttacaagtaaaaccagatatc |
| | | cagaac |
| SEQ ID NO: 1351 | TRAJ38*01 | aaagctttctatgactgtgtaatgctggcaacaaccgtaagctgatttggggattgggaacaagcctggcagtaaatccgagtgagtcttcgtg ttaact |
| SEQ ID NO: 1352 | TRAJ39*01 | cagccgaagatcactgtgtgaataataatgcaggcaacatgctcacctttggaggggaacaaggttaatggtcaaaccccgtgagtatctct gctgaat |
| SEQ ID NO: 1353 | TRAJ4*01 | aagcaccatctgattgtgtgttttctggtggctacaataagctgatttttggagcagggaccaggctggctgtacacccatgtgagtatgaccct gcaag |
| SEQ ID NO: 1354 | TRAJ40*01 | tatgttggtttatgtagagacacataacactgtgactacctcaggaacctacaaatacatctttggaacaggcaccaggctgaaggttttagca agt |
| SEQ ID NO: 1355 | TRAJ41*01 | ttagggagaacgcactgtggaactcaaattccgggtatgcactcaacttcggcaaaggcacctcgctgttggtcacaccccgtgagtttttgtg gtttac |
| SEQ ID NO: 1356 | TRAJ42*01 | agccccataggactgtgtgaattatggaggaagccaaggaaatctcatctttggaaaaggcactaaactctctgttaaaccaagtaagtgttg gggattc |
| SEQ ID NO: 1357 | TRAJ43*01 | ttgttagagcatgtattactgtgacaataacaatgacatgcgctttggagcagggaccagactgacagtaaaaccaagtaagttgggggaatg ggtcaat |
| SEQ ID NO: 1358 | TRAJ44 *01 | aggtttctgttatgaagcatctcacagtgtaaataccggcactgccagtaaactcacctttgggactggaacaagacttcaggtcacgctcggt |
| SEQ ID NO: 1359 | TRAJ45*01 | agggttggcccagagtgtgtattcaggaggaggtgctgacggactcacctttggcaaagggactcatctaatcatccagccctgtaagtgcttt tgcctg |
| SEQ ID NO: 1360 | TRA146"01 | aagctgctgacagccgtgagaagaaaagcagcggagacaagctgacttttgggaccgggactcgtttagcagttaggcccagtaagtctgag cagaaagt |
| SEQ ID NO: 1361 | TRAJ47*01 | gtagaggagtttgacgctgtgtggaatatggaaacaaactggtctttggcgcaggaaccattctgagagtcaagtcctgtgagtataaaacac actcaag |
| SEQ ID NO: 1362 | TRAJ47*02 | gtgtactattgcatctcggccctggaatatggaaacaagctggtctttggcgcaggaaccattctgagagtcaagtcctatatccagaaccctg accctg |
| SEQ ID NO: 1363 | TRAJ48*01 | atgacttagaacactgtgtatctaactttggaaatgagaaattaacctttgggactggaacaagactcaccatcatacccagtaagttcttcatc cttgg |
| SEQ ID NO: 1364 | TRAJ49*01 | tgttgagcttcctatcacagtggaacaccggtaaccagttctattttgggacagggacaagtttgacggtcattccaagtaagtcaaagaaaat tttcca |
| SEQ ID NO: 1365 | TRAJ5*01 | tactgtgatgtaccagggtgtggacacgggcaggagagcacttacttttgggagtggaacaagactccaagtgcaaccaagtaagtacccaa acttaggc |
| SEQ ID NO: 1366 | TRAJ50*01 | taaaggtttggatggctgtgtgaaaacctcctacgacaaggtgatatttgggccagggacaagcttatcagtcattccaagtaagtgtccctgg ggtgct |
| SEQ ID NO: 1367 | TRAJ51*01 | aaactccctgaagcagggagatgcgtgacagctatgagaagctgatatttggaaaggagacatgactaactgtgaagccaagcaagctgga aagacctaa |
| SEQ ID NO: 1368 | TRAJ52*01 | gcctccagtgcagtgctaatgctggtggtactagctatggaaagctgacatttggacaagggaccatcttgactgtccatccaagtaagtgtaa caagac |
| SEQ ID NO: 1369 | TRAJ53*01 | agccttctgtggctgtgagaatagtggaggtagcaactataaactgacatttggaaaaggaactctcttaaccgtgaatccaagtaagtttgaa gggagt |
| SEQ ID NO: 1370 | TRAJ54*01 | taaagcctcgtgctgtggtgtaattcagggagcccagaagctggtatttggccaaggaaccaggctgactatcaacccaagtaagtatgacag ggtgaag |
| SEQ ID NO: 1371 | TRAJ55*01 | gaggatggatccctgttagtgacaagtgctggtaatgctcctgttggggaaaggggatgagtacaaaaataaatccaagtaagtgtggaggg acaagaag |
| SEQ ID NO: 1372 | TRAJ56*01 | agatcctcgtgtcattgtgttatactggagccaatagtaagctgacatttggaaaaggaataactctgagtgttagaccaggtatgttttaatga atgtt |
| SEQ ID NO: 1373 | TRAJ57*01 | aagcagtctgtgggggtgtaactcagggcggatctgaaaagctggtctttggaaagggaacgaaactgacagtaaacccatgtaagtctgaa taatgctt |
| SEQ ID NO: 1374 | TRAJ58*01 | aagcccctcagcacagtgtttaagaaaccagtggctctaggttgacctttggggaaggaacacagctcacagtgaatcctggtaagtggagg ggagcatt |
| SEQ ID NO: 1375 | TRAJ59*01 | atgtaaaggcagcagctcctgtgggaaggaaggaaacaggaaatttacatttggaatggggacgcaagtgagagtgaagctatctttaaacc aaaggtgt |
| SEQ ID NO: 1376 | TRAJ6*01 | caggttttatcaaaggctgtcctcactgtgtgcatcaggaggaagctacatacctacatttggaagaggaaccagccttattgttcatccgtgta agt |
| SEQ ID NO: 1377 | TRAJ60*01 | gtaaagggcctgggcactatgtgaagatcacctagatgctcaactttgggaaggggactgagttaattgtgagcctgggtgagtacctcaact ccagagg |
| SEQ ID NO: 1378 | TRAJ61*01 | taaaggtgcccactcctgtgggtaccgggttaataggaaactgacatttggagccaacactagaggaatcatgaaactcagcaagtaatattt ggcagaa |
| SEQ ID NO: 1379 | TRAJ7*01 | tgtaatacacttacacagtgtgactatgggaacaacagactcgcttttgggaaggggaaccaagtggtggtcataccaagtaagtgagctggg atcctcc |
| SEQ ID NO: 1380 | TRAJ8*01 | tacagagttatgtcagagtgtgaacacaggctttcagaaacttgtatttggaactggcacccgacttctggtcagtccaagtaagtcaaatctg cagaaa |
| SEQ ID NO: 1381 | TRAJ9*01 | cgcagtgcaaatcactgtgggaaatactggaggcttcaaaactatctttggagcaggaacaagactatttgttaaagcaagtaagttccatga aataacc |
| SEQ ID NO: 1382 | TRBJ1-1*01 | ttttcaccttgacccctgtcactgtgtgaacactgaagctttctttggacaaggcaccagactcacagttgtaggtaagacatttttcaggttcttt tgc |
| SEQ ID NO: 1383 | TRBJ1-2*01 | ttttagagtggctatattcttatgtgctaactatggctacaccttcggttcggggaccaggttaaccgttgtaggtaaggctgggggtctctagga gggg |
| SEQ ID NO: 1384 | TRBJ1-3*01 | tttgaagtggccctgggaggctgtgctctggaaacaccatatattttggagagggaagttggctcactgttgtaggtgagtaagtcaaggctgg acagct |
| SEQ ID NO: 1385 | TRBJ1-4*01 | ttccttccagtctttaatgttgtgcaactaatgaaaaactgtttttggcagtggaacccagctctctgtcttgggtatgtaaaagacttctttcgg gat |
| SEQ ID NO: 1386 | TRBJ1-5*01 | tttgccacactcatgatgcactgtgtagcaatcagccccagcattttggtgatgggactcgactctccatcctaggtaagttggcagaatcaggg tggta |
| SEQ ID NO: 1387 | TRBJ1-6*01 | ttatctaagcctctgcagctgtgctcctataattcacccctccactttgggaatggaccaggctcactgtgacaggtatgggggctccactcttg actc |
| SEQ ID NO: 1388 | TRBJ1-6*02 | ttatctaagcctctgcagctgtgctcctataattcacccctccactttgggaacgggaccaggctcactgtgacaggtatgggggctccactcttg actc |
| SEQ ID NO: 1389 | TRBJ2-1*01 | ttctgggcagccccttcccactgtgctcctacaatgagcagttcttcggccagggacacggctcaccgtgctaggtaagaagggggctccagg tgggag |
| SEQ ID NO: 1390 | TRBJ2-2*01 | tgcgccagggtccccagggctgtgcgaacaccggggagctgtttttggagaaggctctaggctgaccgtactgggtaaggaggcggctggg gctccgga |
| SEQ ID NO: 1391 | TRBJ2-2P*01 | agctgccccactctgagaggggctgtgctgagaggcgctgctgggcgtctggcggaggactcctggttctgggtgctgggagagcgatgggg ctctcag |
| SEQ ID NO: 1392 | TRBJ2-3*01 | ttttgtcctgggcctccaggctgtgagcacagatacgcagtattttggcccaggcacccggctgacagtgctcggtaagcggggctcccgctg aagccc |
| SEQ ID NO: 1393 | TRBJ2-4*01 | ttctgtgccgcgtctcggggctgtgagccaaaaacattcagtacttcggcgccgggacccggctctcagtgctgggtaagctggggccgccggg ggaccg |
| SEQ ID NO: 1394 | TRBJ2-5*01 | tttttgtgcggggctcgggggccgtgaccaagagacccagtacttcgggccaggcacgcggctcctggtgctcggtgagcgcgggctgctggg gcgcggg |
| SEQ ID NO: 1395 | TRBJ2-6*01 | ttgcggggagtccccgggctgtgctctggggccaacgtcctgactttcggggccggcagcaggctgaccgtgctgggtgagttttcgcgggacc acccgg |
| SEQ ID NO: 1396 | TRBJ2-7*01 | tttgcatgcgggggtgcacctccgtgctcctacgagcagtacttcgggccgggcaccaggctcacggtcacaggtgagattcgggcgtctcccc accttc |
| SEQ ID NO: 1397 | TRBJ2-7*02 | tttgcatgcggggatgcacctccgtgctcctacgagcagtacgtcgggccgggcaccaggctcacggtcacaggtgagattcgggcgtctcccc accttc |
| SEQ ID NO: 1398 | TRDJ1*01 | ttttggaacgtcctcaagtgctgtgacaccgataaactcatctttggaaaaggaacccgtgtgactgtggaaccaagtaagtaactcattattta tctga |
| SEQ ID NO: 1399 | TRDJ2*01 | tttttcgtaatgacgcctgtggtagtgctttgacagcacaactcttctttggaaagggaacacaactcatcgtggaaccaggtaagttatgcattt tact |
| SEQ ID NO: 1400 | TRDJ3*01 | tgaggcactgtcataatgtgctcctgggacacccgacagatgtttttcggaactggcatcaaactcttcgtggagccccgtgagttgatctttttc ctat |
| SEQ ID NO: 1401 | TRDJ4*01 | atgagacatacaaaaaggtaatgccgccccagacccctgatctttggcaaaggaacctatctggaggtacaacaac |
| SEQ ID NO: 1402 | TRGJ1*01 | ttttgatatggactgaatcactgtggaattattataagaaactctttggcagtggaacaacactggttgtcacaggtaagtatcggaagaatac aacatt |
| . SEQ ID NO: 1403 | TRGJ1*02 | tactgtgccttgtgggaggtgcttattataagaaactctttggcagtggaacaacacttgttgtcacaggt |
| SEQ ID NO: 1404 | TRGJ2*01 | ttttgatatggactgaatcactgtggaattattataagaaactctttggcagtggaacaacacttgttgtcacaggtaagtatcggaagaataca acatt |
| SEQ ID NO: 1405 | TRGJP*01 | ataaaggcttctcaggtggtgggcaagagttgggcaaaaaaatcaaggtatttggtcccggaacaaagcttatcattacaggtaagttttcttt aaattt |
| SEQ ID NO: 1406 | TRGJP1*01 | gatttttctagaagcttagaccggtgtgataccactggttggttcaagatatttgctgaagggactaagctcatagtaacttcacctggtaagt |
| SEQ ID NO: 1407 | TRGJP2*01 | gatttttgtagaagcttagaccagtgtgatagtagtgattggatcaagacgtttgcaaaagggactaggctcatagtaacttcgcctggtaagt , |

**Table 2.3: Dilution Series Design**

| Dilution | Total desired number of cells in 50 µL | Desired cell fraction of Jurkat-configuration lymphocytes | Number of desired Jurkat cells | Required polyclonal cells | Equivalent Jurkat DNA required (µL) * | Equivalent polyclonal (A037) DNA required (µL) | Water (µL) | Theoretical final dilution DNA concentration (ng/ µL) |
|---|---|---|---|---|---|---|---|---|
| 1 | 2.00E+05 | 1 | 2.00E+05 | 0 | 13.21 | 0.00 | 36.79 | 2.80 |
| 2 | 2.00E+05 | 0.1 | 2.00E+04 | 1.80E+05 | 13.21 of 1 in 10 dilution of Dilution 1 | 2.79 | 34.00 | 2.80 |
| 3 | 2.00E+05 | 0.01 | 2.00E+03 | 1.98E+05 | 13.21 of 1 in 10 dilution of Dilution 2 | 3.07 | 33.72 | 2.80 |
| 4 | 2.00E+05 | 0.001 | 2.00E+02 | 2.00E+05 | 13.21 of 1 in 10 dilution of Dilution 3 | 3.09 | 33.70 | 2.80 |
| 5 | 2.00E+05 | 0.0001 | 2.00E+01 | 2.00E+05 | 13.21 of 1 in 10 dilution of Dilution 4 | 3.10 | 33.69 | 2.80 |
| 6 | 2.00E+05 | 0.00001 | 2.00E+00 | 2.00E+05 | 13.21 of 1 in 10 dilution of Dilution 5 | 3.10 | 33.69 | 2.80 |

| | | | | | | | | |
|---|---|---|---|---|---|---|---|---|
| ^{‡} Assumptions of note: Stock Jurkat Cell Line DNA Concentration: 10.6 ng/µL; Presumed lymphocyte DNA content: 0.0007ng/cell | | | | | | | | |

**Table 2.4: Clinical, Pathology & Outcome Data Parameters**

| Clinical Data Parameters | | Pathology Data Parameters | | | Treatment Data Parameters | Outcome Data Parameters |
|---|---|---|---|---|---|---|
| Age at Diagnosis | | Morphology (small cell, large cell, anaplastic) | | | First-line therapy | Birthdate |
| Gender | | Background (mixed or uniform inflammatory infiltrates) | | | Transplant (Yes/No) | Diagnosis Date |
| Primary Site of Involvement | | Bone Marrow Status at Diagnosis (% of involvement by tumor, if applicable) | | | Second-line or subsequent additional therapies | Date of Last Follow-up |
| Performance Status | | *Primary Specimen* Immunohistochemistry (positive/negative) | | | | Disposition (0=Alive; 1=Deceased) |
| B symptoms | | | CD2 | | | |
| International Prognostic Index | | | CD3 | | | |
| Stage | | | CD4 | | | |
| CBC at diagnosis | | | CD5 | | | |
| | Hb | | CD7 | | | |
| | MCV | | CD8 | | | |
| | Pit | | CD10 | | | |
| | Neut | | CD21 | | | |
| | Mono | | CD23 | | | |
| | Eo | | CD30 | | | |
| | Lymph | | CD56 | | | |
| | Other | | CD57 | | | |
| Chemistry | | | BCL6 | | | |
| | LDH | | Ki67 | | | |
| | Uric Acid | | EBER | | | |
| | Albumin | | ALK | | | |
| | Alk Phos | | PD1 | | | |
| | ALT | | CXCL-13 | | | |
| | AST | Primary Specimen Flow Cytometry (positive/negative) | | | | |
| | BUN | | CD45 | | | |
| | Calcium | | CD2 | | | |
| | Chloride | | CD3 | | | |
| | C02 | | CD5 | | | |
| | Creatinine | | CD4 | | | |
| | Glucose | | CD7 | | | |
| | Potassium | | CD8 | | | |
| | Sodium | | CD10 | | | |
| | Total Bilirubin | | CD19 | | | |
| | Total protein | | CD20 | | | |
| | | | CD30 | | | |
| | | | TCR alpha/beta | | | |
| | | | TCR gamma/delta | | | |
| | | Molecular | | | | |
| | | | Clonality (clonal/polyclonal) | | | |
| | | | Other | | | |
| | | Cytogenetics (normal/abnormal) | | | | |
| | | | Classical | | | |
| | | | FISH | | | |
| | | Serology (positive/negative) | | | | |
| | | | HIV | | | |
| | | | HTLV-1 | | | |

**Table 2.5: Sample descriptions and flow cytometry data of the 6 actual patient lymphocyte specimens used for analytical validation**

| Sample Name | Description | Flow-cytometry Features (if available) | Number of Cells Input for DNA Isolation | "Clonal/Oligoclonal" vs "Polyclonal" |
|---|---|---|---|---|
| A037 | Healthy Donor | N/A | 10,000,000 | Polyclonal |
| | Patient Peripheral | | | |
| | Blood | | | |
| | Mononuclear Cells | | | |
| OV7 | Mixed Ovarian | 90% CD3+ | 10,000,000 | Polyclonal |
| | Tumour-Infiltrating | 10% CD4+ | | |
| | Lymphocytes expanded with IL-2 treatment | 70% CD8+ | | |
| EZM | Cell suspension of melanoma tumour with brisk CD3 infiltration | N/A | 10,000,000 (possible admixed tumour cells) | Uncertain |
| TIL2 | Melanoma tumour-infiltrating lymphocytes expanded in IL-2 | 97% CD8+ | 10,000,000 | Oligoclonal |
| STIM1 | MART1-specific cell line made from peptide stimulation of healthy donor PBMCs, FACS sorting and expansion of tetramer+ cells | 99% CD8+ | 10,000,000 | Clonal/Oligoclonal |
| L2D8 | gp100-specific tumour-infiltrating lymphocyte clone | ~100% CD8+ | 10,000,000 | Clonal/Oligoclonal |

**Table 2.6: Cell lines used for analytical validation**

| Cell Line | Reference Collection # | Previously Documented/Known TRGR Configurations |
|---|---|---|
| CEM (CCRF-CEM) | ATCC CCL-119 | TRBV3-1*01 - TRBD1*01 - TRBJ2-3*01 |
| | | TRBJ1-5 - TRBJ2-1 (partial rearrangement) |
| | | TRBV9 - TRBD2 (partial rearrangement) |
| | | TRGV3 - TRGJ1/TRGJ2 |
| | | TRGV4 - TRGJ1/TRGJ2 |
| Jurkat | DSMZ ACC-282 | TRAV8-4 - TRAJ3 |
| | | TRBV12-3 - TRBJ1-2 (partial rearrangement) |
| MOLT4 | ATCC CRL-1582 | TRBV20-1*01 - TRBD2*01 - TRBJ2-1*01 |
| | | TRBV10-3 - TRBD1*01 - TRBJ2-5 |
| | | TRGV2 - TRGJP1 |
| | | TRGV2 - TRGJP2 |
| SUPT1 | ATCC CRL-1942 | TRBV9*01 - TRBD2*01 - TRBJ2-1*01 |
| | | TRGV3 - TRGJ1/TRGJ2 |
| | | TRGV4 - TRGJ1/TRGJ2 |

### Supplemental Tables

**Table 1.1: Capture Sample Method Data**

| Sample | Sample | Protocol Type | Library Input (ng) |
|---|---|---|---|
| **A037 healthy reference** | | | |
| Sample_A037_PBMC_TCR_A_all | A037_PBMC | CapSeq_One-Step V | 100 |
| Sample_A037_PBMC_TCR_B_all | A037_PBMC | CapSeq_One-Step V | 200 |
| Sample_A037PBMC_TCR_D_all | A037_PBMC | CapSeq_One-Step_V | 600 |
| Szmple_A037_PBMC_TCR_E_all | A037_PBMC | CapSeq_One-Step_V | 800 |
| Sample_A037_PBMC_TCR_F_all | A037_PBMC | CapSeq_One-Step_V | 1000 |
| Sample_A037_PBMC_TCR_G_all | A037_PBMC | CapSeq_One-Step_V | 200 |
| Sample_A037_PBMC_TCR_H_all | A037_PBMC | CapSeq_One-Step_V | 600 |
| SampleA037_TCR_J__PBMC_all | A037_PBMC | CapSeq_One-Step_V | 200 |
| Sample_A037_PBMC_TCR_K_all | A037_PBMC | CapSeq_One Step V | 600 |
| Sample_A037_PBMC_TCR_L_all | A037_PBMC | CapSeq_One-Step_V | 1000 |
| Sample_16_01_A037_PBMC_TCR_F_all | A037_PBMC | CapSeq_One-Step_V | 500 |
| Sample_16_01_A037_PBMC_TCR_Hall | A037_PBMC | CapSeq_One-Step V | 250 |
| Sample_A037_S1_all | A037_PBMC | CapSeq_One-Step_VJ | 100 |
| Sample_A037_PBMC_1S_all | A037_PBMC | CapSeq_One-Step VJ | 100 |
| Sample_16_11_A037 PBMC_TCR_VJ_all | A037_PBMC | CapSeq_One-Step_VJ | 100 |
| Sample_A037_CD3_1S_all | A037_CD3 | CapSeq_One-Step_VJ | 100 |

| Cell lines and flow sorted | | | |
|---|---|---|---|
| M36_EZM | flow_sorted | CapSeq_One-Step_VJ | 100 |
| M36_TIL2 | flow_sorted | CapSeq_One-Step_VJ | 100 |
| OV7-TIL2 | flow_sorted | Capseq_One-Step_VJ | 100 |
| SE14-2005 | cell_line | CapSeq_One-Step_VJ | 100 |
| SE14-2033 | cell-line | CapSeq_One-Step V1 | 100 |
| SE14-2034 | cell_line | CapSeq_One-Step_VJ | 100 |
| SE14-2035 | cell_line | CapSeq_One-Step_VJ | 100 |
| STIM1 | flow_sorted | CapSeq_One-Step_VJ | 100 |
| L2D8 | flow_sorted | CapSeq_One-Step_VJ | 100 |

| Patient samples | | | |
|---|---|---|---|
| M14-10124 | patient_tumor | CapSeq_One-Step_VJ | 100 |
| M14-11153 | patient_tumor | CapSeq_One-Step_VJ | 100 |
| M14-11567 | patient_tumor | CapSeq_One-Step_VJ | 100 |
| M14-11587 | patient_tumor | CapSeq_One-Step_VJ | 100 |
| M14-11721 | patient_tumor | CapSeq_One-Step_VJ | 100 |
| M14-11770 | patient_tumor | CapSeq_One-Step_VJ | 100 |
| M14-12217 | patient_tumor | CapSeq_One-Step_VJ | 100 |
| M14-12649 | patient_tumor | CapSeq_One-Step_VJ | 100 |
| M14-12728 | patient tumor | CapSeq_One-Step_VJ | 100 |
| M14-12753 | patient_tumor | CapSeq_One-Step_VJ | 100 |
| M14-13167 | patient_tumor | CapSeq_One-Step_VJ | 100 |
| M14-13300 | patient_tumor | CapSeq_One-Step_VJ | 100 |
| M14-13750 | patient_tumor | CapSeq_One-Step_VJ | 100 |
| M14-14570 | patient_tumor | CapSeq_One-Step_VJ | 100 |
| M14-14625 | patient_tumor | CapSeq_One-Step_VJ | 100 |
| M14-14907 | patient_tumor | CapSeq_One-Step_VJ | 100 |
| M14-14951 | patient_tumor | CapSeq_One-Step_VJ | 100 |
| M14-14962 | patient_tumor | CapSeq_One-Step_VJ | 100 |
| M14-1508 | patient_tumor | CapSeq_One-Step_VJ | 100 |
| M14 1S119 | patient_tumor | CapSeq_One-Step V1 | 100 |
| M14-3271 | patient_tumor | CapSeq_One-Step_VJ | 100 |
| M14 4454 | patient_tumor | CapSeq_Ore-Step V1 | 100 |
| M14-5819 | patient_tumor | CapSeq_One-Step V1 | 100 |
| M14-5875 | patient_tumor | CapSeq_One-Step Vl | 100 |
| M14-6143 | patient_tumor | CapSeq_One-Step_VJ | 100 |
| M14-6430 | patient_tumor | CapSeq_One-Step_VJ | 100 |
| M14 6443 | patient_tumor | CapSeq_One-Step VJ | 100 |
| M14-6502 | patient_tumor | CapSeq_One-Step_VJ | 100 |
| M14-6885 | patient tumor | CapSeq_One-Step_VJ | 100 |
| M14-7046 | patient_tumor | CapSeq_One-Slep_VJ | 100 |
| M14-7049 | patient_tumor | CapSeq_One-Step_VJ | 100 |
| M14-7053 | patient_tumor | CapSeq_One-Step_VJ | 100 |
| M14-7107 | patient_tumor | CapSeq_One-Step VJ | 100 |
| M 14-7554 | patient_tumor | CapSeq_One-Step_Vl | 100 |
| M14-7568 | patient_tumor | CapSeq_One-Step_VJ | 100 |
| M14-7691 | patient_tumor | CapSeq_One-Step_VJ | 100 |
| M14-7700 | patient_tumor | CapSeq_One-Step_VJ | 100 |
| M14-7782 | patient_tumor | CapSeq_One-Step_VJ | 100 |
| M14-7862 | patient_tumor | CapSeq_One-Step_VJ | 100 |
| M14-7884 | patient_tumor | CapSeq_One-Step Vl | 100 |
| M14-7992 | patient_tumor | CapSeq_One_Step_VJ | 100 |
| M14-8132 | patient_tumor | CapSeq_One-Step_VJ | 100 |
| M14-8272 | patient_tumor | CapSeq_One-Step VJ | 100 |
| M14-8639 | patient_tumor | CapSeq_One-Step V1 | 100 |
| M14-8668 | patient_tumor | CapSeq_One-Step Vl | 100 |
| M14-8740 | patient_tumor | CapSeq_One-Step VJ | 100 |
| M14-8913 | patent_tumor | CapSeq_One-Step_VJ | 100 |
| M14-8914 | patient_tumor | CapScq_One-Step VJ | 100 |
| M14-9212 | patient_tumor | CapSeq_One-Step_VJ | 100 |
| M14-9801 | patient_tumor | CapSeq_One-Step_VJ | |
| M15-1195 | patient_tumor | CapSeq_One-Step_VJ | 100 |
| M15-1330 | patient tumor | CapSeq_One Step_VJ | 100 |
| M15-1470 | patient_tumor | CapSeq_One-Step VJ | 100 |
| M15-1556 | patient_tumor | CapSeq_One-Step_VJ | 100 |
| M15-1825 | patient_tumor | CapSeq_One-Step_VJ | 100 |
| M15-1867 | patient_tumor | CapSeq_One-Step VJ | 100 |
| M15-1883 | patient_tumor | CapSeq_One-5tep_VJ | 100 |
| M15-237 | patient_tumor | CapSeq_One-Step_VJ | 100 |
| M15-2603 | patient_tumor | CapSeq_One-Step_VJ | 100 |
| M15-2779 | patient_tumor | CapSeq_One-Step_VJ | 100 |
| M15-3091 | patient_tumor | CapSeq_One-Step_VJ | 100 |
| M15 587 | patient_tumor | CapSeq One-Step_VJ | 100 |
| M15-795 | patient_tumor | CapSeq_One-Step VJ | 100 |
| M15 933 | patient_tumor | CapSeq_One-Step_VJ | 100 |

**Table 1.2: Capture Sample Read Counts**

| Sample | total reads | on-target reads | off-target reads | on-target ratio | merged reads | reads after threshold |
|---|---|---|---|---|---|---|
| **A037 healthy reference** | | | | | | |
| Sample_A037_PBMC_TCR_A_all | 1961529 | 96884 | 1864620 | 0.049392081 | 1961504 | 1900159 |
| Sample_A037_PBMC_TCR_B_all | 9915634 | 865444 | 9050165 | 0.087280753 | 9915609 | 9488814 |
| Sample_A037_PBMC_TCR_D_all | 11554469 | 359807 | 11194637 | 0.031140072 | 11554444 | 10839947 |
| Sample_A037_PBMC_TCR_E_all | 8208382 | 4019972 | 4188385 | 0.489739878 | 8208357 | 8069762 |
| Sample_A037_PBMC_ICR_F_all | 13434420 | 3925996 | 9508399 | 0.292234127 | 13434395 | 13076224 |
| Sample_A037_PBMC_TCR_G_all | 11585206 | 217323 | 11367858 | 0.018758665 | 11585181 | 11162632 |
| Sample_A037_PBMC_TCR_H_all | 8680363 | 1631345 | 7048993 | 0.187935113 | 8680338 | 8302862 |
| Sample_A037_PBMC_TCR_J_all | 17147171 | 504177 | 16642969 | 0.029402926 | 17147146 | 14908072 |
| Sample_A037_PBMC_TCR_K_all | 8812446 | 518449 | 8293972 | 0.058831453 | 8812421 | 7851064 |
| Sample_A037_PBMC_TCR_L_all | 21053845 | 429885 | 20623935 | 0.020418361 | 21053820 | 17568322 |
| Sample_16_01_A037_PBMC_TCR_F_all | 4457394 | 958772 | 3499597 | 0.715096983 | 44573G9 | 4389100 |
| Sample_76_01_A037_PBMC_TCR_H_all | 6835519 | 1719308 | 5116246 | 0.25152339 | 6835550. | 6750376 |
| Sample_A037_S1_all | 1920124 | 1082540 | 837559 | 0.563786505 | 1920099 | 1867339 |
| Sample_A037_PBMC_1S_all | 4868959 | 2120537 | 2748397 | 0.435521638 | 4768430 | 4706036 |
| Sample_16_11_A037_PBMC_TCR_VJ_all | 1433221 | 413057 | 1020139 | 0.288201889 | 1433196 | 1427599 |
| Sample_A037_CD3_1S_all | 4701054 | 2361517 | 2339512 | 0.502337774 | 4701029 | 4651006 |

| Cell lines and flow sorted | | | | | | |
|---|---|---|---|---|---|---|
| M36_EZM | 2318060 | 1380043 | 937992 | 0.595343951 | 2318035 | 2255858 |
| M36_TIL2 | 1569122 | 769525 | 799572 | 0.490417571 | 1569097 | 1518502 |
| OV7-TIL2 | 2392656 | 1271622 | 1121009 | 0.531468795 | 2392631 | 2320790 |
| SE14-2005 | 1291244 | 476090 | 815129 | 0.368706457 | 1291219 | 1216685 |
| SE14-2033 | 1339529 | 662257 | 677247 | 0.494395418 | 1339504 | 1293618 |
| SE14-2034 | 1278441 | 564484 | 713932 | 0.441540908 | 1278416 | 1240462 |
| SE14-2035 | 1678562 | 743158 | 935379 | 0.442734912 | 1678537 | 1611636 |
| STIM1 | 1880814 | 900492 | 980297 | 0.478777806 | 1880789 | 1827853 |
| L2D8 | 1651306 | 910355 | 740976 | 0.551293946 | 1651281 | 1603088 |

| Patient samples | | | | | | |
|---|---|---|---|---|---|---|
| M14-10124 | 3874239 | 1363917 | 2510297 | 0.352047718 | 3874214 | 3641564 |
| M14-11153 | 4921789 | 1618479 | 3303285 | 0.328839574 | 4921764 | 4871138 |
| M14-11567 | 4961317 | 1742809 | 3218483 | 0.351279509 | 4961292 | 4808248 |
| M14-11587 | 4284116 | 1363269 | 2920822 | 0.318214773 | 4284091 | 4230674 |
| M14-11721 | 5480831 | 1885151 | 3595655 | 0.343953499 | 5480806 | 5423859 |
| M14-11770 | 5405827 | 415885 | 4989917 | 0.076932725 | 5405802 | 5177500 |
| M14-12217 | 5135793 | 1690789 | 3444979 | 0.329216734 | 5135768 | 5098364 |
| M14-12649 | 7798007 | 27595G4 | 5038418 | 0.353880677 | 7797982 | 7715502 |
| M14-12728 | 5006452 | 739003 | 4267424 | 0.147610124 | 5006427 | 4799839 |
| M14-12753 | 5044768 | 1512141 | 3532602 | 0.299744408 | 5034743 | 4998359 |
| M14-13167 | 2912824 | 980216 | 1932583 | 0.336517414 | 2912799 | 2891403 |
| M14-13300 | 6403753 | 976423 | 5427305 | 0.15247668 | 6403728 | 6226299 |
| M14-13750 | 6648103 | 894301 | 5753776 | 0.134519877 | 6648078 | 6520478 |
| M14-14570 | 4577658 | 964191 | 3613442 | 0.210629759 | 4577633 | 4516409 |
| M14-14625 | 4919394 | 671943 | 4247426 | 0.136590604 | 4919369 | 4678232 |
| M14-14907 | 6045676 | 1996999 | 4048657 | 0.330318562 | 6045651 | 5967138 |
| M14-14951 | 4339950 | 334232 | 4005693 | 0.077012869 | 4339925 | 4253000 |
| M14-14962 | 2621464 | 397567 | 2223872 | 0.151658386 | 5799400 | 5552790 |
| M14-1508 | 6616839 | 3224927 | 3391887 | 0.487381815 | 6616814 | 6538041 |
| M14-15119 | 4825285 | 658203 | 4167057 | 0.136407072 | 4815260 | 4721235 |
| M14-3271 | 7352598 | 3438740 | 3913833 | 0.467690468 | 7352573 | 7230944 |
| M14-4454 | 7015117 | 3588858 | 3426234 | 0.511599187 | 7015092 | 6912948 |
| M14-5819 | 6427168 | 2297299 | 4129844 | 0.357435654 | 6427143 | 6377748 |
| M14-5875 | 6466998 | 2244807 | 4222166 | 0.347117318 | 6466973 | 6357148 |
| M14-6143 | 5149354 | 740986 | 4408343 | 0.143898827 | 5149329 | 4979117 |
| M14-6430 | 7717729 | 4019388 | 3698316 | 0.520799318 | 7717704 | 7610950 |
| M14-6443 | 5310114 | 1719071 | 3591018 | 0.323735234 | 5310089 | 5258149 |
| M14-6502 | 6854324 | 449983 | 6404316 | 0.065649508 | 6854299 | 6571528 |
| M14-6885 | 4473140 | 636717 | 3836393 | 0.142342292 | 4473115 | 4255663 |
| M14-7046 | 2901414 | 389561 | 2511828 | 0.134265913 | 2901389 | 2690711 |
| M14-7049 | 4194422 | 328356 | 386604) | 0.078283969 | 4194397 | 4104557 |
| M14-7053 | 4534911 | 634273 | 3900613 | 0.139864487 | 4534886 | 4132215 |
| M14-7107 | 3G53179 | 489927 | 3163227 | 0.134109771 | 3653154 | 3443643 |
| M14-7554 | 6905643 | 3346628 | 3558990 | 0.484622214 | 6905618 | 6814973 |
| M14-7568 | 5989679 | 2953254 | 3036400 | 0.49305714 | 5989654 | 5933921 |
| M14-7691 | 4715544 | 2109689 | 2605830 | 0.447390375 | 4715519 | 4633852 |
| M14-7700 | 6664469 | 2293770 | 4370674 | 0.344178959 | 6664444 | 6605136 |
| M14-7782 | 6155725 | 3173681 | 2982019 | 0.515565754 | 6155700 | 6034814 |
| M14-7862 | 5025139 | 361053 | 4664061 | 0.071849356 | 5025114 | 4886216 |
| M14-7884 | 5190944 | 361315 | 4829604 | 0.069604873 | 5190919 | 5085124 |
| M14 7992 | 5745439 | 2811128 | 2931286 | 0.489802085 | 5745414 | 5649598 |
| M14-8132 | S32δ896 | 1787753 | 3541118 | 0.335482809 | 5328871 | 5288026 |
| M14-8272 | 6030251 | 3161144 | 2869082 | 0.524214332 | 6030226 | 5874655 |
| M14-8639 | 7376555 | 3887519 | 3489011 | 0.527010102 | 1376530 | 7249500 |
| M14-8668 | 5401734 | 2916998 | 2484711 | 0.590011411 | 5401709 | 5338260 |
| M14-8740 | 5346366 | 233692 | 5112649 | 0.043710438 | 5346341 | 5202430 |
| M14-8913 | 6495674 | 3372030 | 3123619 | 0.51911934 | 6495649 | 6455304 |
| M14-8914 | 6562054 | 3324004 | 3238025 | 0.506549321 | 6562029 | 6458959 |
| M14-9212 | 4503869 | 1426322 | 3077522 | 0.316688163 | 4503844 | 4452847 |
| M14-9801 | 5502711 | 387341 | 5115345 | 0.07039094 | 5502686 | 5398233 |
| M15-1195 | 6305701 | 392089 | 5913587 | 0.062180081 | 6305676 | 6065963 |
| M15-1330 | 8302037 | 2704496 | 5597516 | 0.325762942 | 8302012 | 8107829 |
| M15-1470 | 38349G7 | 292000 | 3542942 | 0.076141464 | 3834942 | 3767575 |
| M15-1556 | 6935912 | 3615566 | 3320321 | 0.521281989 | 6935887 | 6892616 |
| M15-1825 | 6078396 | 1963007 | 4115364 | 0.311948192 | 6078371 | 6014071 |
| M15-1867 | 6865892 | 3557974 | 3307893 | 0518210016 | 6865867 | 6816073 |
| M15-1883 | 6227227 | 3087220 | 3139982 | 0.495761597 | 6227202 | 6169114 |
| M15 237 | 6215041 | 2213245 | 4001771 | 0.356111086 | 6215016 | 6155386 |
| M15-2603 | 5G39514 | 2766020 | 2873469 | 0.490471342 | 5639489 | 5564062 |
| M15-2779 | 5680891 | 2792325 | 2888541 | 0.49152941 | 5680866 | 5628837 |
| M15-3091 | 6906018 | 3575635 | 3330358 | 0.517756397 | 6905993 | 6843330 |
| M15-587 | 3920359 | 589850 | 3330484 | 0.15045816 | 3920334 | 3808959 |
| M15-795 | 42752G4 | 769512 | 3505727 | 0.179991692 | Λ175739 | 42C5077 |
| M15-933 | 6551470 | 3277319 | 3274126 | 0.500241778 | 6551445 | 6481344 |

**Table 1.3: Capture Sample V and J Calls**

| Sample | alpha VJ calls | beta VJ calls | gamma VJ calls | delta VJ calls | unmatched VJ calls | single V or J | absent V and J |
|---|---|---|---|---|---|---|---|
| **A037 healthy reference** | | | | | | | |
| Sample_A037_PBMC_TCR_A_all | 30 | 111 | 46 | 0 | 0 | 171866 | *1728107* |
| Sample_A037_PBMC_TCR_B_all | 473 | 806 | 538 | 0 | 0 | 1634949 | 7852049 |
| Sample_A037_PBMC_TCR_D_all | 298 | 244 | 127 | 1 | 0 | 583395 | 1025$883 |
| Sample_A037_PBMC_TCR_E_all | 4470 | 1956 | 2916 | 82 | 5 | 5486404 | 257393U |
| Sample_A037_PBMC_TCR_F_all | 3932 | 1815 | 3169 | 84 | 6 | 5949549 | 7117670 |
| Sample_A037_PBMC_TCR_G_all | 101 | 186 | 78 | 15 | 0 | 420033 | 10742220 |
| Sample_A037_PBMC_TCR_H_all | 1607 | 1125 | 252 | 12 | 4 | 2160797 | 6139066 |
| Sample_A037_PBMC_TCR_J_all | 323 | 139 | 135 | 4 | 2 | 1112523 | 13794941 |
| Sample_A037_PBMC_TCR_K_all | 352 | 169 | 200 | 6 | 0 | 1027278 | 6823060 |
| Sample_A037_PBMC_TCR_L_all | 259 | 111 | 136 | 8 | 3 | 1057487 | 16510319 |
| Sample_16_01_A037_PBMC_TCR_F_all | 925 | 363 | 628 | 25 | 1 | 3437777 | 949382 |
| Sample_16_01_A037_PBMC_TCR_H_all | 1397 | 763 | 1015 | 21 | 2 | 4575171 | 2172015 |
| Sample_A037_S1_all | 1052 | 606 | 734 | 12 | 2 | 1255308 | 609626 |
| Sample_A037_PBMC_1S_all | 1008 | 599 | Σf34 | 26 | 1 | 2536312 | 2167257 |
| Sample_1611_A037_PBMC_TCR_VJ_all | 340 | 161 | 329 | 11 | 0 | 934369 | 492390 |
| Sample_A037_CD3_1S_all | 6368 | 3264 | 4805 | 123 | 7 | 2753833 | 1882607 |

| **Cell lines and flow sorted** | | | | | | | |
|---|---|---|---|---|---|---|---|
| M36_EZM | 138 | 94 | 94 | 0 | 0 | 1521931 | 733602 |
| M36 TIL2 | 2136 | 1579 | 1963 | 4 | 7 | 1015955 | 495858 |
| OV7-TIL2 | 2619 | 1879 | 1918 | 52 | 1 | 1515855 | 793467 |
| SE14-2005 | 2450 | 1293 | 2070 | 0 | 0 | 818261 | 392612 |
| SE14-2033 | 1389 | 924 | 1344 | 0 | 0 | 895089 | 394873 |
| SE14-2034 | 1910 | 2833 | 1377 | 0 | 0 | 856362 | 377981 |
| SE14-2035 | 3031 | 2017 | 2157 | 0 | 0 | 1020846 | 583586 |
| STIM1 | 3068 | 1524 | 2503 | 0 | 0 | 1192227 | 628532 |
| 12D8 | 2074 | 962 | 948 | 0 | 0 | 1060361 | 538744 |

| **Patient samples** | | | | | | | |
|---|---|---|---|---|---|---|---|
| M14-10124 | 1971 | 1098 | 1674 | 48 | 0 | 2380500 | 1256274 |
| M14-11153 | S85 | 283 | 628 | 9 | 0 | 2811142 | 2058492 |
| M14-11567 | 1423 | 901 | 1278 | 8 | 6 | 2599812 | 2204821 |
| M14-11587 | 182 | 151 | 142 | 0 | 2 | 2473198 | 1756900 |
| M14-11721 | 210 | 65 | 192 | 0 | 3 | 3272558 | 2150832 |
| M14-11770 | 17 | 36 | 25 | 0 | 0 | 768985 | 4408438 |
| M14-12217 | 343 | 141 | 2481 | 648 | 0 | 2982597 | 2112155 |
| M14-12649 | 1167 | 857 | 1327 | 4 | 3 | 4868928 | 2843117 |
| M14-12728 | 986 | 607 | 9G7 | 14 | 0 | 1069367 | 3727899 |
| M14-12753 | 1600 | 960 | 2053 | 40 | 1 | 2485050 | 2508656 |
| M14-13167 | 215 | 87 | 248 | 22 | 0 | 1710714 | 1180118 |
| M14-13300 | 1620 | 688 | 2344 | 13 | 1 | 1571492 | 4650142 |
| M14-13750 | 1995 | 1039 | 2144 | 108 | 7 | 1527402 | 4987784 |
| M14-14570 | 155 | 163 | 290 | 45 | 0 | 1742539 | 1773118 |
| M14-14625 | 1083 | 562 | 967 | 7 | 1 | 1084783 | 3590830 |
| M14-14907 | 981 | 147 | 494 | 15 | 0 | 3030809 | 2934593 |
| M14-14951 | 106 | 84 | 174 | 4 | 3 | 613083 | 3639487 |
| M14-14962 | 623 | 332 | 545 | 7 | 0 | 1160605 | 4390679 |
| M14-1508 | 3489 | 2054 | 3136 | 19 | 1 | 4047375 | 2481367 |
| M14-15119 | 4118 | 1546 | 1551 | 0 | 3 | 986010 | 3727908 |
| M14-3271 | 9607 | 2563 | 3523 | 64 | 6 | 4297650 | 2922532 |
| M14-4454 | 1974 | 904 | 1199 | 11 | 6 | 4479570 | 2319285 |
| M14-5819 | 186 | 86 | 271 | 2 | 1 | 2435125 | 3942078 |
| M14-5875 | 484 | 371 | 533 | 12 | 0 | 3411599 | 2944150 |
| M14-6143 | 575 | 241 | 481 | 1 | 0 | 1235788 | 3742032 |
| M14-6430 | 863 | 471 | 705 | 39 | 0 | 4942133 | 2666740 |
| M14-6443 | 0 | 0 | 0 | 0 | 0 | 2721814 | 2536336 |
| M14-6502 | 119 | 77 | 140 | 0 | 0 | 913846 | 5657347 |
| M14-6885 | 1274 | 727 | 888 | 4 | 3 | 985106 | 3267662 |
| M14-7046 | 497 | 190 | 442 | 5 | 4 | G15177 | 2074397 |
| M14-7049 | 5 | 2 | 396 | 611 | 0 | 630487 | 3473057 |
| M14-7053 | 409 | 228 | 420 | 23 | 0 | 936724 | 3194412 |
| M14-7107 | 1122 | 577 | 915 | 2 | 1 | 797093 | 2643934 |
| M14-7554 | 901 | 469 | 861 | 24 | 1 | 1741112 | 5071606 |
| M14-7568 | 2181 | 861 | 1674 | 141 | 2 | 3472975 | 2456088 |
| M14-7691 | 5077 | 4087 | 4193 | 0 | 0 | 2889813 | 1730683 |
| M14-7700 | 536 | 342 | 860 | 6 | 0 | 4144765 | 2458G28 |
| M14-7782 | 682 | 417 | 723 | 21 | 0 | 3850370 | 2182602 |
| M14-7862 | 264 | 104 | 232 | 0 | 2 | 735636 | 4149979 |
| M14-7884 | 340 | 228 | 434 | 0 | 0 | 739303 | 4344815 |
| M14-7992 | 1987 | 1338 | 1755 | 12 | 0 | 3223885 | 2420622 |
| M14-3132 | 229 | 150 | 287 | 3 | 0 | 3138235 | 2149123 |
| M14-8272 | 273 | 223 | 299 | 0 | 0 | 3574689 | 2299172 |
| M14-8639 | 638 | 335 | 605 | 19 | 0 | 4377667 | 2920227 |
| M14-8668 | 140 | 107 | 117 | 0 | 2 | 1224632 | 2113263 |
| M14-8740 | 741 | 374 | 842 | 0 | 0 | 643355 | 4557119 |
| M14-8913 | 451 | 268 | 447 | 12 | 0 | 3838965 | 2615162 |
| M14-8914 | 868 | 350 | 718 | 1 | 1 | 4010134 | 2436788 |
| M14-9212 | 1208 | 712 | 1318 | 7 | 0 | 2691103 | 1758500 |
| M14-9801 | 407 | 183 | 387 | 2 | 0 | 779518 | 4617737 |
| M15-1195 | 119 | 84 | 83 | 0 | 0 | 767911 | 5297767 |
| M15-1330 | 8600 | 3192 | 5559 | 101 | 7 | 5264470 | 2815901 |
| M15-1470 | 327 | 203 | 562 | 0 | 1 | 561308 | 3205175 |
| M15-1556 | 446 | 253 | 483 | 6 | 2 | 3805780 | 3085647 |
| M15-1825 | 969 | 508 | 1009 | 13 | 0 | 3034468 | 2977105 |
| M15 1867 | 269 | 127 | 286 | 34 | 0 | 2887666 | 3927692 |
| | | | | 82 | 4 | 3843001 | 2321808 |
| M15-1883 | 2011 | 885 | 1324 | 0 | 1 | 3559414 | 1596230 |
| M15-237 | 276 | 191 | 275 | | 0 | 3448607 | 2111654 |
| M15-2603 | 1559 | 821 | 1398 | 24 41 | 3 | 3503916 | 2121179 |
| M15 2779 | 1475 | 761 | 1463 | | 0 | 3519287 | 3323608 |
| M15-3091 | 200 | 84 | 143 | 9 11 | 1 | 931289 | 2876009 |
| M15 587 | 647 | 375 | 627 | 7 | 3 | 1064180 | 3140014 |
| M15-795 | 360 | 159 | 355 | 13 | 3 | 2942292 | 3536136 |
| M15-933 | 1187 | 596 | 1118 | | | | |

**Table 1.4: Capture Sample Unique V and J Calls**

| Sample | alpha unique VJ counts | beta unique VJ counts | gamma unique VJ counts | delta unique VJ counts | total unique VJ | Unique VJ normalized to input |
|---|---|---|---|---|---|---|
| **A037 healthy reference** | | | | | | |
| Sample_A037_PBMC_TCR_A_all | 11 | 20 | 6 | 0 | 31 | 0.37 |
| Sample_A037_PBMC_TCR_B_all | 44 | 65 | 18 | 0 | 127 | 0.64 |
| Sample_A037_PBMC_TCR_D_all | 213 | 158 | 25 | 1 | 397 | 0.66 |
| Sample_A037_PBMC_TCR_E_all | 955 | 405 | 49 | 3 | 1412 | 1.77 |
| | 1343 | 527 | 49 | 6 | 1925 | 1.93 |
| Sample_A037_PBMC_TCR_F_all Sample_A037_PBMC_TCR_G_all | 8 | 18 | 5 | 1 | 32 | 0.16 |
| Sample_A037_PBMC_TCR_H_all | 502 | 305 | 24 | 1 | 833 | 1.39 |
| Sample_A037_PBMC_TCR_J_all | 192 | 90 | 21 | 3 | 306 | 1.53 |
| Sample_A037_PBMC_TCR_K_all | 268 | 122 | 32 | 4 | 426 | 0.71 |
| Sample_A037_PBMC_TCR_L_all | 220 | 85 | 24 | 3 | 332 | 0.33 |
| Sample_16_01_A037_PBMC_TCR_F_all | 414 | 175 | 41 | 1 | 632 | 1.26 |
| Sample_16_01_A037_PBMC_TCR_H_all | 463 | 235 | 34 | 3 | 735 | 2.94 |
| Sample_A037_S1_all | 446 | 227 | 36 | 3 | 712 | 7.12 |
| Sample_A037_PBMC_1S_all | 466 | 253 | 36 | 4 | 759 | 7.59 |
| Sample_16_11_A037_PBMC_TCR_VJ_all | 263 | 125 | 36 | 3 | 427 | 4.27 |
| Sample_A037_CD3_1S_all | 1704 | 710 | 54 | 7 | 2475 | 24.75 |

| **Cell lines and flow sorted** | | | | | | |
|---|---|---|---|---|---|---|
| M36_EZM | 67 | 41 | 15 | 0 | 123 | 1.23 |
| M36_TIL2 | 244 | 163 | 38 | 1 | 446 | 4.46 |
| OV7-TIL2 | 143 | 114 | 49 | 5 | 311 | 3.11 |
| SE14-2005 | 6 | 13 | 5 | 0 | 24 | 0.24 |
| SE14-2033 | 14 | 3 | 5 | 0 | 22 | 0.22 |
| SE14-2034 | 5 | 16 | 7 | 0 | 28 | 0.28 |
| SE14-2O3S | 9 | 9 | 6 | 0 | 24 | 0.24 |
| STIM1 | 101 | 71 | 23 | 0 | 195 | 1.95 |
| L2DB | 6 | 3 | 3 | 0 | 12 | 012 |

| **Patient samples** | | | | | 402 | |
|---|---|---|---|---|---|---|
| M14-10124 | 225 | 142 | 33 | 2 | | 4.02 |
| M14-11153 | 137 | 63 | 28 | 2 | 230 | 2.30 |
| M14-11567 | 242 | 147 | 39 | 1 | 429 | 4.29 |
| M14-11587 | 37 | 39 | 15 | 0 | 91 | 0.91 |
| M14-11721 | 35 | 14 | 21 | 0 | 70 | 0.70 |
| M14-11770 | 14 | 16 | 8 | 0 | 38 | 0.38 |
| M14-12217 | 59 | 32 | 15 | 1 | 107 | 1.07 |
| M14-12649 | 174 | 132 | 34 | 1 | 341 | 3.41 |
| M14-12728 | 433 | 229 | 47 | 4 | 713 | 7.13 |
| M14-12753 | 178 | 104 | 25 | 4 | 311 | 3.11 |
| M14-13167 | 44 | 19 | 21 | 2 | 86 | 0.86 |
| M14-13300 | 221 | 146 | 33 | 2 | 402 | 4.02 |
| M14-13750 | 410 | 201 | 46 | 5 | 662 | 6.62 |
| M14-14570 | 34 | 33 | 18 | 3 | 88 | 0.88 |
| M14-14625 | 485 | 242 | 50 | 2 | 779 | 7.79 |
| M14-14907 | 227 | 62 | 26 | 2 | 317 | 3.17 |
| M14-14951 | 73 | 43 | 24 | 1 | 141 | 1.41 |
| M14-14962 | 377 | 173 | 41 | 3 | 544 | 5.44 |
| M14-1508 | 352 | 203 | 46 | 5 | 606 | 6.06 |
| M14-15119 | 19 | 18 | 7 | 0 | 44 | 0.44 |
| M14-3271 | 798 | 405 | 53 | 4 | 1260 | 12.60 |
| M14-4454 | 260 | 132 | 31 | 2 | 425 | 4.25 |
| M14-5819 | 53 | 23 | 24 | 1 | 101 | 1.01 |
| M14-5875 | 99 | 79 | 32 | 1 | 211 | 2.11 |
| M14-6143 | 278 | 113 | 40 | 1 | 432 | 4.31 |
| M14-6430 | 173 | 112 | 19 | 3 | 317 | 3.17 |
| M14-6443 | 0 | 0 | 0 | 0 | 0 | 0.00 |
| M14-6502 | 66 | 37 | 27 | 0 | 130 | 1.30 |
| M14-6885 | 513 | 262 | 32 | 3 | 810 | 8.10 |
| M14-7046 | 157 | 70 | 23 | 1 | 251 | 2.51 |
| M14-7049 | 3 | 1 | 3 | 3 | 10 | 0.10 |
| M14-7053 | 148 | 89 | 35 | 4 | 276 | 2.76 |
| M14-7107 | 456 | 205 | 45 | 1 | 707 | 7.07 |
| M14-7554 | 164 | 103 | 29 | 5 | 301 | 3.01 |
| M14-7568 | 480 | 186 | 39 | 5 | 710 | 7.10 |
| M14-7691 | 237 | 146 | 43 | 0 | 426 | 4.26 |
| M14-7700 | 105 | 64 | 26 | 1 | 196 | 1.96 |
| M14-7782 | 150 | 99 | 34 | 2 | 285 | 2.85 |
| M14-7862 | 76 | 32 | 22 | 0 | 130 | 1.30 |
| M14-1884 | 171 | 106 | 39 | 0 | 316 | 3.16 |
| M14-7992 | 258 | 160 | 34 | 1 | 454 | 4.54 |
| M14-8132 | 34 | 28 | 21 | 1 | 84 | 0.84 |
| M14-8272 | 72 | 60 | 29 | 0 | 161 | 1.51 |
| M14-8639 | 125 | 77 | 27 | 3 | 232 | 2.32 |
| M14 8668 | 44 | 32 | 19 | 0 | 95 | 0.95 |
| M14-8740 | 17 | 9 | 13 | 0 | 39 | 0.39 |
| M14-8913 | 90 | 61 | 30 | 2 | 183 | 1.83 |
| M14-8914 | 1// | 75 | 29 | 1 | 282 | 2.82 |
| M14-9212 | 190 | 128 | 27 | 3 | 348 | 3.48 |
| M14-9801 | 85 | 41 | 29 | 1 | 156 | 1.56 |
| M15-1195 | 45 | 32 | 24 | 0 | 101 | 101 |
| M15-1330 | 1019 | 362 | 55 | 6 | 1442 | 14.42 |
| M15-1470 | 50 | 38 | 28 | 0 | 116 | 1.16 |
| M15-1556 | 120 | 59 | 24 | 1 | 204 | 2.04 |
| M15-1825 | 214 | 121 | 30 | 1 | 366 | 3.66 |
| M15-1867 | 90 | 46 | 32 | 2 | 170 | 1.70 |
| M15-1883 | 435 | 194 | 33 | 6 | 668 | 6.68 |
| M15 237 | 51 | 36 | 21 | 0 | 108 | 1.08 |
| M15-2603 | 294 | 169 | 57 | 3 | 523 | 5.23 |
| M15-2779 | 349 | 185 | 31 | 5 | 570 | 5.70 |
| M15-3091 | 44 | 25 | 15 | 1 | 85 | 0.85 |
| M15-587 | 309 | 159 | 39 | 4 | 511 | 5.11 |
| M15-795 | 174 | 73 | 38 | 2 | 287 | 2.87 |
| M15-933 | 353 | 170 | 57 | 1 | 581 | 5.81 |

**Table 1.5: Capture Sample Unique CDR3 Calls**

| Sample | alpha total unique CDR3 | beta total unique CDR3 | gamma total unique CDR3 | delta total unique CDR3 | total unique CDR3 | Unique CDR3 normalized to input |
|---|---|---|---|---|---|---|
| **A037 healthy reference** | | | | | | |
| Sample_A037_PBMC_TCR_A_all | 12 | 27 | 9 | 0 | 48 | 0.48 |
| Sample_A037_PBMC_TCR_B_all | 63 | 104 | 31 | 0 | 198 | 0.99 |
| Sample_A037_PBMC_TCR_D_all | 229 | 188 | 65 | 2 | 484 | 0.81 |
| Sample_A037_PBMC_TCR_E_all | 1367 | 778 | 348 | 21 | 2514 | 3.14 |
| Sample_A037_PBMC_TCR_F_all | 2066 | 1100 | 540 | 24 | 3730 | 3.73 |
| Sample_A037_PBMC_TCR_G_all | 11 | 23 | 11 | 3 | 48 | 0.24 |
| Sample_A037_PBMCTCR_H_all | 633 | 482 | 62 | 3 | 1180 | 1.97 |
| Sample_A037_PBMC_TCR_J_all | 216 | 104 | 48 | 4 | 372 | 1.86 |
| Sample_A037_PBMC_TCR_K_all | 297 | 148 | 82 | 5 | 532 | 0.89 |
| Sample_A037_PBMC_TCR_L_all | 242 | 99 | 63 | 8 | 112 | 0.41 |
| Sample_16_01_A037_PBMC_TCR_F_all | 482 | 229 | 155 | 14 | 880 | 1.76 |
| Sample_1601_A037_PBMC_TCR_H_all | 555 | 330 | 158 | 4 | 1047 | 4.19 |
| Sample_A037_S1_all | 509 | 303 | 141 | 5 | 958 | 9.58 |
| Sample_A037_PBMC_15_all | 539 | 344 | 157 | 13 | 1053 | 10.53 |
| Sample_16_11_A037_PRMC_TCR_VJ_all | 293 | 142 | 114 | 8 | 557 | 5.57 |
| Sample_A037_CD3_1S_all | 2840 | 1672 | 691 | 47 | 5250 | 52.50 |

| **Cell lines and flow sorted** | | | | | | |
|---|---|---|---|---|---|---|
| M3G_EZM | 70 | 48 | 26 | 0 | 144 | 1.44 |
| M36_TIL2 | 310 | 25 | 101 | 2 | 438 | 4.38 |
| OV7-TIL2 | 219 | 192 | 83 | 9 | 503 | 5.03 |
| SE14-2005 | 32 | 29 | 21 | 0 | 82 | 0.82 |
| SE14-2033 | 32 | 21 | 10 | 0 | 63 | 0.63 |
| SE14-2034 | 10 | 66 | 8 | 0 | 84 | C 84 |
| SE14-2035 | 33 | 39 | 23 | 0 | 95 | 0.95 |
| STIM1 | 160 | 136 | 55 | 0 | 351 | 3.51 |
| L2D8 | 14 | 21 | 10 | 0 | 45 | 0.45 |

| **Patient samples** | | | | | | |
|---|---|---|---|---|---|---|
| M14-10124 | 279 | 201 | 101 | 3 | 584 | 5.84 |
| M14-11153 | 151 | 80 | 54 | 1 | 287 | 2.87 |
| M14-11567 | 287 | 193 | 97 | 1 | 578 | 5.78 |
| M14 11587 | 41 | 57 | 30 | 0 | 128 | 1.28 |
| M14-11721 | 39 | 17 | 28 | 0 | 84 | 0.84 |
| M14-11770 | 14 | 16 | 11 | 0 | 41 | 0.41 |
| M14-12217 | 66 | 43 | 52 | 18 | 179 | 1.79 |
| M14-12649 | 206 | 185 | 89 | 1 | 481 | 4.81 |
| M14-12728 | 494 | 323 | 183 | 7 | 1007 | 10.07 |
| M14-12753 | 223 | 164 | 79 | 10 | 476 | 4.76 |
| M14-13167 | 55 | 23 | 32 | 6 | 116 | 1.16 |
| M14-13300 | 253 | 216 | 102 | 6 | 577 | 5.77 |
| M14-13750 | 516 | 313 | 167 | 20 | 1016 | 10.16 |
| M14-14570 | 35 | 40 | 34 | 8 | 117 | 1.17 |
| M14-14625 | 562 | 321 | 193 | 3 | 1079 | 10.79 |
| M14-14907 | 255 | 75 | 66 | 3 | 399 | 3.99 |
| M14-14951 | 76 | 47 | 42 | 1 | 167 | 1.67 |
| M14-14962 | 371 | 224 | 140 | 5 | 740 | 7.40 |
| M14-1508 | 448 | 314 | 163 | 8 | 933 | 9.33 |
| M14-15119 | 83 | 67 | 10 | 0 | 160 | 1.60 |
| M14-3271 | 1084 | 714 | 275 | 12 | 2085 | 20.85 |
| M14-4454 | 303 | 170 | 84 | 4 | 561 | 5.61 |
| M14-5819 | 57 | 31 | 40 | 1 | 129 | 1.29 |
| M14-5875 | 114 | 101 | 68 | 3 | 286 | 2.86 |
| M14-6143 | 308 | 140 | 108 | 1 | 557 | 5.57 |
| M14-6430 | 202 | 139 | 71 | 5 | 417 | 4.17 |
| M14-6443 | 0 | 0 | 0 | 0 | 0 | 0.00 |
| M14-6502 | 69 | 38 | 50 | 0 | 157 | 1.57 |
| M14-6885 | 613 | 391 | 164 | 3 | 1161 | 11.61 |
| M14-7046 | 177 | 78 | 72 | 3 | 330 | 330 |
| M14-7049 | 3 | 1 | 13 | 11 | 28 | 0.28 |
| M14-7053 | 162 | 109 | 79 | 10 | 360 | 3.60 |
| M14-7107 | 532 | 290 | 158 | 1 | 981 | 9.81 |
| M14-7554 | 189 | 129 | 78 | 13 | 409 | 4.09 |
| M14-7568 | 583 | 252 | 138 | 10 | 983 | 9.83 |
| M14-7691 | 317 | 301 | 99 | 0 | 717 | 7.17 |
| M14-7700 | 123 | 82 | 74 | 1 | 280 | 2.80 |
| M14 7782 | 166 | 125 | 75 | 4 | 370 | 3.70 |
| M14-7862 | 82 | 38 | 37 | 0 | 157 | 1.57 |
| M14-7884 | 181 | 125 | 102 | 0 | 408 | 4.08 |
| M14-7992 | 306 | 231 | 118 | 3 | 658 | 6.58 |
| M14-8132 | 37 | 34 | 33 | 1 | 105 | 1.05 |
| M14-8272 | 77 | 73 | 50 | 0 | 200 | 2.00 |
| M14-8639 | 140 | 99 | 65 | 8 | 312 | 3.12 |
| M14-8668 | 45 | 35 | 16 | 0 | 106 | *1.06* |
| M14-8740 | 31 | 21 | 16 | 0 | 68 | 0.68 |
| M14-8913 | 114 | 78 | 53 | 5 | 250 | 2.50 |
| M14-8914 | 212 | 100 | 78 | 1 | 391 | 3.91 |
| M14-9212 | 224 | 168 | 85 | 3 | 480 | 4.80 |
| M14-9801 | 104 | 52 | 42 | 1 | 199 | 1.99 |

| | | | | | | |
|---|---|---|---|---|---|---|
| M15-1195 | 48 | 36 | 32 | 0 | 116 | 1.16 |
| M15-1330 | 14G9 | 619 | 279 | 15 | 2382 | 23.82 |
| M15-1470 | 57 | 44 | 50 | 0 | 151 | 1.51 |
| M15-1556 | 127 | 71 | 56 | 1 | 255 | 2.55 |
| M15-1825 | 259 | 147 | 108 | 2 | 516 | 5.16 |
| M15-1867 | 96 | 54 | 59 | 4 | 213 | 2.13 |
| M15-1883 | 520 | 284 | 120 | 11 | 935 | 9.35 |
| M15-237 | SΘ | 45 | 32 | 0 | 135 | 1.35 |
| M15-2603 | 351 | 220 | 123 | 4 | 698 | 6.98 |
| M15-2779 | 408 | 247 | 123 | 7 | 785 | 7.85 |
| M15-3091 | 47 | 29 | 25 | 2 | 103 | 1.03 |
| M15-587 | 346 | 214 | 113 | 6 | 679 | 6.79 |
| M15-795 | 188 | 85 | 87 | 3 | 363 | 3.63 |
| M15-933 | 418 | 242 | 162 | 3 | 825 | 8.25 |

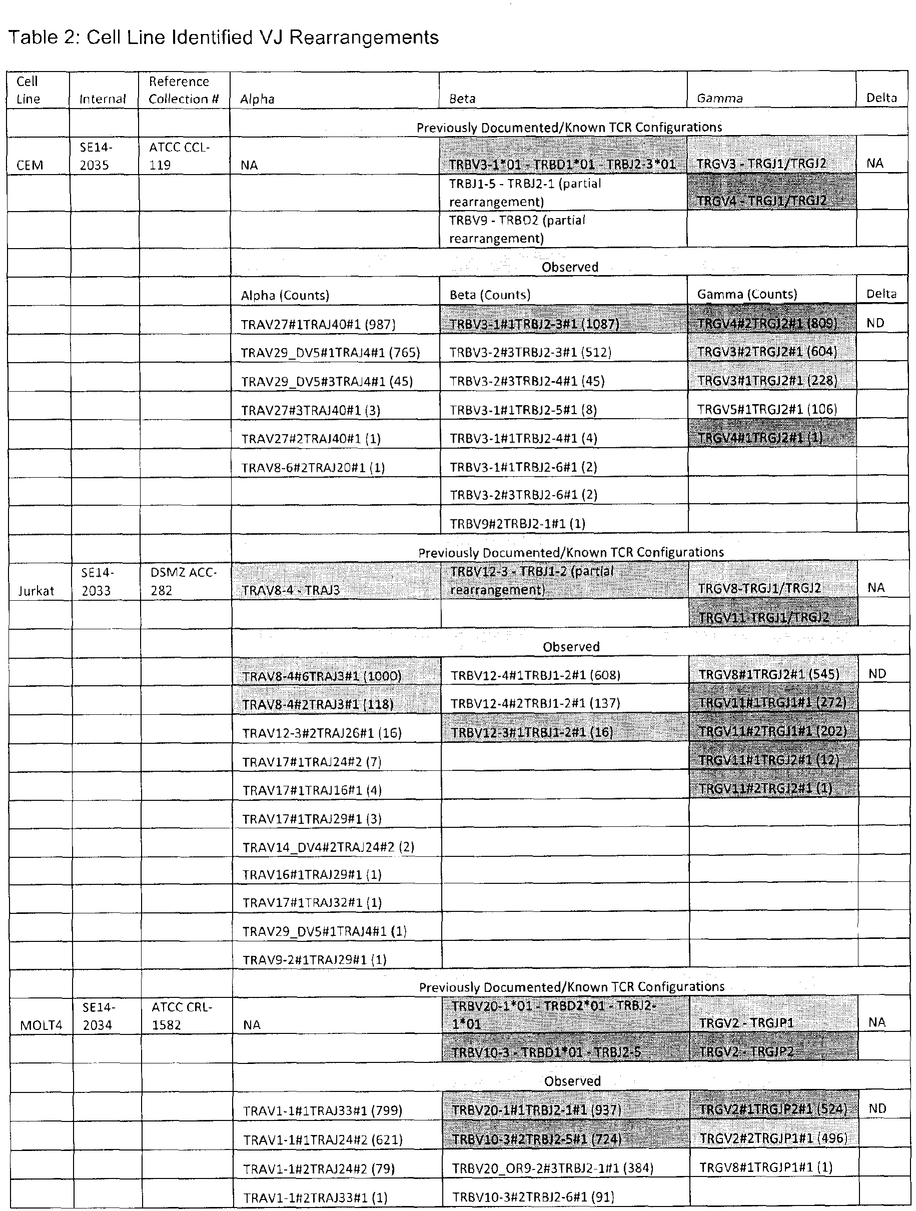

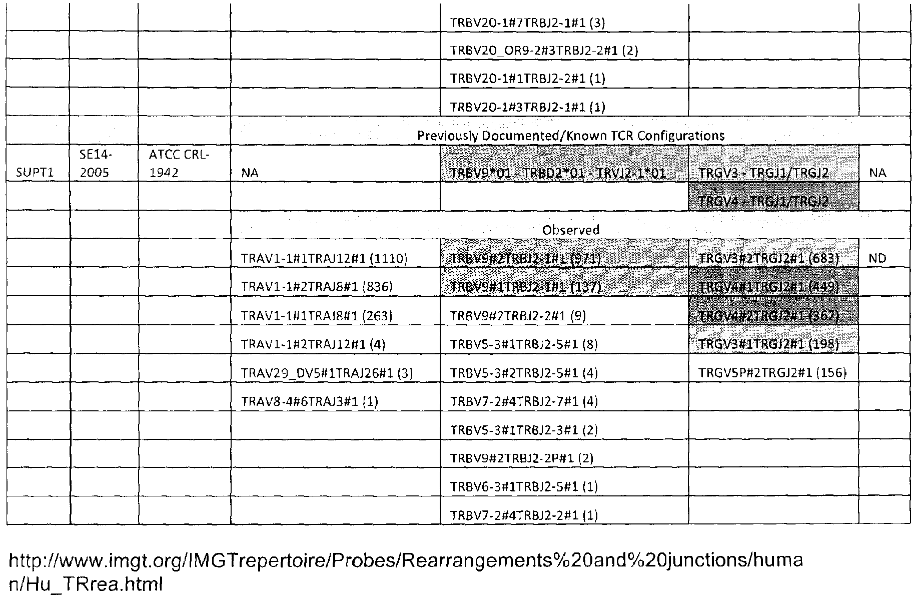

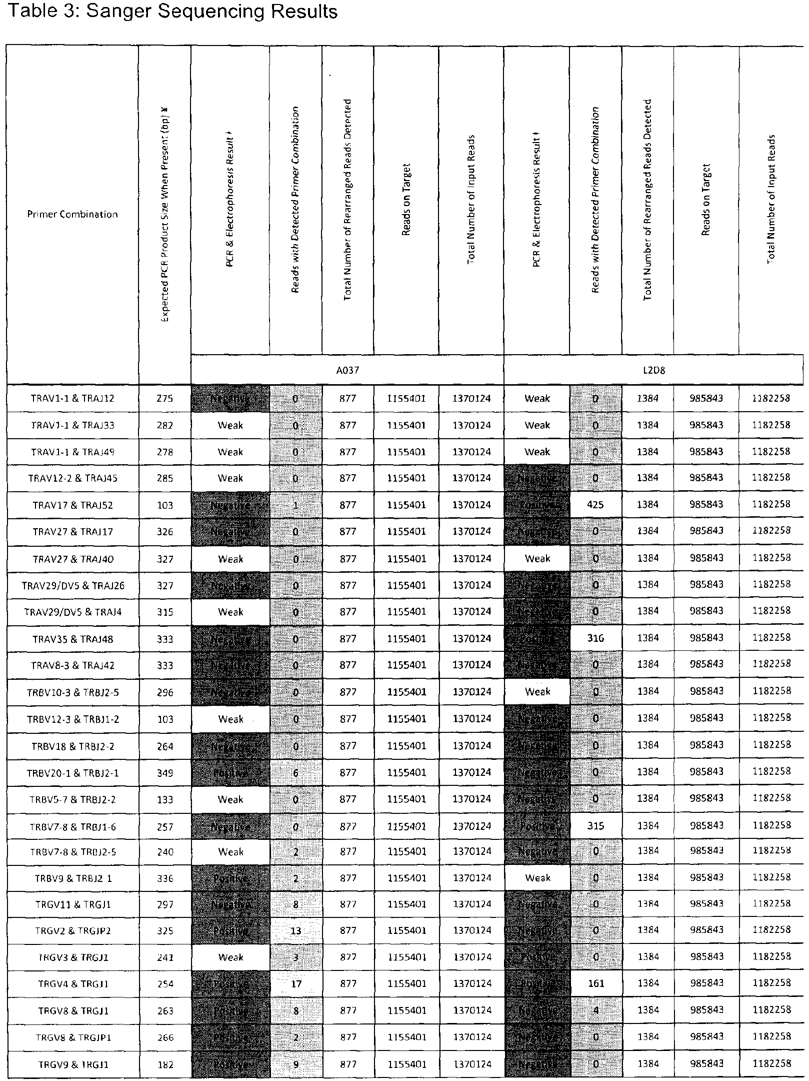

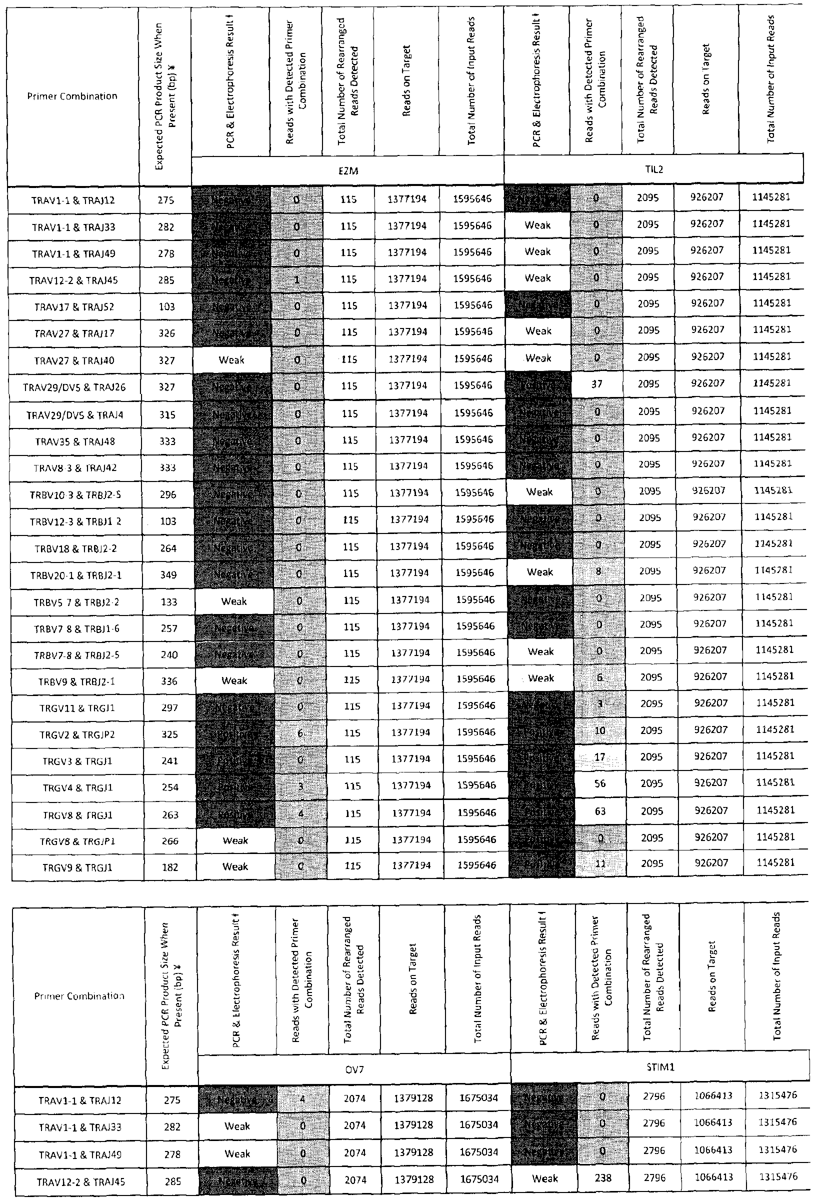

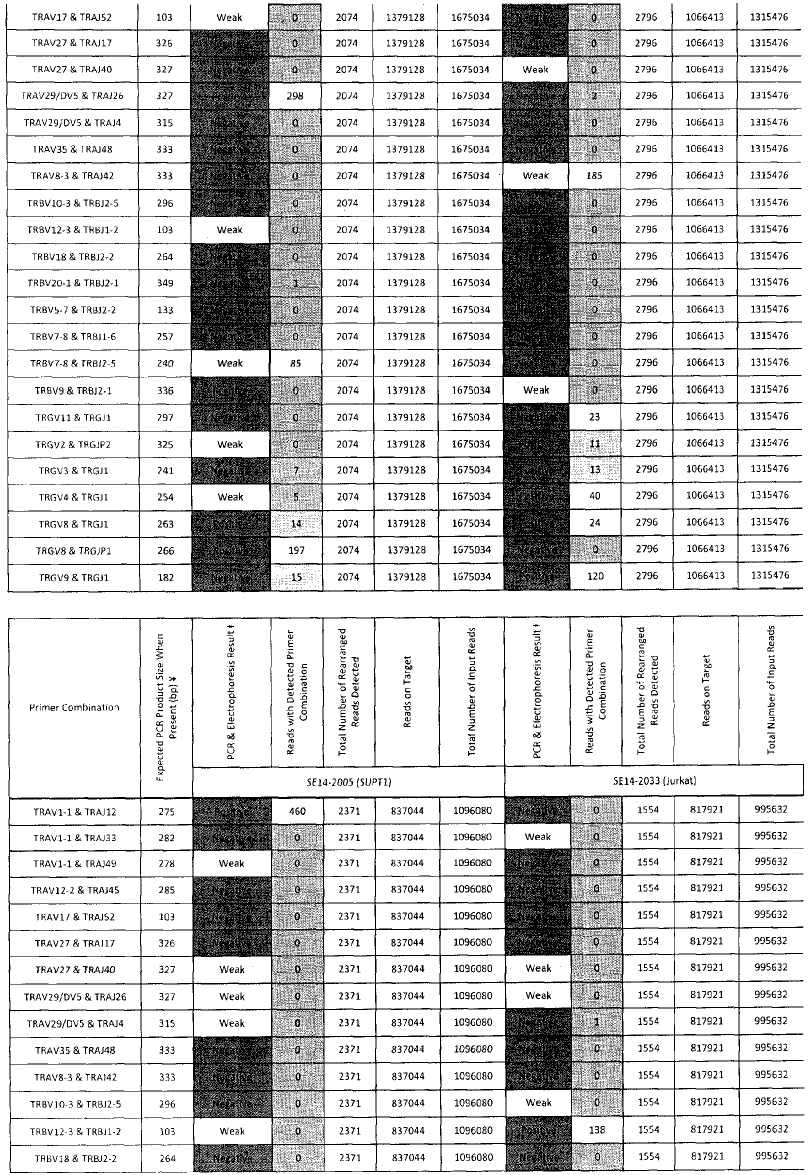

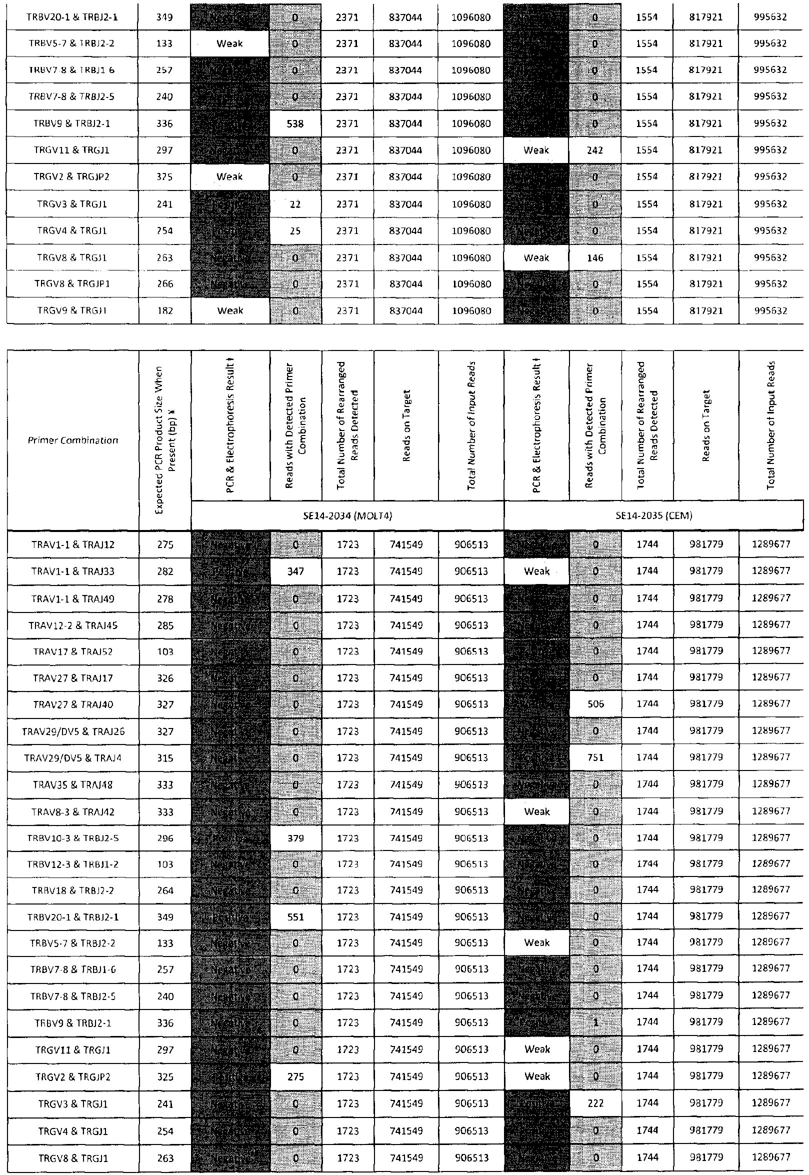

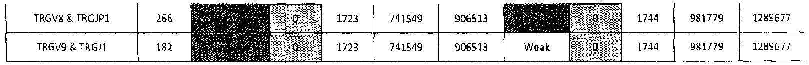

## Claims

1. A method of capturing a population of T-Cell receptor and/or immunoglobulin sequences with variable regions within a patient sample, said method comprising:
extracting/preparing DNA fragments from the patient sample;
ligating a nucleic acid adapter to the DNA fragments, the nucleic acid adapter suitable for recognition by a pre-selected nucleic acid probe;
capturing DNA fragments existing in the patient sample using a collection of nucleic acid hybrid capture probes, wherein each capture probe is designed to hybridize to a known V gene segment and/or a J gene segment within the T cell receptor and/or immunoglobulin genomic loci,
wherein the hybrid capture probes are designed based on all unique V genes and J genes and these probe sets specifically target the 3' regions of V gene coding regions and the 5' regions of J gene coding regions.

2. The method of claim 1, further comprising sequencing the captured DNA fragments, wherein the sequencing can be used to determine clonotypes within the patient sample.

3. The method of claim 1, wherein said sequencing is optimized for short read sequencing, preferably further comprising amplifying the population of sequences using nucleic acid amplification probes/oligonucleotides that recognize the adapter prior to said sequencing.

4. The method of any one of claims 1-3, further comprising fragmenting DNA extracted from the patient sample to generate the DNA fragments.

5. The method of any one of claims 1-4, wherein the ligating step is performed before the capturing step or the capturing step is performed before the ligating step.

6. The method of any one of claims 1-5, wherein the patient sample comprises tissue, urine, cerebral spinal fluid, saliva, feces, ascities, pleural effusion, blood or blood plasma, preferably the patient sample comprises cell-free nucleic acids in blood plasma.

7. The method of any one of claims 1-6, wherein the hybrid capture probes are at least 30bp in length, preferably wherein the hybrid capture probes are between 60bp and 150bp in length, preferably between 80bp and 120bp in length, and further preferably about 100bp in length.

8. The method of any one of claims 1-7, wherein hybridizing comprises hybridizing under stringent conditions, preferably very stringent conditions.

9. The method of any one of claims 1-8, wherein the hybrid capture probes comprise a label, preferably wherein the label is used to distinguish between sequences bound to the screening probes and unbound double stranded fragments.

10. The method of any one of claims 1-9, wherein the adapter is designed to permit sequencing of the DNA fragment and/or barcoding of the DNA fragment.

11. The method of any one of claims 1-10, wherein preparing the DNA fragments comprises extracting RNA from the patient sample and preparing corresponding cDNA.

12. The method of any one of claims 1-11, further comprising a depletion step, comprising depleting the DNA fragments of non-rearranged sequences using probes that recognize nucleic acid sequences adjacent to V and/or J gene segments in the genome.

13. The method of claim 12, wherein the capturing of DNA fragments using V gene segment and J gene segment hybrid capture probes is performed in separate steps, and in any order with the depletion step, preferably in the following order: J gene capture, depletion, then V gene capture.

14. A method for characterizing the immune repertoire of a subject, the immune repertoire comprising the subject's T-Cell population, the method comprising the method of any one of claims 1-13, followed by a method comprising:
(j) identifying all sequences containing a V gene segment from the sequences of the DNA fragments by aligning the sequences of the DNA fragments to a library of known V gene segment sequences;
(k) trimming the identified sequences in (j) to remove any sequences corresponding to V gene segments to produce a collection of V-trimmed nucleotide sequences;
(l) identifying all sequences containing a J gene segment in the population of V-trimmed nucleotide sequences by aligning the V-trimmed nucleotide sequences to a library of known J gene segment sequences;
(m) trimming the V-trimmed nucleotide sequences identified in (l) to remove any sequences corresponding to J gene segments to produce VJ-trimmed nucleotide sequences;
(n) identifying any D gene segment comprised in the VJ-trimmed nucleotide sequences identified in (m) by aligning the VJ-trimmed nucleotide sequences to a library of known D gene segment sequences;
(o) for each VJ-trimmed nucleotides sequence identified in (m), assembling a nucleotide sequence comprising the V gene segment, any D gene segment, and the J gene segment identified in steps (j), (n) and (l) respectively ;
(p) selecting from the nucleotide sequence assembled in step (o) a junction nucleotide sequence comprising at least the junction between the V gene segment and the J gene segment, including any D gene segment, the junction nucleotide sequence comprising between 18bp and 140bp;
and optionally (q) and (r):
(q) translating each reading frame of the junction nucleotide sequence and its complementary strand to produce 6 translated sequences; and
(r) comparing the 6 translated sequences to a library of known CDR3 regions of T-Cell receptor and/or immunoglobulin sequences to identify the CDR3 region in the DNA fragments.

## Patentansprüche

1. Verfahren zum Einfangen einer Population von T-Zell-Rezeptor- und/oder Immunglobulin-Sequenzen mit variablen Regionen in einer Patientenprobe, wobei das Verfahren umfasst:
Extrahieren/Präparieren von DNA-Fragmenten aus der Patientenprobe;
Ligieren eines Nukleinsäureadapters an die DNA-Fragmente, wobei der Nukleinsäureadapter zur Erkennung durch eine vorgewählte Nukleinsäuresonde geeignet ist;
Einfangen von DNA-Fragmenten, die in der Patientenprobe vorhanden sind, unter Verwendung einer Sammlung von Nukleinsäure-Hybrid-Einfangsonden, wobei jede Einfangsonde so ausgelegt ist, dass sie an ein bekanntes V-Gen-Segment und/oder ein J-Gen-Segment innerhalb der genomischen T-Zell-Rezeptor- und/oder Immunglobulin-Loci hybridisiert,
wobei die Hybrid-Einfangsonden auf der Grundlage aller einzigartigen V-Gene und J-Gene entworfen werden und diese Sondensätze spezifisch auf die 3'-Regionen von V-Gen-codierenden Regionen und die 5'-Regionen von J-Gencodierenden Regionen abzielen.

2. Verfahren nach Anspruch 1, zudem umfassend das Sequenzieren der eingefangenen DNA-Fragmente, wobei die Sequenzierung zur Bestimmung von Klonotypen innerhalb der Patientenprobe verwendet werden kann.

3. Verfahren nach Anspruch 1, wobei die Sequenzierung für Short-Read-Sequenzierung optimiert ist, vorzugsweise zudem umfassend die Amplifikation der Population von Sequenzen unter Verwendung von Nukleinsäure-Amplifikationssonden, die den Adapter erkennen, vor der Sequenzierung.

4. Verfahren nach einem der Ansprüche 1 bis 3, zudem umfassend das Fragmentieren der aus der Patientenprobe extrahierten DNA zur Erzeugung der DNA-Fragmente.

5. Verfahren nach einem der Ansprüche 1 bis 4, wobei der Ligationsschritt vor dem Einfangschritt durchgeführt wird oder der Einfangschritt vor dem Ligationsschritt durchgeführt wird.

6. Verfahren nach einem der Ansprüche 1 bis 5, wobei die Patientenprobe Gewebe, Urin, Cerebrospinal-Flüssigkeit, Speichel, Fäkalien, Aszites, Pleuraerguss, Blut oder Blutplasma umfasst, und wobei die Patientenprobe vorzugsweise zellfreie Nukleinsäuren im Blutplasma umfasst.

7. Verfahren nach einem der Ansprüche 1 bis 6, wobei die Hybrid-Einfangsonden mindestens 30 bp lang sind, wobei die Hybrid-Einfangsonden vorzugsweise zwischen 60 bp und 150 bp lang sind, vorzugsweise zwischen 80 bp und 120 bp lang sind und weiter vorzugsweise etwa 100 bp lang sind.

8. Verfahren nach einem der Ansprüche 1 bis 7, wobei das Hybridisieren das Hybridisieren unter stringenten Bedingungen, vorzugsweise hochstringenten Bedingungen, umfasst.

9. Verfahren nach einem der Ansprüche 1 bis 8, wobei die Hybrid-Einfangsonden eine Markierung umfassen, wobei die Markierung vorzugsweise zur Unterscheidung zwischen an die Screening-Sonden gebundenen Sequenzen und ungebundenen doppelsträngigen Fragmenten verwendet wird.

10. Verfahren nach einem der Ansprüche 1 bis 9, wobei der Adapter so gestaltet ist, dass er die Sequenzierung des DNA-Fragments und/oder die Strichcodierung des DNA-Fragments ermöglicht.

11. Verfahren nach einem der Ansprüche 1 bis 10, wobei die Herstellung der DNA-Fragmente die Extraktion von RNA aus der Patientenprobe und die Herstellung der entsprechenden cDNA umfasst.

12. Verfahren nach einem der Ansprüche 1 bis 11, zudem umfassend einen Abreicherungsschritt, umfassend die Abreicherung der DNA-Fragmente von nicht umgelagerten Sequenzen unter Verwendung von Sonden, die Nukleinsäuresequenzen erkennen, die an V- und/oder J-Gensegmente im Genom angrenzen.

13. Das Verfahren nach Anspruch 12, wobei das Einfangen von DNA-Fragmenten unter Verwendung von V-Gensegment- und J-Gensegment-Hybrid-Einfangsonden in getrennten Schritten und in beliebiger Reihenfolge mit dem Abreicherungsschritt durchgeführt wird, vorzugsweise in der folgenden Reihenfolge: J-Gen-Einfang, Abreicherung, dann V-Gen-Einfang.

14. Verfahren zur Charakterisierung des Immunrepertoires eines Individuums, wobei das Immunrepertoire die T-Zell-Population des Individuums umfasst, wobei das Verfahren das Verfahren nach einem der Ansprüche 1 bis 13 umfasst, gefolgt von einem Verfahren, umfassend:
(j) Identifizierung aller Sequenzen, die ein V-Gensegment enthalten, aus den Sequenzen der DNA-Fragmente durch Alignment der Sequenzen der DNA-Fragmente mit einer Bibliothek bekannter V-Gensegmentsequenzen;
(k) Trimmen der identifizierten Sequenzen in (j), um alle Sequenzen zu entfernen, die V-Gensegmenten entsprechen, um eine Sammlung von V-getrimmten Nukleotidsequenzen zu erzeugen;
(l) Identifizierung aller Sequenzen, die ein J-Gensegment in der Population der V-getrimmten Nukleotidsequenzen enthalten, durch Alignment der V-getrimmten Nukleotidsequenzen mit einer Bibliothek bekannter J-Gensegmentsequenzen;
(m) Trimmen der in (l) identifizierten V-getrimmten Nukleotidsequenzen, um alle Sequenzen zu entfernen, die J-Gensegmenten entsprechen, um VJ-getrimmte Nukleotidsequenzen zu erzeugen;
(n) Identifizierung jedes D-Gensegments, das in den in (m) identifizierten VJ-getrimmten Nukleotidsequenzen enthalten ist, durch Alignment der VJ-getrimmten Nukleotidsequenzen mit einer Bibliothek bekannter D-Gensegmentsequenzen;
(o) für jede in (m) identifizierte VJ-getrimmte Nukleotidsequenz Assemblieren einer Nukleotidsequenz, umfassend das V-Gensegment, irgendein D-Gensegment und das J-Gensegment, die in den Schritten (j), (n) bzw. (l) identifiziert wurden,;
(p) Auswählen aus der in Schritt (o) assemblierten Nukleotidsequenz einer Verbindungsnukleotidsequenz, die mindestens die Verbindung zwischen dem V-Gensegment und dem J-Gensegment, einschließlich eines beliebigen D-Gensegments, umfasst, wobei die Verbindungsnukleotidsequenz zwischen 18 bp und 140 bp umfasst,
und gegebenenfalls (q) und (r):
(q) Translatieren jedes Leserasters der Verbindungsnukleotidsequenz und ihres komplementären Strangs, um 6 translatierte Sequenzen zu erzeugen; und
(r) Vergleichen der 6 translatierten Sequenzen mit einer Bibliothek bekannter CDR3-Regionen von T-Zell-Rezeptor- und/oder Immunglobulinsequenzen, um die CDR3-Region in den DNA-Fragmenten zu identifizieren,

## Revendications

1. Procédé de capture d'une population de séquences de récepteur des lymphocytes T et/ou d'immunoglobuline avec des régions variables dans un échantillon de patient, ledit procédé comprenant :
l'extraction/préparation de fragments d'ADN à partir de l'échantillon de patient ;
la ligature d'un adaptateur d'acide nucléique aux fragments d'ADN, l'adaptateur d'acide nucléique étant adapté pour la reconnaissance par une sonde d'acide nucléique présélectionnée ;
la capture de fragments d'ADN présents dans l'échantillon de patient à l'aide d'une collection de sondes de capture hybrides d'acide nucléique, chaque sonde de capture étant conçue pour s'hybrider à un segment de gène V et/ou un segment de gène J connu au sein des locus génomiques de récepteur des lymphocytes T et/ou d'immunoglobuline,
dans lequel les sondes de capture hybrides sont conçues sur la base de tous les gènes V et gènes J uniques et ces ensembles de sondes ciblent spécifiquement les régions 3' de régions codant pour le gène V et les régions 5' de régions codant pour le gène J.

2. Procédé selon la revendication 1, comprenant en outre le séquençage des fragments d'ADN capturés, le séquençage pouvant être utilisé pour déterminer des clonotypes dans l'échantillon de patient.

3. Procédé selon la revendication 1, dans lequel ledit séquençage est optimisé pour le séquençage de lectures courtes, comprenant de préférence en outre l'amplification de la population de séquences au moyen de sondes d'amplification d'acide nucléique/oligonucléotides qui reconnaissent l'adaptateur avant ledit séquençage.

4. Procédé selon l'une quelconque des revendications 1 à 3, comprenant en outre la fragmentation de l'ADN extrait de l'échantillon de patient pour générer les fragments d'ADN.

5. Procédé selon l'une quelconque des revendications 1 à 4, dans lequel l'étape de ligature est réalisée avant l'étape de capture ou bien l'étape de capture est réalisée avant l'étape de ligature.

6. Procédé selon l'une quelconque des revendications 1 à 5, dans lequel l'échantillon de patient comprend un tissu, de l'urine, du liquide céphalo-rachidien, de la salive, des selles, des ascites, un épanchement pleural, du sang ou du plasma sanguin, de préférence l'échantillon de patient comprend des acides nucléiques acellulaires dans le plasma sanguin.

7. Procédé selon l'une quelconque des revendications 1 à 6, dans lequel les sondes de capture hybrides ont une longueur d'au moins 30 pb, de préférence dans lequel les sondes de capture hybrides ont une longueur comprise entre 60 pb et 150 pb, de préférence une longueur comprise entre 80 pb et 120 pb, et de préférence en outre une longueur d'environ 100 pb.

8. Procédé selon l'une quelconque des revendications 1 à 7, dans lequel l'hybridation comprend l'hybridation dans des conditions stringentes, de préférence des conditions très stringentes.

9. Procédé selon l'une quelconque des revendications 1 à 8, dans lequel les sondes de capture hybrides comprennent un marqueur, de préférence dans lequel le marqueur est utilisé pour distinguer les séquences liées aux sondes de criblage et les fragments double brin non liés.

10. Procédé selon l'une quelconque des revendications 1 à 9, dans lequel l'adaptateur est conçu pour permettre le séquençage du fragment d'ADN et/ou le codage à barres du fragment d'ADN.

11. Procédé selon l'une quelconque des revendications 1 à 10, dans lequel la préparation des fragments d'ADN comprend l'extraction d'ARN à partir de l'échantillon de patient et la préparation de l'ADNc correspondant.

12. Procédé selon l'une quelconque des revendications 1 à 11, comprenant en outre une étape de déplétion, comprenant la déplétion des fragments d'ADN de séquences non réarrangées au moyen de sondes qui reconnaissent des séquences d'acide nucléique adjacentes aux segments des gènes V et/ou J dans le génome.

13. Procédé selon la revendication 12, dans lequel la capture de fragments d'ADN au moyen de sondes de capture hybrides de segment de gène V et de segment de gène J est effectuée dans des étapes séparées, et dans n'importe quel ordre avec l'étape de déplétion, de préférence dans l'ordre suivant : Capture du gène J, épuisement, puis capture du gène V.

14. Procédé de caractérisation du répertoire immunitaire d'un sujet, le répertoire immunitaire comprenant la population de lymphocytes T du sujet, le procédé comprenant le procédé selon l'une quelconque des revendications 1 à 13, suivi d'un procédé comprenant :
(j) l'identification de toutes les séquences contenant un segment de gène V parmi les séquences des fragments d'ADN par alignement des séquences des fragments d'ADN avec une banque de séquences de segment de gène V connues ;
(k) la troncature des séquences identifiées dans (j) pour retirer toutes les séquences correspondant aux segments de gène V afin de produire une collection de séquences nucléotidiques tronquées pour V ;
(l) l'identification de toutes les séquences contenant un segment de gène J dans la population de séquences nucléotidiques tronquées pour V par alignement des séquences nucléotidiques tronquées pour V avec une banque de séquences de segment de gène J connues ;
(m) la troncature des séquences nucléotidiques tronquées pour V identifiées dans (1) pour retirer toutes les séquences correspondant aux segments de gène J afin de produire des séquences nucléotidiques tronquées pour VJ ;
(n) l'identification de tout segment de gène D compris dans les séquences nucléotidiques tronquées pour VJ identifiées dans (m) par alignement des séquences nucléotidiques tronquées pour VJ sur une banque de séquences de segment de gène D connues ;
(o) pour chaque séquence nucléotidique tronquée pour VJ identifiée dans (m), l'assemblage d'une séquence nucléotidique comprenant le segment de gène V, un segment quelconque de gène D et le segment du gène J identifié aux étapes (j), (n) et (1) respectivement ;
(p) la sélection à partir de la séquence nucléotidique assemblée dans l'étape (o) d'une séquence nucléotidique de jonction comprenant au moins la jonction entre le segment de gène V et le segment de gène J, y compris tout segment de gène D, la séquence nucléotidique de jonction comprenant entre 18 pb et 140 pb ;
et éventuellement (q) et (r) :
(q) la traduction de chaque cadre de lecture de la séquence nucléotidique de jonction et son brin complémentaire pour produire 6 séquences traduites ; et
(r) la comparaison des 6 séquences traduites à une banque de régions CDR3 connues de séquences de récepteur des lymphocytes T et/ou d'immunoglobuline pour identifier la région CDR3 dans les fragments d'ADN.
